# EUROPÄISCHE PATENTSCHRIFT

(11) **EP 2 010 185 B1**
(45) Veröffentlichungstag und Bekanntmachung des Hinweises auf die Patenterteilung: **13.06.2012**
(21) Anmeldenummer: 07727726.7
(22) Anmeldetag: 03.04.2007
(51) Int. Cl.: A61K 31/519

(54) **DIHYDROTHIENOPYRIMIDINE ZUR BEHANDLUNG VON ENTZÜNDLICHEN ERKRANKUNGEN**
DIHYDROTHIENOPYRIMIDINE FOR TREATING INFLAMMATORY DISORDERS
DIHYDROTHIÉNOPYRIMIDINES DESTINÉES AU TRAITEMENT DE MALADIES INFLAMMATOIRES

(30) Priorität: 19.04.2006 EP 06112779
(43) Veröffentlichungstag der Anmeldung: 07.01.2009
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: POUZET, Pascale, 88400 Biberach (DE); FIEGEN, Dennis, 88400 Biberach (DE); FOX, Thomas, 88400 Biberach (DE); GOEGGEL, Rolf, 89073 Ulm (DE); HOENKE, Christoph, 55218 Ingelheim am Rhein (DE); JUNG, Birgit, 88471 Laupheim (DE); NICKOLAUS, Peter, 88447 Warthausen (DE)
(74) Vertreter: Hammann, Heinz
(86) Internationale Anmeldenummer: PCT/EP2007/053255
(87) Internationale Veröffentlichungsnummer: WO 2007/118793

(56) Entgegenhaltungen:
- WO-A-2006/111549
- CHAKRABORTI A K ET AL: "3D-QSAR Studies on thieno[3,2-d]pyrimidines as Phosphodiesterase IV Inhibitors" BIOORGANIC & MEDICINAL CHEMISTRY LETTERS, OXFORD, GB, Bd. 13, Nr. 8, 2003, Seiten 1403-1408, XP002392463 ISSN: 0960-894X

## Beschreibung

Die Erfindung betrifft neue Dihydrothienopyrimidine der Formel 1, sowie pharmakologisch verträgliche Salze, Diastereomere, Enantiomere, Racemate, Hydrate oder Solvate davon, 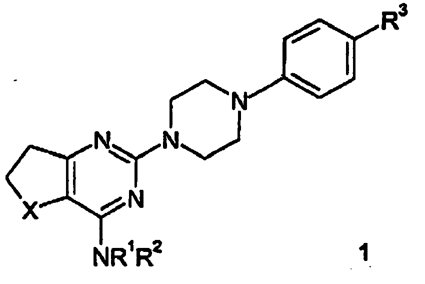 worin X SO ist,
die geeignet sind zur Behandlung von Atemwegs- oder gastrointestinalen Beschwerden oder Erkrankungen, entzündlichen Erkrankungen der Gelenke, der Haut oder der Augen, Erkrankungen des periphären oder zentralen Nervensystems oder Krebserkrankungen, sowie pharmazeutische Zusammensetzungen die diese Verbindungen beinhalten.

### STAND DER TECHNIK

US 3,318,881 und BE 663693 offenbaren die Herstellung von Dihydrothieno-[3,2-d]pyrimidine die kardiovaskulären und sedative Eigenschaften besitzen.

Chakraborti et al, Bioorganic & Medicinal Chemistry Lettes, Band 13, Nr. 8, 2003, Seiten 1403-1408 beschreibt Thienopyrimidine, die jedoch neben der zusätzlichen Doppelbindung im Ringsystem auch noch völlig anders substituiert sind als die erfindungsgemäßen Verbindungen.

WO 2006/111549 offenbart hingegen oxidierte Dihydrothienopyrimidine, die sich jedoch in ihrem Substitutionsmuster von den erfindungsgemäßen Verbindungen unterscheiden.

### BESCHREIBUNG DER ERFINDUNG

Überraschenderweise konnte nun gefunden werden, dass Dihydrothienopyrimidine der Formel **1**, insbesondere solche, in denen X SO bedeutet, geeignet sind zur Behandlung entzündlicher Erkrankungen.

Gegenstand der vorliegenden Erfindung sind deshalb Verbindungen der Formel 1 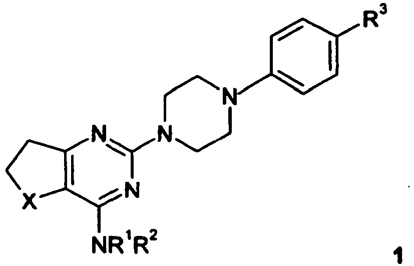 worin
- **X**: SO,
- **R¹**: H, C₁₋₆-Alkyl, C₂₋₆-Alkenyl, C₂₋₆-Alkinyl, C₆₋₁₀-Aryl, C₆₋₁₀-Aryl-C₁₋₆-alkylen oder C₅₋₁₀-Heteroaryl-C₁₋₆-alkylen
- **R²**: H ist oder ein Rest ausgewählt aus der Gruppe bestehend aus C₁₋₁₀-Alkyl, C₂₋₆-Alkenyl und C₂₋₆-Alkinyl ist, der gegebenenfalls durch Halogen substituiert sein kann und der gegebenenfalls durch einen oder mehrere Reste aus der Gruppe bestehend aus OR^{2.1}, COOR^{2.1}, CONR^{2.2}R^{2.3}, SR^{2.1}, C₆₋₁₀-Aryl, einem mono- oder bicyclischen C₃₋₁₀-Heterocyclus, einem mono- oder bicyclischen C₅₋₁₀-Heteroaryl, einem mono- oder bicyclischem C₃₋₁₀-Cycloalkyl, CH₂-NR^{2.2}R^{2.3} und NR^{2.2}R^{2.3} substituiert sein kann, welcher wiederum gegebenenfalls durch einen oder mehrere Reste ausgewählt aus der Gruppe bestehend aus OH, Halogen, OR^{2.1}, Oxo, C₁₋₆-Alkyl, C₆₋₁₀-Aryl, COOR^{2.1}, CH₂-NR^{2.2}R^{2.3} und NR^{2.2}R^{2.3} substituiert sein kann, wobei **R^{2.1}** H ist oder ein Rest ausgewählt ist aus der Gruppe bestehend aus C₁₋₆-Alkyl, C₁₋₆-Alkanol, C₁₋₃-Haloalkyl, mono- oder bicyclisches C₃₋₁₀ Cycloalkyl, C₆₋₁₀-Aryl-C₁₋₆-alkylen, mono- oder bicyclisches C₅₋₁₀-Heteroaryl-C₁₋₆-alkylen, C₃₋₁₀-Heterocyclus-C₁₋₆-alkylen, C₃₋₁₀-Cycloalkyl-C₁₋₆-alkylen, ein mono- oder bicyclisches C₆₋₁₀-Aryl, ein mono- oder bicyclisches C₅₋₁₀-Heteroaryl und ein mono- oder bicyclischer, gesättigter oder ungesättigter Heterocyclus, der gegebenenfalls durch einen oder mehrere Reste ausgewählt aus der Gruppe bestehend aus OH, Halogen, C₁₋₆-Alkyl und C₆₋₁₀-Aryl substituiert sein kann, wobei **R^{2.2}** und **R^{2.3}** unabhängig voneinander H sind oder ein Rest ausgewählt aus der Gruppe bestehend aus Halogen, C₁₋₆-Alkyl, mono- oder bicyclisches C₃₋₁₀ Cycloalkyl, C₆₋₁₀-Aryl-C₁₋₆-alkylen, C₅₋₁₀-Heteroaryl-C₁₋₆-alkylen, mono- oder bicyclisches C₆₋₁₀-Aryl, mono- oder bicyclischer C₃₋₁₀-Heterocyclus, mono- oder bicyclisches C₅₋₁₀-Heteroaryl, CO-NH₂, CO-NHCH₃, CO-N(CH₃)₂, SO₂(C₁-C₂-Alkyl), CO-R^{2.1} und COOR^{2.1}, der gegebenenfalls durch einen oder mehrere Reste ausgewählt aus der Gruppe bestehend aus OH, Halogen, C₁₋₆-Alkyl, C₆₋₁₀-Aryl und COOR^{2.1} substituiert sein kann,
- oder **R²**: ein mono- oder polycyclisches C₃₋₁₀ Cycloalkyl, das gegebenenfalls einfach oder mehrfach über C₁₋₃-Alkylgruppen verbrückt sein kann und das gegebenenfalls mit einem Rest ausgewählt aus der Gruppe bestehend aus verzweigtes oder unverzweigtes C₁₋₆-Alkanol, OR^{2.1}, COOR^{2.1}, SO₂NR^{2.2}R^{2.3}, C₃₋₁₀-Heterocyclus, C₆₋₁₀-Aryl, C₁₋₆-Alkyl, C₆₋₁₀-Aryl-C₁₋₆-alkylen, C₅₋₁₀Heteroaryl-C₁₋₆-alkylen, mono- oder bicyclisches C₃₋₁₀ Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, Halogen, C₁₋₆-Alkyl, C₆₋₁₀-Aryl und NR^{2.2}R^{2.3} substituiert sein kann,
- oder **R²**: ein mono- oder polycyclisches C₆₋₁₀-Aryl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3},C₃₋₁₀-Cycloalkyl, C₃₋₁₀-Heterocyclus, C₁₋₆-Alkyl, C₆₋₁₀-Aryl-C₁₋₆-alkylen, C₃₋₁₀-Heterocyclus-C₁₋₆-alkylen, C₅-₁₀-Heteroaryl-C₁₋₆-alkylen, C₆₋₁₀-Aryl, SO₂-CH₃, SO₂-CH₂CH₃ und SO₂-NR^{2.2}R^{2.3} substituiert sein kann, der wiederum gegebenenfalls durch einen oder mehrere oder mehrere Reste ausgewählt aus der Gruppe bestehend aus OH, OR^{2.1}, Oxo, Halogen, C₁₋₆-Alkyl, C₆₋₁₀-Aryl und NR^{2.2}R^{2.3} substituiert sein kann,
- oder **R²**: ein Rest ausgewählt aus einer Gruppe bestehend aus mono- oder bicyclischer, gesättigtem oder ungesättigtem C₃₋₁₀-Heterocyclus und einem mono- oder bicyclischen C₅₋₁₀-Heteroaryl, welcher 1 bis 4 Heteroatome ausgewählt aus S, O und N umfasst und gegebenenfalls durch einen oder mehrere Reste ausgewählt bedeuten aus der Gruppe Halogen, OH, Oxo und SH oder durch einen oder mehrere Reste ausgewählt aus der Gruppe OR^{2.1}, SR^{2.1}, COOR^{2.1}, COR^{2.1}, C₁₋₆-Alkanol, C₃₋₁₀-Cycloalkyl, C₆₋₁₀-Aryl, C₁₋₆-Alkyl, C₆₋₁₀-Aryl-C₁₋₆-alkylen, C₅₋₁₀-Heteroaryl-C₁₋₆-alkylen, C₅₋₁₀-Heterocyclus, C₅₋₁₀-Heteroaryl, C₁₋₆-Alkanol und NR^{2.2}R^{2.3} substituiert sein kann, der gegebenenfalls wiederum durch einen oder mehrere Reste ausgewählt aus der Gruppe bestehend aus OH, OR^{2.1}, Oxo, Halogen, C₁₋₆-Alkyl, C₆₋₁₀-Aryl, CH₂-NR^{2.2}R^{2.3} und NR^{2.2}R^{2.3} substituiert sein kann,
- oder worin **NR¹R²**: gemeinsam einen heterocyclischen C₄₋₇-Ring bedeuten, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₆-Alkanol, Oxo, Halogen, C₁₋₆-Alkyl, C₆₋₁₀-Aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3} und NR^{2.2}R^{2.3} substituiert sein kann, und worin
- **R³**: ausgewählt ist aus der Gruppe bestehend aus Fluor, Chlor, Brom, Iod, Hydroxy, SO₂-CH₃, COOR^{2.1}, Nitril-Gruppe und C₃₋₁₀-Heterocyclus-C₁₋₆-alkylen, wobei der C₃₋₁₀-Heterocyclus mono- oder bicyclisch sein kann und gegebenenfalls durch einen Rest ausgewählt aus der Gruppe bestehend aus OH, Halogen, Oxo,, C₁₋₆-Alkyl und C₆₋₁₀-Aryl substituiert sein kann,
- oder: ein Rest ausgewählt ist aus der Gruppe bestehend aus C₁₋₆-Alkyl, C₂₋₆-Alkenyl, C₂₋₆-Alkinyl, C₆₋₁₀-Aryl; C₆₋₁₀-Aryl-C₁₋₆-alkylen C₅₋₁₀-Heteroaryl-C₁₋₆-alkylen, C₃₋₁₀-Heterocyclus und C₃₋₁₀-Cycloalkyl, der gegebenenfalls mit einem Rest ausgewählt aus der Gruppe bestehend aus OH, Halogen, Oxo,, C₁₋₆-Alkyl und C₆₋₁₀-Aryl substituiert sein kann, oder
- **R³**: die Gruppe -CO-NR^{3.1}R^{3.2} bedeutet,
- wobei **R^{3.1}** und **R^{3.2}**: unabhängig voneinander H sind oder Reste sind, die ausgewählt sind aus der Gruppe bestehend aus C₁₋₆-Alkyl, C₂₋₆-Alkenyl, C₂₋₆-Alkinyl, C₆₋₁₀-Aryl; C₆-₁₀-Aryl-C₁₋₆-alkylen, C₅₋₁₀-Heteroaryl-C₁₋₆-alkylen, C₅₋₁₀Heteroaryl-C₁₋₆-alkinylen, C₅-₁₀-Heteroaryl-C₁₋₆-alkenylen, mono- oder bicyclischer, C₃₋₁₀-Heterocyclus, C₃₋₁₀-Heterocyclus-C₁₋₆-alkylen und mono- oder bicyclisches C₅₋₁₀-Heteroaryl, wobei der Rest jeweils gegebenenfalls mit einem oder mehreren Resten ausgewählt aus der Gruppe OH, Oxo, Halogen, C₁₋₆-Alkyl und O-C₁₋₆-Alkyl substituiert sein kann,
- oder: wobei
- **R³**: die Gruppe -NR^{3.3}-CO-R^{3.4} bedeutet,
- wobei: **R^{3.3}** H ist oder ein Rest ausgewählt aus der Gruppe bestehend aus C₁₋₆-Alkyl, C₂₋₆-Alkenyl, C₂₋₆-Alkinyl, C₆₋₁₀-Aryl; C₆₋₁₀-Aryl-C₁₋₆-alkylen, C₅₋₁₀-Heteroaryl-C₁₋₆-alkylen, C₃₋₁₀-Heterocyclus und einem C₅₋₁₀₋ Heteroaryl ist, der gegebenenfalls mit einem oder mehreren Resten ausgewählt aus der Gruppe bestehend aus OH, OR^{2.1}, Oxo, NH₂, NR^{2.2}R^{2.3}, Halogen, C₁₋₆-Alkyl und C₆₋₁₀-Aryl substituiert sein kann, und wobei **R^{3.4}** H ist oder ein Rest ausgewählt aus der Gruppe bestehend aus C₁₋₆-Alkyl, C₂₋₆-Alkenyl, C₂₋₆-Alkinyl, C₁₋₆-Alkanol, OR^{2.1}, CH₂-O-CO-C₁₋₆-Alkyl, CH₂-NR^{2.2}R^{2.3}, NR^{2.2}R^{2.3}, C₆₋₁₀-Aryl; C₆₋₁₀-Aryl-C₁₋₆-alkylen, C₅₋₁₀-Heteroaryl-C₁₋₆-alkylen, mono- oder bicyclischer, gesättigter, teilweise gesättigter oder ungesättigter C₃₋₁₀-Heterocyclus mit 1, 2 oder 3 Heteroatomen aus der Gruppe S, O und N und einem mono- oder bicyclischen C₅₋₁₀- Heteroaryl mit 1, 2 oder 3 Heteroatomen aus der Gruppe S, O und N ist, der gegebenenfalls mit einem oder mehreren Resten ausgewählt aus der Gruppe bestehend aus OH, OR^{2.1}, Oxo, NH₂, NR^{2.2}R^{2.3}, Halogen, C₁₋₆-Alkyl und C₆₋₁₀-Aryl substituiert sein kann, oder wobei
- **R³**: eine gegebenenfalls einfach oder zweifach N-substituierte Sulfonamidgruppe SO₂-NR^{3.5}R^{3.6} bedeutet,
- wobei R^{3.5} und R^{3.6}: jeweils unabhängig voneinander C₁₋₆-Alkyl oder C₆₋₁₀-Aryl sein können,
sowie pharmakologisch verträgliche Salze, Diastereomere, Enantiomere, Racemate, Hydrate oder Solvate davon.

Ein bevorzugter Gegenstand der Erfindung sind die obigen Verbindungen der Formel 1, worin
- **X**: SO
- **R¹**: H, C₁₋₆-Alkyl, C₆₋₁₀-Aryl, C₆₋₁₀-Aryl-C₁₋₆-alkylen oder C₅₋₁₀-Heteroaryl-C₁₋₆-alkylen
- **R²**: H ist oder C₁₋₆-Alkyl, das gegebenenfalls durch Halogen substituiert sein kann und das gegebenenfalls durch einen oder mehrere Reste ausgewählt aus der Gruppe bestehend aus OR^{2.1}, COOR^{2.1}, CONR^{2.2}R^{2.3}, SR^{2.1}, C₆₋₁₀-Aryl, einem mono- oder bicyclischen C₃₋₁₀-Heterocyclus, einem mono- oder bicyclischen C₅₋₁₀-Heteroaryl, einem mono- oder bicyclische C₃₋₁₀-Cycloalkyl, CH₂-NR^{2.2}R^{2.3} und NR^{2.2}R^{2.3} substituiert sein kann, welcher wiederum gegebenenfalls durch einen oder mehrere Reste ausgewählt aus der Gruppe bestehend aus OH, Halogen, OR^{2.1}, Oxo, C₁₋₆-Alkyl, C₆₋₁₀-Aryl, COOR^{2.1}, CH₂-NR^{2.2}R^{2.3} und NR^{2.2}R^{2.3} substituiert sein kann, wobei **R^{2.1}** H ist oder ein Rest ausgewählt ist aus der Gruppe bestehend aus C₁₋₆-Alkyl, C₁₋₆-Alkanol, C₁₋₃-Haloalkyl, ein mono- oder bicyclisches C₃₋₁₀-Cycloalkyl, ein C₆₋₁₀Aryl-C₁₋₆-alkylen oder C₅₋₁₀Heteroaryl-C₁₋₆-alkylen, C₃₋₁₀-Heterocyclus-C₁₋₆-alkylen, C₃₋₁₀-Cycloalkyl-C₁₋₆-alkylen, ein mono- oder bicyclisches C₆₋₁₀-Aryl, ein mono- oder bicyclisches C₅₋₁₀-Heteroaryl und ein mono- oder bicyclischer, gesättigter oder ungesättigter C₃₋₁₀-Heterocyclus ist, der gegebenenfalls durch einen oder mehrere Reste ausgewählt aus der Gruppe bestehend aus OH, Halogen, C₁₋₆-Alkyl, C₆₋₁₀-Aryl substituiert sein kann, wobei **R^{2.2}** und **R^{2.3}** unabhängig voneinander H sind oder ausgewählt sind aus der Gruppe bestehend aus Halogen, C₁₋₆-Alkyl, mono- oder bicyclisches C₃₋₁₀ Cycloalkyl, C₆₋₁₀-Aryl-C₁₋₆-alkylen, C₅₋₁₀-Heteroaryl-C₁₋₆-alkylen, mono- oder bicyclisches C₆₋₁₀-Aryl, mono- oder bicyclischer, gesättigter oder ungesättigter C₃₋₁₀-Heterocyclus, mono- oder bicyclisches C₅₋₁₀-Heteroaryl, CO-NH₂, CO-NHCH₃, CO-N(CH₃)₂, SO₂(C₁-C₂-Alkyl), CO-R^{2.1} und COOR^{2.1}, der gegebenenfalls durch einen oder mehrere Reste ausgewählt aus der Gruppe bestehend aus OH, Halogen, C₁₋₆-Alkyl, C₆₋₁₀-Aryl und COOR^{2.1} substituiert sein kann, oder
- **R²**: ein mono- oder polycyclisches C₃₋₁₀-Cycloalkyl, das gegebenenfalls einfach oder mehrfach über C₁₋₃-Alkylgruppen verbrückt sein kann und das gegebenenfalls einfach oder mehrfach durch OH oder Halogen oder durch einen oder mehrere Reste ausgewählt aus der Gruppe bestehend aus verzweigtes oder unverzweigtes C₁₋₆-Alkanol, OR^{2.1}, COOR^{2.1}, SO₂NR^{2.2}R^{2.3}, C₃₋₁₀-Heterocyclus, C₆₋₁₀-Aryl, C₁₋₆-Alkyl, C₆₋₁₀-Aryl-C₁₋₆-alkylen, C₁₅₋₁₀-Heteroaryl-C₁₋₆-alkylen, mono- oder bicyclisches C₃₋₁₀-Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, Halogen, C₁₋₆-Alkyl, C₆₋₁₀-Aryl und NR^{2.2}R^{2.3} substituiert sein kann, oder
- **R²**: ein mono- oder polycyclisches C₆₋₁₀-Aryl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3},C₃₋₁₀-Cycloalkyl, C₃₋₁₀-Heterocyclus, C₁₋₆-Alkyl, C₆₋₁₀-Aryl-C₁₋₆-alkylen, C₃₋₁₀-Heterocyclus-C₁₋₆-alkylen, C₅-₁₀-Heteroaryl-C₁₋₆-alkylen, C₆₋₁₀-Aryl, SO₂-CH₃, SO₂-CH₂CH₃ und SO₂-NR^{2.2}R^{2.3} substituiert sein kann, der wiederum gegebenenfalls durch einen oder mehrere Reste ausgewählt aus der Gruppe bestehend aus OH, OR^{2.1}, Oxo, Halogen, C₁₋₆-Alkyl, C₆₋₁₀-Aryl und NR^{2.2}R^{2.3} substituiert sein kann, oder
- **R²**: ein Rest ausgewählt aus einer Gruppe bestehend aus mono- oder bicyclischen, gesättigtem oder ungesättigtem C₃₋₁₀-Heterocyclus und einem mono- oder bicyclischen C₅₋₁₀-Heteroaryl, welcher 1 bis 4 Heteroatome ausgewählt aus S, O und N umfasst und gegebenenfalls durch einen oder mehrere Reste ausgewählt aus der Gruppe Halogen, OH, Oxo und SH oder durch einen oder mehrere Reste ausgewählt aus der Gruppe bestehend aus OR^{2.1}, SR^{2.1}, COOR^{2.1}, COR^{2.1}, C₁₋₆-Alkanol, C₃₋₁₀-Cycloalkyl, C₆₋₁₀-Aryl, C₁₋₆-Alkyl, C₆₋₁₀-Aryl-C₁₋₆-alkylen, C₅₋₁₀- Heteroaryl-C₁₋₆-alkylen, C₅₋₁₀-Heterocyclus, C₅₋₁₀-Heteroaryl, C₁₋₆-Alkanol und NR^{2.2}R^{2.3} substituiert sein kann, der gegebenenfalls wiederum durch einen oder mehrere Reste ausgewählt aus der Gruppe bestehend aus OH, OR^{2.1}, Oxo, Halogen, C₁₋₆-Alkyl, C₆₋₁₀-Aryl und NR^{2.2}R^{2.3} substituiert sein kann,
- oder **NR¹R²**: bedeutet gemeinsam einen heterocyclischen C₄₋₇-Ring, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₆-Alkanol, Oxo, Halogen, C₁₋₆-Alkyl, C₆₋₁₀-Aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂NR^{2.2}R^{2.3} und NR^{2.2}R^{2.3} substituiert sein kann,
sowie pharmakologisch verträgliche Salze, Diastereomere, Enantiomere, Racemate, Hydrate oder Solvate davon.

Bevorzugt sind weiterhin die oben genannten Verbindungen der Formel 1, worin
- **X**: SO, und
- **R¹**: H, C₁₋₆-Alkyl, C₆₋₁₀-Aryl, C₆₋₁₀-Aryl-C₁₋₆-alkylen oder C₅₋₁₀-Heteroaryl-C₁₋₆-alkylen, und
- **R²**: H ist oder C₁₋₆-Alkyl, das gegebenenfalls durch Halogen substituiert sein kann und das gegebenenfalls durch einen oder mehrere Reste ausgewählt aus der Gruppe bestehend aus OR^{2.1}, COOR^{2.1}, CONR^{2.2}R^{2.3}, SR^{2.1}, Phenyl, einem mono- oder bicyclischen C₅₋₁₀-Heterocyclus, C₅₋₆-Heteroaryl, einem mono- oder bicyclischem C₅₋₁₀-Cycloalkyl, CH₂-NR^{2.2}R²⁻³ und NR^{2.2}R^{2.3} substituiert sein kann, welcher wiederum gegebenenfalls durch einen oder mehrere Reste ausgewählt aus der Gruppe bestehend aus OH, Halogen, OR^{2.1} Oxo, C₁₋₆-Alkyl, Phenyl, COOR^{2.1}, CH₂-NR^{2.2}R^{2.3} und NR^{2.2}R^{2.3} substituiert sein kann, wobei **R^{2.1}** H ist oder ein Rest ausgewählt ist aus der Gruppe bestehend aus C₁₋₆-Alkyl, C₁₋₆-Alkanol, C₁₋₃-Haloalkyl, ein mono- oder bicyclisches C₅₋₁₀ Cycloalkyl, ein Phenyl-C₁₋₆-alkylen, ein C₅₋₆-Heteroaryl-C₁₋₆-alkylen, C₅₋₁₀-Heterocyclus-C₁₋₆-alkylen, C₅₋₁₀-Cycloalkyl-C₁₋₆-alkylen, Phenyl, ein mono- oder bicyclische C₅₋₁₀-Heteroaryl und ein mono- oder bicyclischer, gesättigter oder ungesättigter C₅₋₁₀-Heterocyclus ist, der gegebenenfalls durch einen oder mehrere Reste ausgewählt aus der Gruppe bestehend aus OH, Halogen, C₁₋₆-Alkyl und Phenyl substituiert sein kann, wobei **R^{2.2}** und **R^{2.3}** unabhängig voneinander H sind oder ein Rest ausgewählt aus der Gruppe bestehend aus Halogen, C₁₋₆-Alkyl, mono- oder bicyclisches C₅₋₁₀ Cycloalkyl, Phenyl-C₁₋₆-alkylen, C₅₋₆-Heteroaryl-C₁₋₆-alkylen, Phenyl, mono- oder bicyclischer C₅₋₁₀-Heterocyclus, mono- oder bicyclisches C₅₋₆-Heteroaryl, CO-NH₂, CO-NHCH₃, CO-N(CH₃)₂, SO₂(C₁-C₂-Alkyl), CO-R^{2.1} und COOR^{2.1} sind, der gegebenenfalls durch einen oder mehrere Reste ausgewählt aus der Gruppe bestehend aus OH, Halogen, C₁₋₆-Alkyl, Phenyl und COOR^{2.1} substituiert sein kann, oder
- **R**²: ein mono- oder polycyclisches C₅₋₁₀-Cycloalkyl, das gegebenenfalls einfach oder mehrfach über C₁₋₃-Alkylgruppen verbrückt sein kann und das gegebenenfalls einfach oder mehrfach durch OH oder Halogen oder durch einen oder mehrere Reste ausgewählt aus der Gruppe verzweigtes oder unverzweigtes C₁₋₃-Alkanol, OR^{2.1}, COOR^{2.1}, SO₂NR^{2.2}R^{2.3}, C₅₋₁₀-Heterocyclus, Phenyl, C₁₋₆-Alkyl, Phenyl-C₁₋₆-alkylen, C₅₋₆-Heteroaryl-C₁₋₆-alkylen, mono- oder bicyclisches C₅₋₁₀-Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, Halogen, C₁₋₆-Alkyl, Phenyl und NR^{2.2}R^{2.3} substituiert sein kann,
- **R²**: oder Phenyl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3},C₅₋₁₀-Cycloalkyl, C₅₋₁₀-Heterocyclus, C₁₋₆-Alkyl, Phenyl-C₁-₆-alkylen, C₅₋₁₀-Heterocyclus-C₁₋₆-alkylen, C₅₋₆-Heteroaryl-C₁₋₆-alkylen, Phenyl, SO₂-CH₃, SO₂-CH₂CH₃ und SO₂-NR^{2.2}R^{2.3} substituiert sein kann, der wiederum gegebenenfalls durch einen oder mehrere Reste ausgewählt aus der Gruppe bestehend aus OH, OR^{2.1}, Oxo, Halogen, C₁₋₆-Alkyl, C₆₋₁₀-Aryl und NR^{2.2}R^{2.3} substituiert sein kann,
- **R²**: oder ein Rest ausgewählt aus einer Gruppe bestehend aus mono- oder bicyclischem, gesättigtem oder ungesättigtem C₅₋₁₀-Heterocyclus und mono- oder bicyclisches C₅₋₆-Heteroaryl, welcher 1 bis 4 Heteroatome ausgewählt aus S, O und N umfasst und gegebenenfalls durch einen oder mehrere Reste ausgewählt aus der Gruppe Halogen, OH, Oxo und SH oder durch einen oder mehrere Reste ausgewählt aus der Gruppe OR^{2.1}, SR^{2.1}, COOR^{2.1}, COR^{2.1}, C₁₋₆-Alkanol, C₃₋₁₀-Cycloalkyl, Phenyl, C₁₋₆-Alkyl, Phenyl-C₁₋₆-alkylen, C₅₋₆-Heteroaryl-C₁₋₆alkylen, C₅-₁₀-Heterocyclus, C₅₋₆-Heteroaryl, C₁₋₆-Alkanol und NR^{2.2}R^{2.3} substituiert sein kann, der gegebenenfalls wiederum durch einen oder mehrere Reste ausgewählt aus der Gruppe bestehend aus OH, OR^{2.1}, Oxo, Halogen, C₁₋₆-Alkyl, Phenyl und **NR^{2.2}R^{2.3}** substituiert sein kann,
sowie pharmakologisch verträgliche Salze, Diastereomere, Enantiomere, Racemate, Hydrate oder Solvate davon.

Besonders bevorzugt sind weiterhin die obigen Verbindungen der Formel **1**, worin **NR¹R²** gemeinsam einen heterocyclischen C₄₋₇-Ring bedeutet, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₆-Alkanol, Oxo, Halogen, C₁₋₆-Alkyl, C₆₋₁₀-Aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,

sowie pharmakologisch verträgliche Salze, Diastereomere, Enantiomere, Racemate, Hydrate oder Solvate davon.

Ein bevorzugter Gegenstand der Erfindung sind weiterhin die obigen Verbindungen nach Formel **1**, worin
- **R¹**: H oder Methyl ist,
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel 1, worin
- **NR¹R²**: gemeinsam einen Pyrrolidinring bilden, der gegebenenfalls durch einen oder mehrere Reste ausgewählt aus der Gruppe bestehend aus OH, OR^{2.1}, CH₂-OH, CH₂-CH₂-OH, Oxo, Cl, F, Br, Methyl, Ethyl, Propyl, Phenyl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}SO₂-C₁₋₃-Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel 1, worin
- **R²**: Phenyl bedeutet, das einfach oder mehrfach durch OH, SH oder Halogen und/oder durch einen oder mehrere Reste ausgewählt aus der Gruppe bestehend aus OR^{2.1}, COOR^{2.1}, NR^{2.2}R^{2.3}, CH₂-NR^{2.2}R^{2.3},C₅₋₁₀-Cycloalkyl, C₅₋₁₀-Heterocyclus, C₁₋₆-Alkyl, Phenyl-C₁₋₆-alkylen, C₅₋₁₀-Heterocyclus-C₁₋₆-alkylen, C₅₋₆-Heteroaryl-C₁₋₆-alkylen, Phenyl, SO₂-CH₃, SO₂-CH₂CH₃ und SO₂-NR^{2.2}R^{2.3} an beliebiger Position substituiert ist, der wiederum gegebenenfalls durch einen oder mehrere Reste ausgewählt aus der Gruppe bestehend aus OH, OR^{2.1}, Oxo, Halogen, C₁₋₆-Alkyl, Phenyl und NR^{2.2}R^{2.3} substituiert sein kann,
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel 1, worin
- **R²**: Phenyl ist, das in mindestens einer der beiden meta-Stellungen durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3}, C₅₋₁₀-Cycloalkyl, C₅₋₁₀-Heterocyclus, C₁₋₆-Alkyl, Phenyl-C₁₋₆-alkylen, C₅₋₁₀-Heterocyclus-C₁₋₆-alkylen, C₅₋₆-Heteroaryl-C₁₋₆-alkylen, Phenyl SO₂-CH₃, SO₂-CH₂CH₃ und SO₂-NR^{2.2}R^{2.3} substituiert sein kann, der wiederum gegebenenfalls durch einen oder mehrere Reste ausgewählt aus der Gruppe bestehend aus OH, OR^{2.1}, Oxo, Halogen, C₁₋₆-Alkyl, Phenyl und NR^{2.2}R^{2.3} substituiert sein kann,
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel **1**, worin
- **R²**: Phenyl ist, das in mindestens einer der beiden meta-Stellungen durch einen oder mehreren Reste ausgewählt aus der Gruppe bestehend aus Methyl, F, Cl, OH, OR^{2.1}, COOR^{2.1}, NH₂ und N(CH₃)₂ substituiert ist,
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel **1**, worin
- **R²**: C₁₋₆-Alkyl ist, das gegebenenfalls durch Halogen substituiert sein kann und das gegebenenfalls durch einen oder mehrere Reste ausgewählt aus der Gruppe bestehend aus OR^{2.1}, COOR^{2.1}, CONR^{2.2}R^{2.3}, SR^{2.1}, Phenyl, einem mono- oder bicyclischen C₅₋₁₀-Heterocyclus, C₅₋₆-Heteroaryl, einem mono- oder bicyclischem C₅₋₁₀-Cycloalkyl, CH₂-NR^{2.2}R^{2.3} und NR^{2.2}R^{2.3} substituiert sein kann, welcher wiederum durch einen oder mehrere Reste ausgewählt aus der Gruppe bestehend aus OH, Halogen, OR^{2.1}, Oxo, C₁₋₆-Alkyl, Phenyl, COOR^{2.1}, CH₂-NR^{2.2}R^{2.3} und NR^{2.2}R^{2.3} substituiert sein kann,
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel **1**, worin
- **R²**: Methyl, Ethyl oder Propyl ist,
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel 1, worin
- **R²**: C₁₋₆-Alkyl ist, das durch einen oder mehrere Reste ausgewählt aus der Gruppe bestehend aus OH, COOR^{2.1}, CON(CH₃)₂, C₁₋₆-Alkyl, Phenyl, Cyclopropyl und NR^{2.2}R^{2.3} substituiert ist, welcher wiederum gegebenenfalls durch einen oder mehrere Reste ausgewählt aus der Gruppe bestehend aus OH, Fluor, Chlor, Brom, Iod, OR^{2.1}, Oxo, C₁₋₆-Alkyl, Phenyl, C₁₋₃-Alkanol, CH₂-NR^{2.2}R^{2.3} und NR^{2.2}R^{2.3} substituiert sein kann,
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel **1**, worin
- **R²**: C₁₋₆-Alkyl ist, das durch einen oder mehrere Reste ausgewählt aus der Gruppe bestehend aus OH, Phenyl, COOR², NH₂ substituiert ist, wobei das Phenyl wiederum gegebenenfalls durch einen oder mehrere Reste ausgewählt aus der Gruppe bestehend aus OH, , Fluor, Chlor, Brom, Iod, OR^{2.1}, C₁₋₆-Alkyl, CH₂-NH₂, CH₂(CH₃)₂, NH₂ und N(CH₃)₂ substituiert sein kann.
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel **1**, worin
- **R²**: ein Rest nach Formel 2 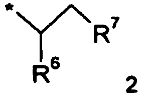
ist,
- worin **R⁷**: OH oder NH₂ ist und
- worin **R⁶**: ein Rest ausgewählt aus der Gruppe bestehend aus C₁₋₆-Alkyl, C₅₋₁₀-Heteroaryl und C₆₋₁₀-Aryl, vorzugsweise Phenyl, ist, das gegebenenfalls mit einem oder mehreren Resten ausgewählt aus der Gruppe bestehend aus Halogen, OH, COOR^{2.1}, OR^{2.1}, NH₂, C₁₋₆-Alkyl, C₁₋₆-Haloalkyl und C₁₋₆-Alkanol substituiert sein kann,
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen der Formel **1**, worin
- **R²**: ein Rest nach Formel **2** 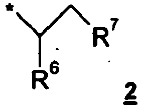 ist,
worin
- **R⁷**: OH oder NH₂ ist und
- **R⁶**: Methyl, Ethyl, Propyl, Isopropyl ist.

Ein weiterer bevorzugter Gegenstand der Erfindung sind die obigen Verbindungen der Formel **1**, worin
- R²: ein monocyclischer C₃₋₇-Cycloalkyl-Ring ist, der in spiro-Stellung mit einem Rest ausgewählt aus der Gruppe bestehend aus -OH; -CH₂-OH, -CH₂-CH₂-OH, verzweigtes oder unverzweigtes C₃₋₆-Alkanol, -OR^{2.1}, Methyl, Ethyl, Propyl, Isopropyl, Butyl, Isobutyl und Halogen, substituiert sein kann, wobei R^{2.1} ausgewählt aus der Gruppe bestehend aus Methyl, Ethyl, Propyl, Isopropyl, Butyl, Isobutyl sein kann.

Ein weiterer bevorzugter Gegenstand der Erfindung sind die obigen Verbindungen der Formel **1,** worin
- **R²**: ein Rest ausgewählt aus einer Gruppe bestehend aus monocyclischen, gesättigten drei-, vier-, fünf-, sechs- oder siebengliedrigem Heterocyclus mit 1, 2 oder 3 Heteroatomen jeweils ausgewählt aus der Gruppe bestehend aus N, O und S, der gegebenenfalls durch einen oder mehrere Reste ausgewählt aus der Gruppe Fluor, Chlor, Brom, OH, Oxo und SH oder durch einen oder mehrere Reste ausgewählt aus der Gruppe OR^{2.1}, SR^{2.1}, COOR^{2.1}, COR^{2.1}, C₁₋₈-Alkanol, C₃-₁₀-Cycloalkyl, Phenyl, C₁₋₈-Alkyl, Phenyl-C₁₋₈-alkylen, C₅₋₁₀-Heteroaryl-C₁₋₆-alkylen, C₅₋₁₀-Heterocyclus, C₅₋₁₀-Heteroaryl und NR^{2.2}R^{2.3} substituiert sein kann, der gegebenenfalls wiederum durch einen oder mehrere Reste ausgewählt aus der Gruppe bestehend aus OH, OR^{2.1}, Oxo, Halogen, C₁₋₈-Alkyl, Phenyl und NR^{2.2}R^{2.3} substituiert sein kann, wobei
- **_{R}^{2.1}**: H oder ein Rest ausgewählt aus der Gruppe bestehend aus C₁₋₆-Alkyl, C₁₋₆-Alkanol, C₁₋₃-Haloalkyl, mono- oder bicyclisches C₃₋₁₀ Cycloalkyl, Phenyl-C₁₋₆-alkylen, mono- oder bicyclisches C₅₋₁₀-Heteroaryl-C₁₋₆-alkylen, C₃₋₁₀-Heterocyclus-C₁₋₈-alkylen, C₃₋₇-Cycloalkyl-C₁₋₆-alkylen, Phenyl, ein mono- oder bicyclisches C₅₋₁₀-Heteroaryl und ein monocyclischer, gesättigter oder ungesättigter, fünf-, sechs- oder siebengliedriger Heterocyclus mit 1, 2 oder 3 Heteroatomen aus der Gruppe bestehend aus N, O und S, der gegebenenfalls durch einen oder mehrere Reste ausgewählt aus der Gruppe bestehend aus OH, Halogen, C₁₋₆-Alkyl und Phenyl substituiert sein kann, wobei **R^{2.2}** und **R^{2.3}** unabhängig voneinander H sind oder ein Rest ausgewählt sind aus der Gruppe bestehend aus Halogen, C₁₋₈-Alkyl, mono- oder bicyclisches C₃₋₁₀ Cycloalkyl, Phenyl-C₁₋₆-alkylen, C₅₋₁₀-Heteroaryl-C₁₋₆-alkylen, Phenyl, mono- oder bicyclischer C₃₋₁₀-Heterocyclus, mono- oder bicyclisches C₅₋₁₀-Heteroaryl, CO-NH₂, CO-NHCH₃, CO-N(CH₃)₂, SO₂(C₁-C₂-Alkyl), CO-R^{2.1} und COOR^{2.1}, der gegebenenfalls durch einen oder mehrere Reste ausgewählt aus der Gruppe bestehend aus OH, Halogen, C₁₋₈-Alkyl, Phenyl und COOR^{2.1} substituiert sein kann.

Ein weiterer bevorzugter Gegenstand der Erfindung sind die obigen Verbindungen nach Formel **1,** worin
- **R³**: Fluor, Chlor, Brom, Iod oder CN bedeutet, sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel 1, worin
- **R³**: die Gruppe -CO-NR^{3.1}R^{3.2} bedeutet,
- wobei **R^{3.1}** und **R^{3.2}**: unabhängig voneinander H sind oder Reste sind, die ausgewählt sind aus der Gruppe bestehend aus C₁₋₆-Alkyl, C₂₋₆-Alkenyl, C₂₋₆-Alkinyl, C₆₋₁₀-Aryl; C₆- ₁₀-Aryl-C₁₋₆-alkylen, C₅₋₁₀-Heteroaryl-C₁₋₆-alkylen, C₅₋₁₀-Heteroaryl-C₁₋₆-alkinylen, C₅-₁₀Heteroaryl-C₁₋₆-alkenylen, mono- oder bicyclischer, C₅₋₁₀-Heterocyclus, C₅₋₁₀-Heterocyclus-C₁₋₆-alkylen und mono- oder bicyclisches C₅₋₁₀-Heteroaryl, wobei der Rest jeweils gegebenenfalls mit einem oder mehreren Resten ausgewählt aus der Gruppe bestehend aus OH, Oxo, Halogen, C₁₋₆-Alkyl und O-C₁₋₆-Alkyl substituiert sein kann,
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel **1**, worin
- **R³**: die Gruppe -CO-NR^{3.1}R^{3.2} bedeutet, wobei
- **R^{3.1}**: Wasserstoff oder Methyl ist und wobei
- **R^{3.2}**: ein Rest ausgewählt aus der Gruppe bestehend aus C₁₋₆-Alkyl, C₂₋₆-Alkenyl, C₂₋₆-Alkinyl, C₆₋₁₀-Aryl; C₆₋₁₀-Aryl-C₁₋₆-alkylen, C₅₋₁₀-Heteroaryl-C₁₋₆-alkylen, C₅₋₁₀-Heteroaryl-C₁₋₆-alkinylen, C₅₋₁₀-Heteroaryl-C₁₋₆-alkenylen, mono- oder bicyclischer, C₅₋₁₀-Heterocyclus, C₅₋₁₀-Heterocyclus-C₁₋₆-alkylen und mono- oder bicyclisches C₅-₁₀-Heteroaryl ist, wobei der Rest jeweils gegebenenfalls mit einem oder mehreren Resten ausgewählt aus der Gruppe bestehend aus OH, Oxo, Halogen, C₁₋₆-Alkyl und O-C₁₋₆-Alkyl substituiert sein kann,
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel **1**, worin
- **R³**: die Gruppe -CO-NR^{3.1}R^{3.2} bedeutet,
- wobei **R^{3.1}** und **R^{3.2}**: unabhängig voneinander H sind oder Reste sind, die ausgewählt sind aus der Gruppe bestehend aus C₁₋₆-Alkyl, Phenyl; Phenyl-C₁₋₆-alkylen, C₅₋₆-Heteroaryl-C₁₋₆-alkylen, C₅₋₆-Heteroaryl-C₁₋₆-alkinylen, C₅₋₆-Heteroaryl-C₁₋₆- alkenylen, mono- oder bicyclischer, C₅₋₁₀-Heterocyclus, C₅₋₁₀-Heterocyclus-C₁₋₆-alkylen und mono- oder bicyclisches C₅₋₁₀-Heteroaryl, wobei der Rest jeweils gegebenenfalls mit einem oder mehreren Resten ausgewählt aus der Gruppe bestehend aus OH, Oxo, Halogen, C₁₋₆-Alkyl und O-C₁₋₆-Alkyl substituiert sein kann
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel 1, worin
- R³: die Gruppe -NR^{3.3}-CO-R ^{3.4} bedeutet,
- wobei R^{3.3}: H ist oder ein Rest ausgewählt aus der Gruppe bestehend aus C₁₋₆-Alkyl, C₂₋₆-Alkenyl, C₂₋₆-Alkinyl, C₆₋₁₀-Aryl; C₆₋₁₀-Aryl-C₁₋₆-alkylen, C₅₋₁₀-Heteroaryl-C₁₋₆-alkylen, C₃₋₁₀-Heterocyclus und einem C₅₋₁₀- Heteroaryl ist, der gegebenenfalls mit einem oder mehreren Resten ausgewählt aus der Gruppe bestehend aus OH, OR^{2.1}, Oxo, NH₂, NR^{2.2}R^{2.3}, Halogen,C₁₋₆-Alkyl und C₆₋₁₀-Aryl substituiert sein kann, und
- wobei R^{3.4}: H ist oder ein Rest ausgewählt aus der Gruppe bestehend aus C₁₋₆-Alkyl, C₂₋₆-Alkenyl, C₂₋₆-Alkinyl, C₁₋₆-Alkanol, OR^{2.1}, CH₂-O-CO-C₁₋₆-Alkyl, CH₂NR^{2.2}R^{2.3}, NR^{2.2}R^{2.3}, C₆₋₁₀-Aryl; C₆₋₁₀-Aryl-C₁₋₆-alkylen, C₅₋₁₀-Heteroaryl-C₁₋₆-alkylen, mono- oder bicyclischer, gesättigter oder ungesättigter C₃₋₁₀-Heterocyclus mit 1, 2 oder 3 Heteroatomen aus der Gruppe O, S und N und einem mono- oder bicyclischen C₅₋₁₀-Heteroaryl mit 1, 2 oder 3 Heteroatomen aus der Gruppe O, S und N ist, der gegebenenfalls mit einem oder mehreren Resten ausgewählt aus der Gruppe bestehend aus OH, OR^{2.1}, Oxo, NH₂, NR^{2.2}R^{2.3}, Halogen, C₁₋₆₋Alkyl und C₆₋₁₀-Aryl substituiert sein kann,
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel 1, worin
- R³: die Gruppe -NR^{3..3}-CO-R^{3.4} bedeutet,
- wobei R^{3.3}: Wasserstoff oder Methyl und
- wobei R^{3.4}: ein Rest ausgewählt aus der Gruppe bestehend aus C₁₋₆-Alkyl, C₂₋₆-Alkenyl, C₂₋₆-Alkinyl, C₁₋₆-Alkanol, OR^{2.1}, CH₂-O-CO-C₁₋₆-Alkyl, CH₂NR^{2.2}R^{2.3} , NR^{2.2}R^{2.3}, C₆₋₁₀₋Aryl; C₆₋₁₀-Aryl-C₁₋₆ₐ-alkylen, C₅₋₁₀-Heteroaryl;. C₁₋₆-alkylen, mono- oder bicyclischer, gesättigter oder ungesättigter C₃₋₁₀-Heterocyclus mit 1, 2 oder 3 Heteroatomen aus der Gruppe N; S und **O** und einem mono- oder bicyclischen C₅₋₁₀- Heteroaryl mit 1, 2 oder 3 Heteroatomen aus der Gruppe N; S und O ist, der gegebenenfalls mit einem oder mehreren Resten ausgewählt aus der Gruppe bestehend aus OH, OR^{2.1}, Oxo, NH₂, NR^{2.2}R^{2.3}, Halogen, C₁₋₆-Alkyl und C₆₋₁₀-Aryl substituiert sein kann,
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel 1, worin
- R³: die Gruppe -NR^{3.3}-CO-R^{3.4} bedeutet,
- wobei R^{3.3}: H ist oder ein Rest ausgewählt aus der Gruppe bestehend aus C₁₋₆-Alkyl, Phenyl; Phenyl-C₁₋₆-alkylen, C₅₋₁₀-Heteroaryl-C₁₋₆-alkylen, C₅₋₁₀-Heterocyclus und einem C₅₋₁₀- Heteroaryl ist, der gegebenenfalls mit einem oder mehreren Resten ausgewählt aus der Gruppe bestehend aus OH, OR^{2.1}, Oxo, NH₂, N(CH₃)₂, Halogen, C₁₋₆-Alkyl und Phenyl substituiert sein kann; und
- wobei R^{3.4}: H ist oder ein Rest ausgewählt aus der Gruppe bestehend aus C₁₋₈-Alkyl, ^{.} C₁₋₈-Alkanol, OR^{2.1}, CH₂-O-CO-C₁₋₆-Alkyl, CH₂-NH₂, CH₂-N(CH₃)₂, NH₂, N(CH₃)₂, Phenyl; Phenyl-C₁₋₆-alkylen, C₅₋₁₀-Heteroaryl-C₁₋₆-alkylen, mono- oder bicyclischer, gesättigter oder ungesättigter C₅₋₁₀-Heterocyclus mit 1, 2 oder 3 Heteroatomen aus der Gruppe N; S und O und einem mono- oder bicyclischen C₅₋₁₀- Heteroaryl mit 1, 2 oder 3 Heteroatomen aus der Gruppe N; S und O ist, der gegebenenfalls mit einem oder mehreren Resten ausgewählt aus der Gruppe bestehend aus OH, OR^{2.1}, Oxo, NH₂, N(CH₃)₂, Halogen, C₁₋₆-Alkyl und Phenyl substituiert sein kann, -
sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel 1, worin
- R¹: H ist und
- R²: ausgewählt ist aus der Gruppe bestehend aus H, Methyl, Ethyl; Propyl. 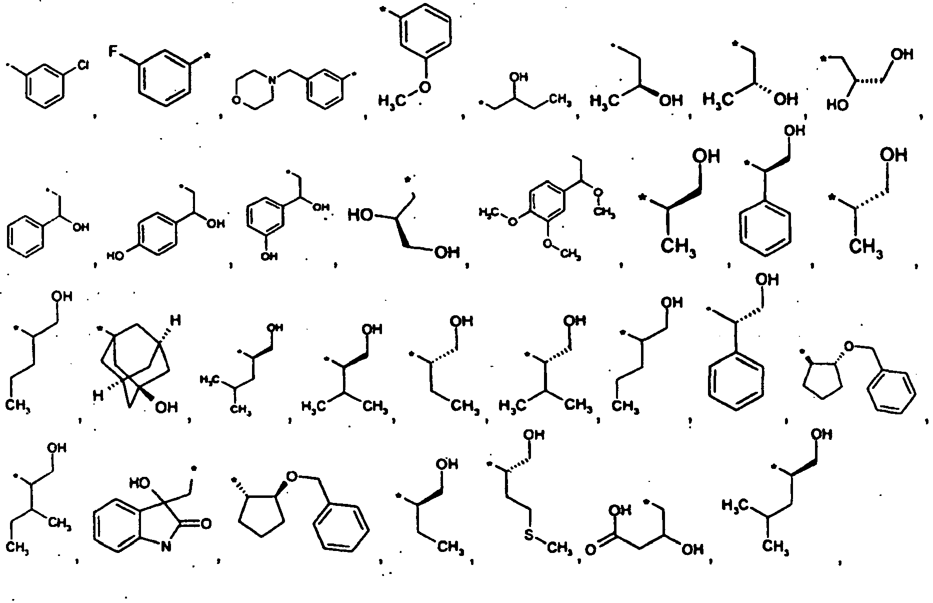 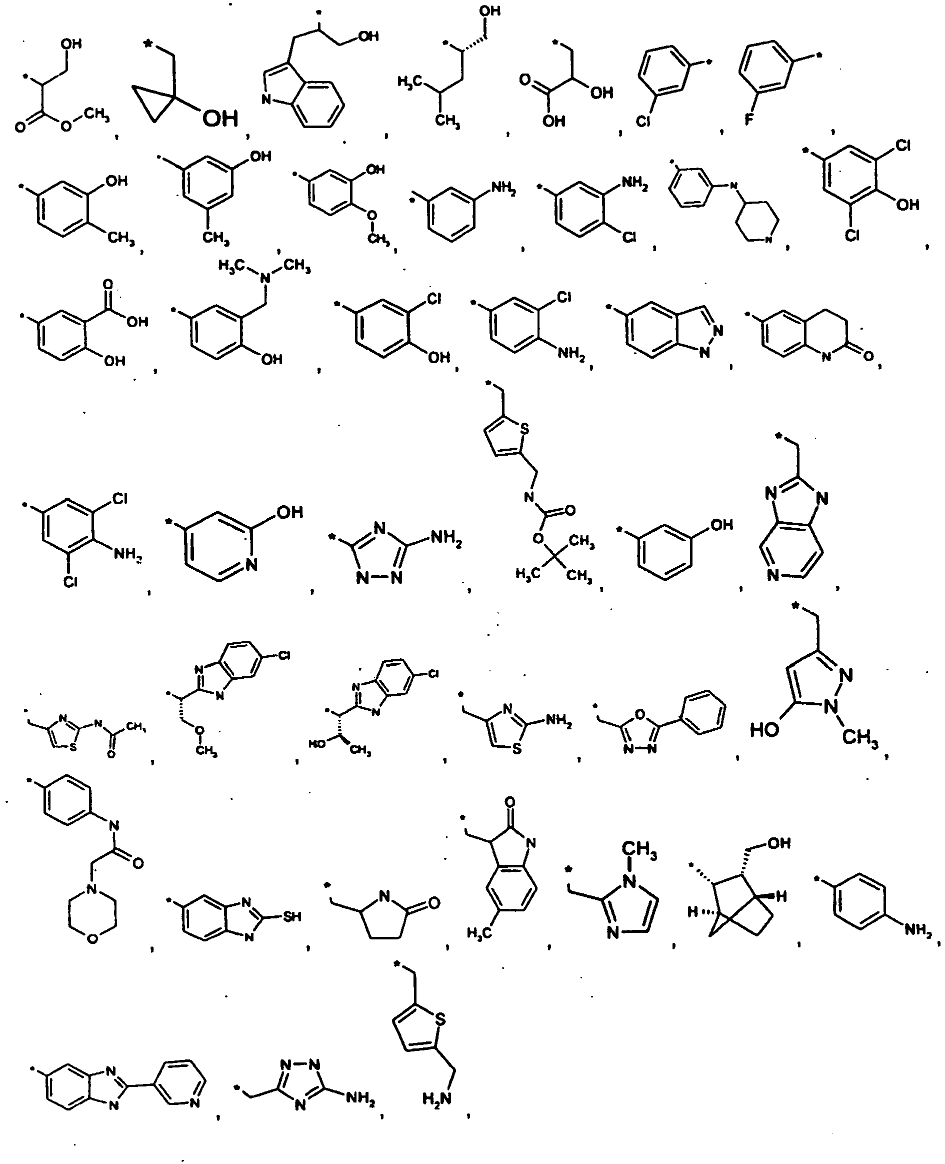 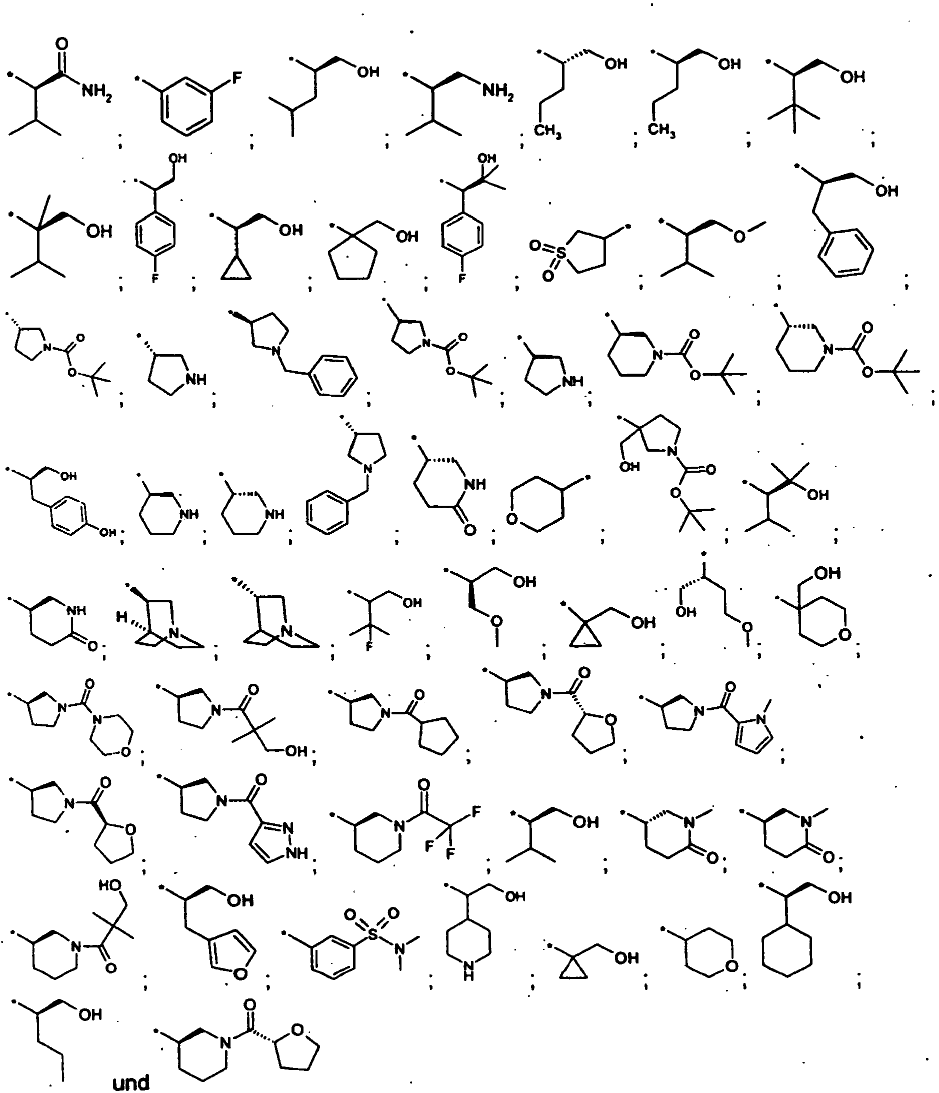
und worin
- R³: ausgewählt ist aus der Gruppe bestehend aus Chlorid, Cyanid, 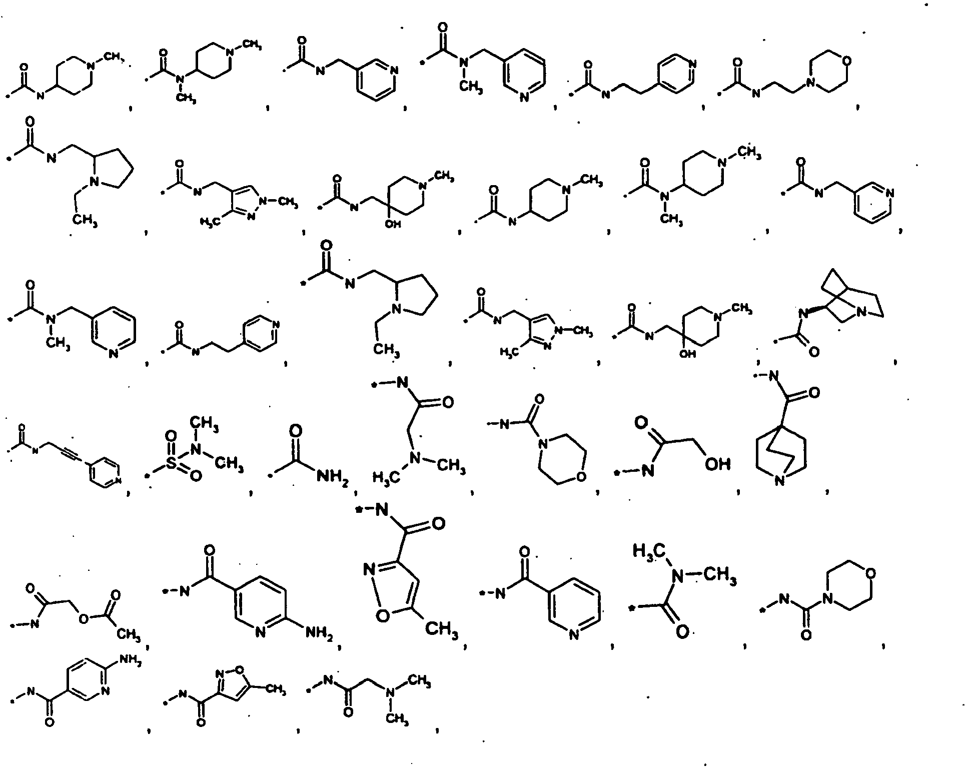 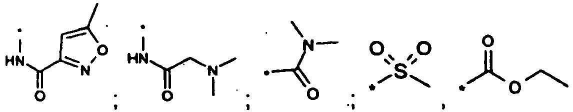
und Bromid, sowie pharmakologisch verträgliche Salze, Diastereomere, Enantiomere, Racemate, Hydrate oder Solvate davon.

Ein weiterer bevorzugter Gegenstand der Erfindung sind die obigen Verbindungen nach Formel **1**, worin
- **R¹**: H ist und worin
- **NR¹R²**: ausgewählt ist aus der Gruppe bestehend aus 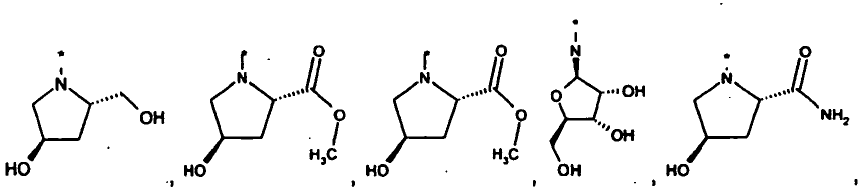 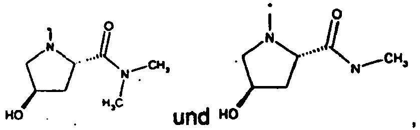 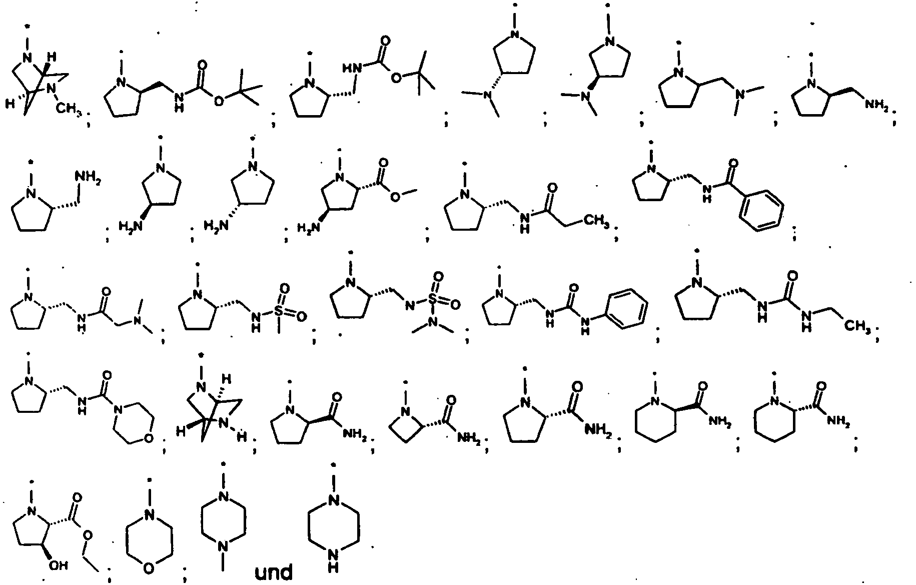
und worin
- **R³**: Fluorid, Chlorid, Bromid, Nitril oder Hydroxy ist, sowie pharmakologisch verträgliche Salze, Diastereomere, Enantiomere, Racemate, Hydrate oder Solvate davon.
Ein weiterer Gegenstand der Erfindung sind Zwischenprodukte des Synthese-Schemas 2 der Formel 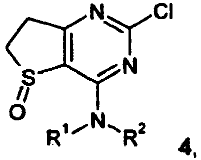 worin R¹ und R² wie vorstehend beschrieben definiert sind,
sowie pharmakologisch verträgliche Salze, Diastereomere, Enantiomere, Racemate, Hydrate oder Solvate davon.

Ein weiterer Gegenstand der Erfindung sind Zwischenprodukte des Synthese-Schemas 3 der Formel 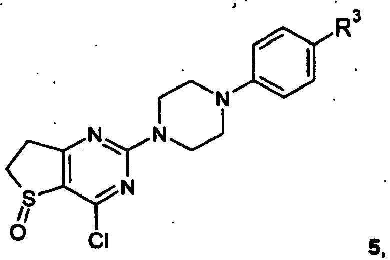 worin R³ wie vorstehend beschrieben definiert ist,
sowie pharmakologisch verträgliche Salze, Diastereomere, Enantiomere, Racemate, Hydrate oder Solvate davon.

Ein weiterer Gegenstand der Erfindung sind Zwischenprodukte des Synthese-Schemas 4 der Formel 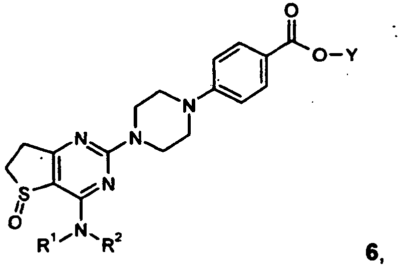 worin R¹ und R² wie vorstehend beschrieben definiert sind und worin
- Y: H, Methyl oder Ethyl bedeutet,
sowie pharmakologisch verträgliche Salze, Diastereomere, Enantiomere, Racemate, Hydrate oder Solvate davon.

Ein weiterer Gegenstand der Erfindung sind Zwischenprodukte des Synthese-Schemas 5 der Formel 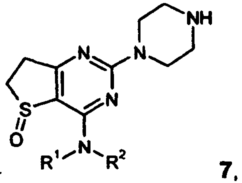 worin R¹ und R² wie vorstehend beschrieben definiert sind,
sowie pharmakologisch verträgliche Salze, Diastereomere, Enantiomere, Racemate. Hydrate oder Solvate davon.

Ein weiterer Gegenstand der Erfindung sind Zwischenprodukte des Synthese-Schemas 6 der Formel 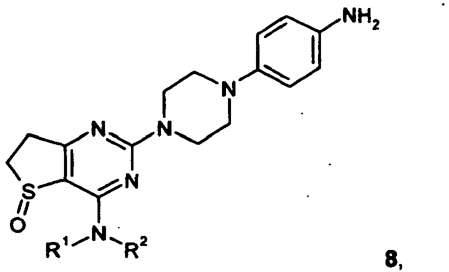 worin R¹ und R² wie vorstehend beschrieben definiert sind,
sowie pharmakologisch verträgliche Salze, Diastereomere, Enantiomere, Racemate, Hydrate oder Solvate davon.

Ein weiterer Gegenstand der Erfindung sind die obigen Verbindungen der Formel **1** als Arzneimittel.

Ein weiterer Gegenstand der Erfindung ist die Verwendung der obigen Verbindungen nach Formel **1** zur Herstellung eines Medikaments zur Behandlung von Krankheiten, die sich durch Inhibition des PDE4-Enzyms behandeln lassen.

Ein weiterer Gegenstand der Erfindung ist die Verwendung der obigen Verbindungen nach Formel **1** zur Herstellung eines Medikaments zur Behandlung von Atemwegs- oder gastrointestinalen Beschwerden oder Erkrankungen, wie auch entzündliche Erkrankungen der Gelenke, der Haut oder der Augen, Krebserkrankungen, sowie von Erkrankungen des periphären oder zentralen Nervensystems.

Ein weiterer Gegenstand der Erfindung ist die Verwendung der obigen Verbindungen nach Formel **1** zur Herstellung eines Medikaments zur Vorbeugung und Behandlung von Atemwegs- oder Lungenerkrankungen, die mit einer erhöhten Schleimproduktion, Entzündungen und / oder obstruktiven Erkrankungen der Atemwege einhergehen.

Ein weiterer Gegenstand der Erfindung ist die Verwendung der obigen Verbindungen nach Formel **1** zur Herstellung eines Medikament zur Behandlung von entzündlichen und obstruktiven Erkrankungen wie COPD, chronischer Sinusitis, Asthma, Morbus Crohn, Colitis ulcerosa.

Ein weiterer Gegenstand der Erfindung ist die Verwendung der obigen Verbindungen nach Formel **1** zur Herstellung eines Medikaments zur Behandlung von entzündlichen Erkrankungen des Gastrointestinaltraktes.

Ein weiterer Gegenstand der Erfindung ist die Verwendung der obigen Verbindungen nach Formel **1** zur Herstellung eines Medikaments zur Vorbeugung und Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Him-Trauma.

Ein weiterer Gegenstand der Erfindung sind pharmazeutische Formulierungen, die eine oder mehrere der obigen Verbindungen nach Formel **1** enthalten.

### VERWENDETE BEGRIFFE UND DEFINITIONEN

Soweit nicht anders angegeben, sind alle Substituenten voneinander unabhängig. Sollten an einer Gruppe beispielsweise mehrere C₁₋₆-Alkylgruppen als Substituenten möglich sein, so könnte, beispielsweise im Fall von drei Substituenten C₁₋₆-Alkyl unabhängig voneinander beispielsweise einmal Methyl, einmal n-Propyl und einmal tert-Butyl bedeuten.

Im Rahmen dieser Anmeldung können bei der Definition von möglichen Substituenten, diese auch in Form einer Strukturformel dargestellt werden. Dabei wird ein Stern (*) in der Strukturformel des Substituenten als der Verknüpfungspunkt zum Rest des Moleküls verstanden. Weiterhin wird das auf den Verknüpfungspunkt folgende Atom des Substituenten als das Atom mit der Positionsnummer 1 verstanden. So werden zum Beispiel die Reste N-Piperidinyl (I), 4-Piperidinyl (II), 2-Tolyl (III), 3-Tolyl (IV) und 4-Tolyl (V) wie folgt dargestellt:

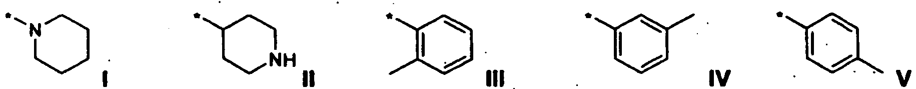

Befindet sich in der Strukturformel des Substituenten kein Stern (*), so kann an dem Substituenten jedes Wasserstoffatom entfernt werden und die dadurch frei werdende Valenz als Bindungsstelle zum Rest eines Molekül dienen. So kann zum Beispiel VI die Bedeutung von 2-Tolyl, 3-Tolyl, 4-Tolyl und Benzyl haben.

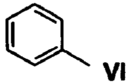

Unter dem Begriff "C₁₋₁₀-Alkyl" (auch soweit sie Bestandteil anderer Reste sind) werden verzweigte und unverzweigte Alkylgruppen mit 1 bis 10 Kohlenstoffatomen, unter dem Begriff "C₁₋₆-Alkyl" dementsprechend verzweigte und unverzweigte Alkylgruppen mit 1 bis 6 Kohlenstoffatomen verstanden. "C₁₋₄-Alkyl" steht entsprechend für verzweigte und unverzweigte Alkylgruppen mit 1 bis 4 Kohlenstoffatomen. Bevorzugt sind Alkylgruppen mit 1 bis 4 Kohlenstoffatomen. Beispielsweise werden hierfür genannt: Methyl, Ethyl, *n-*Propyl, *iso*-Propyl, *n*-Butyl, *iso*-Butyl, *sec*-Butyl, *tert*-Butyl, *n*-Pentyl, *iso*-Pentyl, *neo*-Pentyl oder Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₁₋₆-Alkylen" (auch soweit sie Bestandteil anderer Reste sind) werden verzweigte und unverzweigte Alkylengruppen mit 1 bis 6 Kohlenstoffatomen verstanden und unter dem Begriff "C₁₋₄-Alkylen" verzweigte und unverzweigte Alkylengruppen mit 1 bis 4 Kohlenstoffatomen verstanden. Bevorzugt sind Alkylengruppen mit 1 bis 4 Kohlenstoffatomen. Beispielsweise werden hierfür genannt: Methylen, Ethylen, Propylen, 1-Methylethylen, Butylen, 1-Methylpropylen, 1, 1-Dimethylethylen, 1, 2-Dimethylethylen, Pentylen, 1, 1-Dimethylpropylen, 2, 2, -Dimethylpropylen, 1, 2-Dimethylpropylen, 1, 3-Dimethylpropylen oder Hexylen. Sofern nicht anders beschrieben, umfassen die Definitionen Propylen, Butylen, Pentylen und Hexylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1, 1-Dimethylethylen, 1, 2-Dimethylethylen.

Sollte die Kohlenstoffkette mit einem Rest substituiert sein, der gemeinsam mit einem oder zwei Kohlenstoffatomen der Alkylenkette einen carbocyclischen Ring mit 3, 5 oder 6 Kohlenstoffatomen bildet, so sind damit unter anderem folgende Beispiele der Ringe umfasst:

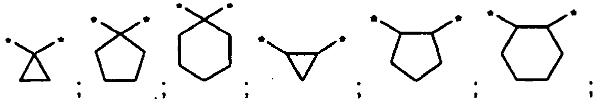

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatome. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc.

Unter dem Begriff "C₂₋₆-Alkenylen" (auch soweit sie Bestandteil anderer Reste sind) werden verzweigte und unverzweigte Alkenylengruppen mit 2 bis 6 Kohlenstoffatomen verstanden und unter dem Begriff "C₂₋₄-Alkenylen" verzweigte und unverzweigte Alkylengruppen mit 2 bis 4 Kohlenstoffatomen verstanden. Bevorzugt sind Alkenylengruppen mit 2 bis 4 Kohlenstoffatome. Beispielsweise werden hierfür genannt: Ethenylen, Propenylen, 1-Methylethenylen, Butenylen, 1-Methylpropenylen, 1, 1-Dimethylethenylen, 1, 2-Dimethylethenylen, Pentenylen, 1, 1-Dimethylpropenylen, 2, 2, - Dimethylpropenylen, 1, 2-Dimethylpropenylen, 1, 3-Dimethylpropenylen oder Hexenylen. Sofern nicht anders beschrieben, umfassen die Definitionen Propenylen, Butenylen, Pentenylen und Hexenylen alle denkbaren isomeren Formen der jeweiligen Reste gleicher Kohlenstoffanzahl. So umfasst beispielsweise Propenyl auch 1-Methylethenylen und Butenylen umfasst 1-Methylpropenylen, 1, 1-Dimethylethenylen, 1, 2-Dimethylethenylen.

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Bevorzugt sind Alkinylgruppen mit 2 bis 4 Kohlenstoffatome. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc.

Unter dem Begriff "C₂₋₆-Alkinylen" (auch soweit sie Bestandteil anderer Reste sind) werden verzweigte und unverzweigte Alkinylengruppen mit 2 bis 6 Kohlenstoffatomen verstanden und unter dem Begriff "C₂₋₄-Alkinylen" verzweigte und unverzweigte Alkylengruppen mit 2 bis 4 Kohlenstoffatomen verstanden. Bevorzugt sind Alkinylengruppen mit 2 bis 4 Kohlenstoffatome. Beispielsweise werden hierfür genannt: Ethinylen, Propinylen, 1-Methylethinylen, Butinylen, 1-Methylpropinylen, 1, 1-Dimethylethinylen, 1, 2-Dimethylethinylen, Pentinylen, 1, 1-Dimethylpropinylen, 2, 2, - Dimethylpropinylen, 1, 2-Dimethylpropinylen, 1, 3-Dimethylpropinylen oder Hexinylen. Sofern nicht anders beschrieben, umfassen die Definitionen Propinylen, Butinylen, Pentinylen und Hexinylen alle denkbaren isomeren Formen der jeweiligen Reste gleicher Kohlenstoffanzahl. So umfasst beispielsweise Propinyl auch 1-Methylethinylen und Butinylen umfasst 1-Methylpropinylen, 1, 1-Dimethylethinylen, 1, 2-Dimethylethinylen.

Unter dem Begriff "Aryl" (auch soweit sie Bestandteil anderer Reste sind) werden aromatische Ringsysteme mit 6 bis 10 Kohlenstoffatomen verstanden. Beispielsweise werden hierfür genannt: Phenyl oder Naphthyl, bevorzugter Arylrest ist Phenyl. Soweit nicht anders beschrieben, können die Aromaten substituiert sein mit einem oder mehreren Resten ausgewählt aus der Gruppe bestehend aus Methyl, Ethyl, *iso*-Propyl, *tert-Butyl,* Hydroxy, Fluor, Chlor, Brom und Jod.

Unter dem Begriff "Aryl-C₁₋₆-alkylen" (auch soweit sie Bestandteil anderer Reste sind) werden verzweigte und unverzweigte Alkylengruppen mit 1 bis 6 Kohlenstoffatomen verstanden, die mit einem aromatischen Ringsystem mit 6 oder 10 Kohlenstoffatomen substituiert sind. Beispielsweise werden hierfür genannt: Benzyl, 1- oder 2-Phenylethyl oder 1- oder 2-Naphthylethyl. Soweit nicht anders beschreiben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Heteroaryl-C₁₋₆-alkylen" (auch soweit sie Bestandteil anderer Reste sind) werden - obwohl auch bereits unter "Aryl-C₁₋₆-alkylen umfasst - verzweigte und unverzweigte Alkylengruppen mit 1 bis 6 Kohlenstoffatomen verstanden, die mit einem Heteroaryl substituiert sind.

Ein solches Heteroaryl umfasst fünf- oder sechsgliedrige heterocyclische Aromaten oder 5-10 gliedrige, bicyclische Heteroarylringe die ein, zwei oder drei Heteroatome, ausgewählt aus der Gruppe Sauerstoff; Schwefel und Stickstoff enthalten können und so viele konjugierte Doppelbindungen enthalten, dass ein aromatisches Systeme gebildet wird. Als Beispiele für fünf- oder sechsgliedrige heterocyclische Aromaten, werden genannt:

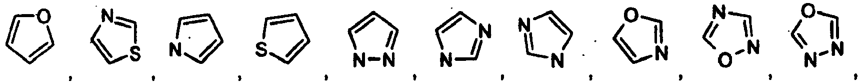

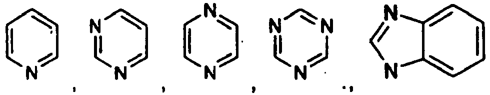

Soweit nicht anders beschreiben, können diese Heteroaryle substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Für die Heteroaryl-C₁₋₆-alkylene werden die folgenden Beispiele genannt:

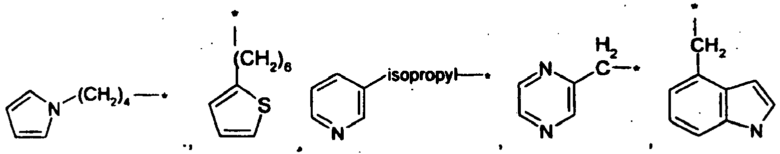

Unter dem Begriff "C₁₋₆-Haloalkyl" (auch soweit sie Bestandteil anderer Reste sind) werden verzweigte und unverzweigte Alkylgruppen mit 1 bis 6 Kohlenstoffatomen verstanden, die mit einem oder mehreren Halogenatomen substituiert sind. Unter dem Begriff "C₁₋₄-Alkyl" verzweigte und unverzweigte Alkylgruppen mit 1 bis 4 Kohlenstoffatomen verstanden, die mit einem oder mehreren Halogenatomen substituiert sind. Bevorzugt sind Alkylgruppen mit 1 bis 4 Kohlenstoffatome. Beispielsweise werden hierfür genannt: CF₃, CHF₂, CH₂F, CH₂CF₃.

Unter dem Begriff "C₃₋₇-Cycloalkyl" (auch soweit sie Bestandteil anderer Reste sind) werden cyclische Alkylgruppen mit 3 bis 7 Kohlenstoffatomen verstanden. Beispielsweise werden hierfür genannt: Cyclopropyl, Cyclobutyl, Cyclopentyl, Cyclohexyl oder Cycloheptyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *ter*t-Butyl, Hydroxy, Fluor, Chlor, Brom und Jod.

Unter dem Begriffe "C₃₋₁₀-Cycloalkyl" werden zudem monocyclische Alkylgruppen mit 3 bis 7 Kohlenstoffatomen und auch bicyclische Alkylgruppen mit 7 bis 10 Kohlenstoffatomen verstanden oder auch monocyclische Alkylgruppen, die durch mindestens eine C₁₋₃-Kohlenstoffbrücke überbrückt sind.

Unter dem Begriff "heterocyclische Ringe" oder auch "Heterocyclus" werden fünf-, sechs- oder siebengliedrige, gesättigte oder ungesättigte heterocyclische Ringe verstanden die ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Obwohl unter dem Begriff "heterocyclische Ringe" oder "Heterocyclus" umfasst, definiert der Begriff "heterocyclische, nichtaromatische Ringe" fünf- , sechs- oder siebengliedrige ungesättigte Ringe. Als Beispiele werden genannt:

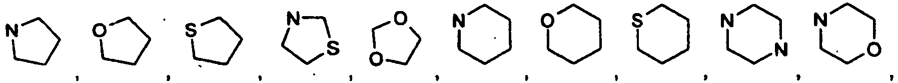

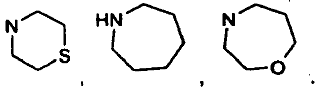

Obwohl unter dem Begriff "heterocyclische Ringe" oder "Heterocyclus" umfasst, definiert der Begriff "heterocyclische, aromatische Ringe" oder "Heteroaryl" fünf- oder sechsgliedrige heterocyclische Aromaten oder 5-10 gliedrige, bicyclische Heteroarylringe die ein, zwei, drei oder vier Heteroatome, ausgewählt aus der Gruppe Sauerstoff, Schwefel und Stickstoff, enthalten können und so viele konjugierte Doppelbindungen enthalten, dass ein aromatisches gebildet Systeme wird. Als Beispiele für fünf oder sechsgliedrige heterocyclische Aromaten, werden genannt:

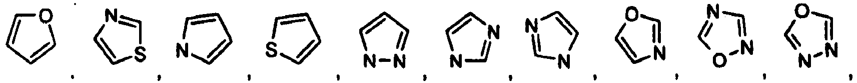

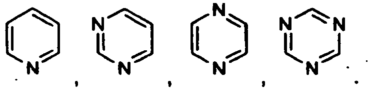

Soweit nicht anders erwähnt, kann ein heterocyclischer Ring (oder "Heterocyclus) mit einer Ketogruppe versehen sein. Als Beispiel hierfür werden genannt.

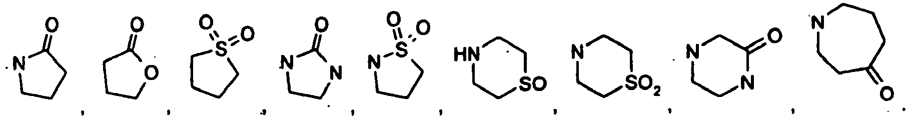

Obwohl unter "Cycloalkyl" bereits umfasst, werden unter dem Begriff "bicyclische Cycloalkyle" in der Regel acht-, neun- oder zehngliedrige, bicyclische Kohlenstoff-Ringe verstanden. Beispielhaft werden genannt:

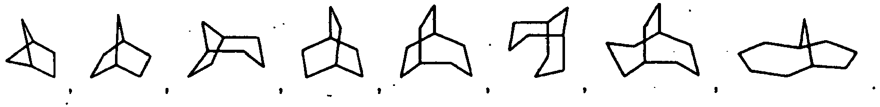

Obwohl unter "Heterocyclus" bereits umfasst, werden unter dem Begriff "bicyclische Heterocyclen" in der Regel acht-, neun- oder zehngliedrige, bicyclische Ringe verstanden, die ein oder mehrere Heteroatome, vorzugsweise 1-4, stärker bevorzugt, 1-3, noch stärker bevorzugt 1-2, insbesondere ein Heteroatom, ausgewählt aus der Gruppe Sauerstoff, Schwefel und Stickstoff enthalten können. Der Ring kann dabei über ein Kohlenstoffatom des Rings oder falls vorhanden über ein Stickstoffatom des Rings mit dem Molekül verknüpft sein. Beispielhaft werden genannt,

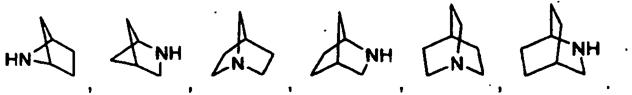

Obwohl unter "Aryl" bereits umfasst, wird unter einem "bicyclischen Aryl" ein 5-10 gliedriger, bicyclischer Arylring verstanden, der so viele konjugierte Doppelbindungen enthält, dass ein aromatisches System gebildet wird. Ein Beispiel für ein bicyclisches Aryl ist Naphthyl.

Obwohl unter "Heteroaryl" bereits umfasst, wird unter einem "bicyclischen Heteroaryl" ein 5-10 gliedriger, bicyclischer Heteroarylring verstanden, der ein, zwei, drei oder vier Heteroatome, ausgewählt aus der Gruppe Sauerstoff, Schwefel und Stickstoff, enthalten kann und so viele konjugierte Doppelbindungen enthält, dass ein aromatisches System gebildet wird.

Obwohl unter dem Begriff "bicyclische Cycloalkyle" oder "bicyclisches Aryl" umfasst, definiert der Begriff "kondensiertes Cycloalkyl" oder "kondensiertes Aryl" bicyclische Ringe, in denen die die Ringe trennende Brücke eine direkte Einfachbindung bedeutet. Als Beispiel für einen kondensiertes, bicyclisches Cycloalkyl werden genannt:

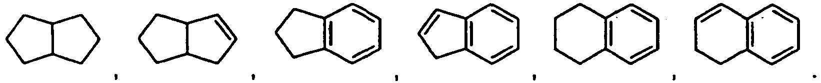

Obwohl unter dem Begriff "bicyclische Heterocyclen" oder "bicyclische Heteroaryle" umfasst, definiert der Begriff "kondensierte, bicyclische Heterocyclen " oder "kondensierte, bicyclische Heteroaryle"bicyclische 5-10 gliedrige Heteroringe die ein, zwei oder drei Heteroatome, ausgewählt aus der Gruppe Sauerstoff, Schwefel und Stickstoff enthalten und in denen die die Ringe trennende Brücke eine direkte Einfachbindung bedeutet. Die "kondensierten, bicyclischen Heteroaryle" enthalten zudem so viele konjugierte Doppelbindungen, dass ein aromatisches System gebildet wird. Beispielhaft werden genannt Pyrrolizin, Indol, Indolizin, Isoindol, Indazol, Purin, Chinolin, Isochinolin. Benzimidazol, Benzofuran, Benzopyran, Benzothiazol, Benzothiazol, Benzoisothiazol, Pyridopyrimidin, Pteridin, Pyrimidopyrimidin,

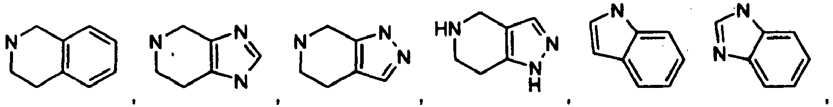

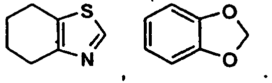

Unter dem Begriff "heterocyclische Spiroringe" (Spiro) werden 5-10 gliedrige, spirocyclische Ringe verstanden die gegebenenfalls ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Soweit nicht anders erwähnt, kann ein spirocyclischer Ring mit einer Oxo-, Methyl- oder Ethylgruppe versehen sein. Als Beispiele hierfür werden genannt:

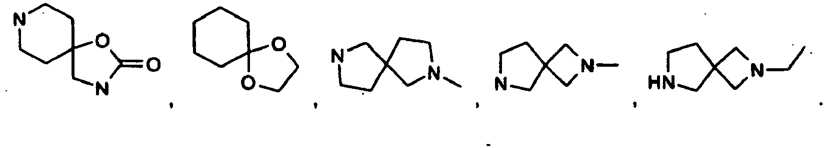

"Halogen" steht im Rahmen der vorliegenden Erfindung für Fluor, Chlor, Brom oder Jod. Sofern nicht gegenteilig angegeben, gelten Fluor, Chlor und Brom als bevorzugte Halogene.

Verbindungen der allgemeinen Formel 1 können Säuregruppen besitzen, hauptsächlich Carboxylgruppen, und/oder basische Gruppen wie z.B. Aminofunktionen. Verbindungen der allgemeinen Formel 1 können deshalb als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Wie vorstehend genannt, können die Verbindungen der Formel 1 in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel 1 mit anorganischen oder organischen Säuren vorliegen. Andererseits kann die Verbindung der Formel 1 im Falle von R gleich Wasserstoff durch Umsetzung mit anorganischen Basen auch in physiologisch und pharmakologisch verträgliche Salze mit Alkali- oder Erdalkalimetallkationen als Gegenion überführt werden. Zur Darstellung der Säureadditionssalze kommen beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden. Zur Darstellung der Alkali- und Erdalkalimetallsalze der Verbindung der Formel **1** in der R Wasserstoff bedeutet, kommen vorzugsweise die Alkali- und Erdalkalihydroxide und -hydride in Betracht, wobei die Hydroxide und Hydride der Alkalimetalle, besonders des Natriums und Kaliums bevorzugt, Natrium- und Kaliumhydroxid besonders bevorzugt sind.

Gegebenenfalls können die Verbindungen der allgemeinen Formel (1) in ihre Salze, insbesondere für die pharmazeutische Anwendung, in ihre pharmakologisch unbedenklichen Säureadditionssalze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure oder Zitronensäure in Betracht. Ferner können Mischungen der vorgenannten Säuren eingesetzt werden.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure

Die erfindungsgemäßen Verbindungen können gegebenenfalls als Racemate vorliegen, sie können aber auch als reine Enantiomere, d.h. in (R)- oder (S)-Form gewonnen werden. Bevorzugt sind Verbindungen die als Racemate bzw. als (S)-Form vorliegen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

### SYNTHESEVORSCHRIFTEN

Im folgenden werden die Synthese-Schemata 1, 2, 3, 4, 5 und 6 zur Herstellung der Beispiele aus Tabelle 1 erläutert.

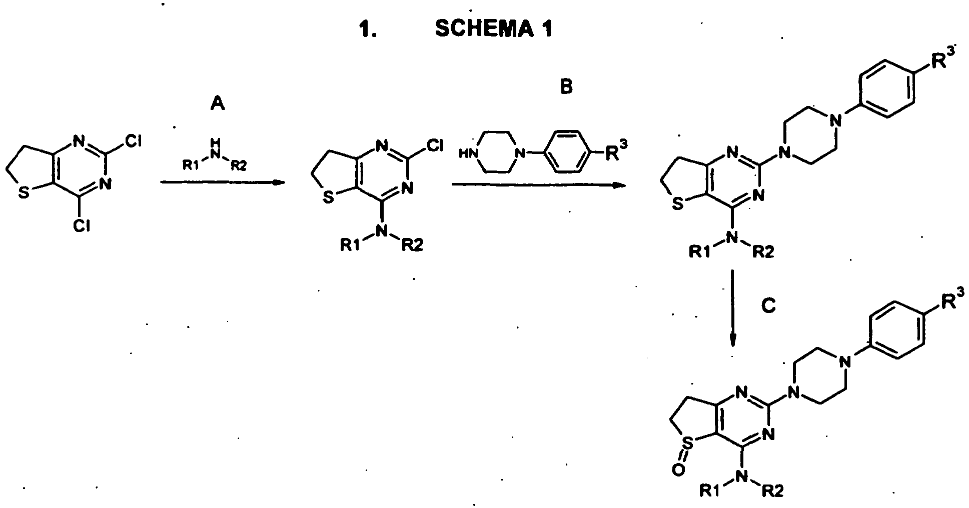

### 1.1 SYNTHESE VON {2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-(3-MORPHOLIN-4-YLMETHYL-PHENYL)-AMIN, BEISPIEL 21 (SIEHE SCHEMA 1)

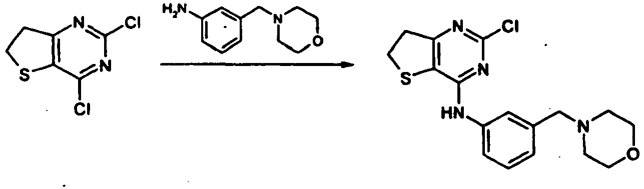

### 1.1.1 (2-Chlor-6,7-dihydro-thieno[3,2-d]pyrimidin-4-yl)-(3-morpholin-4-ylmethyl-phenyl)-amin (Schema 1, Schritt A):

1,25 g 2,4-Dichlor-6,7-dihydro-thieno[3,2-*d*]pyrimidin werden in 5 ml Dimethylformamid vorgelegt, erst 1,05 ml Diisopropylethylamin, dann 0,960 g 3-Morpholin-4-ylmethyl-phenylamin (J. Med. Chem. 1990, 33, 327) zugegeben. Das Reaktionsgemisch wird 20 Stunden bei 60° C gerührt, nach Abkühlen eingedampft. Der Rückstand wird mit Dichlormethan und Wasser extrahiert, die organische Phase getrocknet und zur Trockene eingedampft. Der Rückstand wird mit Petrolether / Ethylacetat kristallisiert. 1,28 g des Produkts werden als Pulver erhalten.

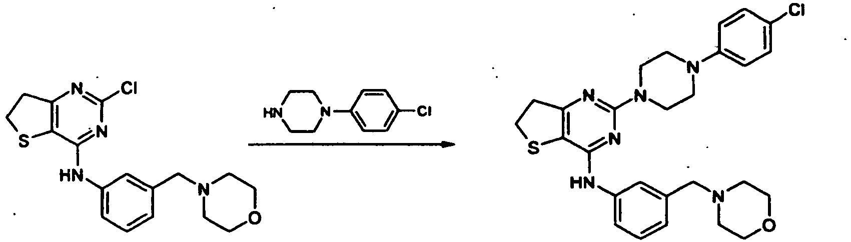

### 1.1.2 {2-[4-(4-Chlor-phenyl)-piperazin-1-yl]-6,7-dihydro-thieno[3,2-d]pyrimidin-4-yl}-(3-morpholin-4-ylmethyl-phenyl)-amin (Schema 1, Schritt B):

1,09 g (2-Chlor-6,7-dihydro-,thieno[3,2-*d*]pyrimidin-4-yl)-(3-morpholin-4-ylmethyl-phenyl)-amin , 1,77 g 1-(4-Chlor-phenyl)-piperazin und 1,03 ml Diisopropylethylamin werden in 12 ml Dioxan vorgelegt, dann in der Mikrowelle 1 Sturide bei 160° C erhitzt. Anschließend wird Wasser zugegeben und mit Dichlormethan extrahiert. Die organische Phase wird mittels Phasentransferkartusche abgetrennt und zur Trockene eingedampft. Der Rückstand wird mit Methanol kristallisiert. 1,17 g des Produkts werden als Pulver (Smp 177°-178° C) erhalten.

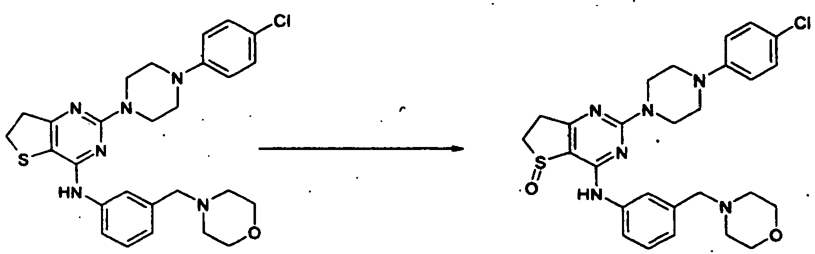

### 1.1.3 {2-[4-(4-Chlor-phenyl)-piperazin-1-yl]-5-oxo-6,7-dihydro-5H-5λ⁴-thieno[3,2-d]pyrimidin-4-yl}-(3-morpholin-4-ylmethyl-phenyl)-amin (Schema 1, Schritt C):

313,80 mg {2-[4-(4-Chloro-phenyl)-piperazin-1-yl]-6,7-dihydro-thieno[3,2-*d*]pyrimidin-4-yl}-(3-morpholin-4-ylmethyl-phenyl)-amin werden in 2,70 ml Eisessig vorgelegt und auf 10°C gekühlt. 57 µl Wasserstoffperoxid (35%) werden zugetropft, dann 0,25 Stunden nachgerührt. Anschließend wird das Reaktionsgemisch in 30 ml Eiswasser gerührt und

mit kalter Ammoniaklösung basisch gestellt. Der ausgefallene Niederschlag wird abgesaugt, mit Wasser gewaschen und getrocknet. Das Rohprodukt wird mit Petrolether und Diethylether ausgerührt, abgesaugt und getrocknet. Das Produkt wird chromatographisch (10 g Chromabond SiOH-Kartusche) gereinigt. 140,0 mg des Produkts werden als Pulver (Smp 244°-248° C) erhalten.

### 1.2 SYNTHESE VON (R)-N²-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-3-METHYL-BUTAN-1,2-DIAMIN TRIFLUORACETAT, BEISPIEL 141 (SIEHE SCHEMA 1)

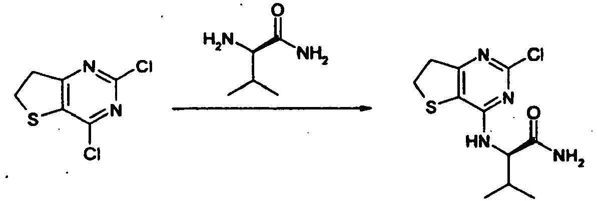

### 1.2.1 (R)-2-(2-Chlor-6,7-dihydro-thieno[3,2-d]pyrimidin-4-ylamino)-3-methyl-butyramid (Schema 1, Schritt A):

800 mg 2,4-Dichlor-6,7-dihydro-thieno[3,2-*d*]pyrimidin und 800 mg D-Valinamid Hydrochlorid werden in 8 ml Dioxan vorgelegt, dann 2,7 ml Diisopropylethylamin zugegeben. Das Reaktionsgemisch wird in der Mikrowelle 2 Stunden auf 120°C erhitzt. Der Rückstand wird mit Wasser versetzt. Der ausgefallene Niederschlag wird abgesaugt und getrocknet. 820 mg des Produkts werden als Pulver erhalten. Analytische HPLC (Methode B): RT = 2,64 min.

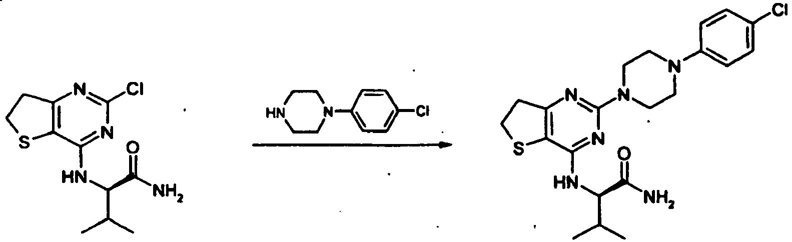

### 1.2.2 (R)-2-{2-[4-(4-Chlor-phenyl)-piperazin-1-yl]-6,7-dihydro-thieno[3,2-d]pyrimidin-4-ylamino}-3-methyl-butyramid (Schema 1, Schritt B):

400 mg (R)-2-(2-Chlor-6,7-dihydro-thieno[3,2-*d*]pyrimidin-4-ylamino)-3-methyl-butyramid, 750 mg 1-(4-Chlor-phenyl)-piperazin und 1,2 ml Diisopropylethylamin werden in 9 ml Dioxan vorgelegt, dann in der Mikrowelle 1 Stunde und 45 Minuten bei 160° C erhitzt. Anschließend wird mit Wasser versetzt. Der ausgefallene Niederschlag wird abgesaugt und getrocknet. 550 mg des Produktes werden als Pulver erhalten. Analytische HPLC (Methode B): RT = 3,03 min.

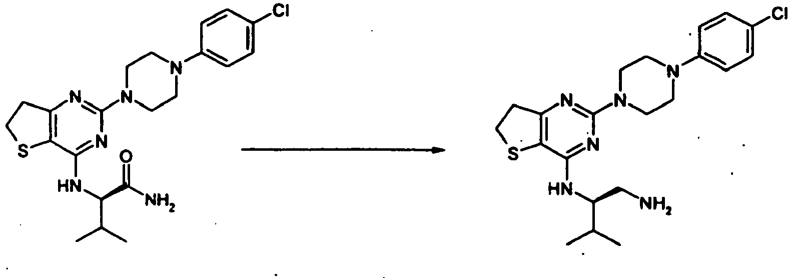

### 1.2.3 (R)-N²-{2-[4-(4-Chlor-phenyl)-piperazin-1-yl]-6,7-dihydro-thieno[3,2-d]pyrimidin-4-yl}-3-methyl-butan-1,2-diamin Trifluoracetat:

400 mg (R)-2-{2-[4-(4-Chlor-phenyl)-piperazin-1-yl]-6,7-dihydro-thieno[3,2-*d*]pyrimidin-4-ylamino}-3-methyl-butyramid werden in 15 ml abs. Tetrahydrofuran vorgelegt und unter Eisbadkühlung mit 3,5 ml einer Lithiumaluminiumhydrid in Tetrahydrofuran Lösung (1 M) versetzt. Das Reaktionsgemisch wird 4 Stunden unter Rückfluss gerührt. Der Rückstand wird mit ein paar Tropfen 1 N NaOH versetzt, getrocknet und über Celite filtriert. Die organische Phase wird zur Trockene eingedampft. Das Produkt wird über präparative HPLC (Methode A) gereinigt. 90 mg des Produktes werden als Trifluoracetat erhalten. Analytische HPLC (Methode B): RT = 2,90 min.

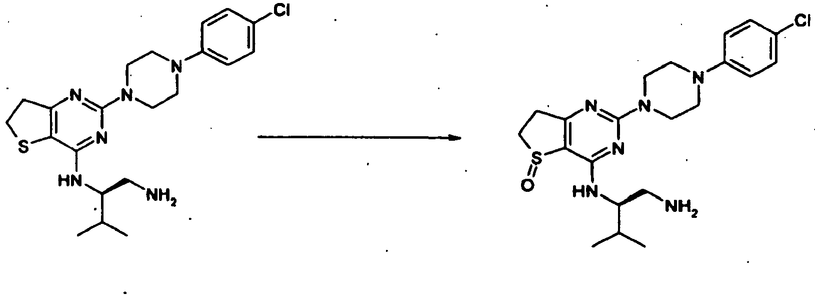

### 1.2.4 (R)-N²-{2-[4-(4-Chlor-phenyl)-piperazin-1-yl]-5-oxo-6,7-dihydro-5H-5λ⁴-thieno[3,2-d]pyrimidin-4-yl}-3-methyl-butan-1,2-diamin Trifluoracetat (Schema 1, Schritt C):

86 mg (R)-*N*²-{2-[4-(4-Chlor-phenyl)-piperazin-1-yl]-6,7-dihydro-thieno[3,2-*d*]pyrimidin-4-yl}-3-methyl-butan-1,2-diamin Trifluoracetat werden in 1,2 ml Eisessig vorgelegt und auf 10°C gekühlt. 18 µl Wasserstoffperoxid (35%) werden zugetropft, dann 2.5 Stunden bei Raumtemperatur gerührt. Anschließend wird das Reaktionsgemisch in 30 ml Eiswasser gerührt und mit kalter Ammoniaklösung basisch gestellt. Das Rohprodukt wird mit Dichlormethan extrahiert. Die Diastereomeren werden über semipräparative HPLC (Methode A) getrennt. 20 mg Diastereomer 1 (Beispiel 141) und 52 mg Diastereomer 2 werden als Trifluoracetate erhalten. Analytische HPLC (MethodeB): Diastereomer 1: RT = 2,75 min; Diastereomer 2: RT: 2,87 min.

### 1.3 SYNTHESE VON (1-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YLAMINO}-CYCLOPENTYL)-METHANOL, BEISPIELE 158 UND 159 (SIEHE SCHEMA 1):

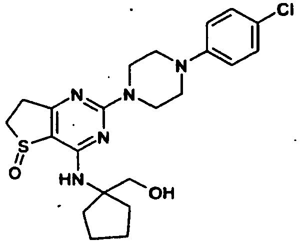

Ausgehend vom 2,4-Dichlor-6,7-dihydro-thieno[3,2-d]pyrimidin und Cycloleucinol kann (1-{2-[4-(4-Chlor-phenyl)-piperazin-1-yl]-5-oxo-6,7-dihydro-5*H*-5λ⁴-thieno[3,2-*d*]pyrimidin-4-ylamino}-cyclopentyl)-methanol, wie bei Beispiel 21 (siehe Schema 1) beschrieben, hergestellt werden. Analytische HPLC-MS (Methode D): RT = 1,29 min.

Beide Enantiomeren können über chirale HPLC (Säule: Diacel AS-H, 250 x 4.6 mm, 5 µm, Fließmittel: (Hexan+Cyclohexylamin (0,2%))/Ethanol (70/30), Flußrate: 1 ml/min) getrennt werden: Enantiomer 1: RT = 4,00 min (Beispiel 159); Enantiomer 2: RT = 5,35 min (Beispiel 158).

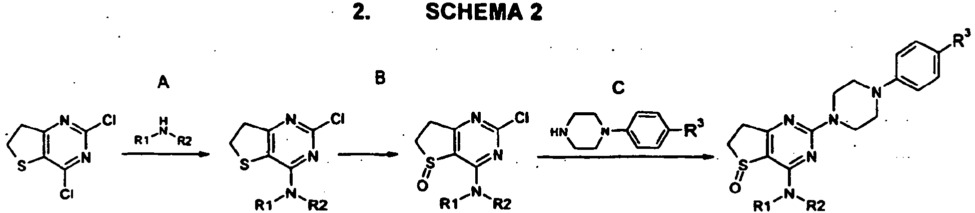

### 2.1 SYNTHESE VON 4-{4-[4-(3-FLUOR-PHENYLAMINO)-5-OXO-6,7-DIHYDRO-5H-5λ⁴-THIENO[3,2-d]PYRIMIDIN-2-YL]-PIPERAZIN-1-YL}-N,N-DIMETHYLBENZOLSULFONAMID, BEISPIEL 28 (SIEHE SCHEMA 2)

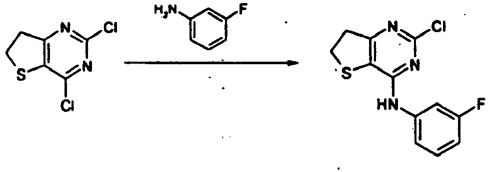

### 2.1.1 (2-Chlor-6,7-dihydro-thieno[3,2-d]pyrimidin-4-yl)-(3-fluor-phenyl)-amin (Schema 2, Schritt A):

4,00 g 2,4-Dichlor-6,7-dihydro-thieno[3,2-*d*]pyrimidin werden in 15 ml Dimethylformamid vorgelegt, erst 4,50 ml Diisopropylethylamin, dann 2,50 ml 3-Fluor-anilin zugegeben. Das Reaktionsgemisch wird 7 Stunden bei 120° C gerührt, nach Abkühlen eingedampft. Der Rückstand wird mit Dichlormethan und Wasser extrahiert, die organische Phase mit gesättigter Natriumchloridlösung gewaschen, getrocknet und zur Trockene eingedampft. Der Rückstand wird chromatographisch (NP_MPLC, Biotage Kartusche (4*15 cm)) gereinigt. 2,60 g des Produkts werden als Pulver erhalten.

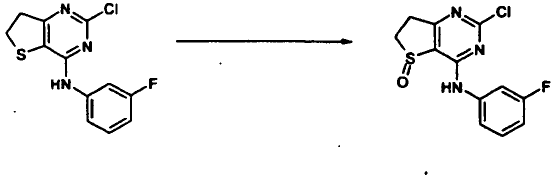

### 2.1.2 2-Chlor-5-oxo-6,7-dihydro-5H-5λ⁴-thieno[3,2-d]pyrimidin-4-yl)-(3-fluor-phenyl)-amin (Schema 2, Schritt B):

2,60 g (2-Chlor-6,7-dihydro-thieno[3,2-*d*]pyrimidin-4-yl)-(3-fluor-phenyl)-amin werden in 40 ml Eisessig vorgelegt. 1,80 ml Wasserstoffperoxid (35%) werden zugetropft, dann 2 Stunden nachgerührt. Anschließend wird das Reaktionsgemisch in Eiswasser gerührt, mit Ammoniaklösung basisch gestellt. Der ausgefallene Niederschlag wird abgesaugt, mit Wasser gewaschen und getrocknet. 2,40 g des Produkts werden als Pulver erhalten.

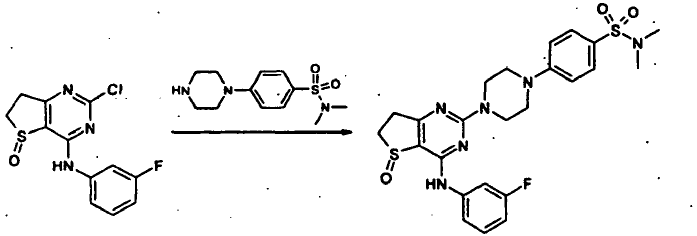

### 2.1.3 4-{4-[4-(3-Fluor-phenylamino)-5-oxo-6,7-dihydro-5H-5λ⁴-thieno[3,2-d]pyrimidin-2-yl]-piperazin-1-yl}-N,N-dimethylbenzolsulfonamid (Schema 2, Schritt C):

180 mg 2-Chlor-5-oxo-6,7-dihydro-5H-5λ⁴-thieno[3,2-*d*]pyrimidin-4-yl)-(3-fluor-phenyl)-amin, 360 mg N,N-Dimethyl-4-piperazin-1-yl-benzolsulfonamid (WO 03/105853) und 230 µl Diisopropylethylamin werden in 2 ml Dioxan vorgelegt, dann in der Mikrowelle 0,75 Stunden bei 160°C erhitzt. Anschließend wird Wasser zugegeben, der ausgefallene Niederschlag abgesaugt, gewaschen und getrocknet. Das Rohprodukt wird über präparative HPLC (Methode A) gereinigt. 109 mg des Produkts werden als Pulver erhalten. Analytische HPLC (Methode A): RT = 3.73 min.

### 2.2 SYNTHESE VON (R)-1-{2-[4-(4-CHLOR-PHENYL)-PEPERAZIN-1-YL]-5-OXO-6,7-DIHYDRO-5H-5λ⁴-THIENO[3,2-d]PYRIMIDIN-4-YLAMINO)-1-(4-FLUOR-PHENYL)-2-METHYL-PROPAN-2-OL, BEISPIELE 160 UND 161 (SIEHE SCHEMA 2)

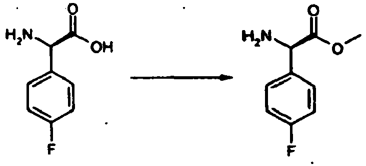

### 2.2.1 (R)-Amino-(4-fluor-phenyl)-essigsäuremethylester:

4 g (R)-4-fluorphenylglycin werden in 80 ml Methanol suspendiert. Unter Eisbadkühlung werden 3,28 ml Thionylclorid langsam zugetropft, so dass die Temperatur zwischen 15°C und 20°C gehalten wird. Das Reaktionsgemisch wird 12 Stunden bei Raumtemperatur gerührt und anschießend zur Trockene eingedampft. 5,1 g des Produkts werden als Hydrochlorid erhalten.

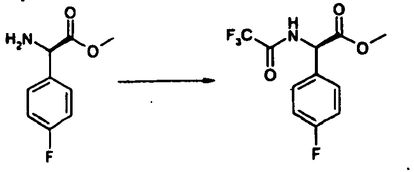

### 2.2.2 (R)-(4-Fluor-phenyl)-(2,2,2-trifluor-acetylamino)-essigsäuremethylester:

5,1 g (R)-Amino-(4-fluor-phenyl)-essigsäuremethylester werden in 36,5 abs. Tetrahydrofuran vorgelegt, dann 3,9 ml Triethylamin zugegeben. Das Reaktionsgemisch wird bei -70°C gekühlt. 3,9 ml Trifluoressiganhydrid werden dann langsam zugetropft, so dass die Temperatur nicht über -60°C übersteigt. Das Reaktionsgemisch wird 12 Stunden bei Raumtemperatur gerührt und anschließend mit Wasser versetzt. Kaliumhydrogencarbonat wird dann zugegeben bis keine Schaumbildung mehr zu beobachten wird. Das Produkt wird mit Essigester extrahiert und anschließend die organische Phase getrocknet und zur Trockene eingedampft. 6,2 g des Produkts werden als Öl erhalten.

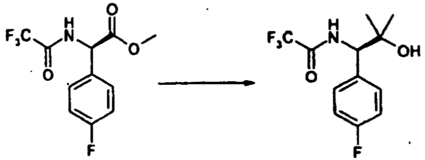

### 2.2.3 2,2,2-Trifluor-N-[(R)-1-(4-fluor-phenyl)-2-hydroxy-2-methyl-propyl]-acetamid:

6,2 g (R)-(4-Fluor-phenyl)-(2,2,2-trifluor-acetylamino)-essigsäuremethylester werden in 195,1 ml abs. Tetrahydrofuran vorgelegt und das Reaktionsgemisch bei +3°C gekühlt. 37,2 ml einer Methylmagnesiumiodid Lösung (3 M) werden langsam zugetropft, so dass die Temperatur nicht über +10°C übersteigt. Das Reaktionsgemisch wird 12 Stunden bei Raumtemperatur gerührt und anschließend in Eiswasser gerührt. Ammoniumchlorid wird zugegeben, so dass der Niederschlag sich löst. Das Produkt wird mit Essigester extrahiert und anschließend die organische Phase getrocknet und zur Trockene eingedampft. 5,6 g des Produkts werden als Öl erhalten.

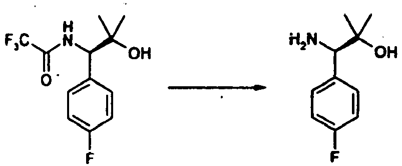

### 2.2.4 (R)-1-Amino-1-(4-fluor-phenyl)-2-methyl-propan-2-ol:

5,6 g 2,2,2-Trifluor-*N*-[(R)-1-(4-fluor-phenyl)-2-hydroxy-2-methyl-propyl]-acetamid und 2,27 g KOH werden in 61,1 ml Methanol suspendiert. Das Reaktionsgemisch wird 20 Stunden bei 60°C gerührt und anschießend mit Wasser und Dichlormethan versetzt, Die organische Phase wird getrocknet und zur Trockene eingedampft. 3,2 g des Produkts werden als Öl erhalten.

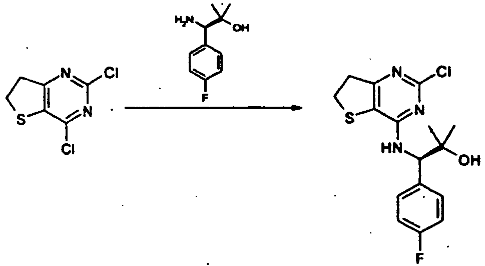

### 2.2.5 (R)-1-(2-Chlor-6,7-dihydro-thieno[3,2-d]pyrimidin-4-ylamino)-1-(4-fluor-phenyl)-2-methyl-propan-2-ol (Schema 2, Schritt A):

533 mg 2,4-Dichlor-6,7-dihydro-thieno[3,2-*d*]pyrimidin, 850 mg (R)-1-Amino-1-(4-fluor-phenyl)-2-methyl-propan-2-ol und 1,3 ml Diisopropylethylamin werden in 9,8 ml Dioxan suspendiert. Das Reaktionsgemisch wird in der Mikrowelle 2 Stunden bei 80°C gerührt und anschießend zur Trockene eingedampft. Der Rückstand wird mit Wasser versetzt. Der ausgefallene Niederschlag wird abgesaugt und chromatographisch (Kieselgel, Petrolether/Essigester 100/0 bis 60/40) gereinigt. 260 mg des Produkts werden als Feststoff erhalten.

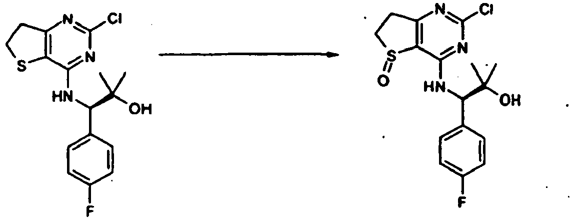

### 2.2.6 (R)-1-(2-Chlor-5-oxo-6,7-dihydro-5H-5λ⁴-thieno[3,2-d]pyrimidin-4-ylamino)-1-(4-fluor-phenyl)-2-methyl-propan-2-ol (Schema 2, Schritt B):

300 mg (R)-1-(2-Chlor-6,7-dihydro-thieno[3,2-*d*]pyrimidin-4-ylamino)-1-(4-fluor-phenyl)-2-methyl-propan-2-ol werden in 2,0 ml Essigsäure vorgelegt. Das Reaktionsgemisch wird auf +10°C gekühlt. 127 µl Wasserstoffperoxid (35%) werden zugetropft. Das Reaktionsgemisch wird 1 Stunde bei +10°C und anschließend in Eiswasser gerührt. Es wird dann mit einer Ammoniaklösung basisch gestellt. Der ausgefallene Niederschlag wird abgesaugt und chromatographisch (Kieselgel, Petrolether/Essigester 50/50 bis 0/100 dann Essigester/Methanol 50/50) gereinigt. 70 mg des Produktes werden als Öl erhalten. Analytische HPLC-MS (Methode D): RT = 1,17 min.

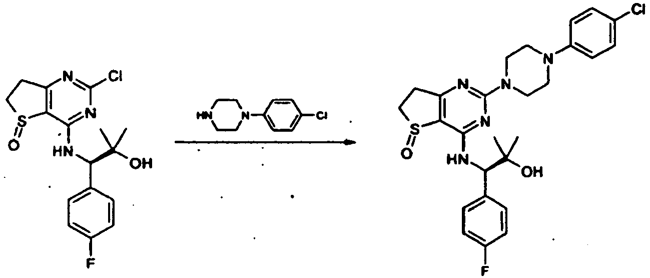

### 2.2.7 (R)-1-{2-[4-(4-Chlor-phenyl)-plperazin-1-yl]-5-oxo-6,7 -dihydro-5H-5λ⁴-thieno[3,2-d]pyrimidin-4-ylamino}-1-(4-fluor-phenyl)-2-methyl-propan-2-ol(Schema 2, Schritt C):

50,0 mg (R)-1-(2-Chlor-5-oxo-6,7-dihydro-5*H*-5λ⁴-thieno[3,2-*d*]pyrimidin-4-ylamino)-1-(4-fluor-phenyl)-2-methyl-propan-2-ol, 76,4 mg 1-(4-Chlorphenylpiperazin) und 99 µl Diisopropylethylamin werden in 850 µl Dioxan vorgelegt, dann in der Mikrowelle 0,3 Stunden bei 120°C erhitzt. Anschließend wird das Reaktionsgemisch zur Trockene eingedampft. Die Diastereomeren werden über semipräparative HPLC (Methode B) getrennt. 10,5 mg des Diastereomers 1 (Beispiel 160) und 13,3 mg des Diastereomers 2 (Beispiel 161) werden als Feststoff erhalten. Analytische HPLC-MS (Methode D): Diastereomer 1: RT = 1,31 min; Diastereomer 2: RT = 1,40 min.

### 2.3 SYNTHESE VON {2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-(TETRAHYDRO-PYRAN-4-YL)-AMIN, BEISPIEL 181 (SIEHE SCHEMA 2)

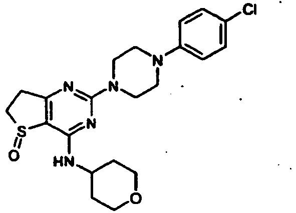

Ausgehend vom 2,4-Dichlor-6,7-dihydro-thieno[3,2-*d*]pyrimidin und 4-Aminotetrahydropyran kann {2-[4-(4-Chlor-phenyl)-piperazin-1-yl)-5-oxo-6,7-dihydro-5*H-*5λ⁴-thieno[3,2-*d*]pyrimidin-4-yl}-(tetrahydro-pyran-4-yl)-amin, wie bei Beispiel 28 (siehe Schema 2) beschrieben, hergestellt werden. Analytische HPLC-MS (Methode D): RT = 1,23 min.

Beide Enantiomeren können über chirale HPLC (Säule: Diacel AS-H, 250 x 4.6 mm, 5 µm, Fließmittel: (Hexan+Cyclohexylamin (0,2%))/Ethanol (80/20), Flußrate: 1 ml/min) getrennt werden: Enantiomer 1: RT = 9,54 min; Enantiomer 2: RT = 11,81 min (Beispiel 181).

### 2.4 SYNTHESE VON (1-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YLAMINO}-CYCLOPROPYL)-METHANOL, BEISPIEL 189 (SIEHE SCHEMA 2)

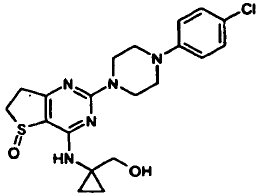

Ausgehend vom 2,4-Dichlor-6,7-dihydro-thieno[3,2-*d*]pyrimidin und 1-Aminocyclopropanmethanol kann (1-{2-[4-(4-Chlor-phenyl)-piperazin-1-yl]-5-oxo-6,7-dihydro-5H 5λ⁴-thieno[3,2-*d*]pyrimidin-4-ylamino}-cyclopropyl)-methanol, wie bei Beispiel 28 (siehe Schema 2) beschrieben, hergestellt werden. Analytische HPLC-MS (Methode D): RT = 1,20 min.

Beide Enantiomeren können über chirale HPLC (Säule: Diacel IA, 250 x 4.6 mm, 5 µm, Fließmittel: (Hexan+Cyclohexylamin (0,2%))/Ethanol (80/20), Flußrate: 1 ml/min) getrennt werden: Enantiomer 1: RT = 17,8 min; Enantiomer 2: RT = 21,9 min (Beispiel 189).

### 2.5 SYNTHESE VON (3-FLUOR-PHENYL)-{2-[4-(4-METHANESULFONYL-PHENYL)-PIPERAZIN-1-YL]-5-OXO-6,7-DIHYDRO-5H-5λ⁴-THIENO[3,2-d]PYRIMIDIN-4-YL}-AMIN, BEISPIEL 202 (SIEHE SCHEMA 2)

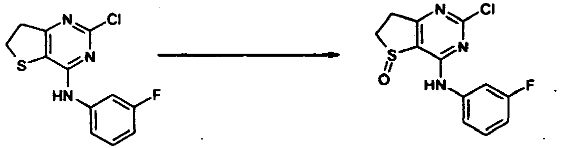

### 2.5.1 2-Chlor-5-oxo-6,7-dihydro-5H-5λ⁴-thieno[3,2-d]pyrimidin-4-yl)-(3-fluor-phenyl)-amin (siehe 2.1.2):

2-Chlor-5-oxo-6,7-dihydro-5H-5λ⁴-thieno[3,2-*d*]pyrimidin-4-yl)-(3-fluor-phenyl)-amin wird, wie bei Beispiel 28 (siehe Schema 2, 2.1.2) beschrieben, hergestellt.

Beide Enantiomeren können über chirale HPLC (Säule: Diacel AS-H, 250 x 4.6 mm, 5 µm, Fließmittel: (Hexan+Cyclohexylamin (0,2%))1(Ethanol+Methanol (1/1)) (80/20), Flußrate: 1 ml/min) getrennt werden: Enantiomer 1: RT = 5,9 min; Enantiomer 2: RT = 7,4 min.

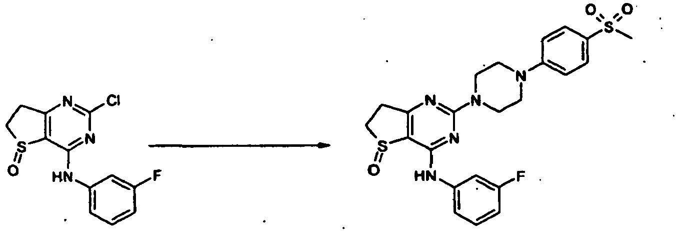

### 2.5.2 (3-Fluor-phenyl)-{2-[4-(4-methanesulfonyl-phenyl)-piperazin-1-yl]-5-oxo-6,7-dihydro-5H-5λ⁴-thieno[3,2-d]pyrimidin-4-yl}-amin:

Ausgehend vom 2-Chlor-5-oxo-6,7-dihydro-5H-5λ⁴-thieno[3,2-*d*]pyrimidin-4-yl)-(3-fluorphenyl)-amin Enantiomer 1 und 1-(4-(Methyl-sulphonyl)phenyl)piperazin kann Beispiel 202, wie bei Beispiel 28 (siehe Schema 2, Schritt C, 2.1.3) beschrieben, hergestellt werden; Analytische HPLC-MS (Methode D): RT = 1,24 min.

### 2.6 SYNTHESE VON (S)-5-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YLAMINO}-1-METHYL-PIPERIDIN-2-ON, BEISPIEL 203 (SIEHE SCHEMA 2)

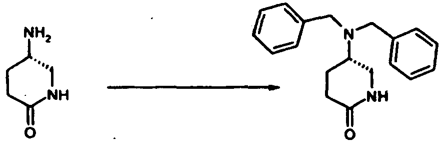

### 2.6.1 (S)-5-Dibenzylamino-piperidin-2-on:

600 mg 4-(S)-Amino-delta-valerolactam Hydrochlorid, 970 µl Benzylbromid und 1,5 g Natriumhydrogencarbonat werden in 30 ml Ethanol suspendiert. Das Reaktionsgemisch wird dann 8 Stunden bei 80°C gerührt und anschießend zur Trockene eingedampft. Der Rückstand wird in Wasser suspensiert und das Produkt mit Dichlormethan extrahiert. Das Produkt wird dann chromatographisch (Kieselgel, Dichlormethan/methanol 100/0 bis 95/5) gereinigt. 500 mg des Produktes werden als Öl erhalten. Analytische HPLC-MS (Methode D): RT = 1,01 min.

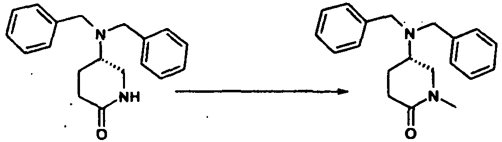

### 2.6.2 (S)-5-Dibenzylamino-1-methyl-piperidin-2-on:

500 mg (S)-5-Dibenzylamino-piperidin-2-on werden in 15 ml Tetrahydrofuran suspendiert. Unter Eisbadkühlung werden 175 mg Kalium-*tert*-butylat zugegeben. Das Reaktionsgemisch wird dann 30 Minuten bei Raumtemperatur gerührt. Unter Eisbadkühlung werden 95 µl Methyliodid zugegeben. Das Reaktionsgemisch wird dann 48 Stunden bei Raumtemperatur gerührt und anschließend mit einer gesättigten NaCl Lösung und Essigester versetzt. Die organische Phase wird getrocknet und zur Trockene eingedampft. 450 mg des Produktes werden als Öl erhalten. Analytische HPLC-MS (Methode D): RT = 1,07 min.

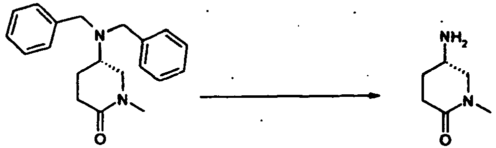

### 2.6.3 (S)-5-Amino-1-methyl-piperidin-2-on:

450 mg (S)-5-Dibenzylamino-1-methyl-piperidin-2-on werden in 25 ml Methanol suspendiert und mit 150 mg Pd/C 10% bei einem Druck von 3 bar hydriert und einer temperatur von 60 °C. Nach 16 Stunden wird der Katalysator abgesaugt und das Filtrat zur Trockene eingedampft. 190 mg des Produktes werden als Öl erhalten.

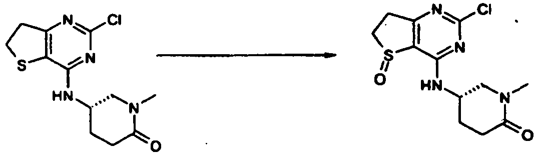

### 2.6.4 (S)-5-(2-Chlor-5-oxo-6,7-dihydro-5H-5λ⁴-thieno[3,2-d]pyrimidin-4-ylamino)-1-methyl-piperidin-2-on:

Ausgehend vom 2,4-Dichlor-6,7-dihydro-thieno[3,2-*d*]pyrimidin und (S)-5-Amino-1-methylpiperidin-2-on kann (S)-5-(2-Chlor-5-oxo-6,7-dihydro-5*H*-5λ⁴-thieno[3,2-d]pyrimidin-4-ylamino)-1-methyl-piperidin-2-on, wie bei Beispiel 28 (siehe Schema 2, 2.1.2) beschrieben, hergestellt werden.

Beide Diastereomeren können über chirale HPLC (Säule: Diacel AS-H, 250 x 4.6 mm, 5 µm, Fließmittel: (Hexan+Cyclohexylamin (0,2%))/Ethanol (70/30), Flußrate: 1 ml/min) getrennt werden: Diastereomer 1: RT = 8,84 min; Diastereomer 2: RT =15,7 min.

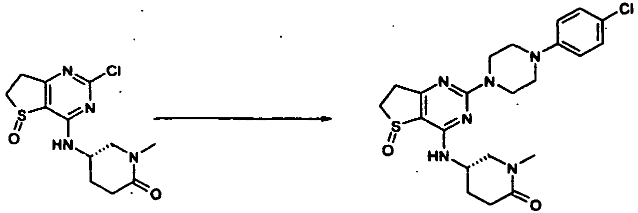

### 2.6.5 (S)-5-{2-[4-(4-Chlor-phenyl)-piperaxin-1-yl]-5-oxo-6,7-dihydro-5H-5λ⁴-thieno[3,2-d]pyrimidin-4-ylamino}-1-methyl-piperidin-2-on:

Ausgehend vom (S)-5-(2-Chlor-5-oxo-6,7-dihydro-5*H*-5λ⁴-thieno[3,2-*d*]pyrimidin-4-ylamino)-1-methyl-piperidin-2-on Diastereomer 1 (siehe 2.6.4) kann Beispiel 203, wie bei Beispiel 28 (siehe Schema 2, Schritt C, 2.1.3) beschrieben, hergestellt werden. Analytische HPLC-MS (Methode D): RT = 1,18 min.

### 2.7 SYNTHESE VON (R)-5-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YLAMINO}-1-METHYL-PIPERIDIN-2-ON. BEISPIEL 204 (SIEHE SCHEMA 2)

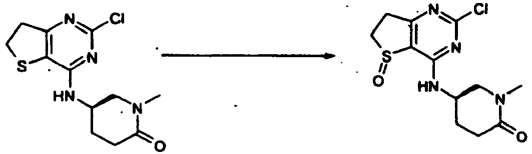

### 2.7.1 (R)-5-(2-Chlor-5-oxo-6,7-dihydro-5H-5λ⁴-thieno[3,2-d]pyrimidin-4-ylamino)-1-methyl-piperidin-2-on:

Ausgehend vom 2,4-Dichlor-6,7-dihydro-thieno[3,2-*d*]pyrimidin und (R)-5-Amino-1-methylpiperidin-2-on (aus dem 4-(R)-Amino-delta-valerolactam Hydrochlorid, wie bei Beispiel 203 (siehe 2.6) beschrieben, hergestellt) kann (R)-5-(2-Chlor-5-oxo-6,7-dihydro-5*H*-5λ⁴-thieno[3,2-*d*]pyrimidin-4-ylamino)-1-methyl-piperidin-2-on, wie bei Beispiel 28 (siehe Schema 2, 2.1.2) beschrieben, hergestellt werden.

Beide Diastereomeren können über chirale HPLC (Säule: Diacel AS-H, 250 x 4.6 mm, 5 µm, Fließmittel: (Hexan+Cyclohexylamin (0,2%))/Ethanol (70/30), Flußrate: 1 ml/min) getrennt werden: Diastereomer 1: RT = 9,4 min; Diastereomer 2: RT = 21,8 min.

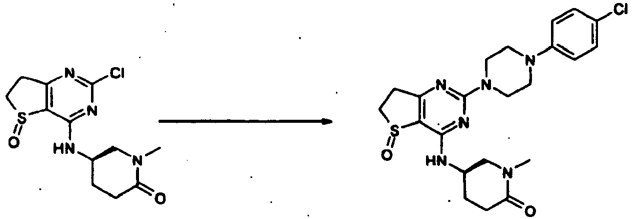

### 2.7.2 (R)-5-{2-[4-(4-Chlor-phenyl)-piperazin-1-yl]-5-oxo-6,7-dihydro-5H-5λ⁴-thieno[3,2-d]pyrimidin-4-ylamino}-1-methyl-piperidin-2-on:

Ausgehend vom (R)-5-(2-Chlor-5-oxo-6,7-dihydro-5*H*-5λ⁴-thieno[3,2-*d*]pyrimidin-4-ylamino)-1-methyl-piperidin-2-on Diastereomer 1 kann Beispiel 204, wie bei Beispiel 28 (siehe Schema 2, Schritt C, 2.1.3) beschrieben, hergestellt werden. Analytische HPLC-MS (Methode D): RT = 1,19 min.

### 2.8 SYNTHESE VON (R)-2-{2-[4-(4-BROM-PHENYL)-PIPERAZIN-1-YL]-5-OXO-6,7-DIHYDRO-5H-5λ⁴-THIENO[3,2-d]PYRIMIDIN-4-YLAMINO}-3-METHYL-BUTAN-1-OL, BEISPIEL 208 (SIEHE SCHEMA 2)

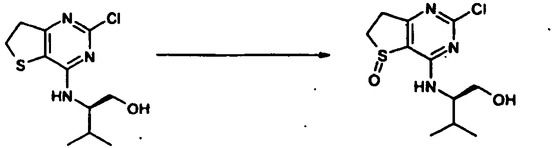

### 2.8.1 (R)-2-(2-Chlor-5-oxo-6,7-dihydro-5H-5λ⁴-thieno[3,2-d]pyrimidin-4-ylamino)-3-methyl-butan-1-ol (Schema 2, Schritt B):

Ausgehend vom 2,4-Dichlor-6,7-dihydro-thieno[3,2-*d*]pyrimidin und D-Valinol können beide Diastereomeren von (R)-2-(2-Chlor-5-oxo-6,7-dihydro-5*H*-5λ⁴-thieno[3,2-*d*]pyrimidin4-ylamino)-3-methyl-butan-1-ol, wie bei Beispiel 28 (siehe 2.1.2) beschrieben, hergestellt werden. Die Diastereomeren können über präparative HPLC (Methode A) getrennt werden. Analytische HPLC-MS (Methode A): Diastereomer 1: RT = 2,13 min; Diastereomer 2: RT= 2,25 min.

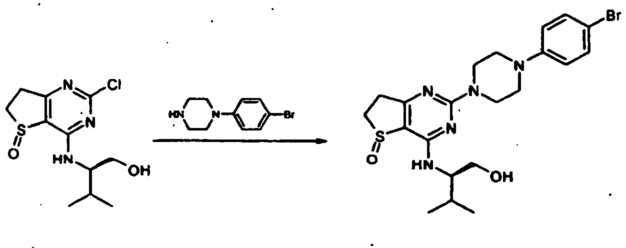

### 2.8.2 (R)-2-{2-[4-(4-Brom-phenyl)-piperazin-1-yl]-5-oxo-6,7-dihydro-5H-5λ⁴-thieno[3,2-d]pyrimidin-4-ylamino}-3-methyl-butan-1-ol (Schema 2, Schritt C):

Ausgehend vom (R)-2-(2-Chlor-5-oxo-6,7-dihydro-5*H*-5λ⁴-thieno[3,2-*d*]pyrimidin-4-ylamino)-3-methyl-butan-1-ol Diastereomer 1 (siehe 2.8.1) kann Beispiel 208, wie bei Beispiel 28 (siehe 2.1.3) beschrieben, hergestellt werden. Analytische HPLC-MS (Methode D): RT = 1,21 min.

### 2.9 SYNTHESE VON (1-{2-[4-(4-BROMO-PHENYL)-PIPERAZIN-1-YL]-5-OXO-6,7-DIHYDRO-5H-5λ⁴-THIENO[3,2-d]PYRIMIDIN-4-YLAMINO}-CYCLOPROPYL)-METHANOL, BEISPIEL 211 (SIEHE SCHEMA 2)

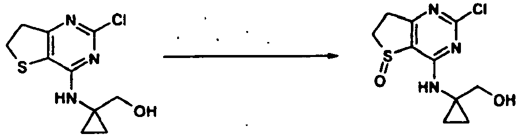

### 2.9.1 [1-(2-Chlor-5-oxo-6,7-dihydro-5H-5λ⁴-thieno[3,2-d]pyrimidin-4-ylamino)-cyclopropyl]-methanol (Schema 2, Schritt B):

Ausgehend vom 2,4-Dichlor-6,7-dihydro-thieno[3,2-*d*]pyrimidin und 1-Aminocyclopropanmethanol können beide Enantiomeren von [1-(2-Chlor-5-oxo-6,7-dihydro-5*H-*5λ⁴-thieno[3,2-*d*]pyrimidin-4-ylamino)-cyclopropyl]-methanol, wie bei Beispiel 28 (siehe 2.1.2) beschrieben, hergestellt werden. Die Enantiomeren können über chirale HPLC (Säule: Diacel IA, 250 x 4.6 mm, 5 µm, Fließmittel: (Hexan+Cyclohexylamin (0,2%))/Ethanol (80/20), Flußrate: 1 ml/min) getrennt werden: Enantiomer 1: RT = 11,1 min; Enantiomer 2: RT = 16,5 min.

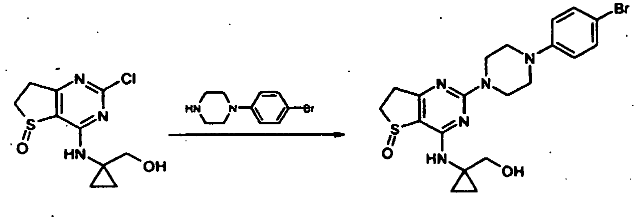

### 2.9.2 (1-{2-[4-(4-Bromo-phenyl)-piperazin-1-yl]-5-oxo-6,7-dihydro-5H-5λ⁴-thieno[3,2-d]pyrimidin-4-ylamino}-cyclopropyl)-methanol (Schema 2, Schritt C):

Ausgehend vom [1-(2-Chlor-5-oxo-6,7-dihydro-5*H*-5λ⁴-thieno[3,2-*d*]pyrimidin-4-ylamino)-cyclopropyl]-methanol Enantiomer 1 (siehe 2.9.1) kann Beispiel 211, wie bei Beispiel 28 (siehe 2.1.3) beschrieben, hergestellt werden. Analytische HPLC-MS (Methode D): RT = 1,18min.

### 2.10 SYNTHESE VON 4-{4-[4-((R)-1-HYDROXYMETHYL-2-METHYL-PROPYLAMINO)-5-OXO-6,7-DIHYDRO-5H-5λ⁴-THIENO[3,2-d]PYRIMIDIN-2-YL]-PIPERAZIN-1-YL}-BENZOESÄUREETHYLESTER, BEISPIEL 212 (SIEHE SCHEMA 2)

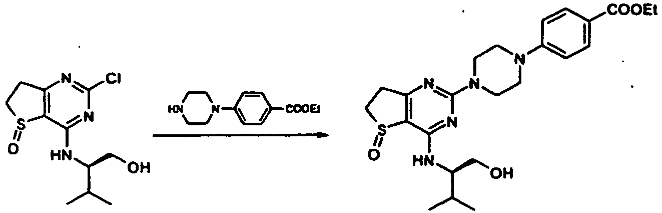

Ausgehend vom (R)-2-(2-Chlor-5-oxo-6,7-dihydro-5*H*-5λ⁴-thieno[3,2-*d*]pyrimidin-4-ylamino)-3-methyl-butan-1-ol Diastereomer 1 (siehe Beispiel 208, 2.8.1) kann Beispiel 212, wie bei Beispiel 28 (siehe 2.1.3) beschrieben, hergestellt werden. Analytische HPLC-MS (Methode D): RT = 1,2 min.

### 2.11 SYNTHESE VON 4-{4-[5-OXO-4-(TETRAHYDRO-PYRAN-4-YLAMINO)-6,7-DIHYDRO-5H-5λ⁴-THIENO[3,2-d]PYRIMIDIN-2-YL]-PIPERAZIN-1-YL}-BENZOESÄUREETHYLESTER, BEISPIEL 214 (SIEHE SCHEMA 2)

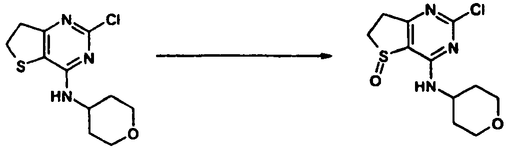

### 2.11.1 (2-Chlor-5-oxo-6,7 -dihydro-5H-5λ⁴-thieno[3,2-d]pyrimidin-4-yl)-(tetrahydropyran-4-yl)-amin (Schema 2, Schritt B):

Ausgehend vom 2,4-Dichlor-6,7-dihydro-thieno[3,2-*d*]pyrimidin und 4-Aminotetrahydropyran können beide Enantiomeren von (2-Chlor-5-oxo-6,7-dihydro-5H-5λ⁴-thieno[3,2-*d*]pyrimidin-4-yl)-(tetrahydro-pyran-4-yl)-amin, wie bei Beispiel 28 (siehe 2.1.2) beschrieben, hergestellt werden. Die Enantiomeren können über chirale HPLC (Säule: Diacel AS-H, 250 x 4.6 mm, 5 µm, Fließmittel: (Hexan+Cyclohexylamin (0,2%))/Ethanol (80/20), Flußrate: 1 ml/min) getrennt werden: Enantiomer 1: RT = 7,51 min; Enantiomer 2: RT = 12,3 min.

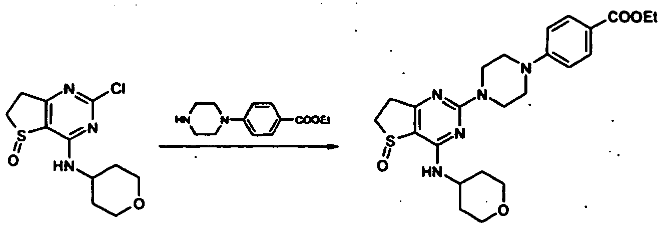

### 2.11.2 4-{4-[5-Oxo-4-(tetrahydro-pyran-4-ylamino)-6,7-dihydro-5H-5λ⁴-thieno[3,2-d]pyrimidin-2-yl]-piperazin-1-yl}-benzoesäureethylester (Schema 2, Schritt C):

Ausgehend vom (2-Chlor-5-oxo-6,7-dihydro-5*H*-5λ⁴-thieno[3,2-*d*]pyrimidin-4-yl)-(tetrahydro-pyran-4-yl)-amin Enantiomer 1 (siehe 2.11.1) kann Beispiel 214, wie bei Beispiel 28 (siehe 2.1.3) beschrieben, hergestellt werden. Analytische HPLC-MS (Methode D): RT = 1,2 min.

### 2.12 SYNTHESE VON (R)-2-{2-[4-(4-BROM-PHENYL)-PIPERAZIN-1-YL]-5-OXO-6,7-DIHYDRO-5H-5λ⁴-THIENO[3,2-d]PYRIMIDIN-4-YLAMINO}-PENTAN-1-OL, BEISPIEL 216 (SIEHE SCHEMA 2)

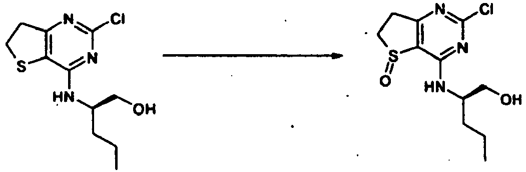

### 2.12.1 (R)-2-(2-Chlor-5-oxo-6,7-dihydro-5H-5λ⁴-thieno[3,2-d]pyrimidin-4-ylamino)-pentan-1-ol (Schema 2, Schritt B):

Ausgehend vom 2,4-Dichlor-6,7-dihydro-thieno[3,2-*d*]pyrimidin und D-norvalinol werden beide Diastereomeren von (R)-2-(2-Chlor-5-oxo-6,7-dihydro-5*H*-5λ⁴-thieno[3,2-*d*]pyrimidin-4-ylamino)-pentan-1-ol, wie bei Beispiel 28 (siehe 2.1.2) beschrieben, hergestell. Die Diastereomeren werden über präparative HPLC (Methode B) getrennt. Analytische HPLC (Methode B): Diastereomer 1: RT = 2,43 min; Diastereomer 2: RT = 2,56 min.

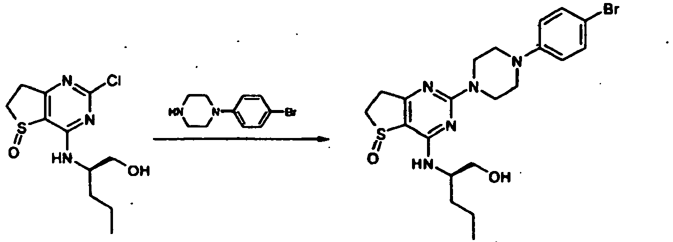

### 2.12.2 (R)-2-{2-[4-(4-Brom-phenyl)-piperazin-1-yl]-5-oxo-6,7-dihydro-5H-5λ⁴-thieno[3,2-d]pyrimidin-4-ylamino}-pentan-1-ol (Schema 2, Schritt C):

Ausgehend von (R)-2-(2-Chlor-5-oxo-6,7-dihydro-5*H*-5λ⁴-thieno[3,2-*d*]pyrimidin-4-ylamino)-pentan-1-ol Diastereomer 1 (siehe 2.12.1) kann Beispiel 216, wie bei Beispiel 28 (siehe 2.1.3) beschrieben, hergestellt werden. Analytische Analytische HPLC-MS (Methode D): RT = 1,23 min.

### 2.13 SYNTHESE VON 2-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YLAMINO}-3-FLUOR-3-METHYLBUTAN-1-OL, BEISPIEL 187 (SIEHE SCHEMA 2)

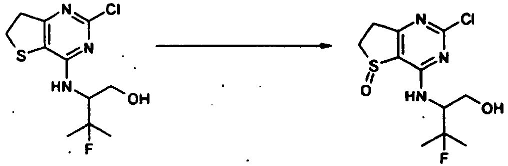

### 2,13.1 2-(2-Chlor-5-oxo-6,7-dihydro-5H-5λ⁴-thieno[3,2-d]pyrimidin-4-ylamino)-3-fluor-3-methyl-butan-1-ol (Schema 2, Schritt B):

Ausgehend vom 2,4-Dichlor-6,7-dihydro-thieno[3,2-*d*]pyrimidin und 3-Fluor-DL-valinol (J. Org. Chem. 2000, 5037) wird ein Stereoisomerengemisch von 2-(2-Chlor-5-oxo-6,7-dihydro-5*H*-5λ⁴-thieno[3,2-*d*]pyrimidin-4-ylamino)-3-fluor-3-methyl-butan-1-ol, wie bei Beispiel 28 (siehe 2.1.2) beschrieben, hergestellt. Die Diastereomeren werden über semipräparative HPLC (Methode A) getrennt. Analytische HPLC-MS (Methode D): Diastereomer 1: RT = 0,92 min; Diastereomer 2: RT = 0,99 min. Beide Diastereomeren werden als Racemate erhalten.

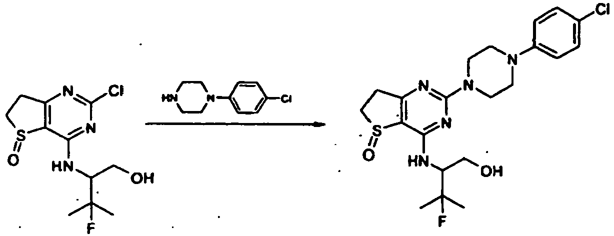

### 2.13.2 2-{2-[4-(4-Chlor-phenyl)-piperazin-1-yl]-5-oxo-6,7-dihydro-5H-5λ⁴-thieno[3,2-d]pyrimidin-4-ylamino}-3-fluor-3-methyl-butan-1-ol (Schema 2, Schritt C):

Ausgehend von 2-(2-Chlor-5-oxo-6,7-dihydro-5*H*-5λ⁴-thieno[3,2-*d*]pyrimidin-4-ylamino)-3-fluor-3-methyl-butan-1-ol Diastereomer 2 (siehe 2.13.1) wird Beispiel 187, wie bei Beispiel 28 (siehe 2.1.3) beschrieben, hergestellt. Das Produkt wird als Racemat erhalten.

Analytische Analytische HPLC-MS (Methode D): RT = 1,26 min.

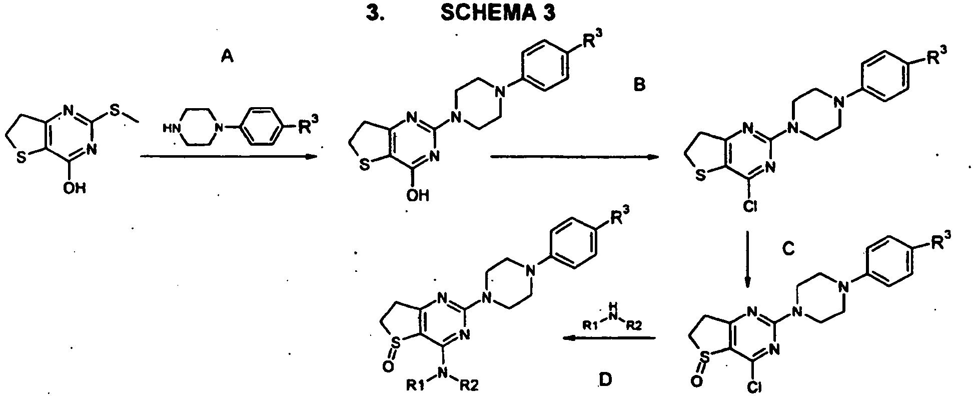

### 3.1 SYNTHESE VON (3R,5S)-1-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-5-HYDROXYMETHYL-PYRROLIDIN-3-OL, BEISPIEL 124 (SIEHE SCHEMA 3)

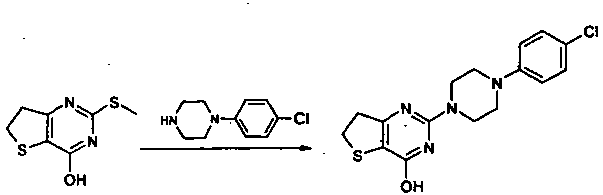

**3.1.1 2-[4-(4-Chlor-phenyl)-piperazin-1-yl]-6,7-dihydro-thieno[3,2-d]pyrimidin-4-ol (Schema 3, Schritt A)**: 20,45 g 1-(4-Chlor-phenyl)-piperazin werden in 5,95 ml (104 mmol) Eisessig vorgelegt und auf 180° C erhitzt. 8,01 g 2-Methylsulfanyl-6,7-dihydro-thieno[3,2-*d*]pyrimidin4-ol werden zugegeben, das nicht rührbare Reaktionsgemisch wird 2,5 Stunden auf 180° C erhitzt. Nach Abkühlen wird Wasser zugegeben, Niederschlag abgesaugt und an Luft getrocknet. Die Substanz wird mit Ethanol im Ultraschall behandelt, abgesaugt und getrocknet. 12,95 g des Produkts werden als Pulver erhalten.

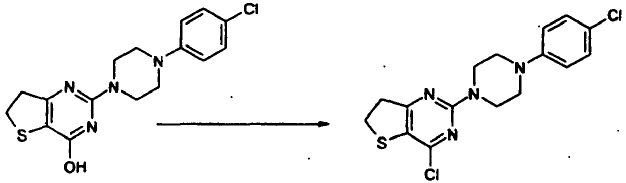

### 3.1.2 4-Chlor-2-[4-(4-chlor-phenyl)-piperazin-1-yl]-6,7-dihydro-thieno[3,2-d]pyrimidin (Schema 3, Schritt B):

12,95 g 2-[4-(4-Chlor-phenyl)-piperazin-1-yl]-6,7-dihydro-thieno[3,2-*d*]pyrimidin-4-ol und 50,89 ml (546 mmol) Phosphoroxychlorid werden 4 Stunden auf 120°C erhitzt. Anschließend wird das Reaktionsgemisch eingedampft, der Rückstand mit Wasser versetzt. Ausgefallener Niederschlag wird abgesaugt, mit Wasser gewaschen und getrocknet. Das Rohprodukt wird mit Methanol verrührt und abgesaugt. 11,78 g des Produkts werden als Pulver erhalten.

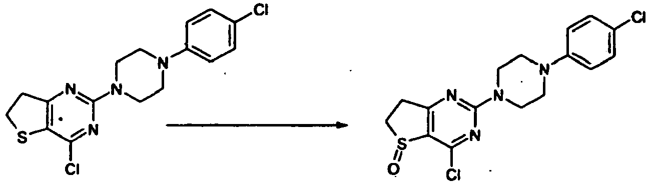

### 3.1.3 4-Chlor-2-[4-(4-chlor-phenyl)-piperazin-1-yl]-6,7-dihydro-thieno[3,2-d]pyrimidine 5-oxid (Schema 3, Schritt C):

14,69 g 4-Chlor-2-[4-(4-chlor-phenyl)-piperazin-1-yl]-6,7-dihydro-thieno[3,2-*d*]pyrimidin werden in 140 ml Eisessig vorgelegt und auf 10° C abgekühlt. 3,79 ml Wasserstoffperoxid (35%) werden zugetropft, dann 2 Stunden nachgerührt. Es werden noch 1 eq Wasserstoffperoxid zugegeben, 8 Stunden bei Raumtemperatur gerührt. Anschließend wird das Reaktionsgemisch in Eiswasser gerührt, mit Ammoniaklösung basisch gestellt. Der ausgefallene Niederschlag wird abgesaugt, mit Wasser gewaschen und getrocknet. 11,90 g des Produkts werden als Pulver erhalten.

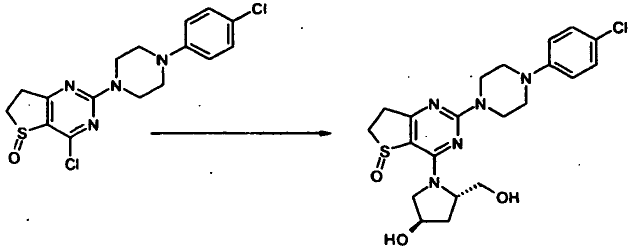

### 3.1.4 (3R,5S)-1-{2-[4-(4-Chlor-phenyl)-piperazin-1-yl]-5-oxo-6,7-dihydro-5H-5λ⁴-thieno[3,2-d]pyrimidin-4-yl}-5-hydroxymethyl-pyrrolidin-3-ol (Schema 3, Schritt D):

220 mg (0,57 mmol) 4-Chlor-2-[4-(4-chlor-phenyl)-piperazin-1-yl]-6,7-dihydro-thieno[3,2-*d*]pyrimidin 5-oxid, 159,23 mg (0,69 mmol) (3R,5S)-5-Hydroxymethyl-pyrrolidin-3-ol trifluoracetat (aus dem käufigen Boc-trans-4-hydroxy-L-prolinol hergestellt) und 197,52 µl (1,15 mmol) Diisopropylethylamin werden in 4 ml Dioxan vorgelegt, in der Mikrowelle 0,3 Stunden auf 120° C erhitzt. Anschließend wird das Reaktionsgemisch mit Wasser und Dichlormethan versetzt und extrahiert. Die organische Phase wird mit Phasentrennkartusche abgetrennt und zur Trockene eingedampft. 200,3 mg des Produkts (75%) werden als racemische Mischung erhalten. Analytische HPLC-MS (Methode A): RT = 2.26 min.

### 3.2. SYNTHESE VON (2S,4R)-1-{2-[4-(4-CHLOR-PHENYL)-PIPERAZIN-1-YL]-5-OXO-6,7-DIHYDRO-5H-5λ⁴-THIENO [3,2-d]PYRIMIDIN-4-YL}-4-HYDROXY-PYRROLIDIN-2-CARBONSÄUREMETHYLESTER, BEISPIELE 125 UND 126 (SIEHE SCHEMA 3, SCHRITT D):

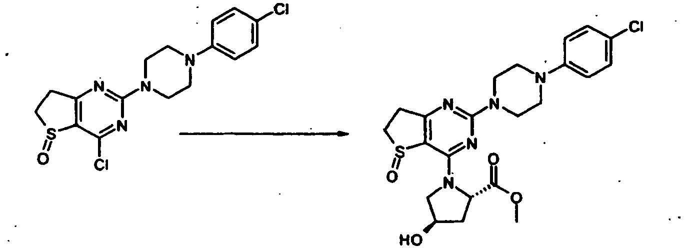

500 mg 4-Chlor-2-[4-(4-chlor-phenyl)-piperazin-1-yl]-6,7-dihydro-thieno[3,2-d]pyrimidin 5-oxid (siehe Beispiel 124), 284,19 mg (2S,4R)-4-Hydroxy-pyrrolidine-2-carbonsäuremethylester und 691,65 µl Diisopropylethylamin werden in 8 ml Dioxan vorgelegt, in der Mikrowelle 0,3 Stunden auf 120° C erhitzt. Anschließend wird das Reaktionsgemisch mit Wasser und Dichlormethan versetzt und extrahiert. Die organische Phase wird mit Phasentrennkartusche abgetrennt und zur Trockene eingedampft. Die Diastereomere werden über präparative HPLC getrennt (Methode B). 200 mg des Diastereomers 1 (Beispiel 125) und 160 mg des Diastereomers 2 (Beispiel 126) als Pulver erhalten. Analytische HPLC-MS (Methode A): Diastereomer 1, RT = 2.51 min, Diastereomer 2, RT = 2,57 min.

### 3.3 SYNTHESE VON (2S,4R)-1-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-4-HYDROXY-PYRROLIDIN-2-CARBONSÄUREAMID, BEISPIEL 128 (SIEHE SCHEMA 3)

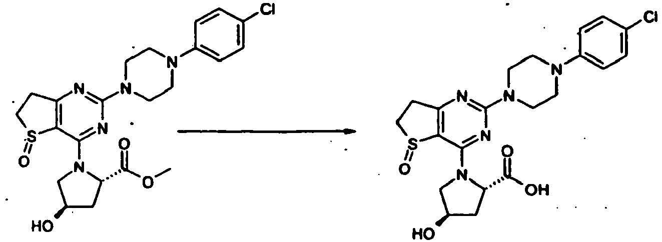

### 3.3.1 (2S,4R)-1-{2-[4-(4-Chlor-phenyl)-piperazin-1-yl]-5-oxo-6,7-dihydro-5H-5λ⁴-thieno[3,2-d]pyrimidin-4-yl}-4-hydroxy-pyrrolidin-2-carbonsäure:

1,12 g (2S,4R)-1-{2-[4-(4-Chlor-phenyl)-piperazin-1-yl]-5-oxo-6,7-dihydro-5*H*-5λ⁴-thieno [3,2-*d*]pyrimidin-4-yl}-4-hydroxy-pyrrolidin-2-carbonsäuremethylester als Diastereomerenmischung (siehe Beispiel 125) und 11,38 ml 1molare Natronlauge werden in 10 ml Methanol vorgelegt, dann 4 Stunden unter Rückfluss gerührt. Die entstandene Suspension wird eingedampft, der Rückstand abgekühlt und mit 2molarer Salzsäure sauer gestellt und die Suspension noch mal eingedampft. Das Produkt wird über präparative HPLC (Methode A) gereinigt. 1,12 g des Produkts werden als Pulver erhalten.

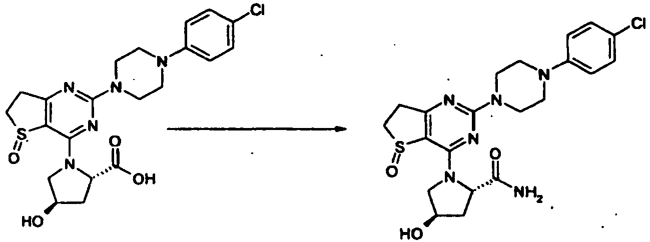

### 3.3.2 (2S,4R)-1-{2-[4-(4-Chlor-phenyl)-piperazin-1-yl]-5-oxo-6,7-dihydro-5H-5λ⁴-thieno[3,2-d]pyrimidin-4-yl}-4-hydroxy-pyrrolidin-2-carbonsäureamid:

80 mg (2S,4R)-1-{2-[4-(4-Chlor-phenyl)-piperazin-1-yl]-5-oxo-6,7-dihydro-5H-5λ⁴-thieno[3,2-d]pyrimidin-4-yl}-4-hydroxy-pyrrolidin-2-carbonsäure werden in 2,00 ml N,N-Dimethylformamid vorgelegt, mit 86,77 mg Diisopropylethylamin und 76,20 mg O-(7-Azabenzotriazol-1-yl-)-N,N,N',N'-tetramethyluronium-hexafluoro-phosphat (HATU) versetzt. Es wird 0,25 Stunden bei Raumtemperatur gerührt, dann 334 µl Ammoniaklösung (0,5 M in Dioxan) zugegeben. Das Reaktionsgemisch wird 3 Stunden bei Raumtemperatur gerührt, anschließend sauer gestellt und Wasser zugegeben. Die Diastereomere werden über präparative HPLC (Methode B) getrennt. 11,7 mg des Diastereomers 1 und 15,3 mg des Diastereomers 2 (Beispiel 128) als Pulver erhalten. Analytische HPLC-MS (Methode A): Diastereomer 1, RT = 1,49 min, Diastereomer 2, RT = 1,49 min.

### 3.4 SYNTHESE VON (2S,4R)-1-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-4-HYDROXY-PYRROLIDIN-2-CARBONSÄUREDIMETHYLAMID, BEISPIEL 129 (SIEHE SCHEMA 3)

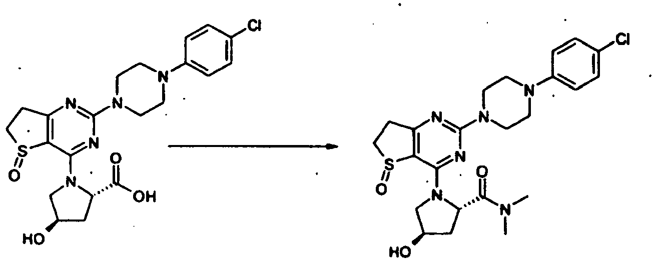

Aus 80 mg (2S,4R)-1-{2-[4-(4-Chlor-phenyl)-piperazin-1-yl]-5-oxo-6,7-dihydro-5H-5λ⁴-thieno[3,2-d]pyrimidin-4-yl}-4-hydroxy-pyrrolidin-2-carbonsäure und 83,7 µl Dimethylamin werden, wie bei Beispiel 128, 13,9 mg des Diastereomers 1 und 23,9 mg des Diastereomers 2 (Beispiel 129) als Pulver erhalten. Analytische HPLC-MS (Methode A): Diastereomer 1, RT = 4,61 min, Diastereomer 2, RT = 4,61 min.

### 3.5 SYNTHESE VON (2S,4R)-1-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-4-HYDROXY-PYRROLIDIN-2-CARBONSÄUREMETHYLAMID, BEISPIEL 130 (SIEHE SCHEMA 3)

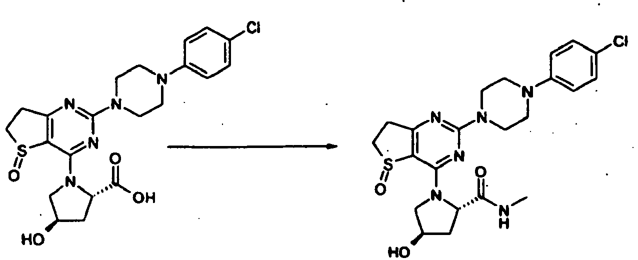

Aus 70 mg (2S,4R)-1-{2-[4-(4-Chlor-phenyl)-piperazin-1-yl]-5-oxo-6,7-dihydro-5H-5λ⁴-thieno[3,2-d]pyrimidin-4-yl}-4-hydroxy-pyrrolidin-2- und 73,0 µl Methylaminlösung (2,0 M in THF) werden, wie bei Beispiel 128, 19,8 mg des Produktes als Pulver erhalten. Analytische HPLC-MS, (Methode A): RT = 1,57 min.

### 3.6 SYNTHESE VON (R)-2-{2-[4(4-CHLOR-PHENYL)-PIPERAZIN-1-YL]-5-OXO-6,7-DIHYDRO-5H-5λ⁴-THIENO₋[3,2-d]PYRIMIDIN-4-YLAMINO}-3-METHYL-BUTAN-1-OL, BEISPIELE 54 UND 57 (SIEHE SCHEMA 3, SCHRITT D):

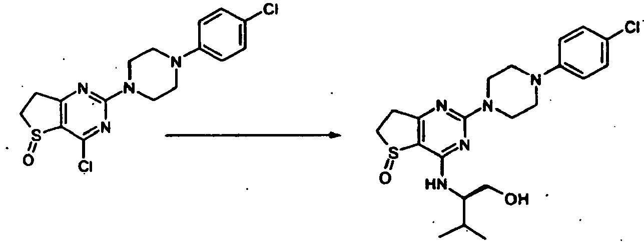

220 mg 4-Chlor-2-[4-(4-chlor-phenyl)-piperazin-1-yl]-6,7-dihydro-thieno[3,2-*d*]pyrimidin 5-oxid (siehe Beispiel 124), 118,43 mg (R)-(-)-2-Amino-3-methyl-butan-1-ol und 197,52 µl Diisopropylethylamin werden in 4 ml Dioxan vorgelegt, in der Mikrowelle 0,3 Stunden auf 120° C erhitzt. Anschließend wird das Reaktionsgemisch mit Wasser versetzt, ausgefallener Niederschlag abgesaugt, gewaschen und getrocknet. Die Diastereomere werden über präparative HPLC (Methode B) getrennt. 25,5 mg des Diastereomers 1 (Beispiel 54) und 40,5 mg des Diastereomers 2 (Beispiel 57) werden als Pulver erhalten. Analytische HPLC (Methode A) : Diastereomer 1: RT = 3,61 min; Diastereomer 2, RT = 3,71 min.

### 3.7 SYNTHESE VON (R)-2-{2-[4-(4-CHLOR-PHENYL)-PIPERAZIN-1-YL]-5-OXO-6,7-DIHYDRO-5H-5λ4-THIENO [3,2-d]PYRIMIDIN-4-YLAMINO}-2-PHENYL-ETHANOL BEISPIELE 49 UND 56 (SIEHE SCHEMA 3, SCHRITT D):

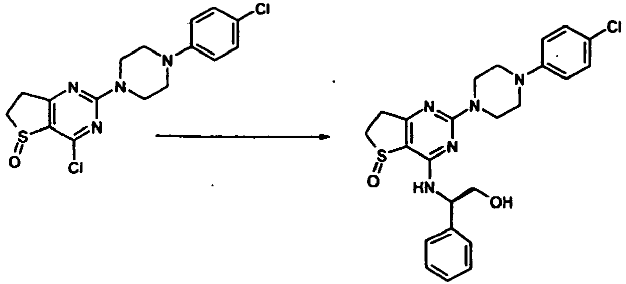

220 mg 4-Chlor-2-[4-(4-chlor-phenyl)-piperazin-1-yl]-6,7-dihydro-thieno[3,2-*d*]pyrimidin 5-oxid (siehe Beispiel 124), 157,48 mg (R)-(-)-2-phenylglycinol und 197,52 µl Diisopropylethylamin werden in 4 ml Dioxan vorgelegt, in der Mikrowelle 0,3 Stunden auf 120° C erhitzt. Anschließend wird das Reaktionsgemisch mit Wasser versetzt, ausgefallener Niederschlag abgesaugt, gewaschen und getrocknet. Die Diastereomere werden über präparative HPLC (Methode B) getrennt. 52,8 mg des Diastereomers 1 (Beispiel 49) und 42,0 mg des Diastereomers 2 (Beispiel 56) werden als Pulver erhalten. ¹H NMR (400 MHz, DMSO): Diastereomer 1: 7.41 - 7.35 (m, 2H), 7.35 - 7.28 (m, 2H), 7.28 - 7.19 (m, 3H), 6.99 - 6.92 (m, 2H); Diastereomer 2: 7.42 - 7.35 (m, 2H), 7.33 - 7.15 (m, 5H), 6.99 - 6.92 (m, 2H).

### 3.8 SYNTHESE VON (R)-2-{2-[4-(4-CHLOR-PHENYL)-PIPERAZIN-1-YL]-5-OXO-6,7-DIHYDRO-5H-5λ4-THIENO[3,2-d]PYRIMIDIN-4-YLAMINO}-4-METHYL-PENTAN-1-OL BEISPIELE 53 UND 69 (SIEHE SCHEMA 3, SCHRITT D):

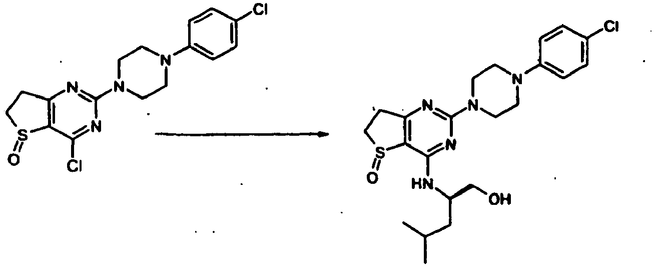

220 mg 4-Chlor-2-[4-(4-chlor-phenyl)-piperazin-1-yl]-6,7-dihydro-thieno[3,2-*d*]pyrimidin 5-oxid (siehe Beispiel 124), 134,53 mg D-Leucinol und 197,52 µl Diisopropylethylamin werden in 4 ml Dioxan vorgelegt, in der Mikrowelle 0,3 Stunden auf 120° C erhitzt. Anschließend wird das Reaktionsgemisch mit Wasser versetzt, ausgefallener Niederschlag abgesaugt, gewaschen und getrocknet. Die Diastereomere werden über präparative HPLC (Methode B) getrennt. 43,9 mg des Diastereomers 1 (Beispiel 53) und 46,9 mg des Diastereomers 2 (Beispiel 69) werden als Öl erhalten. Analytische HPLC-MS (Methode B):Diastereomer 1, RT = 1,86 min, Diastereomer 2, RT = 1,90 min.

### 3.9 SYNTHESE VON (2R,3R,4S,5R)-2-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YLAMINO}-5-HYDROXYMETHYL-TETRAHYDRO-FURAN-3,4-DIOL, BEISPIEL 127 (SIEHE SCHEMA 3, SCHRITT D):

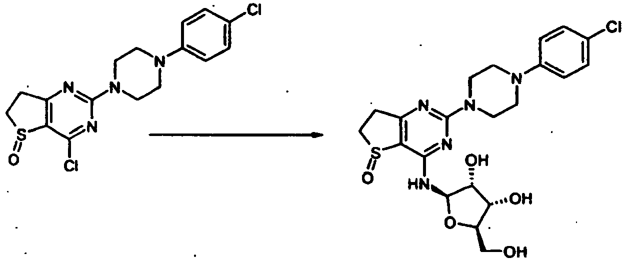

220 mg 4-Chlor-2-[4-(4-chlor-phenyl)-piperazin-1-yl]-6,7-dihydro-thieno[3,2-d]pyrimidin 5-oxid (siehe Beispiel 124), 414,9 mg 2,3-O-Isopropyliden-beta-D-ribofuranosylamin p-Toluolsulfonat und 395,0 µl Diisopropylethylamin werden in 4 ml Dioxan vorgelegt, in der Mikrowelle 0,3 Stunden auf 120° C und nochmal 0,6 Stunden bei 130°C erhitzt. Anschließend wird das Reaktionsgemisch mit Wasser und Essigester versetzt und extrahiert. Die organische Phase wird getrocknet und zur Trockene eingedampft. Das Produkt wird über präparative HPLC (Methode B) gereinigt. 7 mg des Produktes werden als Öl erhalten. HPLC-MS (Methode A): RT = 2.26 min.

### 3.10 SYNTHESE VON [5-({2-[4-(4-CHLOR-PHENYL)-PIPERAZIN-1-YL]-5-OXO-6,7-DIHYDRO-5H-5λ⁴-THIENO[3,2-d]PYRIMIDIN-4-YLAMINO}-METHYL)-THIOPHEN-2-YLMETHYL]-CARBAMIDSÄURE-tert-BUTYLESTER, BEISPIEL 101 (SIEHE SCHEMA 3)

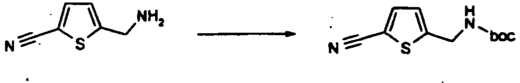

### 3.10.1 5-N-Boc-aminomethyl-2-cyanothiophen Hydrochlorid:

17,5 g 5-Aminomethyl-2-cyanothiophen Hydrochlorid (Bioorg. Med. Chem. Lett. 2002, 743) und 21,8 g Boc-anhydrid werden in 250 ml Chloroform portionsweise eingetragen. In die Suspension werden langsam 50 ml kaltes Wasser gegossen. 8 g NaOH (50%ig) und 25 ml Wasser werden bei Raumtemperatur langsam zugetropft. Die Suspension wird dann übernacht gerührt. Anschließend wird das Reaktionsgemisch mit Chloroform extrahiert. 42,10 g des Produkts werden als Öl erhalten.

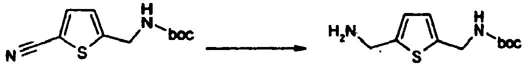

### 3.10.2 5-N-Boc-aminomethyl-2-aminomethylthiophen Hydrochlorid:

23,8 g 5-N-Boc-aminomethyl-2-cyanothiophen werden in 1 l Ethanol und 50 ml methanolischem Ammoniak vorgelegt, mit 40 g Raney-Nickel bei einem Druck von 40 bar hydriert. Der Katalysator wird abgesaugt, das Filtrat eingedampft. Der Rückstand wird in *tert*-Butylmethylether aufgenommen und unter Eiskühlung vorsichtig mit Isopropanol/HCl als Hydrochlorid gefällt. 32,8 g des Produkts werden als Pulver erhalten.

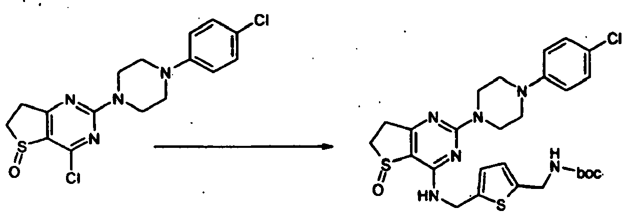

### 3.10.3 [5-({2-[4-(4-Chlor-phenyl)-piperazin-1-yl]-5-oxo-6,7-dihydro-5H-5λ⁴-thieno[3,2-d]pyrimidin-4-ylamino}-methyl)-thiophen-2-ylmethyl]-carbamidsäure-tert-butylester (Schema 3, Schritt D):

200 mg 4-Chlor-2-[4-(4-chlor-phenyl)-piperazin-1-yl]-6,7-dihydro-thieno[3,2-d]pyrimidin 5-oxid (siehe Beispiel 124), 436,6 mg 5-*N*-Boc-aminomethyl-2-aminomethylthiophen Hydrochlorid und 269,4 µl Diisopropylethylamin werden in 4 ml Dioxan vorgelegt, in der Mikrowelle 0,3 Stunden auf 120° C erhitzt. Anschließend wird das Reaktionsgemisch mit Wasser versetzt. Die ausgefallene Substanz wird abgesaugt und mit Wasser gewaschen. Das Produkt wird über semipräparative HPLC (Methode A) gereinigt. 130 mg des Produktes werden als Pulver erhalten. Analytische HPLC-MS, (Methode B): RT = 2.06 min.

### 3.11 SYNTHESE VON (5-AMINOMETHYL-THIOPHEN-2-YLMETHYL)-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-AMIN, BEISPIELE 122 (SIEHE SCHEMA 3)

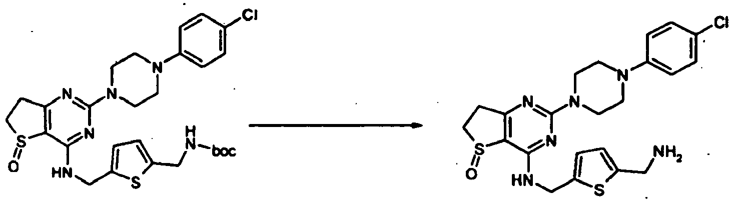

100 mg [5-({2-[4-(4-Chlor-phenyl)-piperazin-1-yl]-5-oxo-6,7-dihydro-5*H*-5λ⁴-thieno[3,2-*d*]pyrimidin-4-ylamino}-methyl)-thiophen-2-ylmethyl]-carbamidsäure-*tert*-butylester (siehe Beispiel 101) werden in 1 ml Dichlormethan suspendiert, 315 µl Trifluoressigsäure zugegeben und 2 Tage bei Raumtemperatur gerührt. Anschließend wird die Reaktionsmischung zur Trockene eingedampft. 134 mg des Produktes werden als Öl erhalten. Analytische HPLC-MS (Methode A): RT = 2.34 min.

### 3.12 SYNTHESE VON 5-({2-[4-(4-CHLOR-PHENYL)-PIPERAZIN-1-YL]-5-OXO-6,7-DIHYDRO-5H-5λ⁴-THIENO[3,2-d]PYRIMIDIN-4-YLAMINO}-METHYL)-2-METHYL-2H-PYRAZOL-3-OL, BEISPIEL 110 (SIEHE SCHEMA 3)

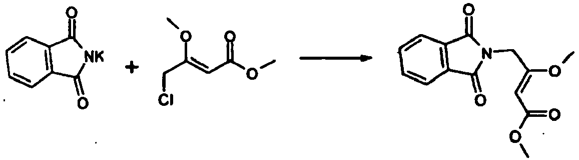

### 3.12.1 (Z)-4-(1,3-Dioxo-1,3-dihydro-isoindol-2-yl)-3-methoxy-but-2-ensäuremethylester:

18,5 Kaliumphtalimid werden in 50 ml DMF suspendiert, bei 60°C geheizt und 16,5 g 4-Chlor-3-methoxy-but-2-en-säuremethylester in 50 ml DMF zugegeben. Nach 2 Tagen wird das Reaktionsgemisch in 500 ml Wasser gegossen. Der entstandene Niederschlag wird abgesaugt und mit Wasser gewaschen. Der Rückstand wird in Essigester gelöst, getrocknet und die Lösung zur Trockene eingedampft. Der Rückstand wird mit Ether verrieben. 21,77 g des Produkts werden als Pulver erhalten.

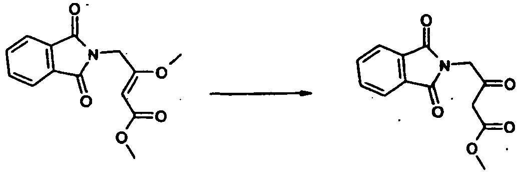

### 3.12.2 4-(1,3-Dioxo-1,3-dihydro-isoindol-2-yl)-3-oxo-buttersäuremethylester:

7,4 g konz. Schwefelsäure werden in 100 ml Methylenchlorid suspendiert und 21,0 g (Z)-4-(1,3-Dioxo-1,3-dihydro-isoindol-2-yl)-3-methoxy-2-en-buttersäuremethylester zugegeben. Die Suspension wird bei Raumtemperatur gerührt. Nach 12 Stunden wird das Reaktionsgemisch in 1 l Ether zugegeben und 30 Min gerührt. Der ausgefallene Niederschlag wird filtriert und mit Ether gewaschen. 16,69 g des Produkts werden als Pulver erhalten.

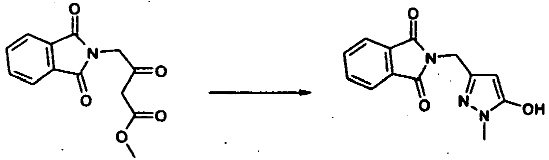

### 3.12.3 2-(5-Hydroxy-1-methyl-1H-pyrazol-3-ylmethyl)-isoindol-1,3-dion:

10,0 g 4-(1,3-Dioxo-1,3-dihydro-isoindol-2-yl)-3-oxo-buttersäuremethylester werden bei 60°C in 200 ml Ethanol gelöst und 1,94 g Methylhydrazin zugegeben. Das Reaktionsgemisch wird abgekühlt und bei Raumtemperatur weitergerührt. Nach 5 Stunden wird die ausgefallene Substanz abgesaugt und mit Ethanol gewaschen. 7,91 g des Produkts werden als Pulver erhalten.

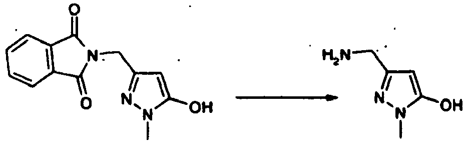

**3.12.4 5-Aminomethyl-2-methyl-2*H*-pyrazol-3-ol**: 7,88 g 2-(5-Hydroxy-1-methyl-1H-pyrazol-3-ylmethyl)-isoindol-1,3-dion werden in 150 ml Salzsäure (6 M) suspendiert und unter Rückfluß gerührt. Nach 4 Stunden wird die Reaktionsmischung abgekühlt und bei 0°C übernacht stehen gelassen. Die ausgefallene Substanz wird filtriert. Das Filtrat wird eingedampft und in Ether/Methanol kristallisiert. 4,9 g des Produkts werden als Pulver erhalten.

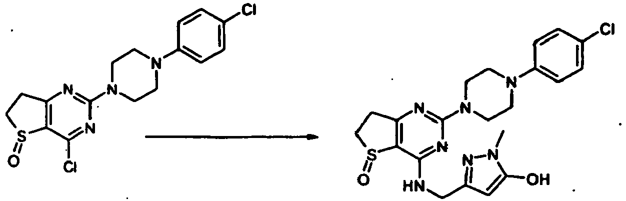

### 3.12.5 5-({2-[4-(4-Chlor-phenyl)-piperazin-1-yl]-5-oxo-6,7-dihydro-5H-5λ⁴-thieno[3,2-d]pyrimidin-4-ylamino}-methyl)-2-methyl-2H-pyrazol-3-ol (Schema 3, Schritt D):

200 mg 4-Chlor-2-[4-(4-chlor-phenyl)-piperazin-1-yl]-6,7-dihydro-thieno[3,2-d]pyrimidin 5-oxid (siehe Beispiel 124), 313,3 mg 5-Aminomethyl-2-methyl-2H-pyrazol-3-ol und 359,25 µl Diisopropylethylamin werden in 4 ml Dioxan vorgelegt, in der Mikrowelle 0,3 Stunden auf 130° C und nochmal 0,3 Stunden auf 130°C erhitzt. Anschließend wird das Reaktionsgemisch mit Wasser versetzt. Die ausgefallene Substanz wird abgesaugt und mit Wasser gewaschen. Das Produkt wird über semipräparative HPLC (Methode A) gereinigt. 130 mg des Produktes werden als Pulver erhalten. Analytische HPLC-MS (Methode B): RT = 1,61 min.

### 3.13 SYNTHESE VON {2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-(3H-IMIDAZO[4,5-c]PYRIDIN-2-YLMETHYL)-AMIN, BEISPIEL 104 (SIEHE SCHEMA 3)

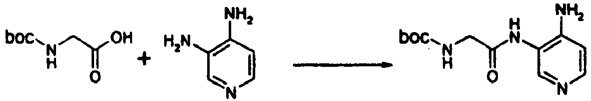

### 3.13.1 [(4-Amino-pyridin-3-ylcarbamoyl)-methyl]-carbamidsäure-tert-butylester:

unter Stickstoff werden 16,05 g N-Boc-glycin und 16,34 g Carbonyldiimidazol in 100 ml DMF gelöst und 15 Minuten bei Raumtemperatur gerührt. 10,0 g 3,4-Diaminopyridin und 22,16 ml N-Metylmorpholin werden zugegeben. Nach 2 Tagen wird der Reaktionsgemisch eingedampft und der Rückstand mit Wasser und Dichlormethan versetzt und extrahiert.

Die Wasser Phase wird eingedampft. Der Rohprodukt wird chromatographisch gereinigt (Kieselgel, CH₂Cl₂/EtOH 95/5 bis 60/40). 23,46 g des Produkts (70%ig) werden als Öl erhalten.

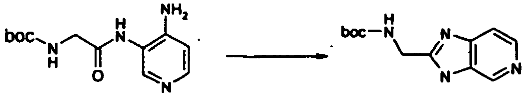

### 3.13.2 (3H-Imidazo[4,5-c]pyridin-2-ylmethyl)-carbamidsäure-tert-butylester

23,0 g [(4-Amino-pyridin-3-ylcarbamoyl)-methyl)-carbamidsäure-*tert*-butylester werden in 80 ml DMF suspendiert und 3,45 ml Eisessig zugegeben. Unter Argon wird der Reaktionsgemisch bei Rückfluß gerührt. Nach 4 Stunden wird die Suspension leicht abgekühlt und 3 ml Essigsäure werden zugegeben. Der Reaktionsgemisch wird bei Rückfluß gerührt. Nach 12 Stunden wird der Reaktionsgemisch eingedampft. Der Rohprodukt wird chromatographisch gereinigt (Kieselgel, CH₂Cl₂/EtOH 100/0 bis 70/30). 14,15 g des Produkts werden erhalten.

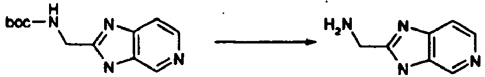

### 3.13.3 C-(3H-Imidazo[4,5-c]pyridin-2-yl)-methylamin:

14,15 g (3*H*-Imidazo[4,5-*c*]pyridin-2-ylmethyl)-carbamidsäure-*tert*-butylester werden in 100 ml ethanolische HCl suspendiert und bei Raumtemperatur gerührt. Der Reaktionsgemisch wird eingedampft. 12,8 g des Produkts werden erhalten. DC (Kieselgel, CH₂Cl₂/EtOH 80/20, Rf = 0,07).

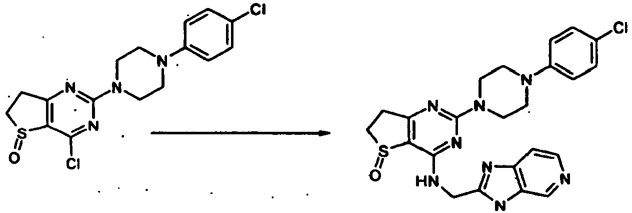

### 3.13.4 {2-[4-(4-Chlor-phenyl)-piperazin-1-yl]-5-oxo-6,7-dihydro-5H-5λ⁴-thieno[3,2-d]pyrimidin-4-yl}-(3H-imidazo[4,5-c]pyridin-2-ylmethyl)-amin (Schema 3, Schritt D):

200 mg 4-Chlor-2-[4-(4-chlor-phenyl)-piperazin-1-yl]-6,7-dihydro-thieno[3,2-d]primidin 5-oxid (siehe Beispiel 124), 346,22 mg C-(3H-Imidazo[4,5-*c*]pyridin-2-yl)-methylamin und 359,24 µl Diisopropylethylamin werden in 4 ml Dioxan vorgelegt, in der Mikrowelle 0,3 Stunden auf 120° C und noch mal 0,3 Stunden bei 120°C erhitzt. Anschließend wird das Reaktionsgemisch mit Wasser versetzt. Die ausgefallene Substanz wird abgesaugt und mit Wasser gewaschen. Das Produkt wird über semipräparative HPLC (Methode A). gereinigt. 50 mg des Produktes werden als Pulver erhalten. Analytische HPLC-MS (Methode B): RT = 1,55 min.

### 3.14 SYNTHESE VON [(R)-1-(6-CHLOR-1H-BENZOIMIDAZOL-2-YL)-2-METHOXYETHYL]-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-AMIN, BEISPIEL 106 (SIEHE SCHEMA 3)

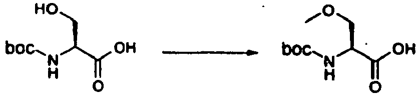

### 3.14.1 (S)-2-tert-Butoxycarbonylamino-3-methoxy-propionsäure:

Unter Stickstoff wird eine Mischung von 74 ml abs. THF und 6 ml abs. Methanol bei -70°C gekühlt und 2 g Natrium Hydrid (Dispersion 55-65%) zugegeben. Die Suspension wird 0,4 Stunde bei Raumtemperatur gerührt. Unter Stickstoff werden 2,05 g *N*-Boc-L-serin in 100 ml abs. THF suspendiert und 40 ml der vorbereitete Natriumethylat Lösung zugegeben.

Die Suspension wird 1 Stunde bei Raumtemperatur gerührt und 1 ml Methyliodid zugegegeben. Nach 1 Stunde wird der Rest der Natriumethylat Lösung sowie weiter 2 ml Methyliodid zugegeben. Nach 18 Stunden wird das Reaktionsgemisch zur Trockene eingedampft. Der Rückstand wird mit Wasser und Äther gewaschen und extrahiert. Die Wasser Phase wird sauer gestellt und mit Essigester und Dichlormethan extrahiert. 0,52 g des Produkts werden erhalten.

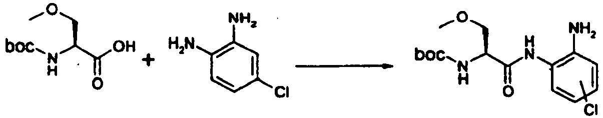

### 3.14.2 [(S)-1-(2-Amino-4/5-chlor-phenylcarbamoyl)-2-methoxy-ethyl]-carbamidsäure-tert-butylester:

Unter Stickstoff werden 6,65 g (S)-2-*tert*-Butoxycarbonylamino-3-methoxy-propionsäure und 4,33 g 4-Chlor-1,2-phenylendiamin in 100 ml abs. THF suspendiert und bei 5°C abgekühlt. 6,26 g Dicyclohexylcarbodiimid in 50 ml abs. THF werden langsam zugetropft. Das Reaktionsgemisch wird 0,5 Stunde bei 5°C und dann bei Raumtemperatur gerührt. Nach 18 Stunden wird das ausgefallene Dicyclohexylharnstoff abfiltriert und das Filtrat zur Trockene eingedampft. Das Rohprodukt wird chromatographisch gereinigt (Kiesegel, Petroläther dann Petroläther/Essigester (90/10), (80/20), (50/50) und (30/70)). 1,85 g des Produkts werden als Isomergemisch erhalten.

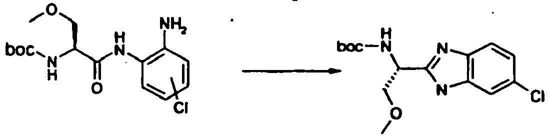

### 3.14,2 [(R)-1-(6-Chlor-1H-benzoimidazol-2-yl)-2-methoxy-ethyl]-carbamidsäure-tert-butylester:

5,0 g ((S)-1-(2-Amino-4/5-chlor-phenylcarbamoyl)-2-methoxy-ethyl]-carbamidsäure-tert-butylester als Isomergemisch werden in 60 ml Eisessig suspendiert und bei 55°C gerührt. Nach 4 Stunden wird das Eisessig bis auf ¼ des Volumens abdestilliert. Die Suspension wird kalt gestellt und mit Ammoniak (2N) basisch gestellt (pH 8). Das ausgefallene Produkt wird abfiltriert, mit Wasser gewaschen und mit 40 ml

Ethanol/Essigester/Äther/Petroläther (1/1/1/1) verrührt. 3,95 g des Produkts werden als Pulver erhalten.

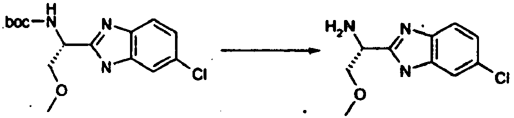

### 3.14.3 (R)-1-(6-Chlor-1H-benzoimidazol-2-yl)-2-methoxy-ethylamin:

1,4 g [(R)-1-(6-Chlor-1H-benzoimidazol-2-yl)-2-methoxy-ethyl]-carbamidsäure-*tert-*butylester werden in 10 ml Dichlormethan suspendiert und unter Eisbadkühlung mit 3,5 ml Trifuoressigsäure versetzt. Das Reaktionsgemisch wird bei Raumtemperatur gerührt und nach 2 Stunden mit 50 ml Dichlormethan und 200 ml Wasser versetzt und extrahiert. Die Wasser Phase wird mit 50 ml Ether extrahiert, mit Ammoniak (konz.) basisch gestellt (pH 9,5) und mit 150 ml Essigester extrahiert. Der Rückstand wird in Ether verrieben. 450 mg des Produkts werden als Pulver erhalten.

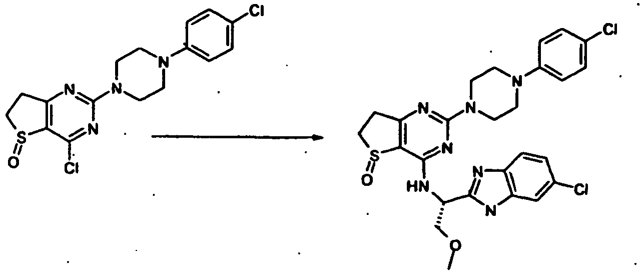

### 3.14.4 [(R)-1-(6-Chlor-1H-benzoimidazol-2-yl)-2-methoxy-ethyl]-{2-[4-(4-chlor-phenyl)-piperazin-1-yl]-5-oxo-6,7-dihydro-5H-5λ⁴-thieno[3,2-d]pyrimidin-4-yl}-amin (Schema 3, Schritt D):

200 mg 4-Chlor-2-[4-(4-chlor-phenyl)-piperazin-1-yl]-6,7-dihydro-thieno[3,2-d]pyrimidin 5-oxid (siehe Beispiel 124), 235 mg (R)-1-(6-Chlor-1H-benzoimidazol-2-yl)-2-methoxyethylamin und 179,62 µl Diisopropylethylamin werden in 4 ml Dioxan vorgelegt, in der Mikrowelle 0,3 Stunden auf 120° C erhitzt. Anschließend wird das Reaktionsgemisch mit Wasser und Dichlormethan versetzt und extrahiert. Das Produkt wird über semipräparative HPLC (Methode A) gereinigt. 113 mg des Produktes werden als Pulver erhalten. Analytische HPLC-MS, (Methode B): RT = 1,88 min.

### 3.15 SYNTHESE (1R,2R)-1-(6-CHLOR-1H-BENZOIMIDAZOL-2-YL)-1-{2-[4-(4-CHLORPHENYL)-PIPERAZIN-1-YL]-5-OXO-6,7-DIHYDRO-5H-5λ⁴-THIENO[3,2-d]PYRIMIDIN-4-YLAMINO}-PROPAN-2-OL, BEISPIEL 107 (SIEHE SCHEMA 3)

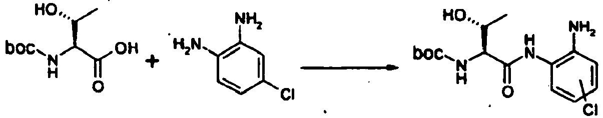

### 3.15.1 [(1S,2R)-1-(2-Amino-4/5-chlor-phenylcarbamoyl)-2-hydroxy-propyl]-carbamidsäure-tert-butylester:

3,48 g (L)-Threonin und 2,15 g 4-Chlor-1,2-phenylendiamin werden in 50 ml THF suspendiert, 6,27 ml Triethylamin und 5,14 g O-(Benzotriazol-1-yl-)-N,N,N',N'-tetramethyluroniumtetrafluoroborat (TBTU) werden zugegeben. Das Reaktionsgemisch wird übernacht bei Raumtemperatur gerührt.und zur Trockene eingedampft. Der Rückstand wird in 150ml Ethylacetat gelöst und mit 2x50 ml Zitronsäure (5%) und 2x50 ml NaOH (2M) gewaschen. Das Rohprodukt wird in tert-Butylmethylether/Petrolether umkristallisiert. 1,93 g des Produkts werden als Isomergemisch erhalten.

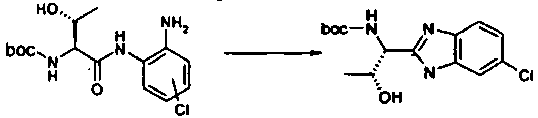

### 3.15.2 [(1R,2R)-1-(6-Chlor-1H-benzoimidazol-2-yl)-2-hydroxy-propyl]-carbamidsäure-tert-butylester:

1,80 g [(1S,2R)-1-(2-Amino-4/5-chlor-phenylcarbamoyl)-2-hydroxy-propyl]-carbamidsäure-tert-butylester als Isomergemisch werden in 15 ml Eisessig suspendiert und bei 60°C gerührt. Nach 1 Stunde wird das Reaktionsgemisch in einer NaOH Lösung (10 g NaOH in 100 ml Wasser) zugegeben. Das Rohprodukt wird mit Ethylacetat extrahiert und chromatographisch gereinigt (Kieselgel, Ethylacetat/Petrolether (50/50)). 1,7 g des Produkts werden als Schaum erhalten.

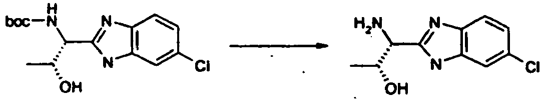

### 3.15.3 (1R,2R)-1-Amino-1-(6-chlor-1H-benzoimidazol-2-yl)-propan-2-ol:

1,65 g [(1R,2R)-1-(6-Chlor-1*H*-benzoimidazol-2-yl)-2-hydroxy-propyl]-carbamidsäure-*tert-*butylester werden in 5 ml Dichlormethan suspendiert und mit 5,0 ml Trifuoressigsäure versetzt. Das Reaktionsgemisch wird bei Raumtemperatur gerührt und nach 3 Stunden mit Wasser versetzt. Die Wasser Phase wird mit NaOH basisch gestellt und das Produkt mit tert-Butylmethylether extrahiert. 1,04 g des Produkts werden als Pulver erhalten.

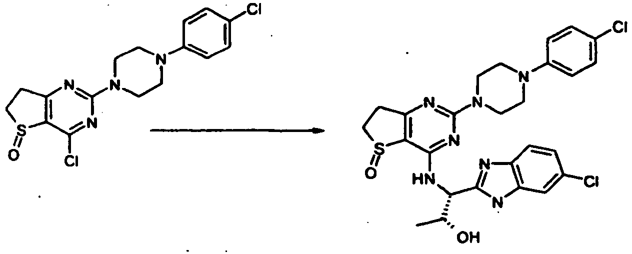

### 3.15.4 (1R,2R)-1-(6-Chlor-1H-benzoimidazol-2-yl)-1-{2-[4-(4-chlor-phenyl)-piperazin-1-yl]-5-oxo-6,7-dihydro-5H-5λ⁴-thieno[3,2-d]pyrimidin-4-ylamino}-propan-2-ol (Schema 3, Schritt D):

200 mg 4-Chlor-2-[4-(4-chlor-phenyl)-piperazin-1-yl]-6,7-dihydro-thieno[3,2-d]pyrimidin 5-oxid (siehe Beispiel 124), 353,41 mg (R)-1-(6-Chlor-1*H*-benzoimidazol-2-yl)-2-methoxyethylamin und 179,62 µl Diisopropylethylamin werden in 4 ml Dioxan vorgelegt, in der Mikrowelle 2x0,3 Stunden auf 120° C erhitzt. Anschließend wird das Reaktionsgemisch mit Wasser und Dichlormethan versetzt und extrahiert. Das Produkt wird über semipräparative HPLC (Methode A). gereinigt. 150 mg des Produktes werden als Pulver erhalten. Analytische HPLC-MS (Methode B): RT = 1,90 min.

### 3.16 SYNTHESE VON C-((R)-1-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-PYRROLIDIN-2-YL)-METHYLAMIN TRIFLUORACETAT, BEISPIEL 224 (SIEHE SCHEMA 3, SCHRITT D)

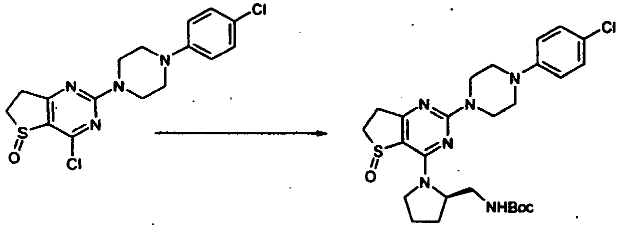

### 3.16.1 ((R)-1-{2-[4-(4-Chlor-phenyl)-piperazin-1-yl]-5-oxo-6,7-dihydro-5H-5λ⁴-thieno[3,2-d]pyrimidin-4-yl}-pyrrolidin-2-ylmethyl)-carbamidsäure-tert-butylester, Beispiel 219 (Schema 3, Schritt D):

200 mg 4-Chlor-2-[4-(4-chlor-phenyl)-piperazin-1-yl]-6,7-dihydro-thieno[3,2-*d*]pyrimidin 5-oxid (siehe Beispiel 124), 314 mg (R)-2-N-BOC-aminomethylpyrrolidin und 179,6 µl Diisopropylethylamin werden in 4 ml DMF vorgelegt, in der Mikrowelle 0,3 Stunden auf 120° C erhitzt. Anschließend wird das Reaktionsgemisch mit Trifuoressigsäure angesäuert und mit Wasser versetzt. Das Produkt wird über semipräparative HPLC (Methode A) getrennt. 68 mg Produkt (Beispiel 219) werden als Pulver erhalten. Analytische HPLC-MS (Methode B): RT = 2,0 min.

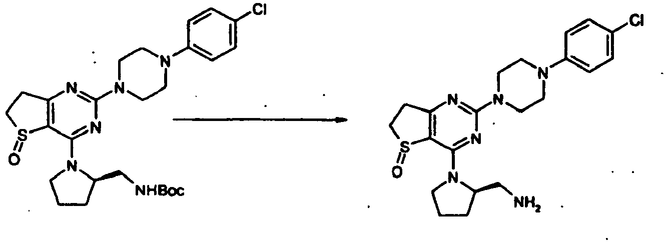

### 3.16.2 C-(((R)-1-{2-[4-(4-Chlor-phenyl)-piperazin-1-yl]-5-oxo-6,7-dihydro-5H-5λ⁴-thieno[3,2-d]pyrimidin-4-yl}-pyrrolidin-2-yl)-methylamin Trifluoracetat:

38 mg ((R)-1-{2-[4-(4-Chlor-phenyl)-piperazin-1-yl]-5-oxo-6,7-dihydro-5*H*-5λ⁴-thieno[3,2-*d*]pyrimidin-4-yl}-pyrrolidin-2-ylmethyl)-carbamidsäure-*tert*-butylester werden in 1 ml Dichlormethan suspendiert und 86 µl Trifuoressigsäure zugegeben. Das Reaktionsgemisch wird bei Raumtemperatur gerührt und nach 12 Stunden zur Trockene eingedampft. Die Diastereomere werden über semipräparative HPLC (Methode A) getrennt. 7,6 mg des Diastereomers 1 (Beispiel 224) und 8,3 mg des Diastereomers 2 werden als Trifluoracetate erhalten. Analytische HPLC-MS (Methode B): Diastereomer 1: RT = 1,56 min; Diastereomer 2: RT = 1,61 min.

### 3.17 SYNTHESE VON C-((S)-1-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-PYRROLIDIN-2-YL)-METHYLAMIN, BEISPIELE 225 UND 226 (SIEHE SCHEMA 3, SCHRITT D)

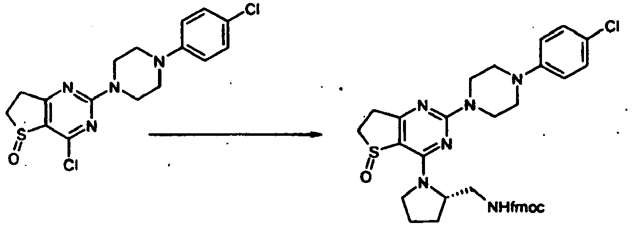

### 3.17.1 ((S)-1-{2-[4-(4-Chlor-phenyl)-piperazin-1-yl]-5-oxo-6,7-dihydro-5H-5λ⁴-thieno[3,2-d]pyrimidin-4-yl}-pyrrolidin-2-ylmethyl)-carbonsäure-9H-fluoren-9-ylmethylester (Schema 3, Schritt D):

3,0 g 4-Chlor-2-[4-(4-chlor-phenyl)-piperazin-1-yl]-6,7-dihydro-thieno[3,2-*d*]pyrimidin 5-oxid (siehe Beispiel 124), 2,8 g (S)-2-N-FMOC-aminomethylpyrrolidin und 2,7 ml Diisopropylethylamin werden in 24 ml DMF vorgelegt. Das Reaktionsgemisch wird in 3 geteilt und in der Mikrowelle 0,3 Stunden auf 120° C erhitzt. Anschließend wird das Reaktionsgemisch mit Dichlormethan verdünnt und filtriert. Das Filtrat wird zur Trockene eingedampft. 6,6 g Produkt werden als Öl erhalten.

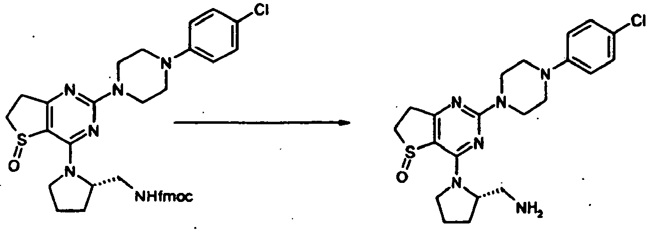

### 3.17.2 C-((S)-1-{2-[4-(4-Chlor-phenyl)-piperazin-1-yl]-5-oxo-6,7-dihydro-5H-5λ⁴-thieno[3,2-d]pyrimidin-4-yl}-pyrrolidin-2-yl)-methylamin Trifluoracetat (siehe Schema 3):

6,6 g ((S)-1-{2-[4-(4-Chlor-phenyl)-piperazin-1-yl]-5-oxo-6,7-dihydro-5*H*-5λ⁴-thieno[3,2-*d*]pyrimidin-4-yl}-pyrrolidin-2-ylmethyl)-carbonsäure-9*H*-fluoren-9-ylmethylester werden in 80 ml Dichlormethan suspendiert und 5,5 ml Piperidin zugegeben. Das Reaktionsgemisch wird 5 Stunden bei Raumtemperatur gerührt und anschießend mit Wasser und einer gesättigten NaCl Lösung versetzt. Die organische Phase wird getrocknet und zur Trockene eingedampft. Die Diastereomere werden über semipräparative HPLC (Methode B) getrennt. 880 mg des Diastereomers 1 (Beispiel 225) und 880 mg des Diastereomers 2 (Beispiel 226) werden als Trifluoracetate erhalten. Analytische HPLC-MS (Methode D): Diastereomer 1: RT = 1,07 min; Diastereomer 2: RT = 1.12 min.

### 3.18 SYNTHESE VON (2S,4R)-4-AMINO-1-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-PYRROLIDIN-2-CARBONSÄUREMETHYLESTER TRIFLUORACETAT, BEISPIELE 229 UND 230 (SIEHE SCHEMA 3, SCHRITT D)

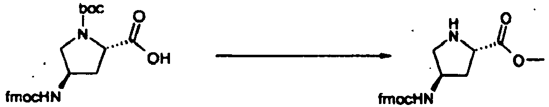

### 3.18.1 (2S,4R)-4-(9H-Fluoren-9-ylmethoxycarbonylamino)-pyrrolidin-2-carbonsäuremethylester Sulfat:

850 mg (2S,4R)-4-(9*H*-Fluoren-9-ylmethoxycarbonylamino)-pyrrolidin-1,2-dicarbonsäure-1-tert-butyl-ester und 60 µl Schwefelsäure werden in 12 ml abs. Methanol vorgelegt. Das eaktionsgemisch wird dann 48 Stunden unter Rückfluss gerührt und anschließend zur Trockene eingedampft. 1,0 g Produktwird als Schaum erhalten. Analytische HPLC-MS (Methode B): RT = 1,80 min.

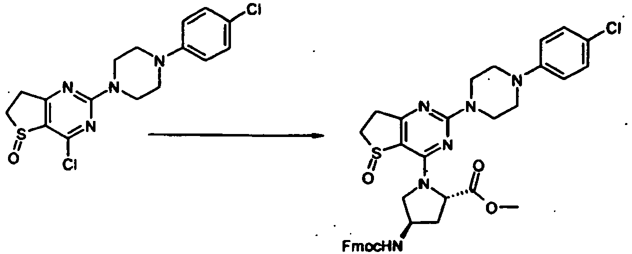

### 3.18.2 (2S,4R)-1-{2-[4-(4-Chlor-phenyl)-piperazin-1-yl]-5-oxo-6,7-dihydro-5H-5λ⁴-thieno[3,2-d]pyrimidin-4-yl}-4-(9H-fluoren-9-ylmethoxycarbonylamino)-pyrrolidin-2-carbonsäuremethylester Trifluoracetat (Schema 3, Schritt D):

280 mg 4-Chlor-2-[4-(4-chlor-phenyl)-piperazin-1-yl]-6,7-dihydro-thieno[3,2-*d*]pyrimidin 5-oxid (siehe Beispiel 124), 600 mg (25,4R)-4-(9*H*-Fluoren-9-ylmethoxycarbonylamino)-pyrrolidin-2-carbonsäuremethylester und 450 µl Diisopropylethylamin werden in 3 ml Dioxan vorgelegt und in der Mikrowelle 0,3 Stunden auf 120° C erhitzt. Anschließend wird das Reaktionsgemisch mit Wasser versetzt. Der ausgefallene Niederschlag wird abgesaugt und über präparative HPLC (Methode B) gereinigt. 200 mg Produkt werden als Trifluoracetat erhalten. Analytische HPLC (Methode B): RT = 3.6 min.

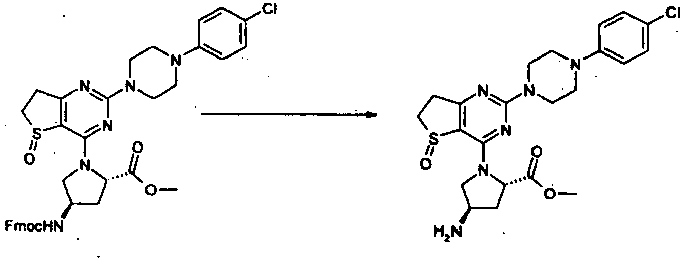

### 3.18.3 Synthese von (2S,4R)-4-Amino-1-{2-[4-(4-chlor-phenyl)-piperazin-1-yl]-5-oxo-6,7-dihydro-5H-5λ⁴-thieno[3,2-d]pyrimidin-4-yl}-pyrrolidin-2-carbonsäure-methylester Trifluoracetat:

200 mg (2S,4R)-1-{2-[4-(4-Chlor-phenyl)-piperazin-1-yl]-5-oxo-6,7-dihydro-5*H*-5λ⁴-thieno[3,2-*d*]pyrimidin-4-yl}-4-(9*H*-fluoren-9-ylmethoxycarbonylamino)-pyrrolidin-2-carbonsäuremethylester werden in 5 ml Dichlormethan suspendiert und mit 300 µl Piperidin versetzt. Das Reaktionsgemisch wird bei Raumtemperatur gerührt und nach 4 Stunden zur Trockene eingedampft. Die Diastereomere werden über präparative HPLC (Methode B) getrennt. 52,8 mg des Diastereomers 1 (Beispiel 229) und 49,5 mg des Diastereomers 2 (Beispiel 230) werden als Trifluoracetat erhalten. Analytische HPLC (Methode B): Diastereomer 1: RT = 2,79 min; Diastereomer 2: RT = 2.83 min.

### 3.19 SYNTHESE VON (R)-2-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YLAMINO}-PENTAN-1-OL TRIFLUORACETAT, BEISPIELE.143 UND 144 (SIEHE SCHEMA 3, SCHRITT D):

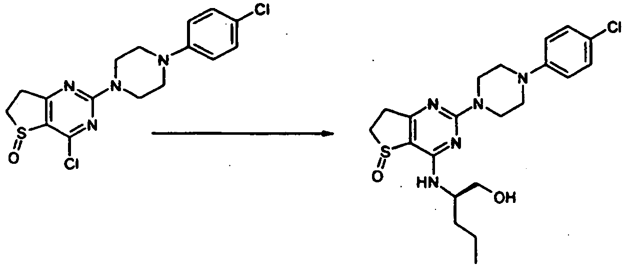

400 mg 4-Chlor-2-[4-(4-chlor-phenyl)-piperazin-1-yl]-6,7-dihydro-thieno[3,2-*d*]pyrimidin 5-oxid (siehe Beispiel 124), 321,9 mg (R)-(-)-2-Amino-1-pentanol und 362,3 µl Diisopropylethylamin werden in 10 ml Dioxan vorgelegt, in der Mikrowelle 0,3 Stunden auf 120° C erhitzt. Anschließend wird das Reaktionsgemisch mit Wasser versetzt, der ausgefallene Niederschlag abgesaugt, gewaschen und getrocknet. Die Diastereomere werden über semipräparative HPLC (Methode B) getrennt. 180 mg des Diastereomers 1 (Beispiel 143) und 110 mg des Diastereomers 2 (Beispiel 144) werden als Trifluoracetate erhalten. Analytische HPLC-MS (Methode D): Diastereomer 1: RT = 1,21 min; Diastereomer 2: RT = 1,22 min.

### 3.20 SYNTHESE VON ((1R,2R)-2-BENZYLOXY-CYCLOPENTYL)-{2-[4-(4-CHLORPHENYL)-PIPERAZIN-1-YL]-5-OXO-6,7-DIHYDRO-5H-5λ⁴-THIENO[3,2-d]PYRIMIDIN-4-YL}-AMIN, BEISPIELE 61 (MISCHUNG VON DIASTEREOMEREN), 151 (DIASTEREOMER 1) UND 152 (DIASTEREOMER 2) (SIEHE SCHEMA 3, SCHRITT D):

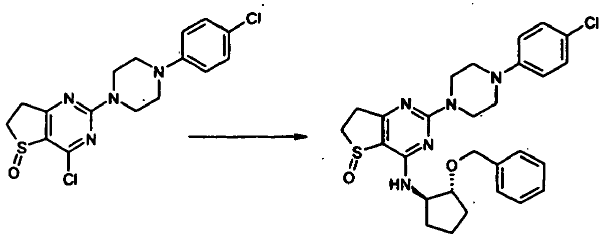

Ausgehend vom 4-Chlor-2-[4-(4-chlor-phenyl)-piperazin-1-yl]-6,7-dihydro-thieno[3,2-d]pyrimidin 5-oxid (siehe Beispiel 124) und (1 R,2R)-2-Benzyloxycyclopentylamin wird ((1R,2R)-2-Benzyloxy-cyclopentyl)-{2-[4-(4-chlor-phenyl)-piperazin-1-yl]-5-oxo-6,7-dihydro-5*H*-5λ⁴-thieno[3,2-*d*]pyrimidin-4-yl}-amin, wie bei Beispiel 124 beschrieben, hergestellt. Das Produkt wird über präparative HPLC (Methode A) gereinigt. Analytische HPLC-MS (Methode A): RT = 3.09 min.

Beide Diastereomeren können über chirale HPLC (Säule: Diacel IB, 250 x 4.6 mm, 5 µm, Fließmittel: (Hexan+Cyclohexylamin (0,2%))/EtOH (60/40), Flußrate: 1 ml/min) getrennt werden: Diastereomer 1: RT = 5,97 min (Beispiel 151); Diastereomer 2: RT = 7,92 min (Beispiel 152).

### 3.21 SYNTHESE VON (R)-2-{2-[4-(4-CHLOR-PHENYL)-PIPERAZIN-1-YL]-5-OXO-6,7-DIHYDRO-5H-5λ⁴-THIENO[3,2-d]-PYRIMIDIN-4-YLAMINO}-2,3-DIMETHYL-BUTAN-1-OL, BEISPIEL 153 (SIEHE SCHEMA 3):

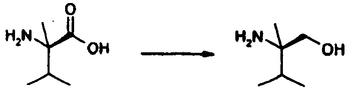

### 3.21.1 (R)-2-Amino-2,3-dimethyl-butan-1-ol:

Unter argon werden 140 mg Natriumborhohydrid in 4 ml Tetrahydrofuran vorgelegt, dann 200 mg H-α-Methyl-D-valinol zugegeben. Unter Eisbadkühlung werden 390 mg Iod in 2 ml THF langsam zugetropft. Das Reaktionsgemisch wird unter Rückfluss gerührt, nach 12 Stunden mit Methanol versetzt und anschliessend zur Trockene eingedampft. Der Rückstand wird in einer wässrigen KOH Lösung (20%) suspendiert und 2 Stunden bei Raumtemperatur gerührt. Das Produkt wird dann mit Dichlormethan extrahiert. Die organische Phase wird getrocknet und zur Trockene eingedampft. 160 mg Produkt werden als Öl erhalten.

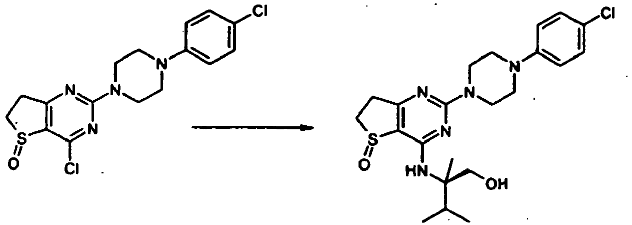

### 3.21.2 (R)-2-{2-[4-[4-(4-Chlor-phenyl)-piperazin-1-yl]-5-oxo-6,7-dihydro-5H-5λ⁴-thieno[3,2-d]pyrimidin-4-ylamino}-2,3-dimethyl-butan-1-ol (Schema 3, Schritt D):

Ausgehend vom 4-Chlor-2-[4-(4-chlor-phenyl)-piperazin-1-yl]-6,7-dihydro-thieno[3,2-d]pyrimidin 5-oxid (siehe Beispiel 124) und (R)-2-Amino-2,3-dimethyl-butan-1-ol wird (R)-2-{2-[4-(4-Chlor-phenyl)-piperazin-1-yl]-5-oxo-6,7-dihydro-5*H*-5λ⁴-thieno[3,2-*d*]pyrimidin-4-ylamino}-2,3-dimethyl-butan-1-ol als Mischung von Diastereomeren hergestellt.

Analytische HPLC-MS (Methode D): RT = 1,27 min (Beispiel 153).

### 3.22 SYNTHESE VON (R)-3-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YLAMINO}-PYRROLIDIN-1-CARBONSÄURE-tert-BUTYLESTER, BEISPIEL 170 UND {2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-(R)-PYRROLIDIN-3-YL-AMIN, BEISPIELE 171 UND 172 (SIEHE SCHEMA 3, SCHRITT D):

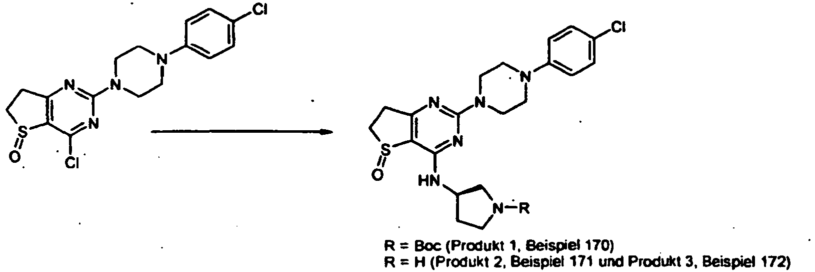

400 mg 4-Chlor-2-[4-(4-chlor-phenyl)-piperazin-1-yl]-6,7-dihydro-thieno[3,2-*d*]pyrimidin 5-oxid (siehe Beispiel 124), 320 mg (R)-(+)-1-BOC-3-Aminopyrrolidin und 370 µl Diisopropylethylamin werden in 4 ml Dioxan vorgelegt, in der Mikrowelle 0,3 Stunden auf 120° C erhitzt. Anschließend wird das Reaktionsgemisch mit Wasser versetzt. Der ausgefallene Niederschlag abgesaugt, gewaschen und getrocknet. 80 mg des Produktes 1 (Beispiel 170) werden als Mischung von Diastereomeren erhalten. Analytische HPLC-MS (Methode D): RT = 1,39 min.

Produkt 1 wird in 2 ml Dichlormethan suspendiert, dann 1,5 ml Trifluoressigsäure zugegeben. Das Reaktionsgemisch wird 2 Stunden bei Raumtemperatur gerührt und dann zur Trockene eingedampft. Die Diastereomeren werden über präparative HPLC (Methode B) getrennt. 96 mg des Diastereomers 1 (Beispiel 171) und 85 mg des Diastereomers 2 (Beispiel 172) werden als Trifluoracetate erhalten. Analytische HPLC (Methode B): Diastereomer 1: RT = 2.62 min; Diastereomer 2: RT = 2.66 min.

### 3.23 SYNTHESE VON 4-((R)-2-{2-[4-(4-CHLOR-PHENYL)-PIPERAZIN-1-YL]-5-OXO-6,7-DIHYDRO-5H-5Λ⁴-THIENO[3,2-D]PYRIMIDIN-4-YLAMINO}-3-HYDROXY-PROPYL)-PHENOL, BEISPIEL 175 (SIEHE SCHEMA 3)

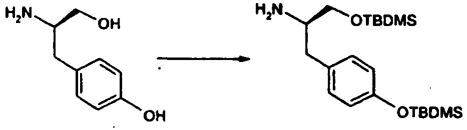

### 3.23.1 (R)-1-(tert-Butyl-dimethyl-silanyloxymethyl)-2-[4-(tert-butyl-dimethyl-silanyloxy)-phenyl]-ethylamin:

100 mg D-Tyrosinol Hydrochlorid werden in 2 ml Dimethylformamid vorgelegt, dann 135 mg Imidazol zugegeben. Das Reaktionsgemisch wird 30 Minuten bei Raumtemperatur gerührt. Unter Eisbadkühlung werden dann 187 mg *tert*-Butyldimethylchlorsilan zugegeben. Das Reaktionsgemisch wird 4 Stunden bei Raumtemperatur gerührt und anschließend mit Wasser und Diethylether versetzt. Die organische Phase wird mit einer gesättigtem NaCl Lösung gewaschen, getrocknet und zur Trockene eingedampft. 208 mg des Produkteswerden als Öl erhalten. Analytische HPLC-MS (Methode D): RT = 1,76 min.

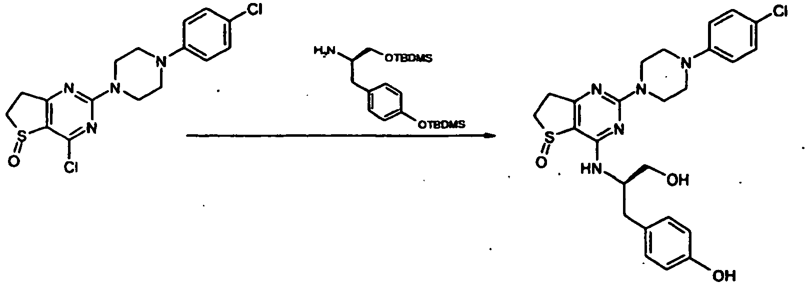

### 3.23.2 4-((R)-2-{2-[4-(4-Chlor-phenyl)-piperazin-1-yl]-5-oxo-6,7-dihydro-5H-5λ⁴-thieno[3,2-d]pyrimidin-4-ylamino}-3-hydroxy-propyl)-phenol (Schema 3, Schritt D):

350 mg 4-Chlor-2-[4-(4-chlor-phenyl)-piperazin-1-yl]-6,7-dihydro-thieno[3,2-*d*]pyrimidin 5-oxid (siehe Beispiel 124), 600 mg (R)-1-(*tert*-Butyl-dimethyl-silanyloxymethyl)-2-[4-(tert-butyl-dimethyl-silanyloxy)-phenyl]-ethylamin und 350 µl Diisopropylethylamin werden in 4 ml Dioxan vorgelegt, in der Mikrowelle 0,2 Stunden auf 120° C erhitzt. Anschließend wird das Reaktionsgemisch zur Trockene eingedampft. Der Rückstand wird in Wasser/Acetonitril/Trifluoressigsäure suspendiert und 3 Stunden bei Raumtemperatur gerührt. Das Reaktionsgemisch wird zur Trockene eingedampft und die Diastereomeren über präparative HPLC (Methode D) getrennt. Analytische HPLC-MS (Methode E): Diastereomer 1: RT = 1.20 min; Diastereomer 2: RT = 1.25 min.

Die Diastereomer 1 Chromatographiefraktionen werden vereint, der Acetonitril eingedampft und 600 µl Trifluoressigsäure zugegeben. Das Reaktionsgemisch wird 3 Stunden bei Raumtemperatur gerührt, mit Ammoniak basisch gestellt und mit Dichlormethan versetzt. Die organische Phase wird getrocknet und zur Trockene eingedampft. 145 mg des Produktes (Beispiel 175) werden als Feststoff erhalten. Analytische HPLC-MS (Methode E): RT = 0.60 min

### 3.24 SYNTHESE VON (R)-2-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YLAMINO}-3-FURAN-3-YL-PROPAN-1-OL, BEISPIELE 206 UND 207 (SIEHE SCHEMA 3)

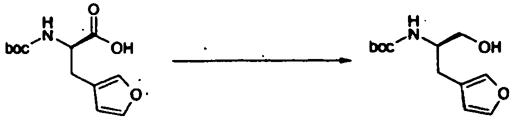

### 3.24.1 ((R)-1-Furan-3-ylmethyl-2-hydroxy-ethyl)-cabonsäure-tert-butylester:

Ausgehend von (R)-2-Amino-3-furan-3-yl-propionsäure (DE3829451) wird (R)-2-*tert-*Butoxycarbonylamino-3-furan-3-yl-propionsäure hergestellt. Unter Argon werden 420 mg (R)-2-*tert*-Butoxycarbonylamino-3-furan-3-yl-propionsäure in 2,5 ml Dimethoxyethan suspendiert. Das Reaktionsgemisch wird auf -30 °C gekühlt. 220 µl N-Methylmorpholin werden zugegeben und anschießend 240 µl Isobutylchlorformiat in 1,5 ml Dimethoxyethan zugetropft. Das Reaktionsgemisch wird auf - 5°C kommen gelassen und der Niederschlag schnell filtriert. Das Filtrat wird auf -15°C gekühlt. 100 mg Natriumborohydrid und einige Tropfen Wasser werden zugegeben. Das Reaktionsgemisch wird 1 Stunde bei Raumtemperatur gerührt und zur Trockene eingedampft. Der Rückstand wird in Essigester suspendiert. Das Reaktionsgemisch wird getrocknet und zur Trockene eingedampft. 370 mg des Produktswerden als Feststoff erhalten. Analytische HPLC-MS (Methode D): RT = 1,21 min.

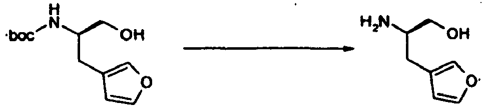

### 3.24.2 (R)-2-Amino-3-furan-3-yl-propan-1-ol Trifluoracetat:

970 mg ((R)-1-Furan-3-ylmethyl-2-hydroxy-ethyl)-cabonsäure-tert-butylester werden in 3 ml Dichlormethan vorgelegt und 3 ml Trifluoressigsäure zugegeben. Das Reaktionsgemisch wird 5 Stunden bei Raumtemperatur gerührt und anschließend zur Trockene eingedampft. 830 mg des Produkteswerden als Öl erhalten. Analytische HPLC-MS (Methode D): RT = 0,28 min.

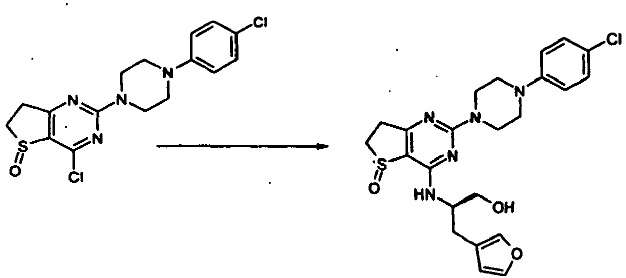

### 3.24.3 (R)-2-{2-[4-(4-Chlor-phenyl)-piperazin-1-yl]-5-oxo-6,7-dihydro-5H-5λ⁴-thieno[3,2-d]pyrimidin-4-ylamino}-3-furan-3-yl-propan-1-ol (Schema 3, Schritt D):

Ausgehend von 4-Chlor-2-[4-(4-chlor-phenyl)-piperazin-1-yl]-6,7-dihydro-thieno[3,2-*d*]pyrimidine 5-oxid (siehe Beispiel 124, 3.1.3) und (R)-2-Amino-3-furan-3-yl-propan-1-ol Trifluoracetat werden beide Diastereomeren von (R)-2-{2-[4-(4-Chlor-phenyl)-piperazin-1-yl]-5-oxo-6,7-dihydro-5*H*-5λ⁴-thieno[3,2-*d*]pyrimidin-4-ylamino}-3-furan-3-yl-propan-1-ol wie bei Beispiel 124 (siehe 3.1.4) beschrieben, hergestellt. Die Diastereomeren werden über präparative HPLC (Methode B) getrennt. Analytische HPLC-MS (Methode D):
Diastereomer 1: RT = 1,23 min (Beispiel 206); Diastereomer 2: RT = 1,24 min (Beispiel 207).

### 3.25 SYNTHESE VON (1-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-PYRROLIDIN-2-YLMETHYL)-DIMETHYL-AMIN, BEISPIEL 223 (SIEHE SCHEMA 3, SCHRITT D)

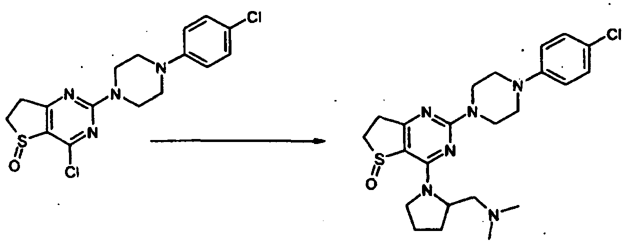

Ausgehend von 4-Chlor-2-[4-(4-chlor-phenyl)-piperazin-1-yl]-6,7-dihydro-thieno[3,2-d]pyrimidine 5-oxid (siehe Beispiel 124, 3.1.3) und Dimethyl-pyrrolidin-2-ylmethyl-amin (Chem. Pharm. Bull. 1970, 1731) wird ein Stereoisomerengemisch von (1-{2-[4-(4-Chlorphenyl)-piperazin-1-yl]-5-oxo-6,7-dihydro-5*H*-5λ⁴-thieno[3,2-*d*]pyrimidin-4-yl}-pyrrolidin-2-ylmethyl)-dimethyl-amin, wie bei Beispiel 124 (siehe 3.1.4) beschrieben, hergestellt. Die Diastereomeren werden über semipräparative HPLC (Methode A) getrennt. Analytische HPLC-MS (Methode A): Diastereomer 1: RT = 2,29 min; Diastereomer 2: RT = 2.38 min (Beispiel 223). Beispiel 223 wird als Racemat erhalten.

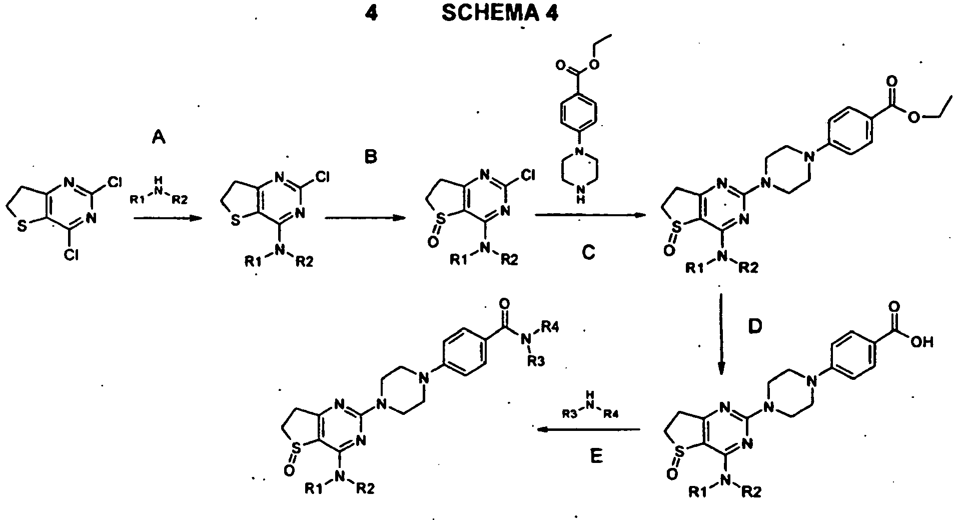

### 4.1 SYNTHESE VON 4-{4-[4-(3-CHLOR-PHENYLAMINO)-5-OXO-6,7-DIHYDRO-5H-5λ⁴-THIENO[3,2-d]PYRIMIDIN-2-YL]-PIPERAZIN-1-YL}-N-METHYL-N-(1-METHYLPIPERIDIN-4-YL)-BENZAMID TRIFLUORACETAT, BEISPIEL 11 (SIEHE SCHEMA 4)

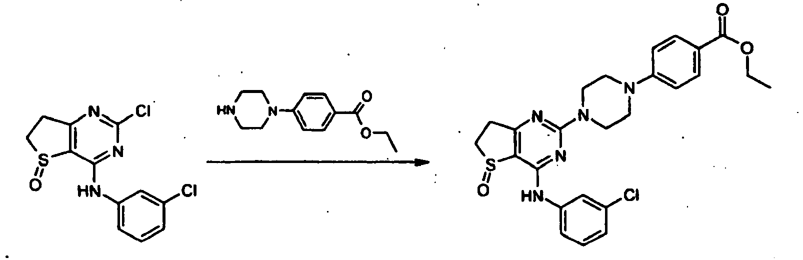

### 4.1.1 4-{4-[4-(3-Chlor-phenylamino)-5-oxo-6,7-dihydro-5H-5λ⁴-thieno[3,2-d]pyrimidin-2-yl]-piperazin-1-yl}-benzoesäureethylester (Schema 4, Schritt C):

5,44 g (2-Chlor-5-oxo-6,7-dihydro-5H-5λ⁴-thieno[3,2-*d*]pyrimidin-4-yl)-(3-chlor-phenyl)-amin (hergestellt aus 2,4-Dichlor-6,7-dihydro-thieno[3,2-d]pyrimidin und 3-Chloranilin wie bei Beispiel 28 beschrieben, siehe Schema 2), 8,00 g 4-Piperazin-1-yl-benzoesäure-ethylester und 4,80 ml Diisopropylethylamin werden in 54 ml Dioxan vorgelegt, dann in der Mikrowelle 0,7 Stunden auf 160° C erhitzt. Anschließend wird das Reaktionsgemisch abgekühlt und mit Wasser versetzt. Ausgefallener Niederschlag wird abgesaugt und mit Petrolether/Ethylacetat 1:1 verrührt, abgesaugt und getrocknet. 6,90 g des Produkts werden als Pulver erhalten.

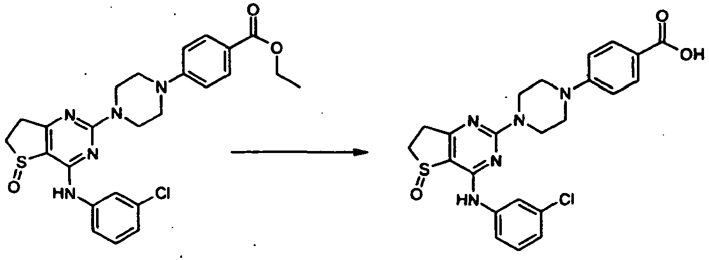

### 4.1.2 4-{4-[4-(3-Chlor-phenylamino)-5-oxo-6,7-dihydro-5H-5λ⁴-thieno[3,2-d]pyrimidin-2-yl]-piperazin-1-yl}-benzoesäure(Schema 4, Schritt D):

6,90 g 4-{4-[4-(3-Chlor-phenylamino)-5-oxo-6,7-dihydro-5H-5λ⁴-thieno[3,2-*d*]pyrimidin-2-yl]-piperazin-1-yl}benzoesäureethylester und 70 ml 1 molare Natronlauge werden in 40 ml Methanol und 40 ml Tetrahydrofuran vorgelegt, dann 1,5 Stunden unter Rückfluss gerührt. Die entstandene Lösung wird eingedampft, der Rückstand abgekühlt und mit 2molarer Salzsäure leicht sauer gestellt. Ausgefallener Niederschlag wird abgesaugt, gewaschen und getrocknet. Die Substanz wird mit Ethylacetat/Methanol 9:1 verrührt, abgesaugt und getrocknet. 3,90 g des Produkts werden als Pulver erhalten.

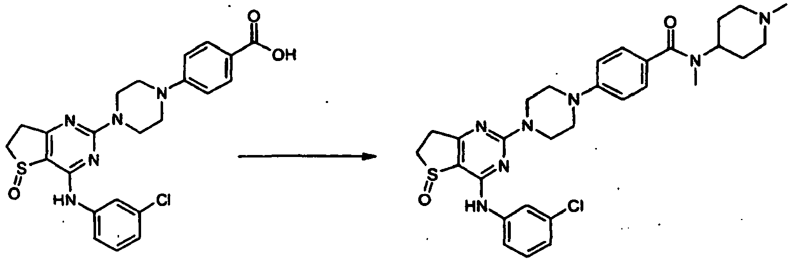

### 4.1.3 4-{4-[4-(3-Chlor-phenylamino)-5-oxo-6,7-dihydro-5H-5λ⁴-thieno[3,2-d]pyrimidin-2-yl]-piperazin-1-yl}-N-methyl-N-(1-methyl-piperidin-4-yl)-benzamid Trifluoracetat (Schema 4, Schritt E):

241,99 mg 4-{4-[4-(3-Chlor-phenylamino)-5-oxo-6,7-dihydro-5H-5λ⁴-thieno[3,2-*d*]pyrimidin-2-yl]-piperazin-1-yl}-benzoesäure werden in 4 ml Dimethylformamid vorgelegt, 69,31 µl Triethylamin und 190,10 mg O-(7-Azabenzotriazol-1-yl-)-N,N,N',N'-tetramethyluronium-hexafluoro-phosphat (HATU) zugegeben. Es wird 0,25 Stunden bei Raumtemperatur gerührt, dann eine Lösung aus 96,16 mg Methyl-(1-methyl-piperidin-4-yl)-amin in 1ml Dimethylformamid zugegeben. Das Reaktionsgemisch wird 16 Stunden bei Raumtemperatur gerührt. Anschließend wird eingedampft. Das Produkt wird über präparative HPLC (Methode C) gereinigt. 213,9 mg des Produkts (60%) werden als Trifluoracetat erhalten. ¹H NMR (400 MHz, DMSO): 7.94 - 7.88 (m, 1 H), 7.70 - 7.62 (m, 1H), 7.41 - 7.27 (m, 3H), 7,17 - 7.10 (m, 1H), 7.04 - 6.95 (m, 2H).

### 4.2 4-{4-[4-(3-FLUOR-PHENYLAMINO)-5-OXO-6,7-DIHYDRO-5H-5λ⁴-THIENO[3,2-d]PYRIMIDIN-2-YL]-PIPERAZIN-1-YL}-N,N-DIMETHYL-BENZAMID TRIFLUORACETAT, BEISPIELE 81 (RACEMAT), 145 (ENANTIOMER 2) UND 146 (ENANTIOMER 1) (SIEHE SCHEMA 4, Schritt E):

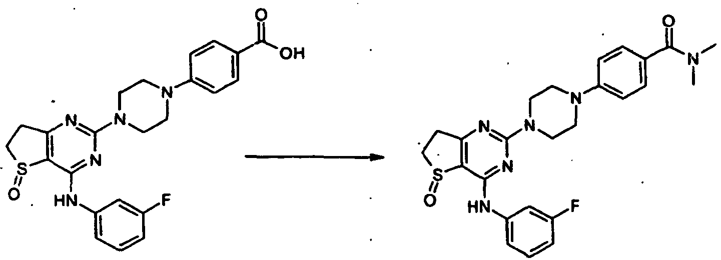

50 mg 4-{4-[4-(3-Fluor-phenylamino)-5-oxo-6,7-dihydro-5H-5λ⁴-thieno[3,2-*d*]pyrimidin-2-yl]-piperazin-1-yl}-benzoesäure (ähnlich hergestellt wie bei Beispiel 11 beschrieben), 44,39 µl Diisopropylethylamin und 39,24 mg O-(7-Azabenzotriazol-1-yl-)-N,N,N',N'-tetramethyluronium-hexafluoro-phosphat (HATU) werden in 2 ml Tetrahydrofuran vorgelegt, 1 ml Dimethylsulfoxid zugegeben. Danach erfolgt die Zugabe von 43 µl Dimethylaminlösung (2molar in Tetrahydrofuran). Das Reaktionsgemisch wird 16 Stunden bei Raumtemperatur gerührt. Anschließend wird eingedampft, der Rückstand mit Wasser und Dichlormethan extrahiert. Die organische Phase wird per Phasentransferkartusche abgetrennt und zur Trockene eingedampft. Das Rohprodukt wird über semipräparative HPLC (Methode A) gereinigt. 13,2 mg des Produkts werden als Trifluoracetat erhalten.
Analytische HPLC-MS, (Methode B): RT = 1,72 min.

Beide Enantiomeren können über chirale HPLC (Säule: Diacel IA, 250 x 4.6 mm, 5 µm, Fließmittel: tBuOMe/EtOH (75/25), Flußrate: 1 ml/min) getrennt werden: Enantiomer 1: RT = 20.4 min (Beispiel 146); Enantiomer 2: RT = 24.3 min (Beispiel 145)

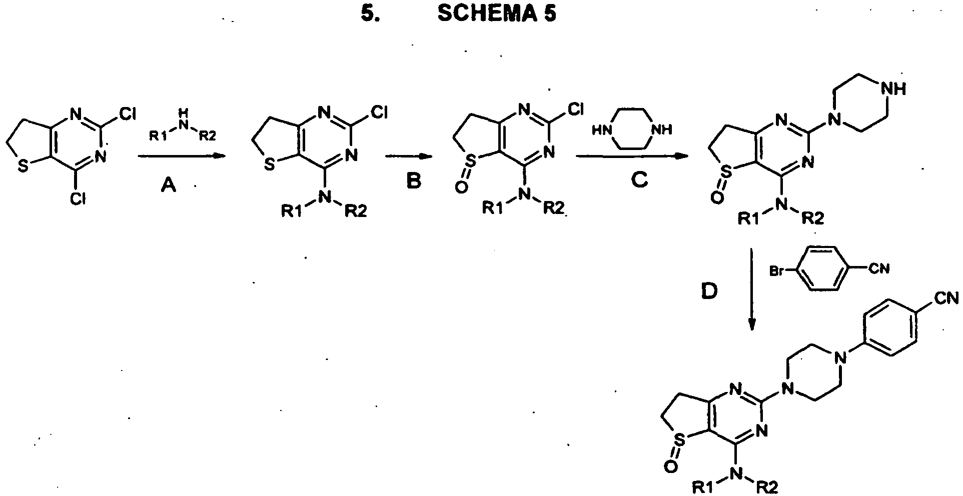

### 5.1 SYNTHESE VON 4-{4-[4-(3-FLUOR-PHENYLAMINO)-5-OXO-6,7-DIHYDRO-5H-5λ⁴-THIENO[3,2-d]PYRIMIDIN-2-YL]-PIPERAZIN-1-YL}-BENZONITRIL BEISPIEL 32 (SIEHE SCHEMA 5)

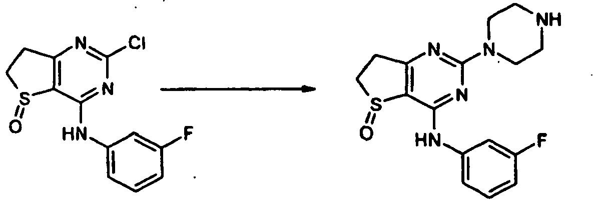

### 5.1.1 (3-Fluor-phenyl)-(5-oxo-2-piperazin-1-yl-6,7-dihydro-5H-5λ⁴-thieno[3,2-d]pyrimidin-4-yl)-amin (Schema 5, Schritt C):

0,500 g 2-Chlor-5-oxo-6,7-dihydro-5H-5λ⁴-thieno[3,2-*d*]pyrimidin-4-yl)-(3-fluor-phenyl)-amin (siehe Beispiel 28, Schema 2) und 1,30 g Piperazin werden in 5 ml Dioxan vorgelegt, dann in der Mikrowelle 0,3 Stunden auf 130° C erhitzt. Anschließend wird das Reaktionsgemisch abgekühlt und mit Wasser versetzt. Der ausgefallene Niederschlag wird abgesaugt, gewaschen und getrocknet. In der Mutterlauge befindliches Produkt wird durch Extraktion mit Dichlormethan erhalten. Beide Rohprodukte werden vereint und über präparative HPLC (Methode A) gereinigt. 360 mg des Produkts werden als Pulver erhalten.

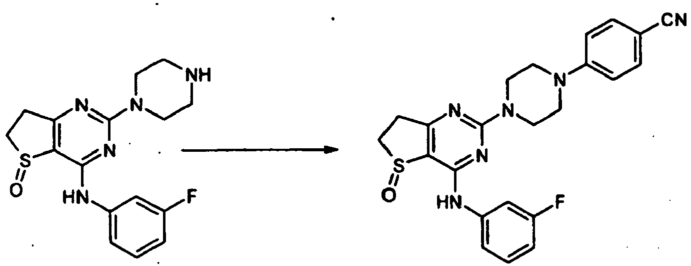

### 5.1.2 4-{4-[4-(3-Fluor-phenylamino)-5-oxo-6,7-dihydro-5H-5λ⁴-thieno[3,2-d]pyrimidin-2-yl]-piperazin-1-yl}-benzonitril (Schema 5, Schritt D):

In Argon Atmosphäre werden 315 mg (3-Fluor-phenyl)-(5-oxo-2-piperazin-1-yl-6,7-dihydro-5H-5λ⁴-thieno[3,2-*d*]pyrimidin-4-yl)-amin, 165 mg 4-Brom-benzonitril, 21 mg Palladium(II)acetat, 80 mg Xantphos und 415 mg Cäsiumcarbonat in 2,50 ml entgastem Toluol 24 Stunden bei 80° C gerührt. Nach Abkühlen wird das Reaktionsgemisch mit Ethylacetat und gesättigter Natriumchloridlösung extrahiert. Die organische Phase wird getrocknet und zur Trockene eingedampft. Der Rückstand wird über präparative HPLC (Methode A) gereinigt. 146 mg des Produkts (36%) werden erhalten. HPLC (Methode A):RT = 3.83 min.

### 5.2 SYNTHESE VON 4-[4-(5-OXO-4-PROPYLAMINO-6,7-DIHYDRO-5H-5λ4-THIENO[3,2-d]PYRIMIDIN-2-YL)-PIPERAZIN-1-YL]-BENZONITRIL BEISPIELE 34 (RACEMAT) UND 148 (ENANTIOMER 1) (SIEHE SCHEMA 5, Schritt D):

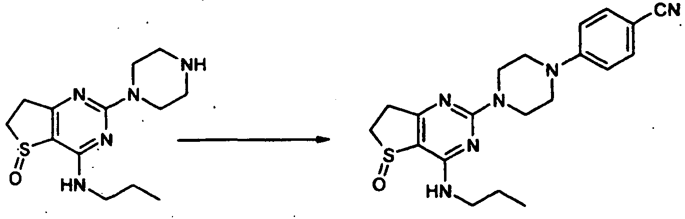

In Argon Atmosphäre werden 400 mg (5-Oxo-2-piperazin-1-yl-6,7-dihydro-5H-5λ⁴-thieno[3,2-*d*]pyrimidin-4-yl)-propylamin (ähnlich hergestellt wie bei Beispiel 32 beschrieben), 245 mg 4-Brombenzonitril, 321 mg Palladium(II)acetat, 120 mg Xantphos und 620 mg Cäsiumcarbonat in 2,50 ml entgastem Toluol 5 Stunden bei 80° C gerührt. Nach Abkühlen wird das Reaktionsgemisch mit Ethylacetat und gesättigter

Natriumchloridlösung extrahiert. Die organische Phase wird getrocknet und zur Trockene eingedampft. Der Rückstand wird über präparative HPLC (Methode A) gereinigt. 282 mg des Produkts werden als Pulver erhalten. Analytische HPLC-MS (Methode A): RT = 2,37 min

Beide Enantiomeren können über chirale HPLC (Säule: Diacel OD-H, 250 x 4.6 mm, 5 µm, Fließmittel: (Hexan+Cyclohexylamin (0,2%))/EtOH (65/35), Flußrate: 1 ml/min) getrennt werden: Enantiomer 1: RT = 7,9 min (Beispiel 148); Enantiomer 2: RT = 9,6 min.

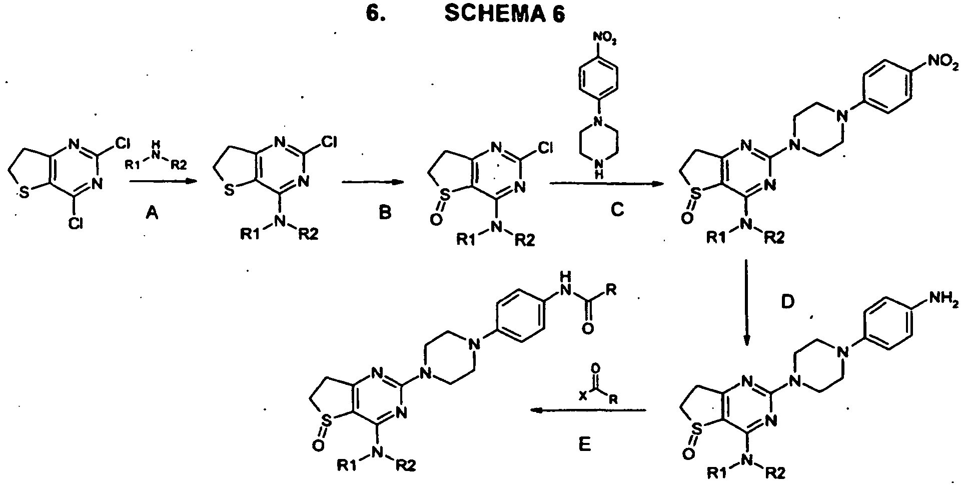

### 6.1 SYNTHESE VON 6-AMINO-N-(4-{4-[4-(3-CHLORO-PHENYLAMINO)-5-OXO-6,7-DIHYDRO-5H-5λ⁴-THIENO[3,2-d]PYRIMIDIN-2-YL]-PIPERAZIN-1-YL}-PHENYL)-NIC OTINAMID TRIFLUORACETAT, BEISPIEL 114 (SIEHE SCHEMA 6)

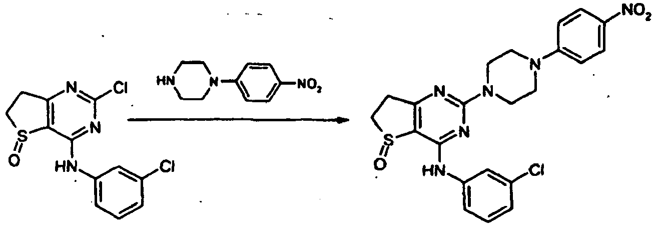

### 6.1.1 (3-Chlor-phenyl)-{2-[4-(4-nitro-phenyl)-piperazin-1-yl]-5-oxo-6,7-dihydro-5H-5λ⁴-thieno[3,2-d]pyrimidin-4-yl}-amin (Schema 6, Schritt D):

1,60 g (2-Chlor-5-oxo-6,7-dihydro-5H-5λ⁴-thieno[3,2-*d*]pyrimidin-4-yl)-(3-chlor-phenyl)-amin (hergestellt aus 2,4-Dichlor-6,7-dihydro-thieno[3,2-*d*]pyrimidin und 3-Chloranilin wie bei Beispiel 28 beschrieben, siehe Schema 2), 2,30 g 1-(4-Nitro-phenyl)-piperazin und 1,00 ml Diisopropylethylamin werden in 10 ml Dioxan vorgelegt, dann in der Mikrowelle 0,75 Stunden auf 160° C erhitzt. Anschließend wird das Reaktionsgemisch mit Wasser versetzt, ausgefallener Niederschlag abgesaugt. Der Niederschlag wird erst mit Wasser, dann mit Ethanol ausgerührt. 2,47 g des Produkts werden als Pulver erhalten.

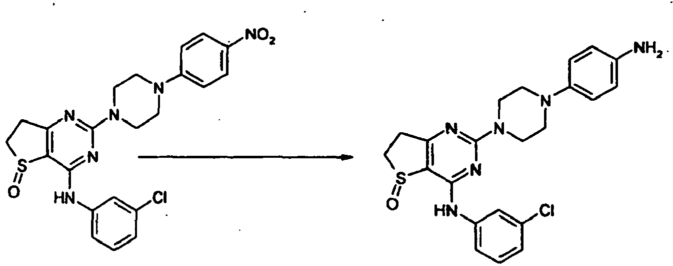

### 6.1.2 {2-[4-(4-Amino-phenyl)-piperazin-1-yl]-5-oxo-6,7-dihydro-5H-5λ⁴-thieno[3,2-d]pyrimidin-4-yl}-(3-chlor-phenyl)-amin Trifluoracetat (Schema 6, Schritt C):

1,00 g (3-Chlor-phenyl)-{2-[4-(4-nitro-phenyl)-piperazin-1-yl]-5-oxo-6,7-dihydro-5H-5λ⁴-thieno[3,2-d]pyrimidin-4-yl}-amin werden in 20 ml Methanol und 40 ml Tetrahydrofuran vorgelegt, mit 100 mg Raney-Nickel 7 Stunden bei Raumtemperatur und einem Druck von 50psi hydriert. Der Katalysator wird abgesaugt, das Filtrat eingedampft. Der Rückstand wird in Acetonitril/ Wasser und Trifluoressigsäure gelöst und über präparative HPLC (Methode A) gereinigt. 0,60 g des Produkts werden als Trifluoracetat erhalten.

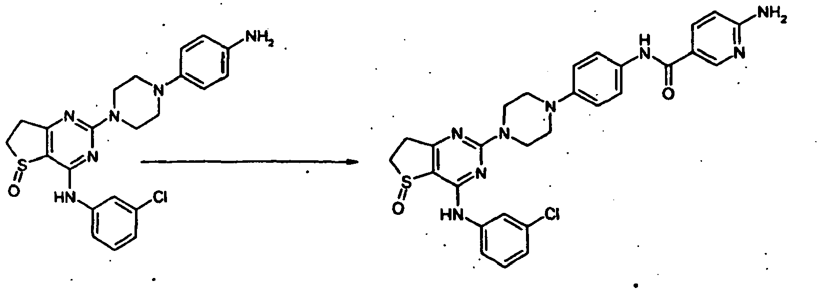

### 6.1.3 6-Amino-N-(4-{4-[4-(3-chlor-phenylamino)-5-oxo-6,7-dihydro-5H-5λ⁴-thieno[3,2-d]pyrimidin-2-yl]-piperazin-1-yl}-phenyl)-nicotinamid Trifluoracetat (Schema 6, Schritt E):

200 mg {2-[4-(4-Amino-phenyl)-piperazin-1-yl]-5-oxo-6,7-dihydro-5H-5λ⁴-thieno[3,2-d]pyrimidin-4-yl}-(3-chlor-phenyl)-amin Trifluoracetat werden in 2,50 ml Dimethylsulfoxid vorgelegt, mit 100 µl Diisopropylethylamin und 150 mg O-(7-Azabenzotriazol-1-yl-)-N,N,N',N'-tetramethyluronium-hexafluoro-phosphat (HATU) versetzt. Es wird 0,25 Stunden bei Raumtemperatur gerührt, dann 50 mg 6-Aminonicotinsäure in 100 µl Diisopropylethylamin zugegeben. Das Reaktionsgemisch wird 16 Stunden bei Raumtemperatur gerührt, anschließend mit Trifluoressigsäure sauer gestellt und 2 ml Wasser zugegeben. Das entstandene Salz wird über präparative HPLC (Methode A) gereinigt. 140 mg des Produkts werden als Trifluoracetat erhalten. Analytische HPLC (Methode B): RT = 2.74 min.

### 6.2 SYNTHESE VON 2-DIMETHYLAMINO-N-(4-{4-[4-(3-FLUOR-PHENYLAMINO)-5-OXO-6,7-DIHYDRO-5H-5λ⁴-THIENO[3,2-d]PYRIMIDIN-2-YL]-PIPERAZIN-1-YL}-PHENYL)-ACETAMID, BEISPIELE 133 (RACEMAT), 149 (ENANTIOMER 2) UND 150 (ENANTIOMER 1) (SIEHE SCHEMA 6):

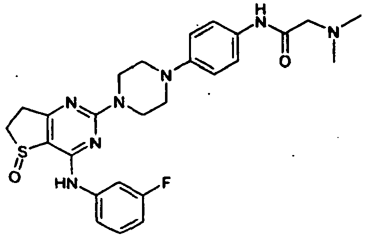

2-Dimethylamino-*N*-(4-{4-[4-(3-fluor-phenylamino)-5-oxo-6,7-dihydro-5*H*-5λ⁴-thieno[3,2-d]pyrimidin-2-yl]-piperazin-1-yl}-phenyl)-acetamid kann, wie bei Beispiel 114 beschrieben, hergestellt werden. Das Produkt kann über präparative HPLC (Methode B) gereinigt werden Analytische HPLC (Methode B): RT = 2.60 min.

Beide Enantiomeren können über chirale HPLC (Säule: Diacel AD-H, 250 x 4.6 mm, 5 µm, Fließmittel: (Hexan+Cyclohexylamin (0,2%))/EtOH (35/65), Flußrate: 1 ml/min) getrennt werden: Enantiomer 1: RT = 17,9 min (Beispiel 150); Enantiomer 2: RT = 37,1 min (Beispiel 149).

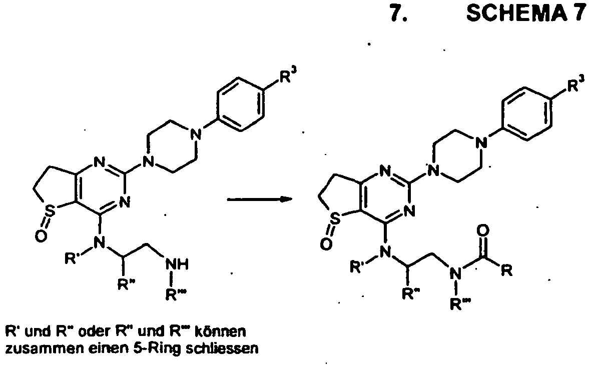

### 7.1 SYNTHESE VON N-((S)-1-{2-[4-(4-CHLOR-PHENYL)-PIPERAZIN-1-YL]-5-OXO-6,7-DIHYDRO-5H-5λ-THIENO[3,2-d]PYRIMIDIN-4-YL}-PYRROLIDIN-2-YLMETHYL)-PROPIONAMID, BEISPIEL 231 (SIEHE SCHEMA 7):

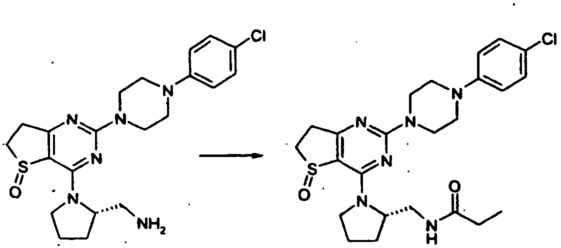

17 µl Propionsäure werden in 800 µl Dimethylsulfoxid vorgelegt, 78 µl Diisopropylethylamin und 94 mg O-(7-Azabenzotriazol-1-yl-)-N,N,N',N'-tetramethyluronium-hexafluoro-phosphat (HATU) zugegeben. Es wird 0,3 Stunden bei Raumtemperatur gerührt, dann eine Lösung aus 100 mg C-((S)-1-{2-[4-(4-Chlor-phenyl)-piperazin-1-yl]-5-oxo-6,7-dihydro-5*H*-5λ-thieno[3,2-*d*]pyrimidin-4-yl}-pyrrolidin-2-yl)-methylamin Trifluoracetat, Diastereomer 2 (Beispiel 226) (siehe Schema 3, 3.17.2), in 700 µl Dimethylsulfoxid zugegeben. Das Reaktionsgemisch wird 15 Stunden bei Raumtemperatur gerührt und anschließend mit Wasser und Dichlormethan versetzt. Die organische Phase wird getrocknet und zur Trockene eingedampft. Das Produkt wird über präparative HPLC (Methode A) gereinigt. 84 mg des Produkts werden als Öl erhalten. Analytische HPLC-MS (Methode D): RT = 1,19 min.

### 7.2 SYNTHESE VON ((R)-3-{2-[4-(4-CHLOR-PHENYL)-PIPERAZIN-1-YL]-5-OXO-6,7-DIHYDRO-5H-5λ-THIENO[3,2-d]PYRIMIDIN-4-YLAMINO}-PYRROLlDIN-1-YL)-(R)-TETRAHYDRO-FURAN-2-YL-METHANON, BEISPIEL 196 (SIEHE SCHEMA 7):

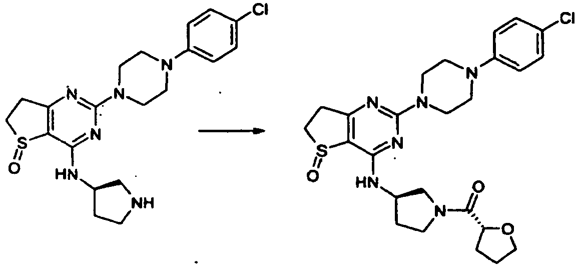

Ausgehend von {2-[4-(4-Chlor-phenyl)-piperazin-1-yl]-5-oxo-6,7-dihydro-5*H*-5λ⁴-thieno[3,2-*d*]pyrimidin-4-yl}-(R)-pyrrolidin-3-yl-amin Diastereomer 1 (Beispiel 171) (siehe Schema 3, 3.22) und (R)-(+)-Tetrahydrofuran-2-carbonsäure wird Beispiel 196, wie: bei Beispiel 231 (siehe Schema 7) beschrieben, hergestellt. Das Produkt wird über präparative HPLC (Methode B) gereinigt. Analytische HPLC-MS (Methode D): RT = 1,18 min.

### 7.3 SYNTHESE VON ((R)-3-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YLAMINO}-PYRROLIDIN-1-YL)-(1-METHYL-1H-PYRROL-2-YL)-METHANON, BEISPIEL 197 (SIEHE SCHEMA 7):

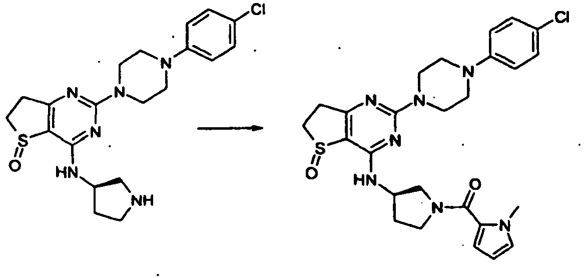

Ausgehend von {2-[4-(4-Chlor-phenyl)-piperazin-1-yl]-5-oxo-6,7-dihydro-5*H*-5λ⁴-thieno[3,2-*d*]pyrimidin-4-yl}-(R)-pyrrolidin-3-yl-amin Diastereomer 1 (Beispiel 171) (siehe Schema 3, 3.22) und 1-Methylpyrrol-2-carbonsäure wird ((R)-3-{2-[4-(4-Chlor-phenyl)-piperazin-1-yl]-5-oxo-6,7-dihydro-5*H*-5λ⁴-thieno[32-*d*]pyrimidin-4-ylamino}-pyrrolidin-1-yl)-(1-methyl-1*H*-pyrrol-2-yl)-methanon, wie bei Beispiel 231 (siehe Schema 7) beschrieben, hergestellt. Das Produkt wird über präparative HPLC (Methode B) gereinigt. Analytische HPLC-MS (Methode D): RT = 1,25 min.

### 7.4 SYNTHESE VON ((R)-3-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YLAMINO}-PYRROLIDIN-1-YL)-(S)-TETRAHYDRO-FURAN-2-YL-METHANON, BEISPIEL 198 (SIEHE SCHEMA 7):

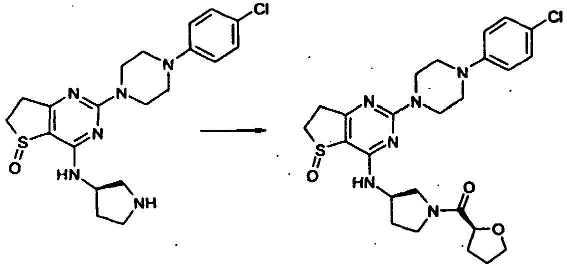

Ausgehend von {2-[4-(4-Chlor-phenyl)-piperazin-1-yl]-5-oxo-6,7-dihydro-5*H*-5λ⁴-thieno[3,2-*d*]pyrimidin-4-yl}-(R)-pyrrolidin-3-yl-amin Diastereomer 1 (Beispiel 171) (siehe Schema 3, 3.22) und (S)-(-)-Tetrahydrofuran-2-carbonsäure wird ((R)-3-{2-[4-(4-Chlor-phenyl)-piperazin-1-yl]-5-oxo-6,7-dihydro-5*H*-5λ⁴-thieno[3,2-*d*]pyrimidin-4-ylamino}-pyrrolidin-1-yl)-(S)-tetrahydro-furan-2-yl-methanon, wie bei Beispiel 231 (siehe Schema 7) beschrieben, hergestellt. Das Produkt wird über präparative HPLC (Methode B) gereinigt. Analytische HPLC-MS (Methode D): RT = 1,17 min.

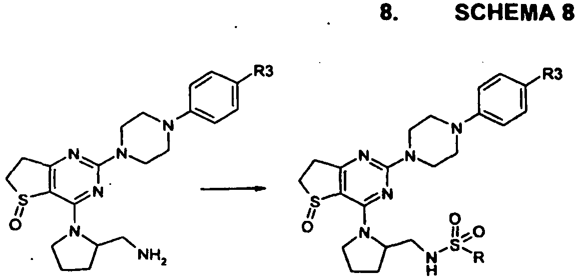

### 8 SYNTHESE VON N-((S)-1-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-PYRROLIDIN-2-YLMETHYL)-METHANSULFONAMID, BEISPIEL 234 (SIEHE SCHEMA 8):

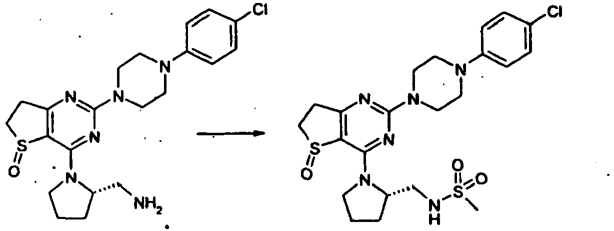

100 mg C-((S)-1-{2-[4-(4-Chlor-phenyl)-piperazin-1-yl]-5-oxo-6,7-dihydro-5*H*-5λ⁴-thieno[3,2-*d*]pyrimidin-4-yl}-pyrrolidin-2-yl)-methylamin Trifluoracetat, Diastereomer 2 (Beispiel 226) (siehe Schema 3, 3.17.2) werden in 2 ml Dichlormethan vorgelegt. Unter Eisbadkühlung werden 38 µl Triethylamin und, nach 5 Minuten, 18 µl Methansulfonylchlorid zugegeben. Das Reaktionsgemisch wird 12 Stunden bei Raumtemperatur gerührt und anschließend mit Wasser versetzt. Die organische Phase wird getrocknet und zur Trockene eingedampft. 120 mg des Produkts werden als Pulver erhalten. Analytische HPLC-MS (Methode D) : RT = 1,20 min.

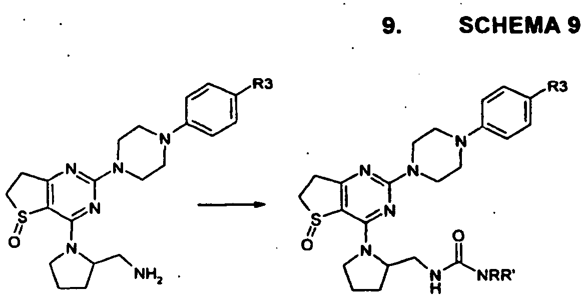

### 9 SYNTHESE VON 1-((S)-1-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YL}-PYRROLIDIN-2-YLMETHYL)-3-PHENYLHARNSTOFF, BEISPIEL 237 (SIEHE SCHEMA 9):

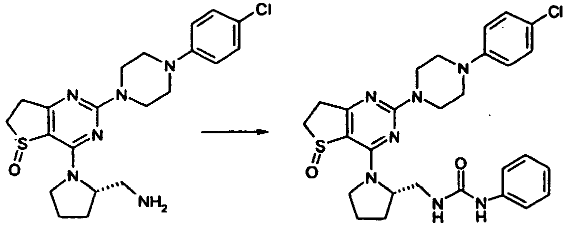

100 mg C-((S)-1-{2-[4-(4-Chlor-phenyl)-piperazin-1-yl]-5-oxo-6,7-dihydro-5*H*-5λ⁴-thieno[3,2-*d*]pyrimidin-4-yl}-pyrrolidin-2-yl)-methylamin Trifluoracetat, Diastereomer 2 (Beispiel 226) (siehe Schema 3, 3.17.2) werden in 2 ml Dichlormethan vorgelegt und 30 µl Phenylisocyanat zugegeben. Das Reaktionsgemisch wird 4 Stunden bei Raumtemperatur gerührt und anschließend zur Trockene eingedampft. Das Produkt wird über präparative HPLC (Methode A) gereinigt. 64 mg des Produkts werden als Pulver erhalten. Analytische HPLC-MS (Methode D): RT = 1,33 min.

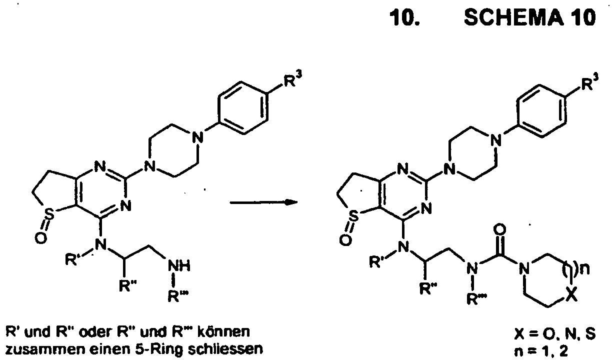

### 10.1 SYNTHESE VON MORPHOLIN-4-CARBONSÄURE ((S)-1-{2-[4-(4-CHLORPHENYL)-PIPERAZIN-1-YL]-5-OXO-6,7-DIHYDRO-5H-5λ⁴-THIENO[3,2-d]PYRIMIDIN-4-YL}-PYRROLIDIN-2-YLMETHYL)-AMID, BEISPIEL 239 (SIEHE SCHEMA 10):

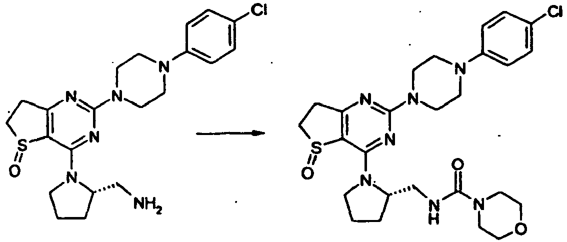

100 mg C-((S)-1-{2-[4-(4-Chlor-phenyl)-piperazin-1-yl]-5-oxo-6,7-dihydro-5*H*-5λ⁴-thieno[3;2-*d*]pyrimidin-4-yl}-pyrrolidin-2-yl)-methylamin Trifluoracetat, Diastereomer 2 (Beispiel 226) (siehe Schema 3, 3.17.2) werden in 2 ml Dichlormethan vorgelegt. Unter Eisbadkühlung werden 38 µl Triethylamin und 28 µl 4-Morpholincarbonylchlorid zugegeben. Das Reaktionsgemisch wird 12 Stunden bei Raumtemperatur gerührt und anschließend mit Wasser versetzt. Die organische Phase wird getrocknet und zur Trockene eingedampft. 121 mg des Produkts werden als Feststoff erhalten. Analytische HPLC-MS (Methode D): RT = 1,18 min.

### 10.2 SYNTHESE VON ((R)-3-{2-[4-(4-CHLOR-PHENYL)-PIPERAZIN-1-YL]-5-OXO-6,7-DIHYDRO-5H-5λ⁴-THIENO[3,2-d]PYRIMIDIN-4-YLAMINO}-PYRROLIDIN-1-YL)-MORPHOLIN-4-YL-METHANON, BEISPIEL 193 (SIEHE SCHEMA 10):

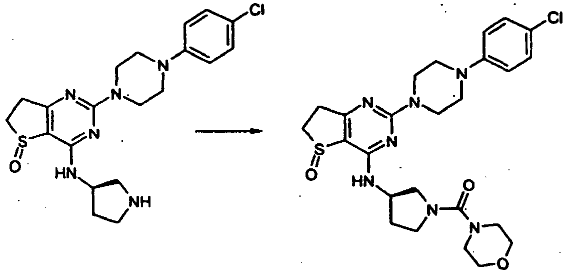

Ausgehend von {2-[4-(4-Chlor-phenyl)-piperazin-1-yl]-5-oxo-6,7-dihydro-5*H*-5λ⁴-thieno[3,2-*d*]pyrimidin-4-yl}-(R)-pyrrolidin-3-yl-amin Diastereomer 1 (Beispiel 171) (siehe Schema 3, 3.22) und 4-Morpholincarbonylchlorid wird ((R)-3-{2-[4-(4-Chlor-phenyl)-piperazin-1-yl]-5-oxo-6,7-dihydro-5*H*-5λ⁴-thieno[3,2-*d*]pyrimidin-4-ylamino}-pyrrolidin-1-yl)-morpholin-4-yl-methanon, wie bei Beispiel 239 (siehe Schema 10) beschrieben, hergestellt. Das Produkt wird über präparative HPLC (Methode A) gereinigt. Analytische HPLC-MS (Methode D): RT = 1,16 min.

### CHROMATOGRAPHIE METHODEN:

Die nach den obigen Synthese-Schemata hergestellten Beispielverbindungen wurden entweder durch die Bestimmung ihrer Schmelzpunkte (siehe Tabelle 1) oder durch die folgenden chromatographischen Methoden, die - sofern sie durchgeführt wurden - im einzelnen in Tabelle 1 angegeben werden, charakterisiert.

### Analytische HPLC-MS, Methode A:

### Bedingungen:

Waters ZMD, Alliance 2690/2695 HPLC, Waters 2700 Autosampler, Waters 996/2996 Diodenarraydetektor. Als mobile Phase wurde eingesetzt:
A: Wasser mit 0.10% TFA
B: Acetonitril mit 0.10% TFA

| Zeit in min | %A | %B | Flussrate in ml/min |
|---|---|---|---|
| 0.0 | 95 | 5 | 1.00 |
| 0.1 | 95 | 5 | 1.00 |
| 3.1 | 2 | 98 | 1.00 |
| 4.5 | 2 | 98 | 1.00 |
| 5.0 | 95 | 5 | 1.00 |

Als stationäre Phase diente eine Säule XTerra®, MS C₁₈ 2.5 µm, 4.6 mm x 30 mm (Säulentemperatur: konstant bei 25°C). Die Diodenarraydetektion erfolgte im Wellenlängenbereich 210-400 nm.

### Analytische HPLC-MS, Methode B:

### Bedingungen:

Waters ZMD, Alliance 2690/2695 HPLC, Waters 2700 Autosampler, Waters 996/2996 Diodenarraydetektor. Als mobile Phase wurde eingesetzt:
A: Wasser mit 0.10% TFA
B: Acetonitril mit 0.10% TFA

| Zeit in min | %A | %B | Flussrate in ml/min |
|---|---|---|---|
| 0.00 | 95 | 5 | 2.00 |
| 0.10 | 95 | 5 | 2.00 |
| 2.10 | 2 | 98 | 2.00 |
| 3.00 | 2 | 98 | 2.00 |
| 3.25 | 95 | 5 | 2.00 |

Als stationäre Phase diente eine Säule Merck Chromolith^{™} SpeedROD RP-18e, 4.6 mm x 50 mm (Säulentemperatur: konstant bei 25°C). Die Diodenarraydetektion erfolgte im Wellenlängenbereich 210-400 nm.

### Analytische HPLC-MS, Methode C:

### Bedingungen:

Waters ZMD Mass Spektrometer, Alliance 2790 + 996 DAD, 2700 Autosampler

| ***Einstellungparametern: ES+*** | |
|---|---|
| Needle | 3.0 kVolts |
| Cone: | 30 Volts |

| *HPLC Parametern* | *Gradient:* | | | |
|---|---|---|---|---|
| *Mobile Phase:* | Zeit [min] A% | B% | Flow | |
| A% H2O mit 0.1 TFA | 0,00 | 95,00 | 5,00 | 1,000 |
| B% ACN mit 0.1% TFA | 0,10 | 95,00 | 5,00 | 1,000 |
| *Stationäre Phase:* | 5,10 | 2,00 | 98,00 | 1,000 |
| X-Terra MS C18 4.6x50mm, 3.5µm | 6,50 | 2,00 | 98,00 | 1,000 |
| Säule Temperatur (°C) 40.0 | 7,00 | 95,00 | 5,00 | 1,000 |

### Aufbereitungsparametern

Retentionzeit Bereich benutzt für MS Integration and DAD Daten: 1.00 - 7.00 Minutes
Wellenlänge Bereich benutzt für die Bestimmung von Flächen %: 210 - 500 nm
Einstellung für MS und UV Peak-to-Peak Amplitude : 2000
Zwei "Smooth" (Savitzky Golay) mit Fenstergrösse (scans) ± 2 für DAD und MS
Chromatogram.

### Analytische HPLC-MS, Methode D:

### Bedingungen:

Waters ZMD, Alliance 2690/2695 HPLC, Waters 2700 Autosampler, Waters 996/2996 Diodenarraydetektor. Als mobile Phase wurde eingesetzt:
A: Wasser mit 0.10% TFA
B: Acetonitril mit 0.10% TFA

| Zeit in min | %A | %B | Flussrate in ml/min |
|---|---|---|---|
| 0.00 | 95 | 5 | 2.50 |
| 0.20 | 95 | 5 | 2.50 |
| 1.50 | 2 | 98 | 2.50 |
| 1.70 | 2 | 98 | 2.50 |
| 1.90 | 95 | 5 | 2.50 |
| 2.20 | 95 | 5 | 2.50 |

Als stationäre Phase diente eine Säule Merck Chromolith^{™} Flash RP-18e, 4.6 mm x 25 mm (Säulentemperatur: konstant bei 25°C). Die Diodenarraydetektion erfolgte im Wellenlängenbereich 210-400 nm.

### Analytische HPLC-MS, Methode E:

### Bedingungen:

| Instrument | Waters Alliance 2695 | | |
|---|---|---|---|
| | PDA Detektor 2996 | | |
| | Waters Micromass ZQ | | |
| Gradient | | | |
| Zeit | % Wasser + 0.13% TFA | % Acetonitril | Fluß [ml/min.] |
| 0.00 | 95 | 5 | 4.25 |
| 0.01 | 95 | 5 | 4.25 |
| 0.89 | 2 | 98 | 4.25 |
| 0.90 | 2 | 98 | 4.25 |
| 0.95 | 95 | 5 | 4.25 |
| 1.05 | 95 | 5 | 4.25 |
| 1.10 | 95 | 5 | 0.1 |
| | | | |

| Parameter | Detektor | Säule | |
|---|---|---|---|
| Start Wellenlänge | 210 | **Waters Xbridge C18** | |
| End Wellenlänge | 380 | Particle Size 3,5 µm | |
| Sampling Rate | 20 | Lenght 20mm | |
| Resolution | 1.2 | Internal diameter 4.6mm | |
| Filter Response | 0 | | |
| Autoexposure | Yes | | |
| Interpolate 656nm | Yes | | |
| Parameter | Integration | | |
| Peak With | 15 | | |
| Threshold | 465 | | |
| Minimum Area | 0 | | |
| Minimum Height | 0 | | |
| Mass(m/z) | 120-850 | | |

### Analytische HPLC, Methode A:

### Bedingungen:

| | | | | | |
|---|---|---|---|---|---|
| Säule: Varian Microsorb, RP C18, 3 µm, 100Å | | | | | |
| DAD-Detektor: von 210 nm bis 380 nm | | | | | |
| Fluß: 1ml/min | | | | | |
| | | | | | |
| | | Fluß [ml/min] | Totzeit [min.] | Säulen ID [mm] | 4,6 |
| | | 1,0 | 0,750 | Säulenlänge [mm] | 50 |
| | | | | Säulenvolumen [ml] | 0,83 |
| | | | | | |
| Fluß [ml/min.] | 1 | | | | |
| Zeit [min.] | % Acetonitril + 0,1% TFA | % Wasser + 0,13% TFA | | | |
| 0,0 | 5 | 95 | | | |
| 0,8 | 5 | 95 | | | |
| 5,0 | 98 | 2 | | | |
| 5,5 | 98 | 2 | | | |
| 5,8 | 5 | 95 | | | |
| 6,7 | 5 | 95 | | | |
| | | | | | |
| Steigung [% / min] | 22,1 | | | | |
| Vg Gradientenvolumen [ml] | 4,2 | | | | |
| Vs Säulenvolumen [ml] | 0,83 | | | | |
| Verhältnis Vg / Vs | 5,06 | | | | |

### Analytische HPLC, Methode B:

### Bedingungen:

| | | | | | |
|---|---|---|---|---|---|
| Säule: Varian Microsorb, RP C18, 3 µm, 100Å | | | | | |
| DAD-Detektor: von 210 nm bis 380 nm | | | | | |
| Fluß: 1,5ml/min | | | | | |
| | | | | | |
| | | Fluß [ml/min] | Totzeit [min.] | Säulen ID [mm] | 4,6 |
| | | 1,5 | 0,500 | Säulenlänge [mm] | 50 |
| | | | | Säulenvolumen [ml] | 0,83 |
| | | | | | |
| Fluß [ml/min.] | 1,5 | | | | |
| Zeit [min.] | % Acetonitril + 0,1% TFA | % Wasser + 0,13% TFA | | | |
| 0,0 | 5 | 95 | | | |
| 0,6 | 5 | 95 | | | |
| 3,4 | 98 | 2 | | | |
| 3,9 | 98 | 2 | | | |
| 4,2 | 5 | 95 | | | |
| 4,9 | 5 | 95 | | | |
| | | | | | |
| Steigung [% / min] | 33,2 | | | | |
| Vg Gradientenvolumen [ml] | 4,2 | | | | |
| Vs Säulenvolumen [ml] | 0,83 | | | | |
| Verhältnis Vg / Vs | 5,06 | | | | |

### Präparative HPLC, Methode A:

### Bedingungen:

| | | | | |
|---|---|---|---|---|
| Gradient Präparative HPLC | Fluß [ml/min] | Totzeit [min.] | Säulen ID [mm] | 41,4 |
| Method A | 120,0 | 3,500 | Säulenlänge [mm] | 250 |
| | | | Säulenvolumen [ml] | 336,4 |

| | | | | |
|---|---|---|---|---|
| Säulenmaterial: Microsorb RP 18, 60Å, 8 µm Fließmittel: Acetonitril + 0,1% TFA, Wasser + 0,13% TFA | | | | |

| Fluß [ml/min.] | 120 |
|---|---|
| Zeit [min.] | % Acetonitril |
| 0,0 | 10 |
| 3,6 | 10 |
| 17,8 | 100 |
| 22,0 | 100 |
| 23,0 | 10 |
| 26,5 | 10 |
| Steigung [% / min] | 6,3 |
| Vg Gradientenvolumen [ml] | 1704 |
| Vs Säulenvolumen [ml] | 336,4 |

### Präparative HPLC, Methode B:

Die Säulenmaterial- und Fließmittelangaben sind die gleiche wie bei Methode A. Der Gradientverlauf bezieht sich auf Methode A, jedoch wird ein isokratischer Schritt eingefügt, der durch die analytische Retentionszeit der jeweiligen zu trennenden Substanz bestimmt ist.

### Präparative HPLC, Methode C:

### Bedingungen:

### Gilson-HPLC-System, Gilson 170 DAD und Gilson 215 Autosampler

### Lösungsmittel:

A% H2O mit 0.1% TFA
B% ACN mit 0.1% TFA

| *Gradient:* | | | |
|---|---|---|---|
| Zeit [min] | A% | B% | Flußrate (ml/min) |
| 0,00 | 95,00 | 5,00 | 0 |
| 0,30 | 95,00 | 5,00 | 0 |
| 0,40 | 95,00 | 5,00 | 5 |
| 0,45 | 95,00 | 5,00 | 40 |
| 1,00 | 95,00 | 5,00 | 40 |
| 16,00 | 40,00 | 60,00 | 40 |
| 17,00 | 5,00 | 95,00 | 40 |
| 20,00 | 5,00 | 95,00 | 40 |
| 22,00 | 95,00 | 5,00 | 40 |
| 25,00 | 95,00 | 5,00 | 40 |

### Stationäre Phase:

50g YMC-Gel ODS-A RP-18 10µm
gepackt mit einem MODCOL springsäule mit 25 mm i.d.
Säuletemperatur 22°C

### Aufbereitungsparameters

Wellenlänge benutzt für die Detektion: 215 nm & 254 nm

| **Präparative HPLC, Methode D:** | | |
|---|---|---|
| Säulenmaterial: Xbridge RP C18, 5 µm | Säulen ID [mm] | 50 |
| Fließmittel: Acetonitril, Wasser + 0,13% TFA | Säulenlänge [mm] | 162 |
| Fluß: 120 ml/min | Säulenvolumen [ml] | 317.9 |

### Gradient

| Zeit [min.] | % Acetonitril |
|---|---|
| 0.0 | 10 |
| 3.0 | 10 |
| 22.0 | 100 |
| 25.5 | 100 |
| 26.5 | 10 |
| 30.0 | 10 |

### Semipräparative HPLC, Methode A:

### Bedingungen:

| | | | | |
|---|---|---|---|---|
| Gradient Semipräperative HPLC | Fluß [ml/min] | Totzeit [min.] | Säulen ID [mm] | 21,4 |
| Method A | 25,0 | 4,4 | Säulenlänge [mm] | 250 |
| | | | Säulenvolumen [ml] | 89,9 |

| | | | | |
|---|---|---|---|---|
| Säulenmaterial: Microsorb RP 18, 300Å, 10 µm Fließmittel: Acetonitril + 0,1% TFA, Wasser + 0,13% TFA | | | | |

| Fluß [ml/min.] | 25 |
|---|---|
| Zeit [min.] | % Acetonitril |
| 0.0 | 10 |
| 4,5 | 10 |
| 22,7 | 100 |
| 26,7 | 100 |
| 27,7 | 10 |
| 32,0 | 10 |
| Steigung [% / min] | 4,9 |
| Vg Gradientenvolumen [ml] | 455 |
| Vs Säulenvolumen [ml] | 89,9 |
| Verhältnis Vg / Vs | 5,06 |

### BEISPIELE

Die folgenden Beispiele wurden analog den oben gezeigten Synthesevorschriften herstellt (je nach Kennzeichnung in der Tabelle). Diese Verbindungen sind als PDE4-Inhibitoren geeignet und besitzen IC₅₀-Werte kleiner oder gleich 1 µmol.

Bei den Beispielen handelt es sich um Verbindungen der folgenden Formel **3,** 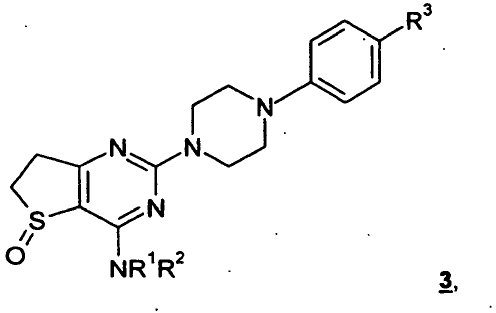 worin die die Variablen R¹, R² und R³ die in der Tabelle 1 stehenden Bedeutungen haben. Die Verbindungen wurden je nach ihrer in Tabelle 1 angegebenen Kennzeichnung nach Schema 1 bis Schema 10 hergestellt.

**Tabelle 1:**

| # | R¹ | R² | R³ | Synthese- Schema / Chiralität (bezogen auf gesamtes Molekül) | Nicht käufliche Bausteine (Herstellung beschrieben in Literatur) | Smp (°C) | Retentionszeit [min] (mit HPLC- MS oder HPLC |
|---|---|---|---|---|---|---|---|
| 1. | H | 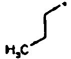 | 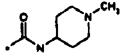 | Schema 3/ | - | - | 2,62 |
| | | | | Racemat | | | Analyt. HPLC-MS Methode C |
| | | | | Schema 4/ | - | - | 2,59 |
| 2. | H | 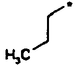 | 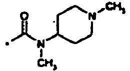 | Racemat | | | Analyt. HPLC-MS Methode C |
| | | | | Schema 4/ | - | - | 2,64 |
| 3. | H | 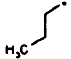 | 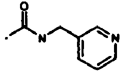 | Racemat | | | Analyt. HPLC-MS Methode C |
| | | | | Schema 4/ | - | - | 2,66 |
| 4. | H | 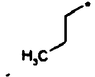 | 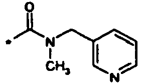 | Racemat | | | Analyt. HPLC-MS Methode C |
| | | | | Schema 4/ | - | - | 2,66 |
| 5. | H | 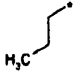 | 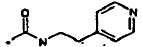 | Racemat | | | Analyt. HPLC-MS Methode C |
| | | | | Schema 4/ | | - | 2,64 |
| 6. | H | 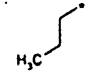 | 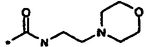 | Racemat | | | Analyt. HPLC-MS Methode C |
| | | | | Schema 4/ | - | - | 2,77 |
| 7. | H | 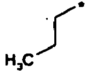 | 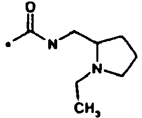 | Racemat | | | Analyt. HPLC-MS Methode C |
| | | | | Schema 4/ | - | - | 2,9 |
| 8. | H | 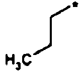 | 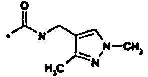 | Racemat | | | Analyt. HPLC-MS Methode C |
| | | | | Schema 4/ | | | 2,6 |
| 9. | H | 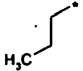 | 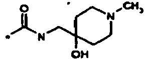 | Racemat | 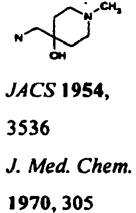 | - | Analyt. HPLC-MS Methode C |
| | | | | Schema 4/ | - | - | 3,21 |
| 10. | H | 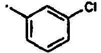 | 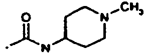 | Racemat | | | Analyt. HPLC-MS Methode C |
| | | | | Schema 4/ | - | - | 3,18 |
| 11. | H | 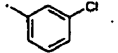 | 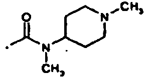 | Racemat | | | Analyt. HPLC-MS Methode C |
| 12. | H | 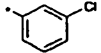 | 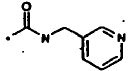 | Schema 4/ | - | - | 3,24 |
| | | | | Racemat | | | Analyt. HPLC-MS Methode C |
| | | | | Schema 4/ | - | - | 3,24 |
| 13. | H | 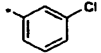 | 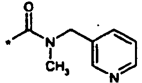 | Racemat | | | Analyt. HPLC-MS Methode C |
| | | | | Schema 4/ | - | - | 3,23 |
| 14. | H | 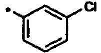 | 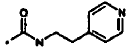 | Racemat | | | Analyt. HPLC-MS Methode C |
| | | | | Schema 4/ | - | - | 3,23 |
| 15. | H | 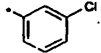 | 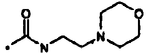 | Racemat | | | Analyt. HPLC-MS Methode C |
| | | | | Schema 4/ | - | - | 3,38 |
| 16. | H | 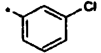 | 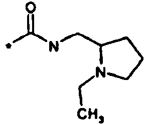 | Mischung von Stereoisomeren | | | Analyt. HPLC-MS Methode C |
| | | | | Schema 4/ | - | - | 3,55 |
| 17. | H | 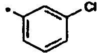 | 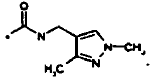 | Racemat | | | Analyt. HPLC-MS Methode C |
| | | | | Schema 4/ | 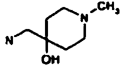 | - | 3,18 |
| 18. | H | 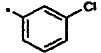 | 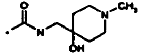 | Racemat | JACS 1954, 3536 J. Med. Chem. 1970, 305 | | Analyt. HPLC-MS Methode C |
| 19. H | | 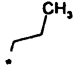 | 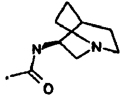 | Schema 4/ | - | - | 2,83 |
| | | | | Mischung von Diastereomeren | | | Analyt. HPLC Methode A |
| 20. | H | 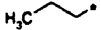 | Cl | Schema 1/ | - | 245- | |
| | | | | Racemat | | 247 | |
| 21. | H | 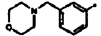 | Cl | Schema 1/ | 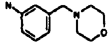 siehe experim. Teil | 244- 248 | |
| 22. | H | 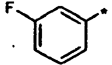 | Cl | Racemat | | | |
| | | | | Schema 1/ | - | 244- | |
| | | | | | | 246 | |
| | | | | Racemat | | | |
| 23. | H | 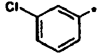 | Cl | Schema 1/ | - | 244- | |
| | | | | | | 245 | |
| | | | | Racemat Schema 4/ | 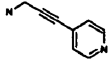 | - | 2,86 |
| 24. | H | 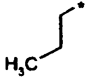 | 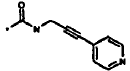 | Racemat | Tet. Lett. 2004, 7069 | | Analyt. HPLC-MS Methode C |
| 25. | H | 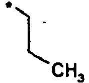 | 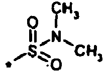 | Schema 2/ Racemat | 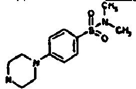 WO 2003/105853 | - | 2,42 HPLC-MS Methode A |
| 26. | H | 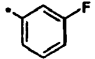 | 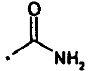 | Schema 2/ Racemat | - | - | 2,29 HPLC-MS Methode A |
| 27. | H | 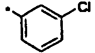 | 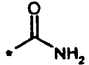 | Schema 2/ Racemat | - | - | 2,39 HPLC-MS Methode A |
| 28. | H | 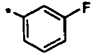 | 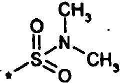 | Schema 2/ Racemat | 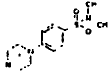 WO 2003/105853 | - | 3,73 Analyt. HPLC Methode A |
| 29. | H | 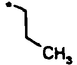 | 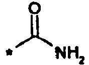 | Schema 2/ Racemat | | - | 2,88 Analyt. HPLC Methode A |
| 30. | H | 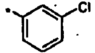 | 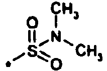 | Schema 2/ Racemat | 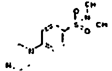 WO 2003/105853 | - | 2,9 HPLC-MS Methode A |
| 31. | H | 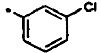 | 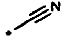 | Schema 5/ | 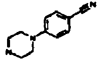 | - | 3,99 |
| | | | | Racemat | siehe experim. Teil | | Analyt. HPLC Methode A |
| | | | | Schema 5/ | 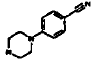 | - | 3,83 |
| 32. | H | 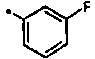 | 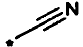 | Racemat | siehe experim. Teil | | Analyt. HPLC Methode A |
| 33. | H | 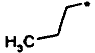 | 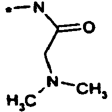 | Schema 6/ Racemat | | - | 1,92 HPLC-MS Methode A |
| 34. | H | 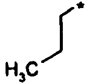 | 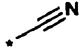 | Schema 5/ Racemat | 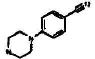 siehe experim. Teil | - | 2,37 HPLC-MS Methode A |
| 35. | H | 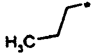 | 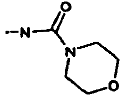 | Schema 6/ | - | 274- 276 | - |
| | | | | Racemat | | | |
| 36. | H | 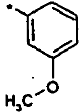 | Cl | Schema 3/ Racemat | - | 238- 241 | - |
| | | | | Schema 3/ | - | 222- 224 | - |
| 37. | H | 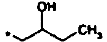 | Cl | Mischung von Stereoisomeren | | | |
| 38. | H | 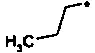 | 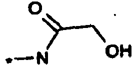 | Schema 6/ | - | 235- 237 | - |
| | | | | Racemat | | | |
| | | | | Schema 3/ | - | - | 2,39 |
| 39. | H | 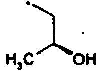 | Cl | Mischung von Diastereomeren | | | HPLC-MS Methode A |
| | | | | | 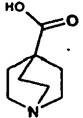 Helv. Chim. Acta 1974, 2332 | - | 1,96 |
| 40. | H | 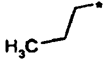 | 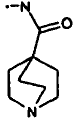 | | | | HPLC-MS Methode A |
| | | | | Schema 3/ | - | 238 | - |
| 41. | H | 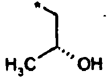 | Cl | Mischung von Diastereomeren | | | |
| | | | | Schema 3/ | - | 241 | - |
| 42. | H | 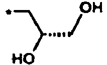 | Cl | Mischung von Diastereomeren | | | |
| | | | | Schema 3/ | - | 240 | - |
| 43. | H | 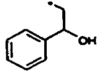 | Cl | Mischung von Stereoisomeren | | | |
| | | | | Schema 3/ | - | 189 | - |
| 44. | H | 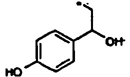 | Cl | Mischung von Stereoisomeren | | | |
| 45. | H | 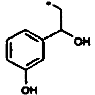 | Cl | Schema 3/ | - | 294 | - |
| | | | | Mischung von Stereoisomeren | | | |
| 46. | H | 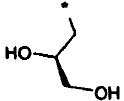 | Cl | Schema 3/ | - | - | 2,2 |
| | | | | Mischung von Diastereomeren | | | HPLC-MS Methode A |
| 47. | H | 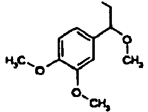 | Cl | Schema 3/ | 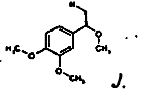 | - | 2,69 |
| | | | | Mischung von Stereoisomeren | Med. Chem. 1983, 174 | | HPLC-MS Methode A |
| 48. | H | 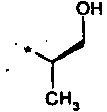 | Cl | Schema 3/ | - | - | 2,32 |
| | | | | Diastereomer | | | HPLC-MS Methode A |
| | | 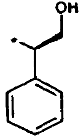 | | Schema 3/ | - | - | 3,04 |
| 49. | H | | Cl | Diastereomer (anderes Diastereomer ist Bsp. 56) | | | Analyt. HPLC Methode A |
| 50. | H | 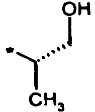 | Cl | Schema 3/ Diastereomer | - | - | 2,37 HPLC-MS Methode A |
| | | | | Schema 3/ | - | - | 2,5 |
| 51. | H | 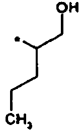 | Cl | Racemat (entsprech.diast ereomracemisc he Mischung ist Bsp. 59) | | | HPLC-MS Methode A |
| 52. | H | 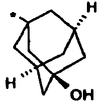 | Cl | Schema 3/ Racemat | - | - | 2,62 HPLC-MS Methode A |
| 53. | H | 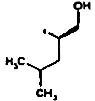 | Cl | Schema 3/ Diastereomer (anderes Diastereomer ist Bsp. 69) | - | - | 1,86 HPLC-MS HPLC-MS Methode B |
| 54. | H | 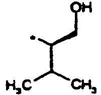 | Cl | Schema 3/ Diastereomer (anderes Diastereomer ist Bsp. 57) | - | - | 1,76 HPLC-MS Methode B |
| 55. | H | 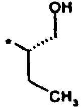 | Cl | Schema 3/ Diastereomer | - | - | 2,47 HPLC-MS Methode A |
| | | | | Schema 3/ | - | - | 3,1 |
| 56. | H | 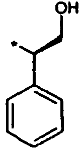 | Cl | Diastereomer (anderes Diastereomer ist Bsp. 49) | | | Analyt. HPLC Methode A |
| | | | | Schema 3/ | - | - | 1,81 |
| 57. | H | 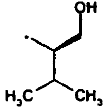 | Cl | Diastereomer | | | HPLC-MS Methode B |
| | | | | Schema 3/ | - | - | 2,57 |
| 58. | H | 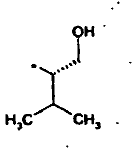 | Cl | Diastereomer | | | HPLC-MS Methode A |
| | | | | Schema 3/ | - | - | 2,57 |
| 59. | H | 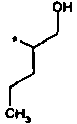 | Cl | Racemat (entsprech.diast ereom racemisc he Mischung ist Bsp. 51) | | | HPLC-MS Methode A |
| | | | | Schema 3/ | - | - | 2,65 |
| 60. | H | 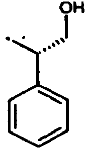 | Cl | Diastereomer | | | HPLC-MS Methode A |
| | | | | Schema 3/ | - | - | 3,09 |
| 61. | H | 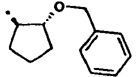 | Cl | Mischung von Diastereomeren | | | HPLC-MS Methode A |
| | | | | Schema 3/ | - | - | 2,66 |
| 62. | H | 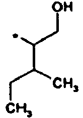 | Cl | Racemat | | | HPLC-MS Methode A |
| | | | | Schema 3/ | | | 2,44 |
| 63. | H | 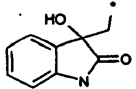 | Cl | Mischung von Stereoisomeren | 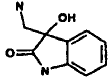 JACS 1936, 1236 | - | HPLC-MS Methode A |
| | | | | Schema 3/ | - | - | 2,99 |
| 64. | H | 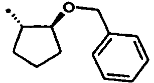 | Cl | Mischung von Diastereomeren | | | HPLC-MS Methode A |
| | | | | Schema 3/ | - | - | 2,4 |
| 65. H | | 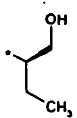 | Cl | Diastereomer (anderes Diastereomer ist Bsp. 67) | | | HPLC-MS Methode A |
| | | | | Schema 3/ | - | - | 2,55 |
| 66. | H | 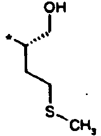 | Cl | Diastereomer (anderes Diastereomer ist Bsp. 69) | | | HPLC-MS Methode A |
| | | | | Schema 3/ | - | - | 2,46 |
| 67. | H | 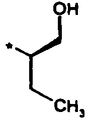 | Cl | Diastereomer (anderes Diastereomer ist Bsp. 65) | | | HPLC-MS Methode A |
| | | | | Schema 3/ | - | - | 3,08 |
| 68. | H | 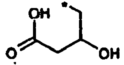 | Cl | Mischung von Stereoisomeren | | | HPLC-MS Methode A |
| | | | | Schema 3/ | - | - | 1,9 |
| 69. | H | 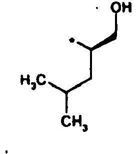 | Cl | Diastereomer (anderes Diastereomer ist Bsp. 66) | | | HPLC-MS Methode B |
| | | | | Schema 3/ | - | - | 2,45 |
| 70. | H | 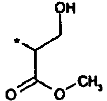 | Cl | Mischung von Stereoisomeren | | | HPLC-MS Methode A |
| | | | | Schema 3/ | 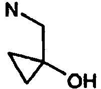 Russ. J. Russ. J. Chem. 2001,1238 | - | 2,41 |
| 71. | H | 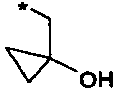 | Cl | Racemat | | | HPLC-MS Methode A |
| 72. | H | 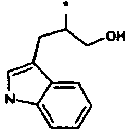 | Cl | Schema 3/ Mischung von Stereoisomeren | - | - | 2,63 HPLC-MS Methode A |
| 73. | H | 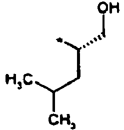 | Cl | Schema 3/ Diastereomer | - | - | 2,59 HPLC-MS Methode A |
| 74. | H | 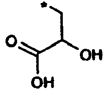 | Cl | Schema 3/ Mischung von Stereoisomeren | - | - | 2,25 HPLC-MS Methode A |
| 75. | H | 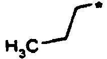 | 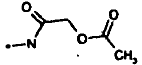 | Schema 6 Racemat | - | - | 2,15 HPLC-MS Methode A |
| | | | | Schema 6 | -- | - | 2,01 |
| 76. | H | 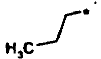 | 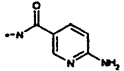 | Racemat | | | HPLC-MS Methode A |
| 77. | H | 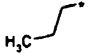 | 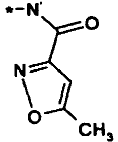 | Schema 6 Racemat | - | - | 2,43 HPLC-MS Methode A |
| 78. | H | 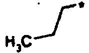 | 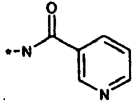 | Schema 6 Racemat | - | - | 2,03 HPLC-MS Methode A |
| 79. | H | 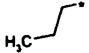 | 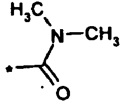 | Schema 4 Racemat | - | - | 2,18 HPLC-MS Methode A |
| 80. | H | 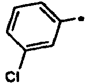 | 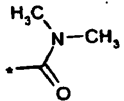 | Schema 4 Racemat | - | - | 2,55 HPLC-MS Methode A |
| 81. | H | 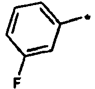 | 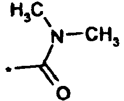 | Schema 4 Racemat | - | - | 1,72 HPLC-MS Methode A |
| | | | | Schema 3 | - | - | 1,93 |
| 82. | H | 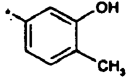 | Cl | Racemat | | | HPLC-MS Methode B |
| | | | | Schema 3 | 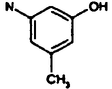 Ber. Deutsch. Chem. Ges. 1882, 2831 | - | 1,89 |
| 83. | H | 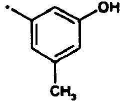 | Cl | Racemat | | | HPLC-MS Methode B |
| | | | | Schema 3 | - | - | 1,79 |
| 84. | H | 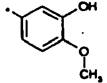 | Cl | Racemat | | | HPLC-MS Methode B |
| | | | | Schema 3 | - | - | 1,64 |
| 85. | H | 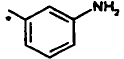 | Cl | Racemat | | | HPLC-MS Methode B |
| | | | | Schema 3 | - | - | 2 |
| 86. | H | 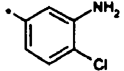 | Cl | Racemat | | | HPLC-MS Methode B |
| 87. | H | 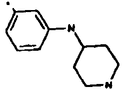 | Cl | Schema 3 | 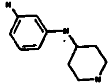 Org. Lett. | | 1,99 |
| | | | | Racemat | | | HPLC-MS |
| | | | | | 2001, 1745 WO 2005/035499 | | Methode B |
| | | | | Schema 3 | - | - | 1,78 |
| 88. | H | 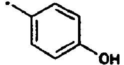 | Cl | Racemat | | | HPLC-MS Methode B |
| | | | | Schema 3 | - | - | 2,02 |
| 89. | H | 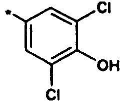 | Cl | Racemat | | | HPLC-MS Methode B |
| | | | | Schema 3 | - | - | 1,84 |
| 90. | H | 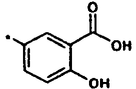 | Cl | Racemat | | | HPLC-MS Methode B |
| | | | | Schema 3 | - | - | 2,88 |
| 91. | H | 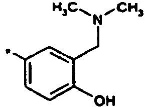 | Cl | Racemat | | | Analyt. HPLC, Methode B |
| | | | | Schema 3 | - | - | 1,88 |
| 92. | H | 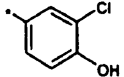 | Cl | Racemat | | | HPLC-MS Methode B |
| | | | | Schema 3 | - | - | 1,89 |
| 93. | H | 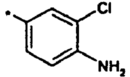 | Cl | Racemat | | | HPLC-MS Methode B |
| | | | | Schema 3 | - | - | 1,76 |
| 94. | H | 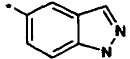 | Cl | Racemat | | | HPLC-MS Methode B |
| | | | | Schema 3 | - | - | 1,75 |
| 95. | H | 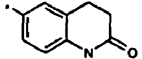 | Cl | Racemat | | | HPLC-MS Methode B |
| | | | | Schema 3 | 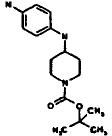 Org. Lett. Org. Lett. 2001,1745 WO 20051035499 | - | 1,95 |
| 96. | H | 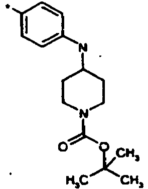 | Cl | Racemat | | | HPLC-MS Methode B |
| | | | | Schema 3 | 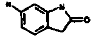 | - | 1,76 |
| 97. | H | 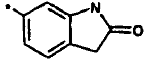 | Cl | Racemat | Hel. Chim. Acta 1937, 373 | | HPLC-MS Methode B |
| | | | | Schema 3 | - | - | 2,08 |
| 98. | H | 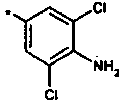 | Cl | Racemat | | | HPLC-MS Methode B |
| | | | | Schema 3 | - | - | 2,91 |
| 99. | H | 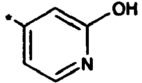 | Cl | Racemat | | | Analyt. HPLC, Methode B |
| | | | | Schema 3 | - | - | 2,98 |
| 100 | H | 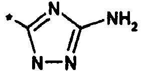 | Cl | Racemat | | | Analyt. HPLC, Methode B |
| | | | | Schema 3 | 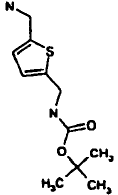 siehe exp. Teil | - | 2,06 |
| 101 | H | 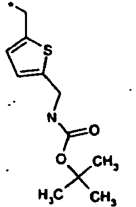 | Cl | Racemat | | | HPLC-MS Methode B |
| | | | | Schema 6 | - | - | 2,39 |
| 102 | H | 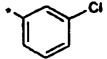 | 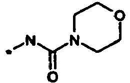 | Racemat | | | HPLC-MS Methode A |
| 103 | H | 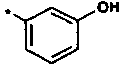 | Cl | Schema 3 | - | - | 1,83 |
| | | | | Racemat | | | HPLC-MS Methode B |
| | | | | Schema 3 | 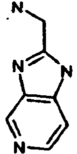 siehe exp. Teil | - | 1,55 |
| 104 H | | 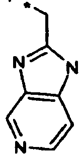 | Cl | Racemat | | | HPLC-MS Methode B |
| | | | | Schema 3 | 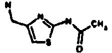 | - | 1,74 |
| 105 | H | 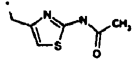 | Cl | Racemat | J. Med. Chem. 1997, *Chem*. 1997, 3726 | | HPLC-MS Methode B |
| | | | | Schema 3 | 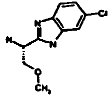 | - | 1,88 |
| 106 | H | 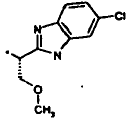 | Cl | Mischung von Diastereomeren | siehe exp. Teil | | HPLC-MS Methode B |
| | | | | Schema 3 | 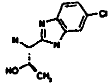 | - | 1,9 |
| 107 | H | 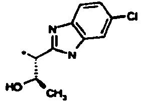 | Cl | Mischung von Diastereomeren | siehe exp. Teil | | HPLC-MS Methode B |
| | | | | Schema 3 | 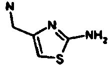 | | 1,64 |
| 108 | H | 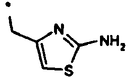 | Cl | Racemat | J. Med. Chem. 1997 , 3726 | | HPLC-MS Methode B |
| | | | | Schema 3 | - | - | 1,93 |
| 109 | H | 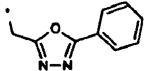 | Cl | Racemat | | | HPLC-MS Methode B |
| | | | | Schema 3 | 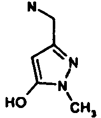 siehe exp. Teil | - | 1,61 |
| 110 | H | 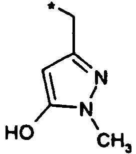 | Cl | Racemat | | | HPLC-MS Methode B |
| | | | | Schema 3 | 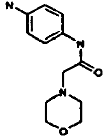 WO 2000/018734 | - | 1,65 |
| 111 | H | 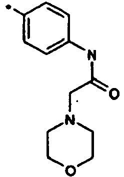 | Cl | Racemat | | | HPLC-MS Methode B |
| | | | | Schema 3 | - | - | 1,74 |
| 112 | H | 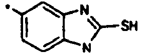 | Cl | Racemat | | | HPLC-MS Methode B |
| | | | | Schema 3 | - | - | 1,65 |
| 113 | H | 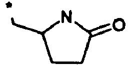 | Cl | Racemat | | | HPLC-MS Methode A |
| 114 | H | 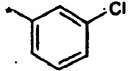 | 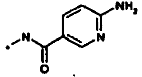 | Schema 6 | - | - | 2,74 |
| | | | | Racemat | | | Analyt. |
| | | | | Schema 3 | - | - | HPLC- Methode B 2,68 |
| 115 | H | 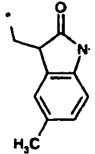 | Cl | Racemat | | | HPLC-MS Methode C |
| | | | | Schema 3 | - | - | 1,63 |
| 116 | H | 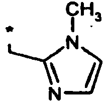 | Cl | Racemat | | | HPLC-MS Methode C |
| | | | | Schema 3 | - | - | 1,88 |
| 117 | H | 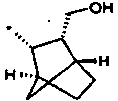 | Cl | Mischung von Diastereomeren | | | HPLC-MS Methode B |
| | | | | Schema 3 | - | - | 2,33 |
| 118 | H | 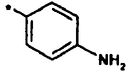 | Cl | Racemat | | | HPLC-MS Methode A |
| | | | | Schema 3 | - | - | 1,71 |
| 119 | H | 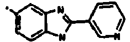 | Cl | Racemat | | | HPLC-MS, Methode B |
| 120 | H | 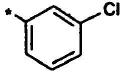 | 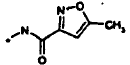 | Schema 6 | - | - | 3,15 |
| | | | | Racemat | | | Analyt. |
| | | | | | | | HPLC Methode B |
| | | | | Schema 3 | - | - | 2,29 |
| 121 | H | 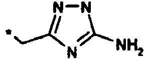 | Cl | Racemat | | | HPLC-MS Methode A |
| 122 | H | 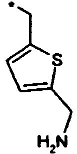 | Cl | Schema 3 Racemat | 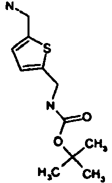 siehe exp. Teil | - | 2,34 HPLC-MS, Methode A |
| | | | | Schema 6 | | | 2,72 |
| 123 | H | 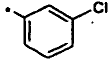 | 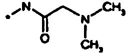 | Racemat | | | Analyt. HPLC Methode B |

Weitere Beispiele betreffen die Verbindungen der folgenden Formel **3**, 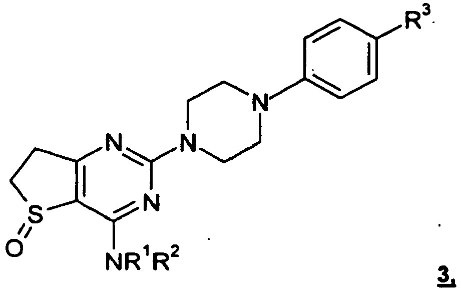 worin die die Variablen R¹, R² und R³ die in der Tabelle 1 stehenden Bedeutungen haben. Die Verbindungen wurden je nach ihrer in Tabelle 1 angegebenen Kennzeichnung nach Schema 1 bis Schema 10 hergestellt.

| **#** | **NR¹R²** | **R³** | **Synthese-Schema / Chiralität (bezogen auf gesamtes Molekül)** | **Nicht käufliche Bausteine (Herstellung beschrieben in Literatur)** | **Smp. (°C)** | **Retentionszeit [min] (mit HPLC-MS oder HPLC** |
|---|---|---|---|---|---|---|
| 124. | 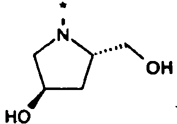 | Cl | Schema 3 Mischung von Diastereomeren | - | - | 2,26 HPLC-MS Methode A |
| 125. | 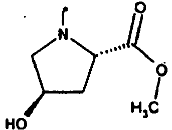 | Cl | Schema 3 Diastereomer | - | - | 2,51 HPLC-MS Methode A |
| 126. | 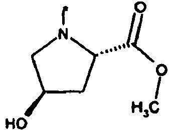 | Cl | Schema 3 Diastereomer | - | - | 2,57 HPLC-MS Methode A |
| 127. | 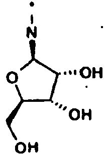 | Cl | Schema 3 Mischung von Diastereomeren | - | - | 2,26 HPLC-MS Methode A |
| 128. | 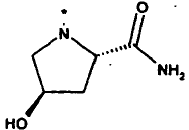 | Cl | Schema 3 Diastereomer | - | - | 1,49 HPLC-MS Methode B |
| 129. | 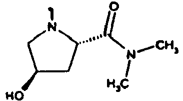 | Cl | Schema 3 Diastereomer | - | - | 4,61 HPLC-MS Methode B |
| 130. | 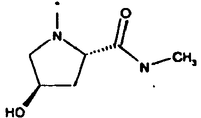 | Cl | Schema 3 Mischung von Diastereomeren | - | - | 1,57 HPLC-MS Methode B |

Weitere Beispiele betreffen die Verbindungen der folgenden Formel **3**, 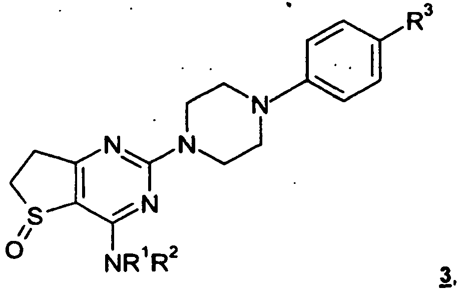 worin die die Variablen R¹, R² und R³ die in der Tabelle 1 stehenden Bedeutungen haben. Die Verbindungen wurden je nach ihrer in Tabelle 1 angegebenen Kennzeichnung nach Schema 1 bis Schema 10 hergestellt.

| # | R¹ | R² | R³ | Synthese- Schema / Chiralität (bezogen auf gesamtes Molekül) | Nicht käufliche Bausteine (Herstellung beschrieben in Literatur) | Retentionszeit [min] (mit HPLC-MS oder HPLC) |
|---|---|---|---|---|---|---|
| 131 | H | 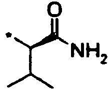 | Cl | Schema 3 (siehe experim. Teil) 1 Diastereomer | | 2.78 HPLC, Methode B |
| 132 | H | 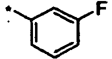 | 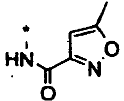 | Schema 6 Racemat | | 1,85 HPLC-MS, Methode B |
| 133 | H | 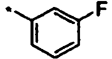 | 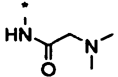 | Schema 6 Racemat | | 2.6 HPLC, Methode B |
| 134 | H | 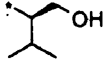 | 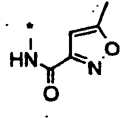 | Schema 6 1 Diastereomer | | 1,66 HPLC-MS, Methode B |
| 135 | H | 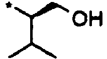 | 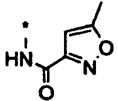 | Schema 6 1 Diastereomer | | 2.73 HPLC, Methode B |
| 136 | H | 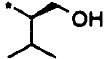 | 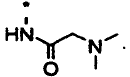 | Schema 6 1 Diastereomer | | 2.28 HPLC, Methode B |
| 137 | H | 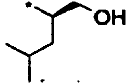 | 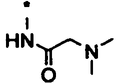 | Schema 6 1 Diastereomer | | 2.49 HPLC, Methode B |
| 138 | H | 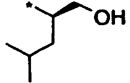 | 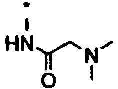 | Schema 6 1 Diastereomer | | 2.39 HPLC, Methode B |
| 139 | H | 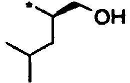 | 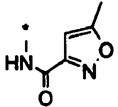 | Schema 6 1 Diastereomer | | 2.91 HPLC, Methode B |
| 140 | H | 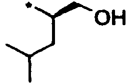 | 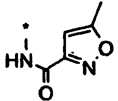 | Schema 6 1 Diastereomer | | 2.85 HPLC, Methode B |
| 141 | H | 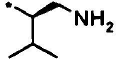 | Cl | Schema 1 (siehe experim. Teil) 1 Diastereomer | | 2.75 HPLC, Methode B |
| 142 | H | 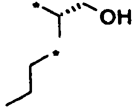 | Cl | Schema 2 1 Diastereomer | | 3.04 HPLC, Methode B |
| 143 | H | 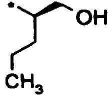 | Cl | Schema 3 (siehe experim. Teil) 1 Diastereomer | | 1,21 HPLC-MS, Methode D |
| 144 | H | 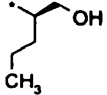 | Cl | Schema 3 (siehe experim. Teil) 1 Diastereomer | | 1,22 HPLC-MS, Methode D |
| 145 | H | 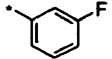 | 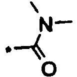 | Schema 4 (siehe experim. Teil) 1 Enantiomer | | 1,17 HPLC-MS, Methode D |
| 146 | H | 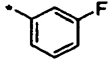 | 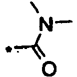 | Schema 4 (siehe experim. Teil) 1 Enantiomer | | 1,17 HPLC-MS, HPLC-MS, Methode D |
| 147 | H | 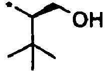 | Cl | Schema 3 1 Diastereomer | | 1,26 HPLC-MS, Methode D |
| 148 | H | 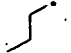 | CN | Schema 5 (siehe experim. Teil) 1 Enantiomer | | 1,16 HPLC-MS, Methode D |
| 149 | H | 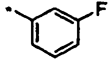 | 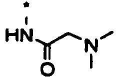 | Schema 6 (siehe experim. Teil) 1 Enantiomer | | 1,05 HPLC-MS, Methode D |
| 150 | H | 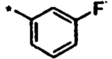 | 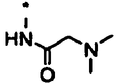 | Schema 6 (siehe experim. Teil) 1 Enantiomer | | 1,07 HPLC-MS, Methode D |
| 151 | H | 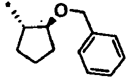 | Cl | Schema 3 (siehe experim. Teil) 1 Diastereomer | | 1,45 HPLC-MS, Methode D |
| 152 | H | 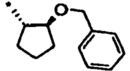 | Cl | Schema 3 (siehe experim. Teil) 1 Diastereomer | | 1,44 HPLC-MS, Methode D |
| 153 | H | 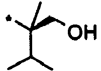 | Cl | Schema 3 (siehe experim. Teil) Mischung von Diastereomer en | 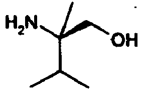 siehe experim Teil experim. | 1,27 HPLC-MS, Methode D |
| 154 | H | 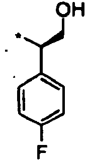 | Cl | Schema 3 1 Diastereomer | 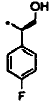 J. Med. Chem. 1999, 4981 | 1,27 HPLC-MS, Methode D |
| 155 | H | 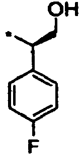 | Cl | Schema 3 1 Diastereomer | 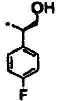 J. Med. Chem. 1999, 4981 | 1,28 HPLC-MS, Methode D |
| 156 | H | 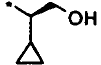 | Cl | Schema 3 1 Diastereomer | 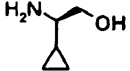 siehe # 153 | 2.9 HPLC, Methode B |
| 157 | H | 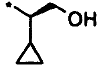 | Cl | Schema 3 1 Diastereomer | 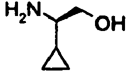 siehe # 153 | 2.94 HPLC, Methode B |
| 158 | H | 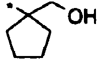 | Cl | Schema 1 (siehe experim. Teil) 1 Enantiomer | | 1,29 HPLC-MS, Methode D |
| 159 | H | 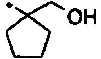 | Cl | Schema 1 (siehe experim. Teil) 1 Enantiomer | | 1,28 HPLC-MS, Methode D |
| 160 | H | 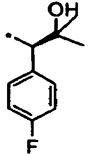 | Cl | Schema 2 (siehe experim. Teil) 1 Diastereomer | 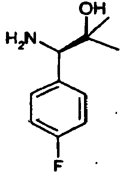 siehe experim. Teil | 1,31 HPLC-MS, Methode D |
| 161 | H | 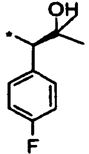 | Cl | Schema 2 (siehe experim. Teil) 1 Diastereomer | 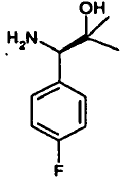 siehe experim. Teil | 1,40 HPLC-MS, Methode D |
| 162 | H | 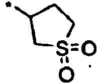 | Cl | Schema 1 Mischung von Diastereomer en | | 1,23 HPLC-MS, Methode D |
| 163 | H | 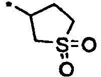 | Cl | Schema 1 Mischung von Diastereomer en | | 1,23 HPLC-MS, Methode D |
| 164 | H | 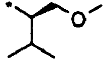 | Cl | Schema 3 1 Diastereomer | 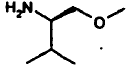 J. Org. Chem. 1978, 892 | 1,34 HPLC-MS, Methode D |
| 165 | H | 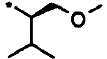 | Cl | Schema 3 1 Diastereomer | 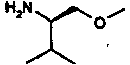 J. Org. Chem. 1978 , | 1,36 HPLC-MS, Methode D |
| 166 | H | 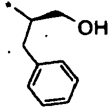 | Cl | Schema 3 1 Diastereomer | | 1,32 HPLC-MS, Methode D |
| 167 | H | 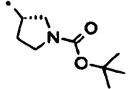 | Cl | Schema 3 (siehe # 170) Mischung von Diastereomer en | | 1,40 HPLC-MS, Methode D |
| 168 | H | 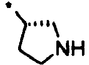 | Cl | Schema 3 (siehe # | | 2.67 HPLC, |
| | | | | 171/172) 1 Diastereomer | | Methode B |
| 169 | H | 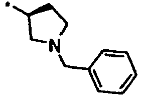 | Cl | Schema 3 Mischung von Diastereomer en | | 1,26 HPLC-MS, Methode D |
| 170 | H | 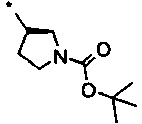 | Cl | Schema 3 (siehe experim. Teil) Mischung von Diastereomer en | | 1,39 HPLC-MS, Methode D |
| 171 | H | 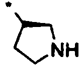 | Cl | Schema 3 (siehe experim. Teil) 1 Diastereomer | | 2.62 HPLC, Methode B |
| 172 | H | 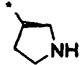 | Cl | Schema 3 (siehe experim. Teil) 1 Diastereomer | | 2.66 HPLC, Methode B |
| 173 | H | 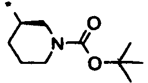 | Cl | Schema 3 (siehe # 170) Mischung von Diastereomer en | | 1,46 HPLC-MS, Methode D |
| 174 | H | 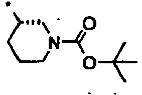 | Cl | Schema 3 (siehe # 170) Mischung von Diastereomer en | | 1,45 HPLC-MS, Methode D |
| 175 | H | 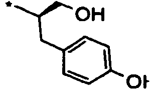 | Cl | Schema 3 (siehe experim. Teil) 1 Diastereomer | | 0,60 HPLC-MS, Methode E |
| 176 | H | 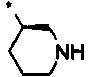 | Cl | Schema 3 (siehe # 171/172) 1 Diastereomer | | 1,08 HPLC-MS, Methode D |
| 177 | H | 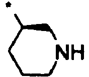 | Cl | Schema 3 (siehe # 171/172) 1 Diastereomer | | 1,11 HPLC-MS, Methode D |
| 178 | H | 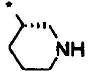 | Cl | Schema 3 (siehe # 171/172) 1 Diastereomer | | 1,13 HPLC-MS, Methode D |
| 179 | | 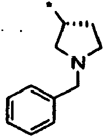 H | Cl | Schema 3 Mischung von Diastereomer en | | 1,25 HPLC-MS, Methode D |
| 180 | H | 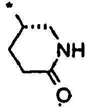 | Cl | Schema 3 Mischung von Diastereomer en | | 1,18 HPLC-MS, Methode D |
| 181 | H | 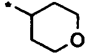 | 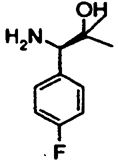 siehe experim. Teil | Schema 2 (siehe experim. Teil) 1 Enantiomer | | 1,23 HPLC-MS, Methode D |
| 182 | H | 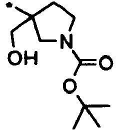 | Cl | Schema 1 Gemisch von Stereoisomer en | | 1,30 HPLC-MS, Methode D |
| 183 | H | 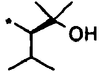 | Cl | Schema 3 Mischung von Diastereomer en | 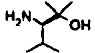 Tet. Lett. 2003, 1129 | 1,31 HPLC-MS, Methode D |
| 184 | H | 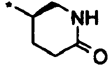 | Cl | Schema 3 Mischung von Diastereomer en | | 1,16 HPLC-MS, Methode D |
| 185 | H | 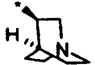 | Cl | Schema 3 1 Diastereomer | | 1.13 HPLC-MS, Methode D |
| 186 | H | 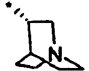 | Cl | Schema 3 1 Diastereomer | | 1,08 HPLC-MS, Methode D |
| 187 | H | 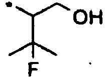 | Cl | Schema 2 (siehe experim. Teil) Racemat | 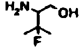 J. Org. Chem 2000, 5037 | 1,26 HPLC-MS, Methode D |
| 188 | H | 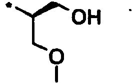 | Cl | Schema 3 1 Diastereomer | 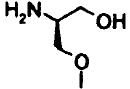 EP1348709 | 1,23 HPLC-MS, Methode D |
| 189 | H | 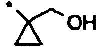 | Cl | Schema 2 (siehe experim. Teil) 1 Enantiomer | | 1,20 HPLC-MS, Methode D |
| 190 | H | 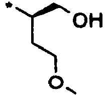 | Cl | Schema 3 1 Diastereomer | 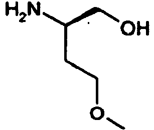 siehe # 153 siehe # 153 | 1,14 HPLC-MS, Methode D |
| 191 | H | 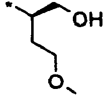 | Cl | Schema 3 1 Diastereomer | 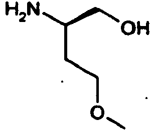 siehe # 153 | 1,16 HPLC-MS, Methode D |
| 192 | H | 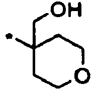 | Cl | Schema 3 Racemat | 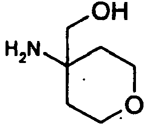 siehe # 153 | 1,13 HPLC-MS, Methode D |
| 193 | H | 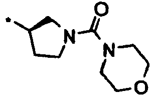 | Cl | Schema 10 (siehe experim. Teil) 1 Diastereomer | | 1,16 HPLC-MS, Methode D |
| 194 | H | 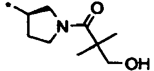 | Cl | Schema 7 (siehe # 196) 1 Diastereomer | | 1,18 HPLC-MS, Methode D |
| 195 | H | 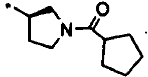 | Cl | Schema 7 (siehe # 196) 1 Diastereomer | | 1,26 HPLC-MS, Methode D |
| 196 | H | 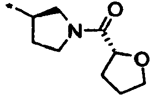 | Cl | Schema 7 (siehe experim. Teil) 1 Diastereomer | | 1,18 HPLC-MS, Methode D |
| 197 | H | 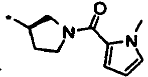 | Cl | Schema 7 (siehe experim. Teil) 1 Diastereomer | | 1,25 HPLC-MS, Methode D |
| 198 | H | 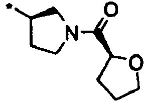 | Cl | Schema 7 (siehe experim. Teil) 1 Diastereomer | | 1,17 HPLC-MS, Methode D |
| 199 | H | 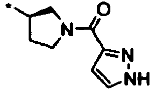 | Cl | Schema 7 (siehe # 196) 1 Diastereomer | | 1,15 HPLC-MS, Methode D |
| 200 | H | 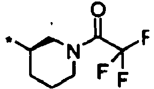 | Cl | Schema 7 (siehe # 196) 1 Diastereomer | | 1,28 HPLC-MS, Methode D |
| 201 | H | 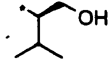 | OH | Schema 2 1 Diastereomer | | 0.95 HPLC-MS, Methode D |
| 202 | H | 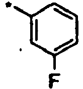 | 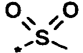 | Schema 2 (siehe experim. Teil) 1 Enantiomer | | 1.24 HPLC-MS, Methode D |
| 203 | H | 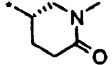 | Cl | Schema 2 (siehe experim. Teil) 1 Diastereomer | 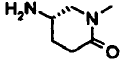 siehe experim. Teil | 1.18 HPLC-MS, Methode D |
| 204 | H | 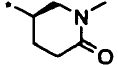 | Cl | Schema 2 (siehe experim. Teil) 1 Diastereomer | 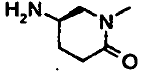 siehe # 203 | 1.19 HPLC-MS, Methode D |
| 205 | H | 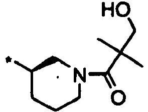 | Cl | Schema 7 (siehe # 196) 1 Diastereomer | | 1.20 HPLC-MS, Methode D |
| 206 | H | 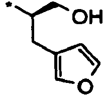 | Cl | Schema 3 (siehe experim. Teil) 1 Diastereomer | 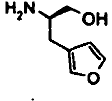 siehe experim. Teil | 1.23 HPLC-MS, Methode D |
| 207 | H | 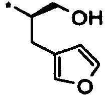 | Cl | Schema 3 (siehe experim. Teil) 1 Diastereomer | 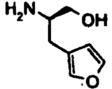 siehe experim. Teil | 1.24 HPLC-MS, Methode D |
| 208 | H | 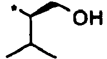 | Br | Schema 2 (siehe experim. Teil) 1 Diastereomer | | 1.21 HPLC-MS, Methode D |
| 209 | H | 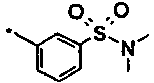 | Cl | Schema 2 Racemat | | 1.42 HPLC-MS, Methode D |
| 210 | H | 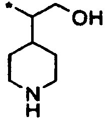 | Cl | Schema 2 Racemat | 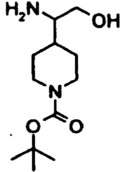 siehe # 153 | 1.04 HPLC-MS, Methode D |
| 211 | H | 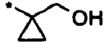 | Br | Schema 2 (siehe experim. Teil) 1 Diastereomer | | 1.18 HPLC-MS, Methode D |
| 212 | H | 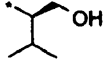 | 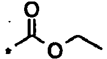 | Schema 2 (siehe experim. Teil) 1 Diastereomer | | 1.2 HPLC-MS, Methode D |
| 213 | H | 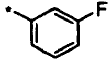 | 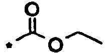 | Schema 2 (siehe # 202) 1 Enantiomer | | 1.4 HPLC-MS, Methode D |
| 214 | H | 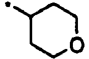 | 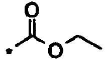 | Schema 2 (siehe experim. Teil) 1 Enantiomer | | 1.2 HPLC-MS, Methode D |
| 215 | H | 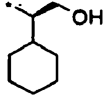 | Cl | Schema 3 1 Diastereomer | | 1.29 HPLC-MS, Methode D |
| 216 | H | 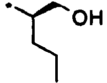 | Br | Schema 2 (siehe experim. Teil) 1 Diastereomer | | 1.23 HPLC-MS, Methode D |
| 217 | H | 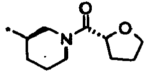 | Cl | Schema 7 (siehe # 196) 1 Diastereomer | | 1.22 HPLC-MS, Methode D |

Weitere Beispiele betreffen die Verbindungen der folgenden Formel **3**, 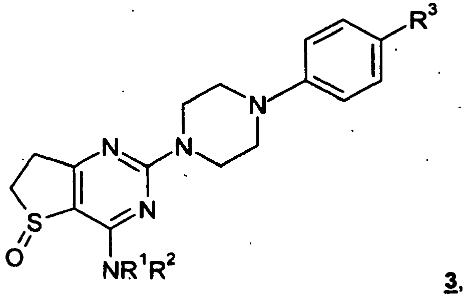 worin die die Variablen R¹, R² und R³ die in der Tabelle 1 stehenden Bedeutungen haben. Die Verbindungen wurden je nach ihrer in Tabelle 1 angegebenen Kennzeichnung nach Schema 1 bis Schema 10 hergestellt.

| # | NR¹R² | R³ | Synthese-Schema / Chiralität (bezogen auf gesamtes Molekül) | Nicht käufliche Bausteine (Herstellung beschrieben in Literatur) | Retentionszeit [min] (mit HPLC-MS oder HPLC |
|---|---|---|---|---|---|
| 218 | 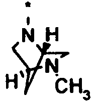 | Cl | Schema 3 1 Diastereomer | | 1,59 HPLC-MS, Methode B |
| 219 | 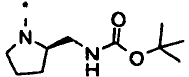 | Cl | Schema 3 (siehe experim. Teil) Mischung von Diastereomeren | | 2,00 HPLC-MS, Methode B |
| 220 | 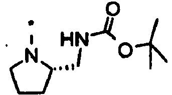 | Cl | Schema 3 (siehe # 219) Mischung von Diastereomeren | | 2,01 HPLC-MS, Methode B |
| 221 | 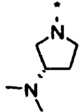 | Cl | Schema 3 Mischung von Diastereomeren | | 2,28 HPLC-MS, Methode A |
| 222 | 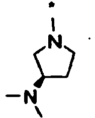 | Cl | Schema 3 Mischung von Diastereomeren | | 2,28 HPLC-MS, Methode A |
| 223 | 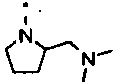 | Cl | Schema 3 (siehe experim. Teil) Racemat | 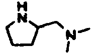 Chem. Pharm. Bull. 1970, 1731 | 2,38 HPLC-MS, Methode A |
| 224 | 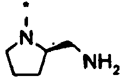 | Cl | Schema 3 (siehe experim. Teil) 1 Diastereomer | | 1,56 HPLC-MS, Methode B |
| 225 | 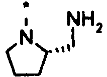 | Cl | Schema 3 (siehe # 224) 1 Diastereomer | | 1,07 HPLC-MS, Methode D |
| 226 | 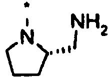 | Cl | Schema 3 (siehe # 224) 1 Diastereomer | | 1,12 HPLC-MS, Methode D |
| 227 | 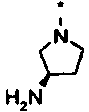 | Cl | Schema 3 Mischung von Diastereomeren | | 1,56 HPLC-MS, Methode B |
| 228 | 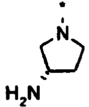 | Cl | Schema 3 Mischung von Diastereomeren | | 2,23 HPLC-MS, Methode B |
| 229 | 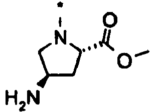 | Cl | Schema 3 (siehe experim. Teil) 1 Diastereomer | | 2.79 HPLC, Methode B |
| 230 | 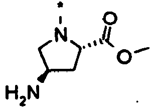 | Cl | Schema 3 (siehe experim. Teil) 1 Diastereomer | | 2.83 HPLC, Methode B |
| 231 | 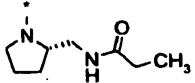 | Cl | Schema 7 (siehe experim. Teil) 1 Diastereomer | | 1,19 HPLC-MS, Methode D |
| 232 | 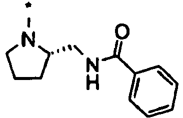 | Cl | Schema 7 (siehe # 231) 1 Diastereomer | | 1,27 HPLC-MS, Methode D |
| 233 | 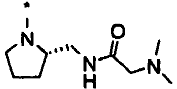 | Cl | Schema 7 (siehe # 231) 1 Diastereomer | | 1,11 HPLC-MS, Methode D |
| 234 | 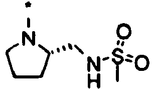 | Cl | Schema 8 (siehe experim. Teil) 1 Diastereomer | | 1,20 HPLC-MS, Methode D |
| 235 | 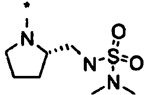 | Cl | Schema 8 (siehe # 235) 1 Diastereomer | | 1,24 HPLC-MS, Methode D |
| 236 | 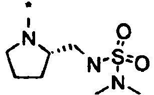 | Cl | Schema 8 (siehe # 235) 1 Diastereomer | | 1,24 HPLC-MS, Methode D |
| 237 | 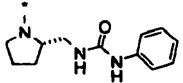 | Cl | Schema 9 (siehe experim. Teil) 1 Diastereomer | | 1,33 HPLC-MS, Methode D |
| 238 | 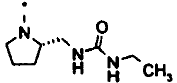 | Cl | Schema 9 (siehe # 237) 1 Diastereomer | | 1,19 HPLC-MS, Methode D |
| 239 | 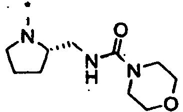 | Cl | Schema 10 (siehe experim. Teil) 1 Diastereomer | | 1,18 HPLC-MS, Methode D |
| 240 | 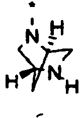 | Cl | Schema 3 1 Diastereomer | | 1,15 HPLC-MS, Methode D |
| 241 | 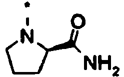 | Cl | Schema 3 1 Diastereomer | | 1,04 HPLC-MS, Methode D |
| 242 | 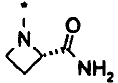 | Cl | Schema 3 (siehe # 128) 1 Diastereomer | | 1,12 HPLC-MS, Methode D |
| 243 | 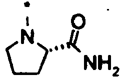 | Cl | Schema 3 1 Diastereomer | | 1,11 HPLC-MS, Methode D |
| 244 | 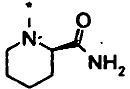 | Cl | Schema 3 (siehe # 128) 1 Diastereomer | | 1,13 HPLC-MS, Methode D |
| 245 | 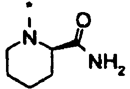 | Cl | Schema 3 (siehe # 128) 1 Diastereomer | | 1,20 HPLC-MS, Methode D |
| 246 | 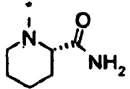 | Cl | Schema 3 (siehe # 128) 1 Diastereomer | | 1,17 HPLC-MS, Methode D |
| 247 | 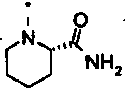 | Cl | Schema 3 (siehe #128) 1 Diastereomer | | 1.19 HPLC-MS, Methode D |
| 248 | 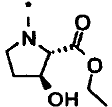 | Cl | Schema 3 1 Diastereomer | 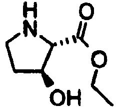 WO 2004/112793 | 1,30 HPLC-MS, Methode D |
| 249 | 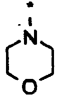 | Cl | Schema 1 Racemat | | 1,24 HPLC-MS, Methode D |
| 250 | 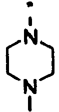 | Cl | Schema 1 Racemat | | 1,16 HPLC-MS, Methode D |
| 251 | 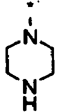 | Cl | Schema 1 Racemat | | 1,13 HPLC-MS, Methode D |

### INDIKATIONSGEBIETE

Wie gefunden wurde, zeichnen sich die Verbindungen der Formel 1 durch vielfältige Anwendungsmöglichkeiten auf therapeutischem Gebiet aus. Hervorzuheben sind solche Anwendungsmöglichkeiten, für welche die erfindungsgemäßen Verbindungen der Formel 1 aufgrund ihrer pharmazeutischen Wirksamkeit als PDE4-Inhibitor bevorzugt zur Anwendung gelangen können. Beispielhaft genannt seinen Atemwegs- oder gastrointestinalen Erkrankungen oder Beschwerden, entzündliche Erkrankungen der Gelenke, der Haut oder der Augen, Krebserkrankungen, sowie Erkrankungen des periphären oder zentralen Nervensystems.

Hierbei bevorzugt genannt sei die Vorbeugung und Behandlung von Atemwegs- oder Lungenerkrankungen, welche mit einer erhöhten Schleimproduktion, Entzündungen und/oder obstruktiven Erkrankungen der Atemwege einher gehen. Beispielhaft hierfür seinen genannt, akute, allergische oder chronische Bronchitis, chronisch obstruktive Bronchitis (COPD), Husten, Lungenemphysem, allergische oder nicht-allergische Rhinitis oder Sinusitis, chronische Rhinitis oder Sinusitis, Asthma, Alveolitis, Farmers' Krankheit, hyperreaktive Atemwege, infektiöse bronchitis oder Pneumonitis, pediatrisches Asthma, Bronchiectasien, Lungenfibrose, ARDS (akutes Atemnotsyndrom des Erwachsenen), Bronchialödem, Lungenödem, Bronchitis, Pneumonie oder interstitielle Pneumonie ausgelöst durch verschiedene Ursachen wie Aspiration, Inhalation von toxischen Gasen oder Bronchitis, Pneumonie oder interstitielle Pneumonie ausgelöst durch Herzinsuffizienz, Bestrahlung, Chemotherapie zystische Fibrose oder Mukoviszidose, alpha1-Antitrypsin-Mangel.

Ebenfalls bevorzugt genannt sei die Behandlung von entzündlichen Erkrankungen des Gastrointestinaltraktes. Beispielhaft hierfür seinen genannt, akute oder chronische entzündliche Veränderungen bei Gallenblasenentzündung, Morbus Crohn, Colitis ulcerosa, entzündliche Pseudopolypen, juvenile Polypen, Colitis cystica profunda, Pneumatosis cystoides intestinales, Erkrankungen der Gallengänge und Gallenblase, z.B. Gallensteine und Konglomerate, zur Behandlung von entzündlichen Erkrankungen der Gelenke wie rheumatoide Arthritis oder entzündliche Erkrankungen der Haut und der Augen.

Ebenfalls bevorzugt genannt sei die Behandlung von Krebserkrankungen. Beispielhaft hierfür seinen genannt alle Formen von akuten und chronischen Leukämien wie, akute lymphatische und akute myeloische Leukämie, chronisch lymphatische und chronisch myeloische Leukämie, sowie Knochentumoren wie das Osteosarkom und sowie alle Arten von Gliomen wie Oligodendrogliom und Glioblastom.

Des Weiteren bevorzugt genannt sei die Vorbeugung und Behandlung von Erkrankungen des periphären oder zentralen Nervensystems. Beispielhaft hierfür seien genannt Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Besonders bevorzugt betrifft die vorliegende Erfindung die Verwendung von Verbindungen der Formel 1 zur Herstellung eines Arzneimittels zur Behandlung entzündlicher oder obstruktiver Erkrankungen der oberen und unteren Atmungsorgane einschließlich der Lunge wie beispielsweise allergische Rhinitis, chronische Rhinitis, Bronchiectasis, zystische Fibrose, idiopathische Lungenfibrose, fibrosierende Alveolitis, COPD, chronische Bronchitis, chronischer Sinusitis, Asthma, Morbus Crohn, Colitis ulcerosa, insbesondere COPD, chronische Bronchitis und Asthma.

Am meisten bevorzugt ist die Verwendung der Verbindungen der Formel 1 zur Behandlung von entzündlichen und obstruktiven Erkrankungen wie COPD, chronische Bronchitis, chronischer Sinusitis, Asthma, Morbus Crohn, Colitis ulcerosa, insbesondere COPD, chronische Bronchitis und Asthma.

Ebenfalls bevorzugt ist die Verwendung der Verbindungen der Formel 1 zur Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Him-Trauma.

Ein herausragender Aspekt der vorliegenden Erfindung ist das reduzierte Profil an Nebenwirkungen. Darunter wird im Rahmen der Erfindung verstanden, eine Dosis einer pharmazeutischen Zusammensetzung verabreichen zu können, ohne beim Patienten Erbrechen, bevorzugt Übelkeit, besonders bevorzugt Unwohlsein auszulösen. Höchst bevorzugt ist die Verabreichung einer therapeutisch wirksamen Substanzmengen, ohne Emesis oder Nausea auszulösen, in jedem Stadium des Krankheitsverlaufs.

### KOMBINATIONEN

Die Verbindungen der Formel **1** können allein oder in Kombination mit anderen erfindungsgemäßen Wirkstoffen der Formel **1** zur Anwendung gelangen. Gegebenenfalls können die Verbindungen der Formel **1** auch in Kombination mit weiteren pharmakologisch aktiven Wirkstoffen eingesetzt werden. Bevorzugt gelangen hierbei solche Wirkstoffe zur Anwendung, die beispielsweise ausgewählt sind aus der Gruppe bestehend aus Betamimetika, Anticholinergika, Corticosteroiden, weiteren PDE4-Inhibitoren, LTD4-Antagonisten, EGFR-Hemmern, MRP4-Inhibitoren, Dopamin-Agonisten, H1-Antihistaminika, PAF-Antagonisten und PI3-Kinase Inhibitoren oder zwei- oder dreifach Kombinationen davon, wie beispielsweise Kombinationen von Verbindungen der Formel **1** mit ein oder zwei Verbindungen aus der Gruppe bestehend aus
- Betamimetika, Corticosteroiden, PDE4-Inhibitoren, EGFR-Hemmer und LTD4-Antagonisten,
- Anticholinergika, Betamimetika, Corticosteroiden, PDE4-Inhibitoren, EGFR-Hemmem und LTD4-Antagonisten,
- PDE4-Inhibitoren, Corticosteroiden, EGFR-Hemmem und LTD4-Antagonisten
- EGFR-Hemmern, PDE4-inhibitoren und LTD4-Antagonisten
- EGFR-Hemmem und LTD4-Antagonisten
- CCR3-Inhibitoren, iNOS-Inhibitoren (inducible nitric oxide synthase-Inhibitoren), (6R)-L-erythro-5,6,7,8-tetrahydrobiopterin (im folgenden "BH4" genannt) und dessen Derivate wie in WO 2006/120176 genannt und SYK-Inhibitoren (spleen tyrosine kinase-Inhibitoren)
- Anticholinergika, Betamimetika, Corticosteroiden, PDE4-Inhibitoren und MRP4-Inhibitoren.

Auch die Kombinationen dreier Wirkstoffe je einer der o.g. Verbindungsklassen ist Bestandteil der Erfindung.

Als Betamimetika gelangen hierbei vorzugsweise Verbindungen zur Anwendung, die ausgewählt sind aus der Gruppe bestehend aus Albuterol, Bambuterol, Bitolterol, Broxaterol, Carbuterol, Clenbuterol, Fenoterol, Formoterol, Arformoterol, Zinterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmeterol, Salmefamol, Soterenol, Sulphonterol, Tiaramide, Terbutaline, Tolubuterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzyl-sulfonamid, 5-[2-(5,6-Diethyl-indan-2-ylamino)-1-hydroxy-ethyl]-8-hydroxy-1H-quinolin-2-on, 4-Hydroxy-7-[2-{[2-{[3-(2-phenylethoxy)propyl]sulphonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol) 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-12-(3,4-Difluor-phenyl)-1,1-dimethyl-ethylamino]-1-hydroxy-ethyl}-6-hydroxy-4H-benzo[1,4]oxazin-3-on und 1-(4-Ethoxy-carbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Bevorzugt sind die Betamimetika ausgewählt aus der Gruppe bestehend aus Bambuterol, Bitolterol, Carbuterol, Clenbuterol, Fenoterol, Formoterol, Hexoprenaline, Ibuterol, Pirbuterol, Procaterol, Reproterol, Salmeterol, Sulphonterol, Terbutaline, Tolubuterol, 3-(4-{6-[2-Hydroxy-2-(4-hydroxy-3-hydroxymethyl-phenyl)-ethylamino]-hexyloxy}butyl)-benzenesulfoneamide, 5-[2-(5,6-Diethyl-indan-2-ylamino)-1-hydroxy-ethyl]-8-hydroxy-1H-quinolin-2-on , 4-hydroxy-7-[2-{[2-{[3-(2-phenylethoxy)propyl]sulphonyl}ethyl]-amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-on, 1-(4-amino-3-chloro-5-trifluormethylphenyl)-2-tert.-butylamino)ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropylphenyl)-1,1 dimethyl-ethylamino]-ethyl}-4H-benzo[1,4]oxazin-3-on, 8-{2-[2-(4-Ethylphenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-ethoxycarbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate. Besonders bevorzugte Betamimetika sind ausgewählt aus der Gruppe bestehend aus Fenoterol, Formoterol, Salmeterol, 3-(4-{6-[2-Hydroxy-2-(4-hydroxy-3-hydroxymethyl-phenyl)-ethylamino]-hexyloxy}-butyl)-benzenesulfoneamid, 5-[2-(5,6-Diethyl-indan-2-ylamino)-1-hydroxy-ethyl]-8-hydroxy-1H-quinolin-2-on, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxyessigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Von diesen Betamimetika sind erfindungsgemäß besonders bevorzugt Formoterol, Salmeterol, 3-(4-{6-[2-Hydroxy-2-(4-hydroxy-3-hydroxymethyl-phenyl)-ethylamino]-hexyloxy}-butyl)-benzenesulfoneamide, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 5-[2-(5,6-Diethyl-indan-2-ylamino)-1-hydroxy-ethyl]-8-hydroxy-1H-quinolin-2-on, gegebenenfalls in Form ihrer Racemate, Enantiomere, Diasteromere und gegebenenfalls in Form ihrer pharmakologisch verträglichen Säureadditionssalze, Solvate oder Hydrate.

Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Als Anticholinergika gelangen hierbei vorzugsweise Verbindungen zur Anwendung, die ausgewählt sind aus der Gruppe bestehend aus Tiotropiumsalzen, Oxitropiumsalzen, Flutropiumsalzen, Ipratropiumsalzen, Glycopyrroniumsalzen, Trospiumsalzen 2,2-Diphenylpropionsäuretropenolester-methobromid, 2,2-Diphenylpropionsäurescopinestermethobromid, 2-Fluor-2,2-Diphenylessigsäurescopinester-methobromid, 2-Fluor-2,2-Diphenylessigsäuretropenolester-methobromid, 3,3',4,4'-Tetrafluorbenzilsäuretropenolester-Methobromid, 3,3',4,4'-Tetrafluorbenzilsäurescopinester-Methobromid, 4,4'-Difluorbenzilsäuretropenolester-Methobromid, 4,4'-Difluorbenzilsäurescopinester-Methobromid, 3,3'-Difluorbenzilsäuretropenolester-Methobromid, 3,3'-Difluorbenzilsäurescopinester-Methobromid, 9-Hydroxy-fluoren-9-carbonsäuretropenolester -Methobromid, 9-Fluor-tluoren-9-carbonsäuretropenolester -Methobromid, 9-Hydroxy-fluoren-9-carbonsäurescopinester -Methobromid, 9-Fluor-fluoren-9-carbonsäurescopinester Methobromid, 9-Methyl-fluoren-9-carbonsäuretropenolester Methobromid, 9-Methyl-fluoren-9-carbonsäurescopinester Methobromid, Benzilsäurecyclopropyltropinester-Methobromid, 2,2-Diphenylpropionsäurecyclopropyltropinester -Methobromid, 9-Hydroxy-xanthen-9-carbonsäurecyclopropyltropinesterMethobromid, 9-Methyl-fluoren-9-carbonsäurecyclopropyltropinester-Methobromid, 9-Methyl-xanthen-9-carbonsäure-cyclopropyltropinester -Methobromid, 9-Hydroxy-fluoren-9-carbonsäurecyclopropyl-tropinester -Methobromid, 4,4'-Difluorbenzilsäuremethylestercyclopropyltropinester - Methobromid, 9-Hydroxy-xanthen-9-carbonsäuretropenolester -Methobromid, 9-Hydroxy-xanthen-9-carbonsäurescopinester Methobromid, 9-Methyl-xanthen-9-carbonsäure-tropenolester -Methobromid, 9-Methyl-xanthen-9-carbonsäurescopinester -Methobromid, 9-Ethyl-xanthen-9-carbonsäuretropenolester Methobromid, 9-Difluormethyl-xanthen-9-carbonsäuretropenolester -Methobromid. 9-Hydroxymethyl-xanthen-9-carbonsäure-scopinester -Methobromid, gegebenenfalls in Form ihrer Solvate oder Hydrate.

In den vorstehend genannten Salzen stellen die Kationen Tiotropium, Oxitropium, Flutropium, Ipratropium, Glycopyrronium und Trospium die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodid und Methansulfonat besonders bevorzugt.

Von besonderer Bedeutung ist das Tiotropiumbromid. Im Falle des Tiotropiumbromids enthalten die erfindungsgemäßen Arzneimittelkombinationen dieses bevorzugt in Form des kristallinen Tiotropiumbromid Monohydrats, welches aus der WO 02/30928 bekannt ist. Wird das Tiotropiumbromid in den erfindungsgemäßen Arzneimittelkombinationen in wasserfreier Form eingesetzt, so gelangt bevorzugt das wasserfreie kristalline Tiotropiumbromid zur Anwendung, welches aus der WO 03/000265 bekannt ist.

Als Corticosteroide gelangen hierbei vorzugsweise Verbindungen zur Anwendung, die ausgewählt sind aus der Gruppe bestehend aus Prednisolon, Prednison, Butixocortpropionat, Flunisolid, Beclomethason, Triamcinolon, Budesonid, Fluticason, Mometason, Ciclesonid, Rofleponid, Dexamethason, Betamethason, Dellazacort, RPR-106541, NS-126, 6,9-Difluoro-17-[(2-furanylcarbonyl)oxy]-11-hydroxy-16-methyl-3-oxo-androsta-1,4-dien-17-carbothionsäure (S)-fluoromethylester und 6,9-Difluoro-11-hydroxy-16-methyl-3-oxo-17-propionyloxy-androsta-1,4-dien-17-carbothionsäure (S)-(2-oxo-tetrahydro-furan-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt ist das Steroid ausgewählt aus der Gruppe bestehend aus Flunisolid, Beclomethason, Triamcinolon, Budesonid, Fluticason, Mometason, Ciclesonid, Rofleponid, Dexamethason,NS-126, 6,9-Difluoro-17-[(2-furanylcarbonyl)oxy]-11-hydroxy-16-methyl-3-oxo-androsta-1,4-dien-17-carbothionsäure (S)-fluoromethylester und 6,9-Difluoro-11-hydroxy-16-methyl-3-oxo-17-propionyloxy-androsta-1,4-dien-17-carbothionsäure (S)-(2-oxo-tetrahydro-furan-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt ist das Steroid ausgewählt aus der Gruppe bestehend aus Budesonid, Fluticason, Mometason, Ciclesonid und 6,9-Difluoro-17-[(2-furanylcarbonyl)oxy)-11-hydroxy-16-methyl-3-oxo-androsta-1,4-dien-17-carbothionsäure (S)-fluoromethylester, gegebenenfalls in Form ihrer Racemate, Enantiomere oder Diastereomere und gegebenenfalls in Form ihrer Salze und Derivate, ihrer Solvate und/oder Hydrate.

Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate. Phosphate, Isonicotinate, Acetate, Propionate, Dihydrögenphosphate, Palmitate, Pivalate oder auch Furoate.

Als weitere PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370,N-(3,5-Dichloro-1-oxo-pyridin-4-yl)-4-difluoromethoxy-3-cyclopropylmethoxy-benzamid, (-)p-[(4aR*,10bS*)-9-Ethoxy-1,2,3,4,4a,10b-hexahydro-8-methoxy-2-methyl-benzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis(4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, 9-Cyclopentyl-5,6-dihydro-7-ethyl-3-(2-thienyl)-9H-pyrazolo[3,4-c].1,2,4-triazölo[4,3-a]pyridin und 9-Cyclopentyl-5,6-dihydro-7-ethyl-3-(tert-butyl)-9H-pyrazolo[3,4-c]-1,2,4-triazolo[4,3-alpyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE4-Inhibitor ausgewählt aus der Gruppe bestehend aus Enprofyllin, Roflumilast, Ariflo (Cilomilast), Arofyllin, Atizorarn,AWD-12-281 (GW-842470), T-440, T-2585, PD-168787, V-11294A, Cl-1018, CDC-801, D-22888, YM-58997, Z-15370, N-(3,5-Dichloro-1-oxo-pyridin-4-yl)-4-difluoromethoxy-3-cyclopropylmethoxybenzamid, Cis[4-Cyano-4-(3-cyclopentyloxy-4-methoxyphenyl)cyclohexan-1-carbonsäure], 2-Carbomethoxy-4-cyano-4-(3-cyclopropylmethoxy-4-difluoromethoxyphenyl)cyclohexan-1-on, Cis[4-cyano-4-(3-cyclopropylmethoxy-4-difluoromethoxyphenyl)cyclohexan-1-ol], 9-Cyclopentyl-5,6-dihydro-7-ethyl-3-(2-thieny)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE4-Inhibitor ausgewählt aus der Gruppe bestehend aus Roflumilast, Ariflo (Cilomilast), Arofyllin,AWD-12-281 (GW-842470), 2-Carbomethoxy-4-cyano-4-(3-cyclopropylmethoxy-4-difluoromethoxyphenyl)cyclohexan-1-on, Cis[4-cyano-4-(3-cyclopropylmethoxy-4-difluoromethoxyphenyl)cyclohexan-1-ol], Atizoram, Z-15370, 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-alpyridin gegebenenfalls in Form ihrer Racemate, Enantiomere oder Diastereomere und gegebenenfalls in Form ihrer pharmakologisch verträglichen Säureadditionssalze, Solvate und/oder Hydrate.

Unter Säureadditionssalzen mit pharmakologisch verträglichen Säuren zu deren Bildung die o.g. PDE4-inhibitoren gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321, 1-(((R)-(3-(2-(6,7-Difluoro-2-quinolinyl)ethenyl)phenyl)-3-(2-(2- hydroxy-2-propyl)phenyl)thio)methylcyclopropan-essigsäure, 1-(((1 (R)-3(3-(2-(2,3-Dichloro-thieno[3,2-b]pyridin-5-yl)-(E)-ethenyl)phenyl)-3-(2-(1-hydroxy-1-methylethyl)phenyl)-propyl)thio)methyl)cyclopropanessigsäure und (2-[[2-(4-tert-Butyl-2-thiazolyl)-5-benzofuranyl]oxymethyl]ophenyl]essigsäure gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Bevorzugt ist der LTD4-Antagonist ausgewählt aus der Gruppe bestehend aus Montelukast, Pranlukast, Zafirlukast, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Besonders bevorzugt ist der LTD4-Antagonist ausgewählt aus der Gruppe bestehend aus Montelukast, Pranlukast, Zafirlukast, MCC-847 (ZD-3523), MN-001 und MEN-91507 (LM-1507) gegebenenfalls in Form ihrer Racemate, Enantiomere oder Diastereomere, gegebenenfalls in Form ihrer pharmakoligisch verträglichen Säureadditionssalze sowie gegebenenfalls in Form ihrer Salze und Derivate, ihrer Solvate und/oder Hydrate.

Unter Säureadditionssalzen mit pharmakologisch verträglichen Säuren zu deren Bildung die LTD4-Antagonisten gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansuffonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Unter Salzen oder Derivaten zu deren Bildung die LTD4-antagonisten gegebenenfalls in der Lage sind, werden beispielsweise verstanden : Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino)-7-cyclopropylmethoxy-chinazolin, 4-[(3-Chlor-4-fluorphenyl)amino]-6-[4-(N,N-dimethylamino)-1-oxo-2-buten-1-yl]amino}-7-cyclopropylmethoxy-chinazolin, 4-[(R)-(1-Phenyl-ethyl)arnino]-6-{[4-(morpholin-4-yl)-1-oxo-2-buten-1-yl]amino)-7-cyclopentyloxy-chinazolin, 4-{(3-Chlor-4-fluor-phenyl)amino]-6-H4-((R)-6-methyl-2-oxo-morphotin-4-yl)-1-oxo-2-buten-1-yl]amino)-7-cyclopropylmethoxy-chinazolin, 4-[(3-Chlor-4 fluorphenyl)aminoj-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxyethyl)-N-methyl-amino)-1-oxo-2-buten-1-yl)amino)-7-cyclcipropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin, 4-[(R)-(1-Phenylethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-(N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(RF(1-Phenyl-ethyl)amino]-6-(4-hydroxy-phenyl)-7H-pyrrolo[2,3-d]pyrimidin, 3-Cyano-4-[(3-chlor-4-fluorphenyl)amirio]-6-{[4-(N,N-dimethytamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonylethyl)amino]methyl}-furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluorphenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-[1-(tert.-butyloxycarbonyl)-piperidin-4-yloxy]-7-methoxy-chinazolin, 4-[(3-Chlor-4-fluorphenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4=fluor-phenyl)amino]-6-{1-[(morpholin-4-yl)carbonylj-piperidin-4-yl-oxy}-7-methoxy-chinazolin, 4-[(3-Chlor-4-fluor-phenyl)amino]-6-{1-[(methoxymethyl)carbonyl]-piperidin-4-yloxy}-7-methoxy-chinazolin, 4-[(3-Chlor-4-fluorphenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-1(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino)-6-(trans-4-[(morpholin-4-yl)carbonylamino]-cyclohexan-1-yloxy}-7-methoxy-chinazolin, 4-[(3-Chlor-4-fluorphenyl)amino]-6-{trans-4-[(morpholin-4-yl)sulfonylamino]-cyclohexan-1-yloxy}-7-methoxy-chinazolin, 4-[(3-Chlor-4-fluor-phenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluorphenyl)amino]-6-(1-aminocarbonylmethyl-piperidin-4-yloxy)-7-methoxy-chinazolin, 4-(3-Chlor-4-fluor-pheny)amino]-6-(cis-4-{N-[(tetrahydropyran-4-y)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sutfonyl]-N-methyl-amino}-cyclohexan-1-yloxy)-7-methoxy- chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluorphenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-aminofcydohexan-1-yloxy)-7-methoxy. chinazolin, 4-[(3-Chlor-4-fluor-phenyl)amino)-6-(cis-4-{N-[(4-methyl-piperazin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl)-piperidin-4-yloxyl-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyh phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7-(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino)-6-(1-(2-methoxyacetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluorphenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-ytoxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-fluor-phenyl)amino]-6-(1-ethyl-piperidin-4-yloxy)-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl. N-methyl-amino)-cyclohexan-1-yloxy]-7-methoxy-chinazolin, 4-[(3-Chlor-4-fluorphenyl)amino]-6-(cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluorphenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinaiolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, Cetuximab, Trastuzumab, ABX-EGF und Mab ICR-62, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Bevorzugte EGFR-Hemmer sind ausgewählt aus der Gruppe bestehend aus 4-[(3-Chlor-4-fluorphenyl)amino]-6{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin; 4-[(3-Chlor-4-fluor-phenyl)amino]-6-{[4-((R)-2-methoxymethyl-6-oxo-morpholin-4-yl)-1-oxo-2-buten-1-yl]amino}-7-cydopropylmethoxy-chinazolin, 4-[(3-Chlor-4-fluorphenyl)amino]-6-[2-((S)-6-methyl-2.oxo.morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(R)-(1-Phenyl-ethyl)amino]-6-{[4-(N,N-bis-(2-methoxy-ethyl)-amino)-1-oxo-2-buten-1-yljamino}-7-cyclopropylmethoxy-chinazolin, 4-[(R)-(1-Phenyl-ethyl)amino]-6-(4-[N-(2-methoxy-ethyl)-N-ethyl-amino]-1-oxo-2-buten-1-yl}amino)-7-cyclopropylmethoxy-chinazolin, 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl}amino}-7-((R)-tetrahydrofuran-3-yloxy)-chinazolin 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4=[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-ylr ethoxy]-7-[(R)-(tetrahydrofuran-2-yl)methoxy]-chinazolin, 4-(3-Chlor-4-fluorphenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-[1-(tert.-butyloxycarbonyl)-piperidin-4-yloxy]-7-methoxy-chinazolin, 4-[(3-Chlor-4-fluorphenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl)carbonyl]-piperidin-4-yl-oxy}-7-methoxy-chinazolin, 4-[(3-Chlor-4-fluor-phenyl)amino)-6-{1-[(methoxymethyl)carbonyl]-piperidin-4-yloxy}-7-methoxy-chinazolin, 4-[(3-Chlor-4-fluorphenyl)amino]-6-(piperidin-3-yloxy)-7-methoxy-chinazolin, 4-[(3-Chlor-4-fluorphenyl)amino]-6-[1-(2-acetylamino-ethyl)-piperidin-4-yloxy]-7-methoxy-7-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4. yl)carbonylamino]-cyclohexan-1-yloxy}-7-methoxy-chinazolin, 4-[(3-Chlor-4-fluor-phenyl)amino]-6-{trans-4-[(morpholin-4-yl)sulfonylamino]-cyclohexan-1-yloxy}-7-methoxy-chinazolin, 4-[(3-Chlor-4-fluor-phenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluorphenyl)amino]-6-(1-aminocarbonylmethyl-piperidin-4-yloxy)-7-methoxy-chinazolin, 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin, 4-[(3-Chlor-4-fluor-phenyl)amino]-6-(cis-4-[(morpholin-4-yl)carbonylamino]-cyclohexan-1-yloxy}-7-methoxy-chinazolin, 4-[3-Chlor-4-fluor-phenyl)amino]-6-(1-[2-(2-oxopyrrolidin-1-yl)ethyl]-piperidin-4-yloxy}-7-methoxy-chinazolin, 4-{(3-Chlor-4-fluor-phenyl)amino)-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxyacetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[3-Chlor-4-fluorphenyl)aminol-6-{1-[(cis-2,6-dimethyl-morpholin-4-yl)carbonyl)-piperidin-4-yloxy)-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluorphenyl)amino]-6-(1-ethyl-piperidin-4-yloxy)-7-methoxy-chinazolin, 4-[(3-Chlor-fluor-phenyl)amino]-6-[1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluorphenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluorphenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyll-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und Cetuximab, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Besonders bevorzugt gelangen im Rahmen der vorliegenden Erfindung diejenigen EGFR-Hemmer zur Anwendung, die ausgewählt sind aus der Gruppe bestehend aus 4-[(3-Chlor. 4-fluophenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino)-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-({4-[N-(2-methoxy-ethyl)-N-methyl-amino)-1-oxo-2-buten-1-yl}amino)-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinyl-phenyl)amino]-6,7-bis-(2-methoxy-ethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-([4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-2-[4-(2-oxo-morpholin-4-yl)-piperidin-1-yl]-ethoxy}-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cydohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluorphenyl)amino]-6-{1-[(morpholin-4-yl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino)-6-(cis-4-(N-[(morpholin-4-yl)carbonyl]-N-methyl-amino}-cyclohexan-1-yloxy)-7-methoxy-chinazolin, 4-(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino)-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Ethinyl-phenyl)amino]-6-(tetrahydropyran-4-yloxy]-7-methoxy-chinazolin-, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluorphenyl)amino]-6-(1-ethyl-piperidin-4-yloxy)-7-methoxy-chinazolin, 4-[(3-Chlor-4-fluorphenyl)amino]-6-[cis-4-(N-methansulfonyl-N-methyl-amino)-cyclohexan-1-yloxyl-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino)-6-(trans-4-methylamino-cyclohexan-1-yloxy)-7-methoxy-chinazolin, 4-[(3-Chlor-4-fluorphenyl)amino]-6-[trans-4-(N-methansulfonyl-N-methyl-amino)-cydohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-(morpholin-4-yl}carbonyl)-N-methyl-amino}-cyclohexan-1-yloxy}-7-methoxy-chihazolin, 4-[(3-Chlo-4-fluor-phenyl)amino]-6-[2-(2,2-dimethyl-6-oxo-morpholin-4-yl)-ethoxy]-7-[(S)-(tetrahydrofuran-2-yl)methoxy)-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxy-chinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Erfindungsgemäß besonders bevorzugt sind als EGFR-Hemmer diejenigen Verbindungen, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-(N-(2-methoxy-ethyl)-N-methyl-amino]-1-oxo-2-buten-1-yl}amino)-7-cyclopropylmethoxy-chinazolin, 4-[(3-Ethinyl-phenyl)amino]-6,7-bis-(2-methoxy-ethoxy)-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluorphenyl)amino]-6-(tetrahydropyran-3-yloxy)-7-methoxy-chinazolin, 4-[(3-Chlor-4-fluorphenyl)amino]-6-{1-[(morpholin-4-yl)carbonyl]-piperidin-4-yloxy}-7-methoxy-chinazolin, 4-[(3-Chlor-4-fluor-phenyl)amino]-6-(1-[2-(2-oxopyrrolidin-1-yl)ethyl)-piperidin-4-yloxy)-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxyl-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluorphenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxyl-7-[(S)-(tetrahydrofuran-2-yl)methoxyl-chinazolin, 4-[(3-Chlor-4-fluor-phenyl)amino]-6-(1-methansuifonyl-piperidin-4-yloxy)-7-methoxy-chinazolin und 4-[(3-Chlor-4-fluor-phenyl)amino]-6-(1-cyano-piperidin-4-yloxy)-7-methoxy-chinazolin gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die EGFR-Hemmer gegebenenfalls in der Lage sind, werden beispielsweise Satze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfit. Hydrophosphat, Hydrofumarat und Hydromethansuffonat verstanden.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopamin-Agonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Eine Bezugnahme auf die vorstehend genannten H1-Antihistaminika schließt im Rahmen der vorliegenden Erfindung eine Bezugnahme auf deren gegebenenfalls existierende pharmakologisch verträgliche Säureadditionssalze ein.

Als PAF-Antagonisten gelangen hierbei vorzugsweise Verbindungen zur Anwendung, die ausgewählt sind aus der Gruppe bestehend aus 4-(2-Chlorphenyl)-9-methyl-2-[3(4-morpholinyl)-3-propanon-1-yl]-6H-thieno-[3,2-f]-[1,2,4]triazolo[4,3-a][1,4]diazepin, 6-(2-Chlorphenyl)-8,9-dihydro-1-methyl-8-[(4-morpholinyl)carbonyl]-4H-7H-cyclo-penta-[4,5)thieno-[3,2-f][1,2,4]triazoto[4,3-a][1,4]diazepin.

Als MRP4-Inhibitoren gelangen hierbei vorzugsweise Verbindungen zur Anwendung, die ausgewählt sind aus der Gruppe bestehend aus N-Acetyl-dinitrophenyl-Cysteine, cGMP, Cholate, Diclofenac, Dehydroepiandrosterone 3-glucuronide, Dehydroepiandrosterone 3-sulphate, Dilazep, Dinitrophenyl-S-glutathione, Estradiol 17-β-glucuronide, Estradiol 3,17-disulphate, Estradiol 3-glucuronide, Estradiol 3-sulphate, Estrone 3-sulphate, Flurbiprofen, Folate, N5-formyl-tetrahydrofolate, Glycocholate, Glycolithocholic acid sulphate, Ibuprofen, Indomethacin, Indoprofen, Ketoprofen, Lithocholic acid sulphate, Methotrexate, MK571 ((*E*)-3-[[[3-[2-(7-Chloro-2-quinolinyl)ethenyl)phenyl]-[[3-dimethylamino)-3-oxopropyl]thio]methyl]thio]-propanoic acid), α-Naphthyl-β-D-glucuronide, Nitrobenzyl mercaptopurine riboside, Probenecid, PSC833, Sildenafil, Sulfinpyrazone, Taurochenodeoxycholate, Taurocholate, Taurodeoxycholate, Taurolithocholate, Taurolithocholic acid sulphate, Topotecan, Trequinsin und Zaprinast, Dipyridamol, gegebenenfalls in Form ihrer Racemate, Enantiomere, Diastereomere und ihrer pharmakologisch verträglichen SäureAdditionssalze und Hydrate.

Vorzugsweise bezieht sich die Erfindung auf die Verwendung von MRP4-Inhibhoren zur Herstellung einer pharmazeutischen Zusammensetzung zur Behandlung von Atemwegserkrankungen enthaltend die erfindungsgemäßen PDE4B-Inhibftoren und MRP4-Inhibitoren, wobei die MRP4-Inhibitoren vorzugsweise ausgewählt sind aus der Gruppe bestehend aus *N*-Acetyl-dinitrophenyl-Cysteine, Dehydroepiandrosterone 3-sulphate, Dilazep, Dinitrophenyl-*S*-glutathione, Estradiol 3,17-disulphate, Flurbiprofen, Glycocholate, Glycolithocholic acid sulphate, Ibuprofen, Indomethacin, Indoprofen, Lithocholic acid sulphate, MK571, PSC833, Sildenafil, Taurochenodeoxycholate, Taurocholate, Taurolithocholate, Taurolithocholic acid sulphate, Trequinsin und Zaprinast, Dipyridamol, gegebenenfalls in Form ihrer Racemate, Enantiomere, Diastereomere und ihrer pharmakologisch verträglichen SäureAdditionssalze und Hydrate.

Stärker bevorzugt bezieht sich die Erfindung auf die Verwendung von MRP4-Inhibitoren zur Herstellung einer pharmazeutischen Zusammensetzung zur Behandlung von Atemwegserkrankungen enthaltend die erfindungsgemäßen PDE4B-Inhibitoren und MRP4-Inhibitoren, wobei die MRP4-Inhibitoren vorzugsweise ausgewählt sind aus der Gruppe bestehend aus Dehydroepiandrosterone 3-sulphate, Estradiol 3,17-disulphate, Flurbiprofen, Indomethacin, Indoprofen, MK571, Taurocholate, gegebenenfalls in Form ihrer Racemate, Enantiomere, Diastereomere und ihrer pharmakologisch verträglichen SäureAdditionssalze und Hydrate. Die Trennung von Enantiomeren aus den Racematen kann durch bekannte Verfahren nach dem Stand der Technik durchgeführt werden (z.B. durch Chromatographie an chiralen Phasen etc.).

Mit Säure-Additionssalzen mit pharmakologisch verträglichen Säuren sind z.B. Salze ausgewählt aus der Gruppe bestehend aus Hydrochloriden, Hydrobromiden, Hydroiodiden, Hydrosulphaten, Hydrophosphaten, Hydromethanesulphonaten, Hydronitraten, Hydromateaten, Hydroacetaten, Hydrobenzoaten, Hydrocitraten, Hydrofumaraten, Hydrotartraten, Hydrooxalaten, Hydrosuccinaten, Hydrobenzoaten and Hydro-*p*-toluenesulphonaten, vorzugsweise Hydrochloride, Hydrobromide, Hydrosulphate, Hydrophosphate, Hydrofumarate and Hydromethanesulphonate gemeint.

Ein weiterer Gegenstand der Erfindung sind pharmazeutische Zubereitungen, die Dreifachkombinationen von den erfindungsgemäßen PDE4B-Inhibitoren, von MRP4-Inhibitoren und einer weiteren aktiven Substanz wie z.B. einem Anticholinergikum, einem Steroid, einem LTD4-Antagonist oder einem Betamimetikum enthalten, sowie deren Herstellung und deren Verwendung zur Behandlung von Atemwegserkrankungen.

Als iNOS-Inhibitoren gelangen hierbei vorzugsweise Verbindungen zur Anwendung, die ausgewählt sind aus der Gruppe bestehend aus: S-(2-Aminoethyl)isothioharnstoff, Aminoguanidin, 2-Aminomethylpyridin, AMT, L-Canavanin, 2-Iminopiperidin, S-Isopropylisothioharnstoff, S-Methylisothioharnstoff, S-Ethylisothiohamstoff, S-Methyltiocitrullin, S-Ethylthiocitrullin, L-NA (N^{w}-Nitro-L-arginin), L-NAME (N^{w}-Nitro-L-argininmethylester), L-NMMA (N^{G}-Monomethyl-L-arginin), L-NIO (N^{w}-Iminoethyl-L-ornithin), L-NIL (N^{ω}-Iminoethyl-lysin), (S)-6-Acetimidoylamino-2-amino-hexanoic acid (1*H-*tetrazol-5-yl)-amid (SC-51) *(*J. Med. Chem. 2002, 45, 1686-1689), 1400W, (S)-4-(2-Acetimidoylamino-ethylsulfanyl)-2-amino-buttersäure (GW274150) *(*Bioorg. Med. Chem. Lett. 2000, 10, 597-600), 2-[2-(4-Methoxy-pyridin-2-yl)-ethyl]-3*H*-imidazo[4,5-b]pyridin (BYK191023) *(*Mol. Pharmacol. 2006, 69, 328-337), 2-((R)-3-Amino-1-phenyl-propoxy)-4-chlor-5-fluorbenzonitril (WO 01/62704), 2-((1R,3S)-3-Amino-4-hydroxy-1-thiazol-5-yl-butylsulfanyl)-6-trifluoromethyl-nicotinonitril (WO 2004/041794), 2-((1R,3S)-3-Amino-4-hydroxy-1-thiazol-5-yl-butylsulfanyl)-4-chlor-benzonitril (WO 2004/041794), 2-((1R,3S)-3-Amino-4-hydroxy-1-5-thiazol-5-yl-butylsulfanyl)-5-chlor-benzonitril (WO 2004/041794), (2S,4R)-2-Amino-4-(2-chlor-5-trifluoromethyl-phenylsulfanyl)-4-thiazol-5-yl-butan-1-ol (WO 2004/041794), 2-((1R,3S)-3-Amino-4-hydroxy-1-thiazol-5-yl-butylsulfanyl)-5-chlor-nicotinonitril (WO 2004/041794), 4-((S)-3-Amino-4-hydroxy-1-phenyl-butylsulfanyl)-6-methoxy-nicotinonitril (WO 02/090332); substituierte 3-Phenyl-3,4-dihydro-l-isoquinolinamin wie z.B. AR-C102222 (J. Med. Chem. 2003, 46, 913-916), (1S,5S,6R)-7-Chlor-5-methyl-2-aza-bicyclo[4.1.0]hept-2-en-3-ylamin (ONO-1714) *(*Biochem. Biophys. Res. Commun. 2000, 270, 663-667), (4R,5R)-5-Ethyl-4-methyl-thiazolidin-2-ylideneamin *(*Bioorg. Med. Chem. 2004, 12, 4101), (4R,5R)-5-Ethyl-4-methyl-selenazolidin-2-*ylideneamin (*Bioorg. Med. Chem. Lett. 2005, 15, 1361), 4-Aminotetrahydrobiopterin *(*Curr. Drug Metabol. 2002, 3,119-121), (E)-3-(4-Chlor-phenyl)-N-(1-{2-oxo-2-[4-(6-trifluormethyl-pyrimidin-4-yloxy)-piperidin-1-yl]-ethylcarbamoyl}-2-pyridin-2-yl-ethyl)-acrylamid (FR260330) *(*Eur. J. Pharmacol. 2005, 509, 71-76), 3-(2,4-Difluor-phenyl)-6-[2-(4-imidazol-1-ylmethyl-phenoxy)-ethoxy]-2-phenyl-pyridin (PPA250) *(*J. Pharmacol. Exp. Ther. 2002, 303, 52-57), 3-{[(Benzo[1,3]dioxol-5-ylmethyl)-carbamoyl]-methyl}-4-(2-imidazol-1-yl-pyrimidin-4-yl)-piperazin-1-carbonsäuremethylester (BBS-1) *(*Drugs Future 2004, 29, .45-52), (R)-1-(2-Imidazol-1-yl-6-methyl-pyrimidin-4-yl)-pyrrolidin-2-carbonsäure (2-benzo[1,3]dioxol-5-yl-ethyl)-amid (BBS-2) *(*Drugs Future 2004, 29, 45-52) und deren pharmazeutischen Salze, Prodrugs oder Solvate.

Als iNOS-Inhibitoren im Rahmen der vorliegenden Erfindung können weiterhin antisense-Oligonucleotide, insbesondere solche antisense-Oligonucleotide, die iNOS-kodierende Nukleinsäuren binden, eingesetzt werden. Z.B. werden in WO 01/52902 antisense-Oligonucleotide, insbesondere antisense-Oligonucleotide, die iNOS kodierende Nukleinsäuren binden, zur Modulierung der Expression von iNOS beschrieben. Solche iNOS-antisense-Oligonucleotide wie insbesondere in WO 01/52902 beschrieben können daher auch aufgrund ihrer ähnlichen Wirkung wie die iNOS-Inhibitoren mit den PDE4-Inhibitoren der vorliegenden Erfindung kombiniert werden.

Als SYK-Inhibitoren gelangen hierbei vorzugsweise Verbindungen zur Anwendung, die ausgewählt sind aus der Gruppe bestehend aus: 2-[(2-aminoethyl)amino]-4-[(3-bromophenyl)amino]-5-Pyrimidinecarboxamide; 2-[[7-(3,4-dimethoxyphenyl)imidazo[1,2-c]pyrimidin-5-yl]amino]-3-Pyridinecarboxamide; 6-[[5-fluoro-2-[3,4,5-trimethoxyphenyl)amino]-4-pyrimidinyl]amino]-2,2-dimethyl-2H-Pyrido[3,2-b]-1,4-oxazin-(4H)-one; N-[3-bromo-7-(4-methoxyphenyl)-1,6-naphthyridin-5-yl]-1,3-Propanediamine 7-(4-methoxyphenyl)-N-methyl-1,6-Naphthyridin-5-amine; N-[7-(4-methoxyphenyl)-1,6-naphthyridin-5-yl]-1,3-Propanediamine; N-[7-(2-thienyl)-1,6-naphthyridin-5-yl-1,3-Propanediamine; N-[7-[4-(dimethylamino)phenyl]-1,6-naphthyridin-5-yl]-1,2-Ethanediamine; N-[7-(4-methoxyphenyl)-2-(trifluoromethyl)-1,6-naphthyridin-5-yl]- 1,3-Propanediamine; N-[7-(4-methoxyphenyl)-3-phenyl-1,6-naphthyridin-5-yl]-1,3-Propanediamine; N-(7-phenyl-1,6-naphthyridin-5-yl)-1,3-Propanediamine; N-[7-(3-fluorophenyl)-1,6-naphthyridin-5-yl]-1,3-Propanediamine; N-[7-(3-chlorophenyl)-1,6-naphthyridin-5-yl]-1,3-Propanediamine; N-[7-[3-(trifluoromethoxy)phenyl]-1,6-naphthyridin-5yl]-1,3-Propanediamine; N-[7-(4-fluorophenyl)-1,6-naphthyridin-5-yl]-1,3-Propanediamine; N-[7-(4-fluorophenyl)-1,6-naphthyridin-5-yl]-1,3-Propanediamine; N-[7-(4-chlorophenyl)-1,6-naphthyridin-5-yl]-1,3-Propanediamine; N-[7-(4'-methyl[1,1'-biphenyl]-4-yl)-1,6-naphthyridin-1,3-Propanediamine; N-[7-[4-(dimethylamino)phenyl]-1,6-naphthyridin-5-yl]-1,3-Propanediamine; N-[7-[4-(diethylamino)phenyl]-1,6-naphthyridin-5-yl]-1,3-Propanediamine; N-[7-[4-(4-morpholinyl)phenyl]-1,6-naphthyridin-5-yl]-1,3-Propanediamine; N-[7-[4-[[2-(dimethylamino)ethyl]methylamino]phenyl]-1,6-naphthyridin-5-yl]-1,3-Propanediamine; N-[7-(4-bromöphenyl)-1,6-naphthyridin-5-yl]-1,3-Propanediamine; N-[7-(4-methylphenyl)-1,6-naphthyridin-5-yl]-1,3-Propanediamine; N-[7-[4-(methylthio)phenyl]-1,6-naphthyridin-5-yl]-1,3-Propanediamine; N-[7-[4-(1-methylethyl)phenyl]-1,6-naphthyridin-5-yl]-1,3-Propanediamine; 7-[4-(dimethylamino)phenyl]-N-methyl-1,6-Naphthyridin-5-amine; 7-[4-(dimethylamino)phenyl]-N,N-dimethyl-1,6-Naphthyridin-5-amine; N-[7-[4-(dimethylamino)phenyl]-1,6-naphthyridin-5-yl]-1,4-Butanediamine; N-[7-[4-(dimethylamino)phenyl]-1,6-naphthyridin-5-ylj-1,5-Pentanediamine; 3-[[7-[4-(dimethylamino)phenyl]-1,6-naphthyridin-5-yl]oxy]-1-Propanol; 4-[5-(4-aminobutoxy)-1,6-naphthyridin-7-yl]-N,N-dimethyl-Benzenamine; 4-[[7-[4-(dimethylamino)phenyl]-1,6-naphthyridin-5-yl]amino]-1-Butanol; N-[7-[4-(dimethylamino)phenyl]-1,6-naphthyridin-5-yl]-N-methyl-1,3-Propanediamine; N-[7-[4-(dimethylamino)phenyl]-1,6-naphthyridin-5-yl]-N'-methyl-1,3-Propanediamine; N-[7-[4-(dimethylamino)phenyl]-1,6-naphthyridin-5-yl]-N,N'-dimethyl-1,3-Propanediamine; 1-amino-3-[[7-[4-(dimethylamino)phenyl]-1,6-naphthyridin-5-yl]amino]-2-Propanol; N-[7-[4-(dimethylamino)phenyl]-1,6-naphthyridin-5-yl]-2,2-dimethyl-1,3-Propanediamine; 7-[4-(dimethylamino)phenyl]-N-(3-pyridinylmethyl)-1,6-Naphthyridin-5-amine; N-[(2-aminophenyl)methyl)-7-(4-(dimethylamino)phenyl]-1,6-Naphthyridin-5-amine; N-[7-[6-(dimethylamino)[1,1'-biphenyl]-3-yl]-1,6-naphthyridin-5-yl]-1,3-Propanediamine,; N-[7-[3-chloro-4-(diethylamino)phenyl]-1,6-naphthyridin-5-yl]-1,3-Propanediamine; N-[7-[4-(dimethylamino)-3-methoxyphenyl]-1,6-naphthyridin-5-yl]-1,3-Propanediamine; N-[7-[4-(diethylamino)phenyl]-3-methyl-1,6-naphthyridin-5-yl]-1,3-Propanediamine; N-[7-(3'-fluoro[1,1'-biphenyl]-3-yl)-1,6-naphthyridin-5-yl]-1,2-Ethanediamine, N-[7-(4-methoxyphenyl)-1,6-naphthyridin-5-yl]-1,6-Naphthyridine-1,3-Propanediamine; N,N'-bis(3-aminopropyl)-7-(4-methoxyphenyl)-2,5-diamine; N-[7-(4-methoxyphenyl)-2-(phenylmethoxy)-1,6-naphthyridin-5-yl]-1,6-Naphthyridine-1,3-Propanediamine; N5-(3-aminopropyl)-7-(4-methoxyphenyl)-N2-(phenylmethyl)-2,5-diamine; N-[7-(2-naphthalenyl)-1,6-naphthyridin-5-yl]-1,3-Propanediamine; N-[7-(2'-fluoro[1,1'-biphenyl]-4-yl)-1,6-naphthyridin-5-yl]-1,3-Propanediamine; N-[7-(3,4,5-trimethoxyphenyl)-1,6-naphthyridin-5-yl]-1,3-Propanediamine; N-[7-(3,4-dimethylphenyl)-1,6-naphthyridin-5-yl]-1,3-Propanediamine; 1-amino-3-[[7-(2-naphthalenyl)-1,6-naphthyridin-5-yl]amino]-2-Propanol; 1-amino-3-[[7-(2'-fluoro[1,1'-biphenyl]-4-yl)-1,6-naphthyridin-5-yl]amino]-2-Propanol; 1-amino-3-[[7-(4'-methoxy[1,1'-biphenyl]-4-yl)-1,6-naphthyridin-5-yl]amino]-2-Propanol; 1-amino-3-[[7-(3,4,5-trimethoxyphenyl)-1,6-naphthyridin-5-yl]amino]-2-Propanol; 1-amino-3-[[7-(4-bromophenyl)-1,6-naphthyridin-5-yl]amino]-2-Propanol; N-[7-(4'-methoxy[1,1'-biphenyl]-4-yl)-1,6-naphthyridin-5-yl]-2,2-dimethyl-1,3-Propanediamine; 1-[[7-[4-(dimethylamino)phenyl]-1,6-naphthyridin-5-yl]amino]-2-Propanol; 2-[[2-[[7-[4-(dimethylamino)phenyl]-1,6-naphthyridin-5-yl]amino]ethyl]thio]-Ethanol; 7-[4-(dimethylam ino)phenyl]-N-(3-methyl-5-isoxazolyl)-1,6-Naphthyridin-5-amine; 7-[4-(dimethylamino)phenyl]-N-4-pyrimidinyl-1,6-Naphthyridin-5-amine; N-[7-[4-(dimethylamino)phenyl]-1,6-naphthyridin-5-yl]-1,3-Cyclohexanediamine; N,N-dimethyl-4-[5-(1-piperazinyl)-1,6-naphthyridin-7-yl]-Benzenamine; 4-[5-(2-methoxyethoxy)-1,6-naphthyridin-7-yl]-N,N-dimethyl-Benzenamine; 1-[7-[4-(dimethylamino)phenyl]-1,6-naphthyridin-5-yl]-4-Piperidinol; 1-[7-[4-(dimethylamino)phenyl]-1,6-naphthyridin-5-yl]-3-Pyrrolidinol; 7-[4-(dimethylamino)phenyl]-N-(2-furanylmethyl)-1,6-Naphthyridin-5-amine; 7-[4-(dimethylamino)phenyl]-N-[3-(1H-imidazol-1-yl)propyl]-1,6-Naphthyridin-5-amine; 1-[7-[4-(dimethylamino)phenyl]-1,6-naphthyridin-5-yl]-4-Piperidinecarboxamide; 1-[3-[[7-[4-(dimethylamino)phenyl]-1,6-naphthyridin-5-yl]amino]propyl]-2-Pyrrolidinone; N-[3'-[5-[(3-aminopropyl)amino]-1,6-naphthyridin-7-yl][1,1'-biphenyl]-3-yl]-Acetamide; N-[7-(4'-fluoro[1,1'-biphenyl]-4-yl)-1,6-naphthyridin-5-yl]-1,3-Propanediamine; N-[4'-[5-[(3-aminopropyl)amino]-1,6-naphthyridin-7-yl][1,1'-biphenyl]-3-yl]-Acetamide; N-[7-[4-(1,3-benzodioxol-5-yl)phenyl]-1,6-naphthyridin-5-yl]-1,3-Propanediamine; N-[7-[4-(2-thienyl)phenyl]-1,6-naphthyridin-5-yl]-1,3-Propanediamine; N-[7-[4-fluoro-3-(trifluoromethyl)phenyl]-1,6-naphthyridin-5-yl]-1,3-Propanediamine; N-[7-[4-(3-pyridinyl)phenyl]-1,6-naphthyridin-5-yl]-1,3-Propanediamine; N-[7-(1,3-benzodioxol-5-yl)-1,6-naphthyridin-5-yl]-1,3-Propanediamine; N-[7-(6-methoxy-2-naphthalenyl)-1,6-naphthyridin-5-yl]-1,3-Propanediamine; 7-[4-(dimethylamino)phenyl]-N-(4-pyridinylmethyl)-1,6-Naphthyridin-5-amine; 3-[[7-[4-(dimethylamino)phenyl]-1,6-naphthyridin-5-yl]methylamino]-Propanenitrile; 7-[4-(dimethylamino)phenyl]-N-[1-(phenylmethyl)-4-piperidinyl]-1,6-Naphthyridin-5-amine; N-[7-[4-(dimethylamino)phenyl]-1,6-naphthyridin-5-yl]-1,2-Cyclohexanediamine, (1R,2S)-rel-N-[7-[4-(dimethylamino)phenyl]-1,6-naphthyridin-5-yl]-1,2-Benzenedimethanamine; N-[7-[4-(diethylamino)phenyl]-1,6-naphthyridin-5-yl]-1,4-Butanediamine; N-[7-[3',5'-bis(trifluoromethyl)[1,1'-biphenyl]-4-yl]-1,6-naphthyridin-5-yl]-3-Propanediamine; N-[7-(3'-methoxy[1,1'-biphenyl]-4-yl)-1,6-naphthyridin-5-yl]-1,3-Propanediamine; N-[7-(3'-fluoro[1,1'-biphenyl]-4-yl)-1,6-naphthyridin-5-yl]-1,3-Propanediamine; 4-[[7-[4-(dimethylamino)phenyl]-1,6-naphthyridin-5-yl]oxy]-1-Butanol; N-[7-[4-(dimethylamino)phenyl]-1,6-naphthyridin-5-yl]-1,4-Cyclohexanediamine; 7-[4-(dimethylamino)phenyl]-N-(2,2,6,6-tetramethyl-4-piperidinyl)-1,6-Naphthyridin-5-amine; N-[7-[3-bromo-4-(dimethylamino)phenyl]-1,6-naphthyridin-5-yl]-1,3-Propanediamine; N-[7-(1-methyl-1H-indol-5-yl)-1,6-naphthyridin-5-yl]-1,3-Propanediamine; N-[7-[3-(trifluoromethyl)phenyl]-1,6-naphthyridin-5-yl]-1,3-Propanediamine; N-[7-[4-(trifluoromethyl)phenyl]-1,6-naphthyridin-5-yl]-1,3-Propanediamine; N-[7-(3-bromo-4-methoxyphenyl)-1,6-naphthyridin-5-yl]-1,3-Propanediamine; N-[7-[4-[[3-(dimethylamino)propyl]methylamino]phenyl]-1,6-naphthyridin-5-yl]-1,4-Cyclohexanediamine; N-[7-[4-[[2-(dimethylamino)ethyl]methylamino]phenyl]-1,6-naphthyridin-5-yl]-1,4-Cyclohexanediamine; N-[7-[4-(dimethylamino)-3-methoxyphenyl]-1,6-naphthyridin-5-yl]-1,4-Cyclohexanediamine; N-[7-[4-(4-morpholinyl)phenyl]-1,6-naphthyridin-5-yl]-1,4-Cyclohexanediamine; N-[7-[3-bromo-4-(4-morpholinyl)phenyl]-1,6-naphthyridin-5-yl]-1,4-Cyclohexanediamine; 4-[[7-[4-[[2-(dimethylamino)ethyl]methylamino]phenyl]-1,6-naphthyridin-5-yl]oxy]-Cyclohexanol; N-[7-[3-bromo-4-(4-morpholinyl)phenyl]-1,6-naphthyridin-5-yl]-1,3-Propanediamine; N,N-dimethyl-4-[5-(4-methyl-1-piperazinyl)-1,6-naphthyridin-7-yl]-Benzenamine; 4-[[7-[4-[[3-(dimethylamino)propyl]methylamino]phenyl]-1,6-naphthyridin-5-yl]oxy]-Cyclohexanol; N-[7-[4-[[2-(dimethylamino)ethyl]methylaminolphenyl]-1,6-naphthyridin-5-yl]-1,4-Butanediamine; [3-[[5-[(3-aminopropyl)amino]-7-(4-methoxyphenyl)-1,6-naphthyridin-2-yl]amino]propyl]-Carbamic acid-1,1-dimethylethyl ester.

### DARREICHUNGSFORMEN

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0, 1 bis 90 Gew.-%, bevorzugt 0, 5 bis 50 Gew.-% der Gesamtzusammensetzung liegen, d.h. in Mengen die ausreichend sind, um den unten angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel 1 gemäß der obigen bevorzugten Ausführungsformen.

Besonders bevorzugt ist es, wenn die Verbindungen der Formel 1 oral verabreicht werden, besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserem oder Farbstoffen versetzt werden.

Ebenfalls bevorzugt ist es, wenn die Verbindungen der Formel 1 inhalativ verabreicht werden, besonders bevorzugt ist es, wenn die Verabreichung ein oder zweimal täglich erfolgt. Hierzu müssen die Verbindungen der Formel 1 in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### Inhalationspulver

Sind die Verbindungen der Formel 1 im Gemisch mit physiologisch unbedenklichen Hilfsstoffen enthalten, können zur Darstellung der erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung. Verfahren zur Herstellung der erfindungsgemäßen Inhalationspulver durch Mahlen und Mikronisieren sowie durch abschließendes Mischen der Bestandteile sind aus dem Stand der Technik bekannt.

### Treibgashaltige Inhalationsaerosole

Die im Rahmen der erfindungsgemäßen Verwendung einsetzbaren treibgashaltigen Inhalationsaerosole können 1 im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind fluorierte Alkanderivate ausgewählt aus TG134a (1, 1, 1, 2-Tetrafluorethan), TG227 (1, 1, 1, 2, 3, 3, 3-Heptafluorpropan) und Mischungen derselben. Die im Rahmen der erfindungsgemäßen Verwendung einsetzbaren treibgashaltigen Inhalationsaerosole können ferner weitere Bestandteile wie Co-Solventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

### Treibgasfreie Inhalationslösungen

Die erfindungsgemäße Verwendung von Verbindungen der Formel **1** erfolgt bevorzugt zur Herstellung von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Die Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Den im Rahmen der erfindungsgemäßen Verwendung einsetzbaren treibgasfreien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.

Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien. Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine. Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration.

Für die oben beschriebenen Behandlungsformen werden gebrauchsfertige Packungen eines Medikaments zur Behandlung von Atemwegserkrankungen, beinhaltend eine beigelegte Beschreibung, welche beispielsweise die Worte Atemwegserkrankung, COPD oder Asthma enthalten, ein Pteridin und ein oder mehrere Kombinationspartner ausgewählt aus der oben beschriebenen Gruppe, bereit gestellt.

## Patentansprüche

1. Verbindungen der Formel **1**, 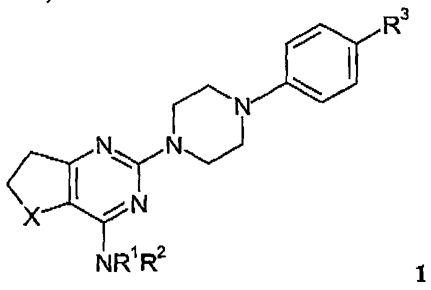 worin
X SO ;
**R¹** H, C₁₋₆-Alkyl, C₂₋₆-Alkenyl, C₂₋₆-Alkinyl, C₆₋₁₀-Aryl, C₆₋₁₀-Aryl-C₁₋₆alkylen oder C₅₋₁₀-Heteroayl-C₁₋₆-alkylen
**R²** H ist oder ein Rest ausgewählt aus der Gruppe bestehend aus C₁₋₁₀-Alkyl, C₂₋₆-Alkenyl und C₂₋₆-Alkinyl ist, der gegebenenfalls durch Halogen substituiert sein kann und der gegebenenfalls durch einen oder mehrere Reste ausgewählt aus der Gruppe bestehend aus OR^{2.1}, COOR^{2.1},CONR^{2.2}R^{2.3}, SR^{2.1}, C₆₋₁₀-Aryl, einem mono- oder bicyclischen C₃₋₁₀-Heterocyclus, einem mono- oder bicyclischen C₅₋₁₀-Heteroaryl, einem mono- oder bicyclischem C₃₋₁₀-Cycloalkyl, CH₂-NR^{2.2}R^{2.3} und NR^{2.2}R^{2.3} substituiert sein kann, welcher wiederum gegebenenfalls durch einen oder mehrere Reste ausgewählt aus der Gruppe bestehend aus OH, Halogen, OR^{2.1}, Oxo, C₁₋₆-Alkyl, C₆₋₁₀-Aryl, COOR^{2.1}, CH₂-NR^{2.2}R^{2.3} und NR^{2.2}R^{2.3} substituiert sein kann, wobei **R^{2.1}** H ist oder ein Rest ausgewählt ist aus der Gruppe bestehend aus C₁₋₆-Alkyl, C₁₋₆-Alkanol, C₁₋₃-Haloalkyl, mono- oder bicyclisches C₃₋₁₀ Cycloalkyl, C₆₋₁₀-Aryl-C₁₋₆-alkylen, mono- oder bicyclisches C₅₋₁₀-Heteroaryl-C₁₋₆-alkylen, C₃₋₁₀-Heterocyclus-C₁₋₆-alkylen, C₃₋₁₀-Cycloalkyl-C₁₋₆-alkylen, ein mono- oder bicyclisches C₆₋₁₀-Aryl, ein mono- oder bicyclisches C₅₋₁₀-Heteroaryl und ein mono- oder bicyclischer, gesättigter oder ungesättigter Heterocyclus, der gegebenenfalls durch einen oder mehrere Reste ausgewählt aus der Gruppe bestehend aus OH, Halogen, C₁₋₆-Alkyl und C₆₋₁₀-Aryl substituiert sein kann, wobei **R^{2.2}** und **R^{2.3}** unabhängig voneinander H sind oder ein Rest ausgewählt sind aus der Gruppe bestehend aus Halogen, C₁₋₆-Alkyl, mono- oder bicyclisches C₃₋₁₀ Cycloalkyl, C₆₋₁₀-Aryl-C₁₋₆-alkylen, C₅₋₁₀-Heteroaryl-C₁₋₆-alkylen, mono- oder bicyclisches C₆₋₁₀-Aryl, mono- oder bicyclischer C₃₋₁₀-Heterocyclus, mono- oder bicyclisches C₅₋₁₀-Heteroaryl, CO-NH₂, CO-NHCH₃, CO-N(CH₃)₂, SO₂(C₁-C₂-Alkyl), CO-R^{2.1} und COOR^{2.1}, der gegebenenfalls durch einen oder mehrere Reste ausgewählt aus der Gruppe bestehend aus OH, Halogen, C₁₋₆-Alkyl, C₆₋₁₀-Aryl und COOR^{2.1} substituiert sein kann, oder
**R²** ein mono- oder polycyclisches C₃₋₁₀ Cycloalkyl, das gegebenenfalls einfach oder mehrfach über C₁₋₃-Alkylgruppen verbrückt sein kann und das gegebenenfalls mit einem Rest ausgewählt aus der Gruppe bestehend aus verzweigtes oder unverzweigtes C₁₋₆-Alkanol, OR^{2.1}, COOR^{2.1}, SO₂NR^{2.2}R^{2.3}, C₃₋₁₀-Heterocyclus, C₆₋₁₀-Aryl, C₁₋₆-Alkyl, C₆₋₁₀-Aryl-C₁₋₆-alkylen, C₅₋₁₀-Heteroaryl-C₁₋₆-alkylen, mono- oder bicyclisches C₃₋₁₀ Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, Halogen, C₁₋₆-Alkyl, C₆₋₁₀-Aryl und NR^{2.2}R^{2.3} substituiert sein kann, oder
**R²** ein mono- oder polycyclisches C₆₋₁₀Aryl, das gegebenenfalls durch OH, SH oder Halogen oder durch einen oder mehrere Reste ausgewählt aus der Gruppe bestehend aus OR^{2.1}, COOR^{2.1} , NR^{2.2}R^{2.3}, CH₂-NR^{2.2}R^{2.3},C₃₋₁₀-Cycloalkyl, C₃₋₁₀-Heterocyclus, C₁₋₆-Alkyl, C₆₋₁₀-Aryl-C₁₋₆-alkylen, C₃₋₁₀-Heterocyclus-C₁₋₆-alkylen, C₅₋₁₀-Heteroaryl-C₁₋₆-alkylen, C₆₋₁₀-Aryl, SO₂-CH₃, SO₂-CH₂CH₃ und SO₂-NR^{2.2}R^{2.3} substituiert sein kann, der wiederum gegebenenfalls durch einen oder mehrere oder mehrere Reste ausgewählt aus der Gruppe bestehend aus OH, OR^{2.1}, Oxo, Halogen, C₁₋₆-Alkyl, C₆₋₁₀-Aryl und NR^{2.2}R^{2.3} substituiert sein kann, oder
**R²** ein Rest ausgewählt aus einer Gruppe bestehend aus mono- oder bicyclischer, gesättigtem oder ungesättigtem C₃₋₁₀-Heterocyclus und einem mono- oder bicyclischen C₅₋₁₀-Heteroaryl, welcher 1 bis 4 Heteroatome ausgewählt aus S, O und N umfasst und gegebenenfalls durch einen oder mehrere Reste ausgewählt aus der Gruppe Halogen, OH, Oxo und SH oder durch einen oder mehrere Reste ausgewählt aus der Gruppe OR^{2.1}, SR^{2.1}, COOR^{1.1}, COR^{2.1}, C₁₋₆-Alkanol, C₃₋₁₀-Cycloalkyl, C₆₋₁₀-Aryl, C₁₋₆-Alkyl, C₆₋₁₀-Aryl-C₁₋₆-alkylen, C₅₋₁₀-Heteroaryl-C₁₋₆-alkylen, C₅₋₁₀-Heterocyclus, C₅₋₁₀-Heteroaryl, C₁₋₆-Alkanol und NR^{2.2}R^{2.3} substituiert sein kann, der gegebenenfalls wiederum durch einen oder mehrere Reste ausgewählt aus der Gruppe bestehend aus OH, OR^{2.1}, Oxo, Halogen, C₁₋₆-Alkyl, C₆₋₁₀-Aryl und NR^{2.2}R^{2.3} substituiert sein kann,
oder **NR¹R²** bedeutet gemeinsam einen heterocyclischen C₄₋₇-Ring, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₆-Alkanol, Oxo, Halogen, C₁₋₆-Alkyl, C₆₋₁₀-Aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann, und worin
**R³** ausgewählt ist aus der Gruppe bestehend aus Fluor, Chlor, Brom, Iod, Hydroxy, SO₂-CH₃, COOR^{2.1}, Nitril-Gruppe und C₃₋₁₀-Heterocyclus-C₁₋₆-alkylen, wobei der C₃₋₁₀-Heterocyclus mono- oder bicyclisch sein kann und gegebenenfalls durch einen Rest ausgewählt aus der Gruppe bestehend aus OH, Halogen, Oxo, C₁₋₆-Alkyl und C₆₋₁₀-Aryl substituiert sein kann,
oder ein Rest ausgewählt ist aus der Gruppe bestehend aus C₁₋₆-Alkyl, C₂₋₆-Alkenyl, C₂₋₆-Alkinyl, C₆₋₁₀-Aryl; C₆₋₁₀-Aryl-C₁₋₆-alkylen, C₅₋₁₀-Heteroaryl-C₁₋₆-alkylen, C₃₋₁₀-Heterocyclus und C₃₋₁₀-Cycloalkyl, der gegebenenfalls mit einem Rest ausgewählt aus der Gruppe bestehend aus OH, Halogen, Oxo, C₁₋₆-Alkyl und C₆₋₁₀-Aryl substituiert sein kann, oder wobei
**R³** die Gruppe -CO-NR^{3.1}R^{3.2}bedeutet,
wobei **R^{3.1}** und **R^{3.2}** unabhängig voneinander H sind oder Reste sind, die ausgewählt sind aus der Gruppe bestehend aus C₁₋₆-Alkyl, C₂₋₆-Alkenyl, C₁₋₆-Alkinyl, C₆₋₁₀-Aryl; C₆₋₁₀-Aryl-C₁₋₆-alkylen, C₅₋₁₀-Heteroaryl-C₁₋₆-alkylen, C₅₋₁₀-Heteroaryl-C₁₋₆-alkinylen, C₅₋₁₀-Heteroaryl-C₁₋₆-alkenylen, mono- oder bicyclischer, C₃₋₁₀-Heterocyclus, C₃₋₁₀-Heterocyclus-C₁₋₆-alkylen und mono- oder bicyclisches C₅₋₁₀-Heteroaryl, wobei der Rest jeweils gegebenenfalls mit einem oder mehreren Resten ausgewählt aus der Gruppe OH, Oxo, Halogen, C₁₋₆-Alkyl und O-C₁₋₆-Alkyl substituiert sein kann, oder wobei
**R³** die Gruppe -NR^{3.3}-CO-R^{3.4} bedeutet,
wobei **R^{3.3}** H ist oder ein Rest ausgewählt aus der Gruppe bestehend aus C₁₋₆-Alkyl, C₂₋₆-Alkenyl, C₂₋₆-Alkinyl, C₆₋₁₀-Aryl; C₆₋₁₀-Aryl-C₁₋₆-alkylen, C₅₋₁₀-Heteroaryl-C₁₋₆-alkylen, C₃₋₁₀-Heterocyclus und einem C₅₋₁₀- Heteroaryl ist, der gegebenenfalls mit einem oder mehreren Resten ausgewählt aus der Gruppe bestehend aus OH, OR^{2.1}, Oxo, NH₂, NR^{2.2}R^{2.3}, Halogen, C₁₋₆-Alkyl und C₆₋₁₀-Aryl substituiert sein kann, und
wobei **R^{3.4}** H ist oder ein Rest ausgewählt aus der Gruppe bestehend aus C₁₋₆-Alkyl, C₂₋₆-Alkenyl, C₂₋₆-Alkinyl, C₁₋₆-Alkanol, OR^{2.1}, CH₂-O-CO-C₁₋₆-Alkyl, CH₂-NR^{2.2}R^{2.3}, NR^{2.2}R^{2.3}, C₆₋₁₀-Aryl; C₆₋₁₀-Aryl-C₁₋₆-alkylen, C₅₋₁₀-Heteroaryl-C₁₋₆-alkylen, mono- oder bicyclischer, gesättigter, teilweise gesättigter oder ungesättigter C₃₋₁₀-Heterocyclus mit 1, 2 oder 3 Heteroatomen aus der Gruppe S, O und N und einem mono- oder bicyclischen C₅₋₁₀- Heteroaryl mit 1, 2 oder 3 Heteroatomen aus der Gruppe S, O und N ist, der gegebenenfalls mit einem oder mehreren Resten ausgewählt aus der Gruppe bestehend aus OH, OR^{2.1}, Oxo, NH₂, NR^{2.2}R^{2.3}, Halogen, C₁₋₆-Alkyl und C₆₋₁₀-Aryl substituiert sein kann, oder wobei
**R³** eine gegebenenfalls einfach oder zweifach N-substituierte Sulfonamidgruppe SO₂-NR^{3.5}R^{3.6} bedeutet,
wobei R^{3.5} und R^{3.6} jeweils unabhängig voneinander C₁₋₆-Alkyl oder C₆₋₁₀-Aryl sein können,
sowie pharmakologisch verträgliche Salze, Diastereomere, Enantiomere, Racemate, Hydrate oder Solvate davon.

2. Verbindungen der Formel 1 nach Anspruch 1, worin
**X** SO ist
**R¹** H, C₁₋₆-Alkyl, C₆₋₁₀-Aryl, C₆₋₁₀-Aryl-C₁₋₆-alkylen oder C₅₋₁₀-Heteroaryl-C₁₋₆-alkylen ist;
**R²** H ist oder C₁₋₆-Alkyl, das gegebenenfalls durch Halogen substituiert sein kann und das gegebenenfalls durch einen oder mehrere Reste ausgewählt aus der Gruppe bestehend aus OR^{2.1}, COOR^{2.1}, CONR^{2.2}R^{2.3}, SR^{2.1}, C₆₋₁₀-Aryl, einem mono- oder bicyclischen C₃₋₁₀-Heterocyclus, einem mono- oder bicyclischen C₅₋₁₀-Heteroaryl, einem mono- oder bicyclischem C₃₋₁₀-Cycloalkyl, CH₂-NR^{2.2}R^{2.3} und NR^{2.2}R^{2.3} substituiert sein kann, welcher wiederum gegebenenfalls durch einen oder mehrere Reste ausgewählt aus der Gruppe bestehend aus OH, Halogen, OR^{2.1}, Oxo, C₁₋₆-Alkyl, C₆₋₁₀-Aryl, COOR^{2.1}, CH₂-NR^{2.2}R^{2.3} und NR^{2.2}R^{2.3} substituiert sein kann, wobei **R^{2.1}** H ist oder ein Rest ausgewählt ist aus der Gruppe bestehend aus C₁₋₆-Alkyl, C₁₋₆-Akanol, C₁₋₃-Haloalkyl, ein mono- oder bicyclisches C₃₋₁₀ Cycloalkyl, ein C₆₋₁₀-Aryl-C₁₋₆-alkylen oder C₅₋₁₀-Heteroaxyl-C₁₋₆-alkylen, C₃₋₁₀-Heterocyclus-C₁₋₆-alkylen, C₃₋₁₀-Cycloalkyl-C₁₋₆-alkylen, ein mono- oder bicyclisches C₆₋₁₀-Aryl, ein mono- oder bicyclisches C₅₋₁₀-Heteroaryl und ein mono- oder bicyclischer, gesättigter oder ungesättigter C₃₋₁₀-Heterocyclus ist, der gegebenenfalls durch einen oder mehrere Reste ausgewählt aus der Gruppe bestehend aus OH, Halogen, C₁₋₆-Alkyl, C₆₋₁₀-Aryl substituiert sein kann, wobei **R^{2.2}** und **R^{2.3}** unabhängig voneinander H sind oder ausgewählt sind aus der Gruppe bestehend aus Halogen, C₁₋₆-Alkyl, mono- oder bicyclisches C₃₋₁₀ Cycloalkyl, C₆₋₁₀-Aryl-C₁₋₆-alkylen, C₅₋₁₀-Heteroaryl-C₁₋₆-alkylen, mono- oder bicyclisches C₆₋₁₀-Aryl, mono- oder bicyclischer, gesättigter oder ungesättigter C₃-₁₀-Heterocyclus, mono- oder bicyclisches C₅₋₁₀-Heteroaryl, CO-NH₂, CO-NHCH₃, CO-N(CH₃)₂, SO₂(C₁-C₂-Alkyl), CO-R^{2.1} und COOR^{2.1}, der gegebenenfalls durch einen oder mehrere Reste ausgewählt aus der Gruppe bestehend aus OH, Halogen, C₁-₆-Alkyl, C₆₋₁₀-Aryl und COOR^{2.1} substituiert sein kann, oder
**R²** ein mono- oder polycyclisches C₃₋₁₀-Cycloalkyl, das gegebenenfalls einfach oder mehrfach über C₁₋₃-Alkylgruppen verbrückt sein kann und das gegebenenfalls einfach oder mehrfach durch OH oder Halogen oder durch einen oder mehrere Reste ausgewählt aus der Gruppe bestehend aus verzweigtes oder unverzweigtes C₁₋₆-Alkanol, OR^{2.1}, COOR^{2.1}, SO₂NR^{2.2}R^{2.3}, C₃₋₁₀-Heterocyclus, C₆₋₁₀-Aryl, C₁₋₆- Alkyl, C₆₋₁₀-Aryl-C₁₋₆-alkylen, C₅₋₁₀-Heteroaryl-C₁₋₆-alkylen, mono- oder bicyclisches C₃₋₁₀-Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, Halogen, C₁₋₆-Alkyl, C₆₋₁₀-Aryl und NR^{2.2}R^{2.3} substituiert sein kann, oder
**R²** ein mono- oder polycyclisches C₆₋₁₀-Aryl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3},C₃₋₁₀-Cycloalkyl, C₃₋₁₀-Heterocyclus, C₁₋₆-Alkyl, C₆₋₁₀-Aryl-C₁₋₆-alkylen, C₃₋₁₀-Heterocyclus-C₁₋₆-alkyle, C₅₋₁₀-Heteroaryl-C₁₋₆-alkylen, C₆₋₁₀-Aryl, SO₂-CH₃, SO₂-CH₂CH₃ und SO₂-NR^{2.2}R^{2.3} substituiert sein kann, der wiederum gegebenenfalls durch einen oder mehrere Reste ausgewählt aus der Gruppe bestehend aus OH, OR^{2.1}, Oxo, Halogen, C₁₋₆-Alkyl, C₆₋₁₀-Aryl und NR^{2.2}R^{2.3} substituiert sein kann, oder
**R²** ein Rest ausgewählt aus einer Gruppe bestehend aus mono- oder bicyclischen, gesättigtem oder ungesättigtem C₃₋₁₀-Heterocyclus und einem mono- oder bicyclischen C₅₋₁₀-Heteroaryl, welcher 1 bis 4 Heteroatome ausgewählt aus S, O und N umfasst und gegebenenfalls durch einen oder mehrere Reste ausgewählt aus der Gruppe Halogen, OH, Oxo und SH oder durch einen oder mehrere Reste ausgewählt aus der Gruppe bestehend aus OR^{2.1}, SR^{2.1}, COOR^{2.1}, COR^{2.1}, C₁₋₆-Alkanol, C₃₋₁₀-Cycloalkyl, C₆₋₁₀-Aryl, C₁₋₆-Alkyl, C₆₋₁₀-Aryl-C₁₋₆-alkylen, C₅₋₁₀-Heteroaryl-C₁₋₆-alkylen, C₅₋₁₀-Heterocyclus, C₅₋₁₀-Heteroaryl, C₁₋₆-Alkanol und NR^{2.2}R^{2.3} substituiert sein kann, der gegebenenfalls wiederum durch einen oder mehrere Reste ausgewählt aus der Gruppe bestehend aus OH, OR^{2.1}, Oxo, Halogen, C₁₋₆-Alkyl, C₆₋₁₀-Aryl und NR^{2.2}R^{2.3} substituiert sein kann,
oder **NR¹R²** bedeutet gemeinsam einen heterocyclischen C₄₋₇-Ring, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₆-Alkanol, Oxo, Halogen, C₁₋₆-Alkyl, C₆₋₁₀-Aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}-R^{2.3}, CH₂-NR^{2.2}-SO₂-C₁₋₃-Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,
sowie pharmakologisch verträgliche Salze, Diastereomere, Enantiomere, Racemate, Hydrate oder Solvate davon.

3. Verbindungen der Formel **1** nach Anspruch 1 und 2, worin
**X** SO ist
**R¹** H, C₁₋₆-Alkyl, C₆₋₁₀-Aryl, C₆₋₁₀-Aryl-C₁₋₆-alkylen oder C₅₋₁₀-Heteroaryl-C₁₋₆-alkylen ist;
**R²** H ist oder C₁₋₆-Alkyl, das gegebenenfalls durch Halogen substituiert sein kann und das gegebenenfalls durch einen oder mehrere Reste ausgewählt aus der Gruppe bestehend aus OR^{2.1}, COOR^{2.1}, CONR^{2.2}R^{2.3}, SR^{2.1}, Phenyl, einem mono- oder bicyclischen C₅₋₁₀-Heterocyclus, C₅₋₆-Heteroaryl, einem mono- oder bicyclischem C₅₋₁₀-Cycloalkyl, CH₂-NR^{2.2}R^{2.3} und NR^{2.2}R^{2.3} substituiert sein kann, welcher wiederum gegebenenfalls durch einen oder mehrere Reste ausgewählt aus der Gruppe bestehend aus OH, Halogen, OR^{2.1}, Oxo, C₁₋₆-Alkyl, Phenyl, COOR^{2.1}, CH₂-NR^{2.2}R^{2.3} und NR^{2.2}R^{2.3} substituiert sein kann, wobei **R^{2.1}** H ist oder ein Rest ausgewählt ist aus der Gruppe bestehend aus C₁₋₆-Alkyl, C₁₋₆-Alkanol, C₁₋₃-Haloalkyl, ein mono- oder bicyclisches C₅₋₁₀-Cycloalkyl, ein Phenyl-C₁₋₆-alkylen, ein C₅₋₆-Heteroaryl-C₁₋₆-alkylen, C₅₋₁₀-Heterocyclus-C₁₋₆-alkylen, C₅₋₁₀-Cycloalkyl-C₁₋₆-alkylen, Phenyl, ein mono- oder bicyclisches C₅₋₁₀-Heteroaryl und ein mono- oder bicyclischer, gesättigter oder ungesättigter C₅₋₁₀-Heterocyclus ist, der gegebenenfalls durch einen oder mehrere Reste ausgewählt aus der Gruppe bestehend aus OH, Halogen, C₁₋₆-Alkyl und Phenyl substituiert sein kann, wobei **R^{2.2}** und **R^{2.3}** unabhängig voneinander H sind oder ein Rest ausgewählt aus der Gruppe bestehend aus Halogen, C₁₋₆-Alkyl, mono- oder bicyclisches C₅₋₁₀ Cycloalkyl, Phenyl-C₁₋₆-alkylen, C₅₋₆-Heteroaryl-C₁₋₆-alkylen, Phenyl, mono- oder bicyclischer C₅₋₁₀-Heterocyclus, mono- oder bicyclisches C₅₋₆-Heteroaryl, CO-NH₂, CO-NHCH₃, CO-N(CH₃)₂, SO₂(C₁-C₂-Alkyl), CO-R^{2.1} und COOR^{2.1} sind, der gegebenenfalls durch einen oder mehrere Reste ausgewählt aus der Gruppe bestehend aus OH, Halogen, C₁₋₆-Alkyl, Phenyl und COOR^{2.1} substituiert sein kann, oder
**R²** ein mono- oder polycyclisches C₅₋₁₀-Cycloalkyl, das gegebenenfalls einfach oder mehrfach über C₁₋₃-Alkylgruppen verbrückt sein kann und das gegebenenfalls einfach oder mehrfach durch OH oder Halogen oder durch einen oder mehrere Reste ausgewählt aus der Gruppe verzweigtes oder unverzweigtes C₁₋₃-Alkanol, OR^{2.1}, COOR^{2.1}, SO₂NR^{2.2}R^{2.3}, C₅-₁₀-Heterocyclus, Phenyl, C₁₋₆-Alkyl, Phenyl-C₁₋₆- alkylen, C₅₋₆-Heteroaryl-C₁₋₆-alkylen, mono- oder bicyclisches C₅₋₁₀-Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, Halogen, C₁₋₆- Alkyl, Phenyl und NR^{2.2}R^{2.3} substituiert sein kann, oder
**R²** ein Phenyl, das gegebenenfalls durch OH, SH oder Halogen oder durch einen oder mehrere Reste ausgewählt aus der Gruppe bestehend aus OR^{2.1}, COOR^{2.1}, NR^{2.1}R^{2.3}, CH₂-NR^{2.2}R^{2.3},C₅₋₁₀-Cycloalkyl, C₅₋₁₀-Heterocyclus, C₁₋₆-Alkyl, Phenyl-C₁₋₆-alkylen, C₅₋₁₀-Heterocyclus-C₁₋₆-alkylen, C₅₋₆-Heteroaryl-C₁₋₆-alkylen, Phenyl, SO₂-CH₃, SO₂- CH₂CH₃ und SO₂-NR^{2.2}R^{2.3} substituiert sein kann, der wiederum gegebenenfalls durch einen oder mehrere Reste ausgewählt aus der Gruppe bestehend aus OH, OR^{2.1}, Oxo, Halogen, C₁₋₆-Alkyl, C₆₋₁₀-Aryl und NR^{2.2}R^{2.3} substituiert sein kann, oder
**R²** ein Rest ausgewählt aus einer Gruppe bestehend aus mono- oder bicyclischen, gesättigtem oder ungesättigtem C₅₋₁₀-Heterocyclus und einem mono- oder bicyclischen C₅₋₆-Heteroaryl, welcher 1 bis 4 Heteroatome ausgewählt aus S, O und N umfasst und gegebenenfalls durch einen oder mehrere Reste ausgewählt aus der Gruppe Halogen, OH, Oxo und SH oder durch einen oder mehrere Reste ausgewählt aus der Gruppe OR^{2.1}, SR^{2.1}, COOR^{2.1}, COR^{2.1}, C₁₋₆-Alkanol, C₃₋₁₀-Cycloalkyl, Phenyl, C₁₋₆-Alkyl, Phenyl-C₁₋₆-alkylen, C₅₋₆-Heteroaryl-C₁₋₆-alkylen, C₅₋₁₀-Heterocyclus, C₅₋₆-Heteroaryl, C₁₋₆-Alkanol und NR^{2.2}R^{2.3} substituiert sein kann, der gegebenenfalls wiederum durch einen oder mehrere Reste ausgewählt aus der Gruppe bestehend aus OH, OR^{2.1}, Oxo, Halogen, C₁₋₆-Alkyl, Phenyl und NR^{2.2}R^{2.3} substituiert sein kann,
oder **NR¹R²** bedeutet gemeinsam einen heterocyclischen C₄₋₇-Ring, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₆-Alkanol, Oxo, Halogen, C₁₋₆-Alkyl, C₆₋₁₀-Aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,
sowie pharmakologisch verträgliche Salze, Diastereomere, Enantiomere, Racemate, Hydrate oder Solvate davon.

4. Verbindungen der Formel **1** nach Anspruch 1 bis 3, worin
**R¹** H oder Methyl ist,
sowie pharmakologisch verträgliche Salze, Diastereomere, Enantiomere, Racemate, Hydrate oder Solvate davon.

5. Verbindungen der Formel **1** nach Anspruch 1 bis 3, worin
**NR¹R²** gemeinsam einen Pyrrolidinring bedeutet, der gegebenenfalls durch einen oder mehrere Reste ausgewählt aus der Gruppe bestehend aus OH, OR^{2.1}, CH₂-OH, CH₂-CH₂-OH, Oxo, Cl, F, Br, Methyl, Ethyl, Propyl, Phenyl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR²-SO₂-C₁₋₃-Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,
sowie pharmakologisch verträgliche Salze, Diastereomere, Enantiomere, Racemate, Hydrate oder Solvate davon.

6. Verbindungen der Formel **1** nach Anspruch 1 bis 4, worin
**R²** Phenyl, das einfach oder mehrfach durch OH, SH oder Halogen oder durch einen oder mehrere Reste ausgewählt aus der Gruppe bestehend aus OR^{2.1}, COO^{2.1}, NR^{2.2}R^{2.3}, CH₂-NR^{2.2}R^{2.3},C₅₋₁₀-Cyloalkyl, C₅₋₁₀-Heterocyclus, C₁₋₆-Alkyl, Phenyl-C₁₋₆-alkylen, C₅₋₁₀-Heterocyclus-C₁₋₆-alkylen, C₅₋₆-Heteroaryl-C₁₋₆-alkylen, Phenyl, SO₂-CH₃, SO₂-CH₂CH₃ und SO₂-NR^{2.2}R^{2.3} an beliebiger Position substituiert ist, der wiederum gegebenenfalls durch einen oder mehrere Reste ausgewählt aus der Gruppe bestehend aus OH, OR^{2.1}, Oxo, Halogen, C₁₋₆-Akyl, Phenyl und NR^{2.2}R^{2.3} substituiert sein kann,
sowie pharmakologisch verträgliche Salze, Diastereomere, Enantiomere, Racemate, Hydrate oder Solvate davon.

7. Verbindungen der Formel **1** nach Anspruch 1 bis 4, worin
**R²** Phenyl ist, das in mindestens einer der beiden meta-Stellungen durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3},C₅₋₁₀-Cycloalkyl, C₅₋₁₀-Heterocyclus, C₁₋₆-Alkyl, Phenyl-C₁₋₆-alkylen, C₅₋₁₀-Heterocyclus-C₁₋₆-alkylen, C₅₋₆-Heteroaryl-C₁₋₆-alkylen, Phenyl, SO₂-CH₃, SO₂-CH₂CH₃ und SO₂-NR^{2.2}R^{2.3} substituiert sein kann, der wiederum gegebenenfalls durch einen oder mehrere Reste ausgewählt aus der Gruppen bestehend aus OH, OR^{2.1}, Oxo, Halogen, C₁₋₆-Alkyl, Phenyl und NR^{2.2}R^{2.3} substituiert sein kann,
sowie pharmakologisch verträgliche Salze, Diastereomere, Enantiomere, Racemate, Hydrate oder Solvate davon.

8. Verbindungen der Formel **1** nach Anspruch 7, worin
**R²** Phenyl ist, das in mindestens einer der beiden meta-Stellungen durch einen oder mehrere Reste ausgewählt aus der Gruppe bestehend aus Methyl, F, Cl, OH, OR^{2.1}, COOR^{2.1}, NH₂ und N(CH₃)₂ substituiert ist,
sowie pharmakologisch verträgliche Salze, Diastereomere, Enantiomere, Racemate, Hydrate oder Solvate davon,

9. Verbindungen der Formeln 1 mach einem der Ansprüche 1 bis 4, worin
**R²** C₁₋₆-Alkyl ist, das gegebenenfalls durch Halogen substituiert sein kann und das gegebenenfalls durch einen oder mehrere Reste ausgewählt aus der Gruppe bestehend aus OR^{2.1}, COOR^{2.1}, CONR^{2.2}R^{2.3}, SR^{2.1}, Phenyl, einem mono- oder bicyclischen C₅-₁₀-Heterocyclus, C₅₋₆-Heteroaryl, einem mono- oder bicyclischem C₅₋₁₀-Cycloalkyl, CH₂-NR^{2.2}R^{2.3} und NR^{2.2}R^{2.3} substituiert sein kann, wobei der Rest aus der zweiten Gruppe wiederum durch einen oder mehrere Reste ausgewählt aus der Gruppe bestehend aus OH, Halogen, OR^{2.1}, Oxo, C₁₋₆-Alkyl, Phenyl, COOR^{2.1}, CH₂-NR^{2.2}R^{2.3} und NR^{2.2}R^{2.3} substituiert sein kann,
sowie pharmakologisch verträgliche Salze, Diastereomere, Enantiomeren, Racemate, Hydrate oder Solvate davon.

10. Verbindungen der Formel 1 nach einem der Ansprüche 1 bis 4 und 9, worin
**R²** Methyl, Ethyl oder Propyl ist,
sowie pharmakologisch verträgliche Salze, Diastereomere, Enantiomere, Racemate, Hydrate oder Solvate davon.

11. Verbindungen der Formel 1 nach einem der Ansprüche 1 bis 4, worin
**R²** C₁₋₆-Alkyl ist, das durch einen oder mehrere Reste ausgewählt aus der Gruppe bestehend aus OH, COOR^{2.1}, CON(CH₃)₂, C₁₋₆-Alkyl, Phenyl, Cyclopropyl und NR^{2.2}R^{2.3} substituiert ist, welcher wiederum gegebenenfalls durch einen oder mehrere Reste ausgewählt aus der Gruppe bestehend aus OH, Fluor, Chlor, Brom, Iod, OR^{2.1}, Oxo, C₁₋₆-Alkyl, Phenyl, C₁₋₃-Alkanol, CH₂-NR^{2.2}R^{2.3} und NR^{2.2}R^{2.3} substituiert sein kann,
sowie pharmakologisch verträgliche Salze, Diastereomere, Enantiomere, Racemate, Hydrate oder Solvate davon,

12. Verbindungen der Formel 1 nach einem der Ansprüche 1 bis 4, worin
**R²** C₁₋₆-Alkyl ist, das durch einen oder mehrere Reste ausgewählt aus der Gruppe bestehend aus OH, Phenyl, COOR^{2.1}, NH₂ substituiert ist, wobei das Phenyl wiederum gegebenenfalls durch einen oder mehrere Reste ausgewählt aus der Gruppe bestehend aus OH, Fluor, Chlor, Brom, Iod, OR^{2.1}, C₁₋₆-Alkyl, CH₂-NH₂, CH₂(CH₃)₂, NH₂ und N(CH₃)₂ substituiert sein kann,
sowie pharmakologisch verträgliche Salze, Diastereomere, Enantiomere, Racemate, Hydrate oder Solvate davon.

13. Verbindungen der Formel **1** nach einem der Ansprüche 1 bis 4 und 11 bis 12, worin
**R²** ein Rest nach Formel **2** 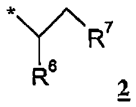
ist,
worin **R⁷** OH oder NH₂ ist und
worin **R⁶** ein Rest ausgewählt aus der Gruppe bestehend aus C₁₋₆-Alkyl, C₅₋₁₀- Heteroaryl und C₆₋₁₀-Aryl, vorzugsweise Phenyl, ist, das gegebenenfalls mit einem oder mehreren Resten ausgewählt aus der Gruppe bestehend aus Halogen, OH, COOR^{2.1}, OR^{2.1}, NH₂, C₁₋₆-Alkyl, C₁₋₆-Haloalkyl und C₁₋₆-Alkanol substituiert sein kann,
sowie pharmakologisch verträgliche Salze, Diastereomere, Enantiomere, Racemate, Hydrate oder Solvate davon.

14. Verbindungen der Formel **1** nach Anspruch 13, worin
**R²** ein Rest nach Formel **2** 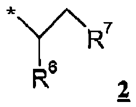
ist, worin
**R⁷** OH oder NH₂ ist und
**R⁶** Methyl, Ethyl, Propyl, Isopropyl ist.

15. Verbindungen der Formel **1** nach einem der Ansprüche 1 bis 4, worin
R² ein monocyclischer C₃₋₇-Cycloakyl-Ring ist, der in spiro-Stehung mit einem Rest ausgewählt aus der Gruppe bestehend aus -OH, -CH₂-OH, -CH₂-CH₂-OH, verzweigtes oder unverzweigtes C₃₋₆-Alkanol, -OR^{2.1}, Methyl, Ethyl, Propyl, Isopropyl, Butyl, Isobutyl und Halogen, substituiert sein kann, wobei R^{2.1} ausgewählt aus der Gruppe bestehend aus Methyl, Ethyl, Propyl, Isopropyl, Butyl, Isobutyl sein kann.

16. Verbindungen der Formel 1 nach einem der Ansprüche 1 bis 4, worin
**R²** ein Rest ausgewählt aus einer Gruppe bestehend aus monocyclischen, gesättigten drei-, vier-, fünf-, sechs- oder siebengliedrigem Heterocyclus mit 1, 2 oder 3 Heteroatomen jeweils ausgewählt aus der Gruppe bestehend aus N, O und S, der gegebenenfalls durch einen oder mehrere Reste ausgewählt aus der Gruppe Fluor, Chlor, Brom, OH, Oxo und SH oder durch einen oder mehrere Reste ausgewählt aus der Gruppe OR^{2.1}, SR^{2.1}, COOR^{2.1}, COR^{2.1}, C₁₋₆-Alkanol, C₃₋₁₀-Cycloalkyl, Phenyl, C₁-₆-Alkyl, Phenyl-C₁₋₆-alkylen, C₅₋₁₀-Heteroaryl-C₁₋₆-alkylen, C₅₋₁₀-Heterocyclus, C₅₋₁₀- Heteroaryl und NR^{2.2}R^{2.3} substituiert sein kann, der gegebenenfalls wiederum durch einen oder mehrere Reste ausgewählt aus der Gruppe bestehend aus OH, OR^{2.1}, Oxo, Halogen, C₁₋₆-Akyl, Phenyl und NR^{2.2}R^{2.3} substituiert sein kann, wobei
**R^{2.1}** H oder ein Rest ausgewählt aus der Gruppe bestehend aus C₁₋₆-Alkyl, C₁₋₆-Alkanol, C₁₋₃-Haloalkyl, mono- oder bicyclisches C₃₋₁₀ Cycloalkyl, Phenyl-C₁₋₆-alkylen, mono- oder bicyclisches C₅₋₁₀-Heteroaryl-C₁₋₆-alkylen, C₃₋₁₀-Heterocyclus-C₁₋₆-alkylen, C₃₋₇-Cycloalkyl-C₁₋₆-alkylen, Phenyl, ein mono- oder bicyclisches C₅₋₁₀-Heteroaryl und ein monocyclischer, gesättigter oder ungesättigter, fünf-, sechs- oder siebengliedriger Heterocyclus mit 1, 2 oder 3 Heteroatomen aus der Gruppe bestehend aus N, O und S, der gegebenenfalls durch einen oder mehrere Reste ausgewählt aus der Gruppe bestehend aus OH, Halogen, C₁₋₆-Alkyl und Phenyl substituiert sein kann, wobei **R^{2.2}** und **R^{2.3}** unabhängig voneinander H sind oder ein Rest ausgewählt sind aus der Gruppe bestehend aus Halogen, C₁₋₆-Alkyl, mono- oder bicyclisches C₃₋₁₀ Cycloalkyl, Phenyl-C₁₋₆-alkylen, C₅₋₁₀-Heteroayl-C₁₋₆-alkylen, Phenyl, mono- oder bicyclischer C₃₋₁₀-Heterocyclus, mono- oder bicyclisches C₅₋₁₀-Heteroaryl, CO- NH₂, CO-NHCH₃, CO-N(CH₃)₂, SO₂(C₁-C₂-Alkyl), CO-R^{2.1} und COOR^{2.1}, der gegebenenfalls durch einen oder mehrere Reste ausgewählt aus der Gruppe bestehend aus OH, Halogen, C₁₋₆-Alkyl, Phenyl und COOR^{2.1} substituiert sein kann.

17. Verbindungen der Formel **1** nach einem der Ansprüche 1 bis 16, worin
**R³** Fluor, Chlor, Brom, Iod oder CN sind,
sowie pharmakologisch verträgliche Salze, Diastereomere, Enantiomere, Racemate, Hydrate oder Solvate davon.

18. Verbindungen der Formel **1** nach einem der Ansprüche 1 bis 16, worin
**R³** die Gruppe -CO-NR^{3.1}R^{3.2} bedeutet,
wobei **R^{3.1}** und **R^{3.2}** unabhängig voneinander H sind oder Reste sind, die ausgewählt sind aus der Gruppe bestehend aus C₁₋₆-Alkyl, C₂₋₆-Alkenyl, C₂₋₆-Alkinyl, C₆₋₁₀-Aryl; C₆₋₁₀-Aryl-C₁₋₆-alkylen, C₅₋₁₀-Heteroaryl-C₁₋₆-alkylen, C₅₋₁₀-Heteroaryl-C₁₋₆-alkinylen, C₅₋₁₀-Heteroaryl-C₁₋₆-alkenylen, mono- oder bicyclischer, C₅₋₁₀-Heterocyclus, C₅₋₁₀-Heterocyclus-C₁₋₆-alkylen und mono- oder bicyclisches C₅₋₁₀-Heteroaryl, wobei der Rest jeweils gegebenenfalls mit einem oder mehreren Resten ausgewählt aus der Gruppe bestehend aus OH, Oxo, Halogen, C₁₋₆-Alkyl und O-C₁₋₆-Alkyl substituiert sein kann,
sowie pharmakologisch verträgliche Salze, Diastereomere, Enantiomere, Racemate, Hydrate oder Solvate davon.

19. Verbindungen der Formel **1** nach einem der Ansprüche 1 bis 16 und 18, worin
**R³** die Gruppe -CO-NR^{3.1}R^{3.2} bedeutet,
wobei **R^{3.1}** und **R^{3.2}** unabhängig voneinander H sind oder Reste sind, die ausgewählt sind aus der Gruppe bestehend aus C₁₋₆-Alkyl, Phenyl; Phenyl-C₁₋₆-alkylen, C₅₋₆-Heteroaryl-C₁-₆-alkylen, C₅₋₆-Heteroaryl-C₁₋₆-alkinylen, C₅₋₆-Heteroaryl-C₁₋₆-alkenylen, mono- oder bicyclischer, C₅₋₁₀-Heterocyclus, C₅₋₁₀-Heterocyclus-C₁₋₆-alkylen und mono- oder bicyclisches C₅₋₁₀-Heteroaryl, wobei der Rest jeweils gegebenenfalls mit einem oder mehreren Resten ausgewählt aus der Gruppe bestehend aus OH, Oxo, Halogen, C₁₋₆-Alkyl und O-C₁₋₆-Alkyl substituiert sein kann,
sowie pharmakologisch verträgliche Salze, Diastereomere, Enantiomere, Racemate, Hydrate oder Solvate davon.

20. Verbindungen der Formel 1 nach einem der Ansprüche 1 bis 16, worin
**R³** die Gruppe -NR^{3..3}-CO-R^{3.4}bedeutet,
wobei **R^{3.3}** H ist oder ein Rest ausgewählt aus der Gruppe bestehend aus C₁₋₆-Alkyl, C₂₋₆-Akenyl, C₂₋₆-Alkinyl, C₆₋₁₀-Aryl; C₆₋₁₀-Aryl-C₁₋₆-alkylen, C₅₋₁₀-Heteroaryl-C₁₋₆-alkylen, C₃₋₁₀-Heterocyclus und einem C₅₋₁₀- Heteroaryl ist, der gegebenenfalls mit einem oder mehreren Resten ausgewählt aus der Gruppe bestehend aus OH, OR^{2.1}, Oxo, NH₂, NR^{2.2}R^{2.3}, Halogen, C₁₋₆-Alkyl und C₆₋₁₀-Aryl substituiert sein kann, und
wobei **R^{3.4}** H ist oder ein Rest ausgewählt aus der Gruppe bestehend aus C₁₋₆-Alkyl, C₂₋₆-Akenyl, C₂₋₆-Alkinyl, C₁₋₆-Alkanol, OR^{2.1}, CH₂-O-CO-C₁₋₆-Alkyl, CH₂NR^{2.2}R^{2.3}, NR^{2.2}R^{2.3}, C₆₋₁₀-Aryl; C₆₋₁₀-Aryl-C₁₋₆-alkylen, C₅₋₁₀-Heteroaryl-C₁₋₆-alkylen, mono- oder bicyclischer, gesättigter oder ungesättigter C₃₋₁₀-Heterocyclus mit 1, 2 oder 3 Heteroatomen aus der Gruppe O, S und N und einem, mono- oder bicyclischen C₅₋₁₀, Heteroaryl mit 1, 2 oder 3 Heteroatomen aus der Gruppe O, S und N ist, der gegebenenfalls mit einem oder mehreren Resten ausgewählt aus der Gruppe bestehend aus OH, OR^{2.1}, Oxo, NH₂, NR^{2.2}R^{2.3}, Halogen, C₁₋₆-Alkyl und C₆₋₁₀-Aryl substituiert sein kann,
sowie pharmakologisch verträgliche Salze, Diastereomere, Enantiomere, Racemate, Hydrate oder Solvate davon.

21. Verbindungen der Formel 1 nach einem der Ansprüche 1 bis 16 und 20, worin
**R³** die Gruppe -NR^{3.3}-CO-R^{3.4}bedeutet,
wobei **R^{3.3}** H ist oder ein Rest ausgewählt aus der Gruppe bestehend aus C₁₋₆-Alkyl, Phenyl; Phenyl-C₁₋₆-alkylen, C₅₋₁₀-Heteroaryl-C₁₋₆-alkylen, C₅₋₁₀-Heterocyclus und einen C₅₋₁₀- Heteroaryl ist, der Gegebenenfalls mit einem oder mehreren Resten ausgewählt aus der Gruppe bestehend aus OH, OR^{2.1}, Oxo, NH₂, N(CH₃)₂, Halogen, C₁₋₆-Alkyl und Phenyl substituiert sein kann, und
wobei **R^{3.4}** H ist oder ein Rest ausgewählt aus der Gruppe bestehend aus C₁₋₆-Alkyl, C₁₋₆-Alkanol, OR^{2.1}, CH₂-O-CO-C₁₋₆-Akyl, CH₂-NH₂, CH₂-N(CH₃)₂, NH₂, N(CH₃)₂, Phenyl, Phenyl-C₁₋₆-alkylen, C₅₋₁₀-Heteroaryl-C₁₋₆-alkylen, mono- oder bicyclischer, gesättigter oder ungesättigter C₅₋₁₀-Heterocyclus mit 1, 2 oder 3 Heteroatomen aus der Gruppe N; S und O und einem mono- oder bicyclischen C₅₋₁₀- Heteroaryl mit 1, 2 oder 3 Heteroatomen aus der Gruppe N, S und O ist, der gegebenenfalls mit einem oder mehreren Resten ausgewählt aus der Gruppe bestehend aus OH, OR^{2.1}, Oxo, NH₂, N(CH₃)₂, Halogen, C₁₋₆-Alkyl und Phenyl substituiert sein kann,
sowie pharmakologisch verträgliche Salze, Diastereomere, Enantiomere, Racemate, Hydrate oder Solvate davon.

22. Verbindungen Nr. 1 bis 123 der Formel 1 nach einem der Ansprüche 1 bis 4 oder 6 bis 21, worin
X für SO steht und
worin R¹ H ist und
und wobei R² und R³ wie in den Verbindungen Nr. 1-123 in der folgenden Tabelle kombiniert sind
| Verbindungs-Nr. | R² | R³ |
|---|---|---|
| 1. | 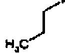 | 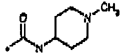 |
| 2. | 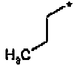 | 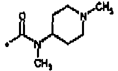 |
| 3. | 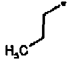 | 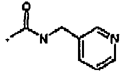 |
| 4. | 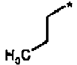 | 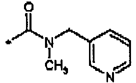 |
| 5. | 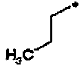 | 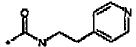 |
| 6. | 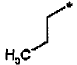 | 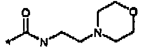 |
| 7. | 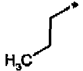 | 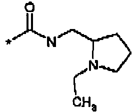 |
| 8. | 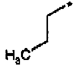 | 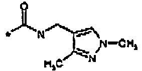 |
| 9. | 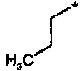 | 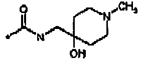 |
| 10. | 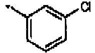 | 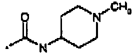 |
| 11. | 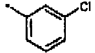 | 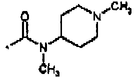 |
| 12. | 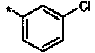 | 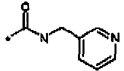 |
| 13. | 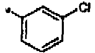 | 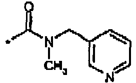 |
| 14. | 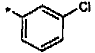 | 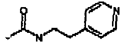 |
| 15. | 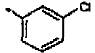 | 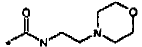 |
| 16. | 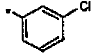 | 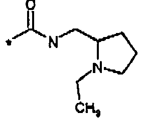 |
| 17. | 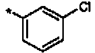 | 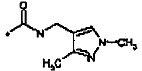 |
| 18. | 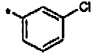 | 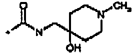 |
| 19. | 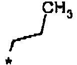 | 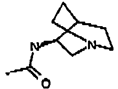 |
| 20. | 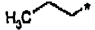 | Cl |
| 21. | 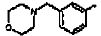 | Cl |
| 22. | 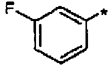 | Cl |
| 23. | 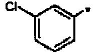 | Cl |
| 24. | 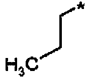 | 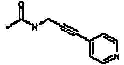 |
| 25. | 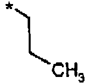 | 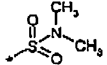 |
| 26. | 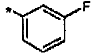 | 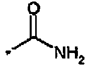 |
| 27. | 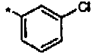 | 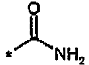 |
| 28. | 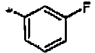 | 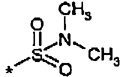 |
| 29. | 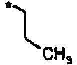 | 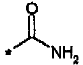 |
| 30. | 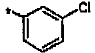 | 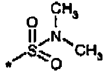 |
| 31. | 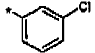 | 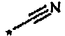 |
| 32. | 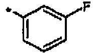 | 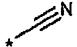 |
| 33. | 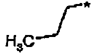 | 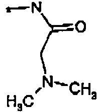 |
| 34. | 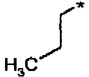 | 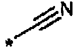 |
| 35. | 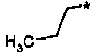 | 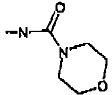 |
| 36. | 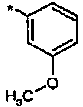 | Cl |
| 37. | 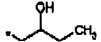 | Cl |
| 38. | 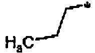 | 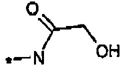 |
| 39. | 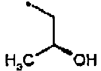 | Cl |
| 40. | 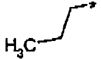 | 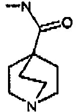 |
| 41. | 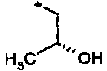 | Cl |
| 42. | 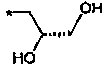 | Cl |
| 43. | 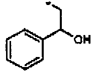 | Cl |
| 44. | 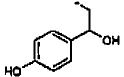 | Cl |
| 45. | 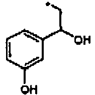 | Cl |
| 46. | 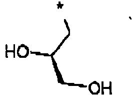 | Cl |
| 47. | 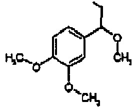 | Cl |
| 48. | 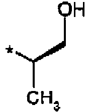 | Cl |
| 49. | 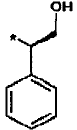 | Cl |
| 50. | 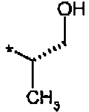 | Cl |
| 51. | 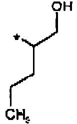 | Cl |
| 52. | 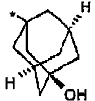 | Cl |
| 53. | 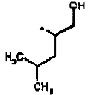 | Cl |
| 54. | 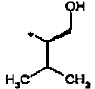 | Cl |
| 55. | 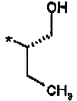 | Cl |
| 56. | 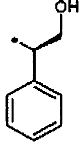 | Cl |
| 57. | 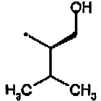 | Cl |
| 58. | 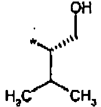 | Cl |
| 59. | 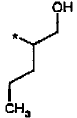 | Cl |
| 60. | 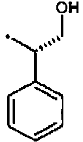 | Cl |
| 61. | 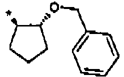 | Cl |
| 62. | 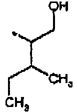 | Cl |
| 63. | 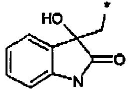 | Cl |
| 64. | 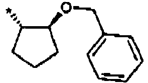 | Cl |
| 65. | 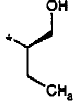 | Cl |
| 66. | 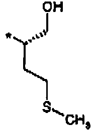 | Cl |
| 67. | 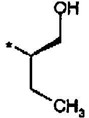 | Cl |
| 68. | 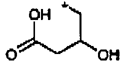 | Cl |
| 69. | 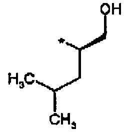 | Cl |
| 70. | 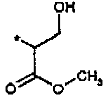 | Cl |
| 71. | 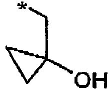 | Cl |
| 72. | 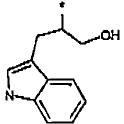 | Cl |
| 73. | 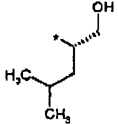 | Cl |
| 74. | 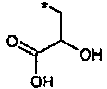 | Cl |
| 75. | 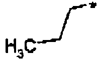 | 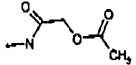 |
| 76. | 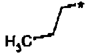 | 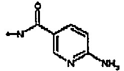 |
| 77. | 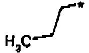 | 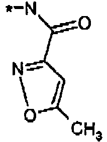 |
| 78. | 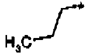 | 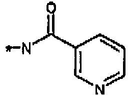 |
| 79. | 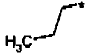 | 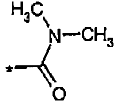 |
| 80. | 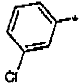 | 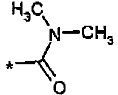 |
| 81. | 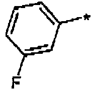 | 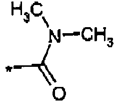 |
| 82. | 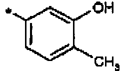 | Cl |
| 83. | 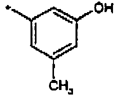 | Cl |
| 84. | 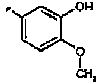 | Cl |
| 85. | 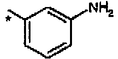 | Cl |
| 86. | 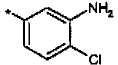 | Cl |
| 87. | 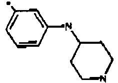 | Cl |
| 88. | 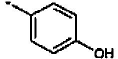 | Cl |
| 89. | 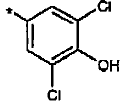 | Cl |
| 90. | 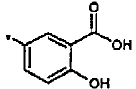 | Cl |
| 91. | 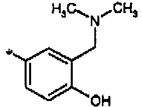 | Cl |
| 92. | 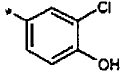 | Cl |
| 93. | 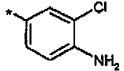 | Cl |
| 94. | 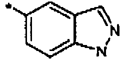 | Cl |
| 95. | 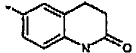 | Cl |
| 96. | 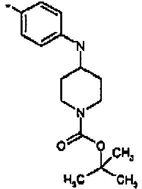 | Cl |
| 97. | 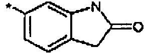 | Cl |
| 98. | 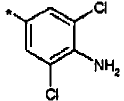 | Cl |
| 99. | 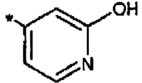 | Cl |
| 100. | 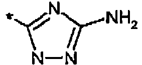 | Cl |
| 101. | 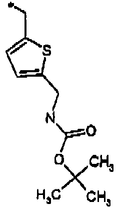 | Cl |
| 102. | 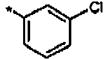 | 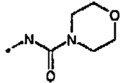 |
| 103. | 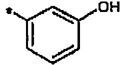 | Cl |
| 104. | 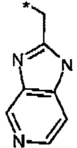 | Cl |
| 105. | 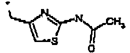 | Cl |
| 106. | 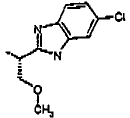 | Cl |
| 107. | 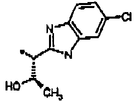 | Cl |
| 108. | 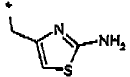 | Cl |
| 109. | 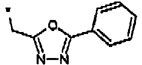 | Cl |
| 110. | 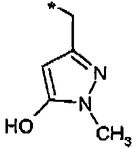 | Cl |
| 111. | 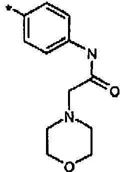 | Cl |
| 112. | 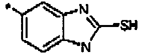 | Cl |
| 113. | 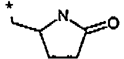 | Cl |
| 114. | 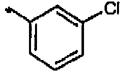 | 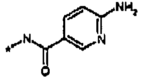 |
| 115. | 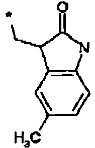 | Cl |
| 116. | 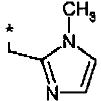 | Cl |
| 117. | 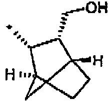 | Cl |
| 118. | 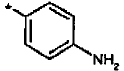 | Cl |
| 119. | 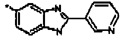 | Cl |
| 120. | 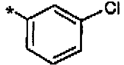 | 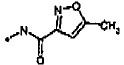 |
| 121. | 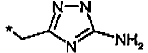 | Cl |
| 122. | 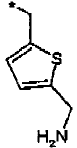 | Cl |
| 123. | 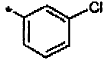 | 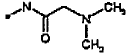 |
sowie pharmakologisch verträgliche Salze, Diastereomere, Enantiomere, Racemate, Hydrate oder Solvate davon.

23. Verbindungen Nr. 131 bis 217 der Formel 1 nach einem der Ansprüche 1 bis 4 oder 6 bis 21, worin
X für SO steht und
worin R¹ H ist und
und wobei R² und R³ wie in den Verbindungen Nr. 131-217 in der folgenden Tabelle kombiniert sind
| Verbindungs-Nr. | R² | R³ |
|---|---|---|
| 131 | 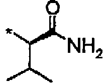 | Cl |
| 132 | 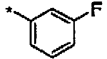 | 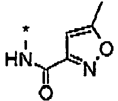 |
| 133 | 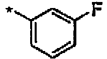 | 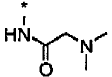 |
| 134 | 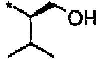 | 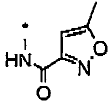 |
| 135 | 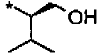 | 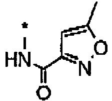 |
| 136 | 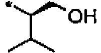 | 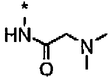 |
| 137 | 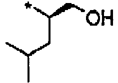 | 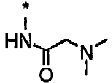 |
| 138 | 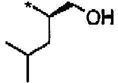 | 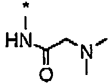 |
| 139 | 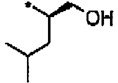 | 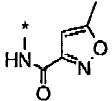 |
| 140 | 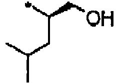 | 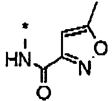 |
| 141 | 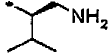 | Cl |
| 142 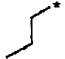 | 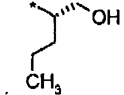 | Cl |
| 143 | 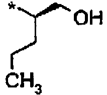 | Cl |
| 144 | 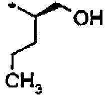 | Cl |
| 145 | 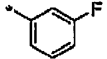 | 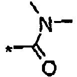 |
| 146 | 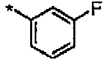 | 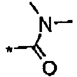 |
| 147 | 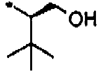 | Cl |
| 148 | 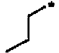 | CN |
| 149 | 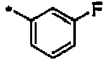 | 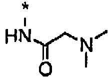 |
| 150 | 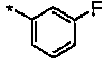 | 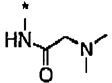 |
| 151 | 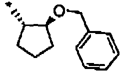 | Cl |
| 152 | 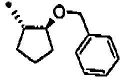 | Cl |
| 153 | 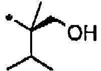 | Cl |
| 154 | 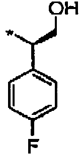 | Cl |
| 155 | 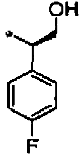 | Cl |
| 156 | 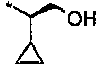 | Cl |
| 157 | 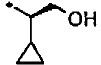 | Cl |
| 158 | 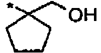 | Cl |
| 159 | 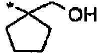 | Cl |
| 160 | 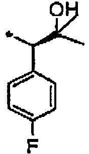 | Cl |
| 161 | 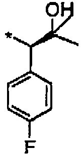 | Cl |
| 162 | 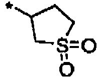 | Cl |
| 163 | 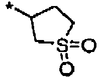 | Cl |
| 164 | 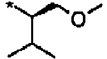 | Cl |
| 165 | 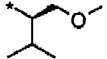 | Cl |
| 166 | 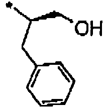 | Cl |
| 167 | 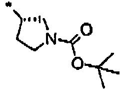 | Cl |
| 168 | 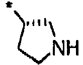 | Cl |
| 169 | 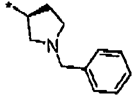 | Cl |
| 170 | 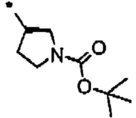 | Cl |
| 171 | 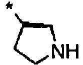 | Cl |
| 172 | 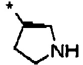 | Cl |
| 173 | 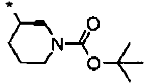 | Cl |
| 174 | 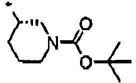 | Cl |
| 175 | 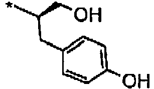 | Cl |
| 176 | 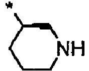 | Cl |
| 177 | 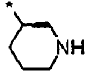 | Cl |
| 178 | 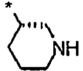 | Cl |
| 179 | 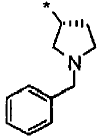 | Cl |
| 180 | 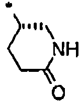 | Cl |
| 181 | 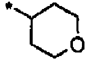 | 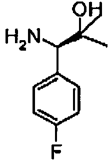 |
| 182 | 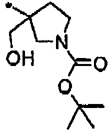 | Cl |
| 183 | 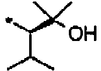 | Cl |
| 184 | 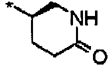 | Cl |
| 185 | 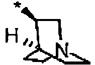 | Cl |
| 186 | 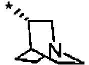 | Cl |
| 187 | 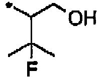 | Cl |
| 188 | 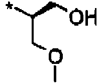 | Cl |
| 189 | 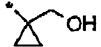 | Cl |
| 190 | 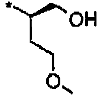 | Cl |
| 191 | 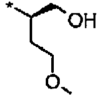 | Cl |
| 192 | 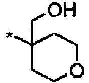 | Cl |
| 193 | 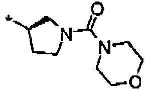 | Cl |
| 194 | 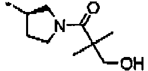 | Cl |
| 195 | 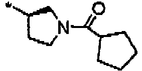 | Cl |
| 196 | 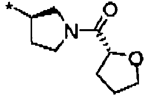 | Cl |
| 197 | 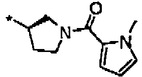 | Cl |
| 198 | 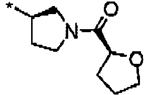 | Cl |
| 199 | 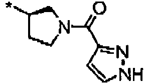 | Cl |
| 200 | 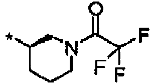 | Cl |
| 201 | 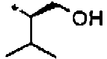 | OH |
| 202 | 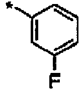 | 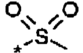 |
| 203 | 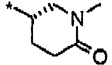 | Cl |
| 204 | 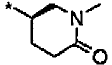 | Cl |
| 205 | 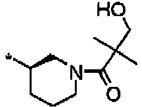 | Cl |
| 206 | 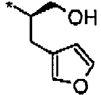 | Cl |
| 207 | 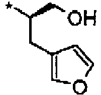 | Cl |
| 208 | 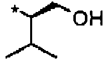 | Br |
| 209 | 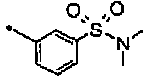 | Cl |
| 210 | 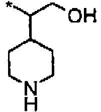 | Cl |
| 211 | 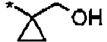 | Br |
| 212 | 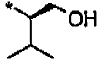 | 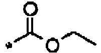 |
| 213 | 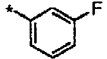 | 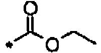 |
| 214 | 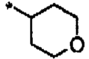 | 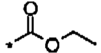 |
| 215 | 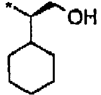 | Cl |
| 216 | 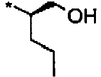 | Br |
| 217 | 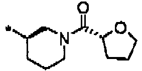 | Cl |
sowie pharmakologisch verträgliche Salze, Diastereomere, Enantiomere, Racemate, Hydrate oder Solvate davon.

24. Verbindungen der Formel 1 nach einem der Ansprüche 1 bis 3 oder 5, worin X für SO steht und
worin NR¹R² ausgewählt ist aus der Gruppe bestehend aus 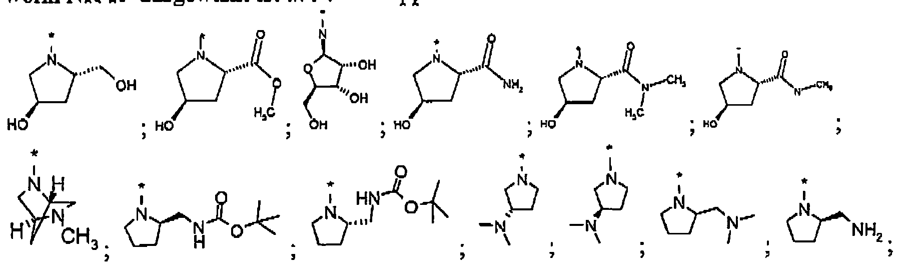 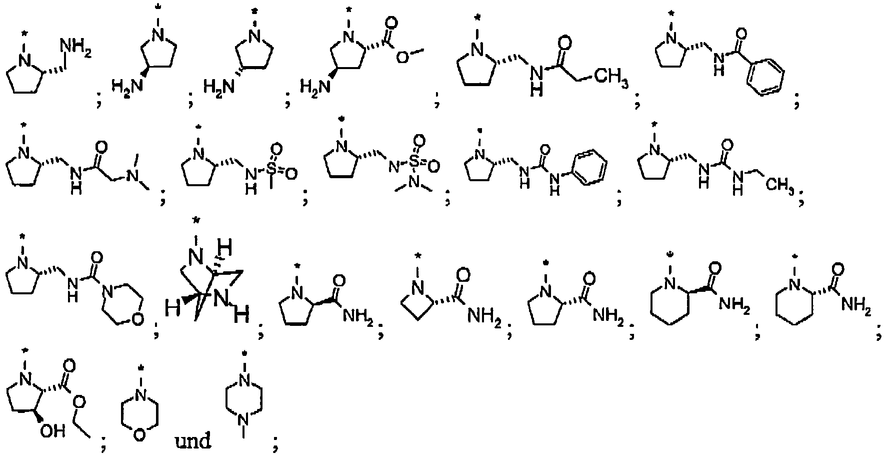 und worin
R³ Cl ist,
sowie pharmakologisch verträgliche Salze, Diastereomere, Enantiomere, Racemate, Hydrate oder Solvate davon.

25. Verbindungen
der Formel 4 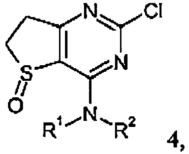 der Formel 5 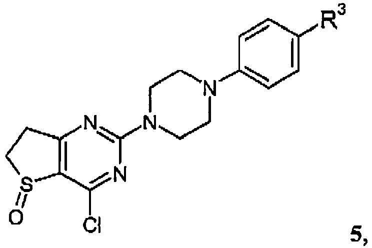 der Formel 6 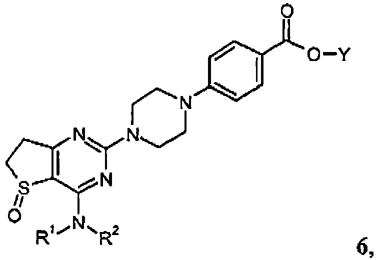 der Formel 7 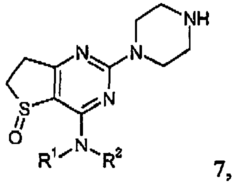 der Formel 8 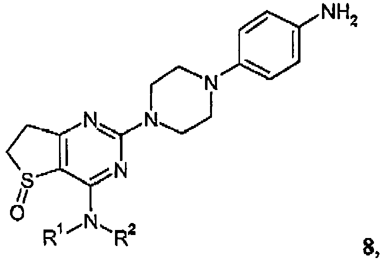
worin R¹, R² und R³ nach einem der Ansprüche 1 bis 23 definiert sind, und worin
Y H, Methyl oder Ethyl bedeutet,
sowie pharmakologisch verträgliche Salze, Diastereomere, Enantiomere, Racemate, Hydrate oder Solvate davon.

26. Verbindungen nach einem der Ansprüche 1 bis 24 als Arzneimittel

27. Verwendung von Verbindungen nach einem der Ansprüche 1 bis 24 zur Herstellung eines Medikaments zur Behandlung von Atemwegs- oder gastrointestinalen Beschwerden oder Erkrankungen, wie auch entzündliche Erkrankungen der Gelenke, der Haut oder der Augen, Krebserkrankungen, sowie von Erkrankungen des periphären oder zentralen Nervensystems.

28. Verwendung von Verbindungen nach einem der Ansprüche 1 bis 24 zur Herstellung eines Medikaments zur Vorbeugung und Behandlung von Atemwegs- oder Lungenerkrankungen, die mit einer erhöhten Schleimproduktion, Entzündungen und / oder obstruktiven Erkrankungen der Atemwege einher gehen.

29. Verwendung von Verbindungen nach einem der Ansprüche 1 bis 24 zur Herstellung eines Medikament zur Behandlung von entzündlichen und obstruktiven Erkrankungen wie COPD, chronischer Sinusitis, Asthma, Morbus Crohn, Colitis ulcerosa, sowie von entzündlichen Erkrankungen des Gastrointestinaltraktes.

30. Verwendung von Verbindungen nach einem der Ansprüche 1 bis 24 zur Herstellung eines Medikaments zur Vorbeugung und Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

## Claims

1. Compounds of formula 1, 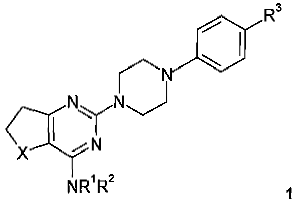 wherein
X denotes SO;
R¹ denotes H, C₁₋₆-alkyl, C₂₋₆-alkenyl, C₂₋₆-alkynyl, C₆₋₁₀-aryl, C₆₋₁₀-aryl-C₁₋₆-alkylene or C₅₋₁₀-heteroaryl-C₁₋₆-alkylene
R² is H or a group selected from among C₁₋₁₀-alkyl, C₂₋₆-alkenyl and C₂₋₆-alkynyl, which may optionally be substituted by halogen and which may optionally be substituted by one or more groups selected from among OR^{2.1}, COOR^{2.1}, CONR^{2.2}R^{2.3}, SR^{2.1}, C₆₋₁₀-aryl, a mono- or bicyclic C₃₋₁₀ heterocycle, a mono- or bicyclic C₅₋₁₀-heteroaryl, a mono- or bicyclic C₃₋₁₀-cycloalkyl, CH₂-NR^{2.2}R^{2.3} and NR^{2.2}R^{2.3}, which may in turn optionally be substituted by one or more groups selected from among OH, halogen, OR^{2.1}, oxo, C₁₋₆-alkyl, C₆₋₁₀-aryl, COOR^{2.1}, CH₂-NR^{2.2}R^{2.3} and NR^{2.2}R^{2.3}, wherein R^{2.1} is H or a group selected from among C₁₋₆-alkyl, C₁₋₆-alkanol, C₁₋₃-haloalkyl, mono- or bicyclic C₃₋₁₀ cycloalkyl. C₆₋₁₀-aryl-C₁₋₆-alkylene, mono- or bicyclic C₅₋₁₀-heteroaryl-C₁₋₆-alkylene, C₃₋₁₀-heterocycle-C₁₋₆-alkylene, C₃₋₁₀-cycloalkyl-C₁₋₆-alkylene, a mono- or bicyclic C₆₋₁₀-aryl, a mono- or bicyclic C₅₋₁₀-heteroaryl and a mono- or bicyclic, saturated or unsaturated heterocycle, which may optionally be substituted by one or more groups selected from among OH, halogen, C₁₋₆-alkyl and C₆₋₁₀-aryl, while **R^{2.2}** and **R^{2.3}** independently of one another are H or a group selected from among halogen, C₁₋₆-alkyl, mono- or bicyclic C₃₋₁₀ cycloalkyl, C₆₋₁₀-aryl-C₁₋₆-alkylene, C₆₋₁₀-heteroaryl-C₁₋₆-alkylene, mono- or bicyclic C₆₋₁₀-aryl, mono- or bicyclic C₃₋₁₀ heterocycle, mono- or bicyclic C₅₋₁₀-heteroaryl, CO-NH₂, CO-NHCH₃, CO-N(CH₃)₂, SO₂(C₁-C₂-alkyl), CO-R^{2.1} and COOR^{2.1}, which may optionally be substituted by one or more groups selected from among OH, halogen, C₁₋₆-alkyl, C₆₋₁₀-aryl and COOR^{2.1},
or **R²** denotes a mono- or polycyclic C₃₋₁₀ cycloalkyl, which may optionally be bridged by one or more C₁₋₃-alkyl groups and which may optionally be substituted by a group selected from among branched or unbranched C₁₋₆-alkanol, OR^{2.1}, COOR^{2.1}, SO₂NR^{2.2}R^{2.3}, C₃₋₁₀ heterocycle, C₆₋₁₀-aryl, C₁-₆-alkyl, C₆₋₁₀-aryl-C₁₋₆-alkylene, C₅₋₁₀-heteroaryl-C₁₋₆-alkylene, mono- or bicyclic C₃₋₁₀ cycloalkyl and NR^{2.2}R^{2.3}, which may optionally be substituted by one or more groups selected from among OH, OR^{2.1}, oxo, halogen, C₁₋₆-alkyl, C₆₋₁₀-aryl and NR^{2.2}R^{2.3},
or **R²** denotes a mono- or polycyclic C₆₋₁₀-aryl, which may optionally be substituted by OH, SH or halogen or by one or more groups selected from among OR^{2.1}, COOR^{2.1}, NR^{2.2}R^{2.3}, CH₂-NR^{2.2}R^{2.3},C₃₋₁₀-cycloalkyl, C₃₋₁₀ heterocycle, C₁₋₆-alkyl, C₆₋₁₀-aryl-C₁₋₆-alkylene, C₃₋₁₀ heterocycle-C₁₋₆-alkylene, C₅₋₁₀-heteroaryl-C₁₋₆-alkylene, C₆₋₁₀-aryl, SO₂-CH₃, SO₂-CH₂CH₃ and SO₂-NR^{2.2}R^{2.3}, which may in turn optionally be substituted by one or more groups selected from among OH, OR^{2.1}, oxo, halogen, C₁₋₆-alkyl, C₆₋₁₀-aryl and NR^{2.2}R^{2.3},
or **R²** denotes a group selected from among mono- or bicyclic, saturated or unsaturated C₃₋₁₀ heterocycle and a mono- or bicyclic C₆₋₁₀-heteroaryl, which includes 1 to 4 heteroatoms selected from among S, O and N and may optionally be substituted by one or more groups selected from among halogen, OH, oxo and SH or by one or more groups selected from among OR^{2.1}, SR^{2.1}, COOR^{2.1}, COR^{2.1}, C₁₋₆-alkanol, C₃₋₁₀-cycloalkyl, C₆₋₁₀-aryl, C₁₋₆-alkyl, C₆₋₁₀-aryl-C₁₋₆-alkylene, C₆₋₁₀-heteroaryl-C₁₋₆-alkylene, C₅₋₁₀ heterocycle, C₅₋₁₀-heteroaryl, C₁₋₆-alkanol and NR^{2.2}R^{2.3}, which may in turn optionally be substituted by one or more groups selected from among OH, OR^{2.1}, oxo, halogen, C₁₋₆-alkyl, C₆₋₁₀-aryl and NR^{2.2}R^{2.3}, or NR¹R² together denote a heterocyclic C₄₋₇ ring, which may optionally be bridged, which contains 1, 2 or 3 heteroatoms selected from among N, O and S and which may optionally be substituted by one or more groups selected from among OH, OR^{2.1}, C₁₋₆-alkanol, oxo, halogen, C₁₋₆-alkyl, C₆₋₁₀-aryl, COOR^{2.1}, CH₂-NR^{2,2}-COO-R^{2.1}, CH₂-NR^{2,2-}CO-R^{2.1}, CH₂-NR^{2,2-}CO-CH₂-NR^{2.2}R^{2.3}, CH₂-NR^{2,2-}SO₂-C₁₋₃-alkyl , CH₂-NR^{2,2}-SO₂-NR^{2.2}R^{2.3}, CH₂-NR^{2,2}-CO-NR^{2.2}R^{2.3}, CO-NR^{2.2}R^{2.3}, CH₂-NR^{2.2}R^{2.3} and NR^{2.2}R^{2.3}, and wherein
R³ is selected from among fluorine, chlorine, bromine, iodine, hydroxy, SO₂-CH₃, COOR^{2.1}, nitrile group and C₃₋₁₀ heterocycle-C₁₋₆-alkylene, wherein the C₃₋₁₀ heterocycle may be mono- or bicyclic and may optionally be substituted by a group selected from among OH, halogen, oxo, C₁₋₆-alkyl and C₆₋₁₀-aryl, or is a group selected from among C₁₋₆-alkyl, C₂₋₆-alkenyl, C₂₋₆-alkynyl, C₆-₁₀-aryl; C₆₋₁₀-aryl-C₁₋₆-alkylene, C₅₋₁₀-heteroaryl-C₁₋₆-alkylene, C₃₋₁₀ heterocycle and C₃₋₁₀-cycloalkyl, which may optionally be substituted by a group selected from among OH, halogen, oxo" C₁₋₆-alkyl and C₆₋₁₀-aryl, or wherein
R³ denotes the group -CO-NR^{3.1}R^{3.2},
wherein R^{3.1} and R^{3.2} independently of one another are H or groups selected from among C₁₋₆-alkyl, C₂₋₆-alkenyl, C₂₋₆-alkynyl, C₆₋₁₀-aryl; C₆₋₁₀-aryl-C₁₋₆-alkylene, C₅₋₁₀-heteroaryl-C₁₋₆-alkylene, C₅₋₁₀-heteroaryl-C₁₋₆-alkynylene, C₅₋₁₀-heteroaryl-C₁₋₆-alkenylene, mono- or bicyclic, C₃₋₁₀ heterocycle, C₃-₁₀ heterocycle-C₁₋₆-alkylene and mono- or bicyclic C₅₋₁₀-heteroaryl, wherein the group in each case may optionally be substituted by one or more groups selected from among OH, oxo, halogen, C₁₋₆-alkyl and O-C₁₋₆-alkyl, or wherein
R³ denotes the group -NR^{3.3}-CO-R^{3.4}, wherein R^{3.3} is H or a group selected from among C₁₋₆-alkyl, C₂₋₆-alkenyl, C₂₋₆-alkynyl, C₆₋₁₀-aryl; C₆₋₁₀-aryl-C₁₋₆-alkylene, C₅₋₁₀-heteroaryl-C₁₋₆-alkylene, C₃₋₁₀ heterocycle and a C₅₋₁₀- heteroaryl, which may optionally be substituted by one or more groups selected from among OH, OR^{2.1}, oxo, NH₂, NR^{2.2}R^{2.3}, halogen, C₁₋₆-alkyl and C₆₋₁₀-aryl, and wherein R^{3.4} is H or a group selected from among C₁₋₆-alkyl, C₂₋₆-alkenyl, C₂₋₆-alkynyl, C₁₋₆-alkanol, OR^{2.1}, CH₂-O-CO-C₁₋₆-alkyl, CH₂-NR^{2.2}R^{2.3}, NR^{2.2}R^{2.3}, C₆₋₁₀-aryl ; C₆₋₁₀-aryl-C₁₋₆-alkylene, C₅₋₁₀-hoteroaryl-C₁₋₆-alkylene, mono- or bicyclic, saturated, partially saturated or unsaturated C₃₋₁₀ heterocycle with 1, 2 or 3 heteroatoms selected from among S, O and N and a mono- or bicyclic C₅₋₁₀-heteroaryl with 1, 2 or 3 heteroatoms selected from among S, O and N, which may optionally be substituted by one or more groups selected from among OH, OR^{2.1}, oxo, NH₂, NR^{2.2}R^{2.3}, halogen, C₁₋₆-alkyl and C₆₋₁₀-aryl, or wherein
R³ denotes an optionally mono- or di-N-substituted sulphonamide group SO₂-NR^{3.5}R^{3.6},
wherein R^{3.5} and R^{3.6} may each independently of one another be C₁₋₆-alkyl or C₆-₁₀-aryl,
as well as pharmacologically acceptable salts, diastereomers, enantiomers, racemates, hydrates or solvates thereof.

2. Compounds of formula 1 according to claim 1, wherein
**X** denotes SO
**R¹** denotes H, C₁₋₆-alkyl, C₆₋₁₀-aryl, C₆₋₁₀-aryl-C₁₋₆-alkylene or C₅₋₁₀-hoteroaryl-C₁₋₆-alkylene
**R²** is H or C₁₋₆-alkyl, which may optionally be substituted by halogen and which may optionally be substituted by one or more groups selected from among OR^{2.1}, COOR^{2.1}, CONR^{2.2}R^{2.3}, SR^{2.1}, C₆₋₁₀-aryl, a mono- or bicyclic C₃₋₁₀ heterocycle, a mono- or bicyclic C₅₋₁₀-heteroaryl, a mono- or bicyclic C₃₋₁₀-cycloalkyl, CH₂-NR^{2.2}R^{2.3} and NR^{2.2}R^{2.3}, which may in turn optionally be substituted by one or more groups selected from among OH, halogen, OR^{2.1}, oxo, C₁₋₆-alkyl, C₆₋₁₀-aryl, COOR^{2.1}, CH₂-NR^{2.2}R^{2.3} and NR^{2.2}R^{2.3}, wherein **R^{2.1}** is H or a group selected from among C₁₋₆-alkyl, C₁₋₆-alkanol, C₁₋₃-haloalkyl, a mono- or bicyclic C₃₋₁₀-cycloalkyl, a C₆₋₁₀-aryl-C₁₋₆-alkylene or C₅₋₁₀-heteroaryl-C₁₋₆-alkylene, C₃₋₁₀ heterocycle-C₁₋₆-alkylene, C₃₋₁₀-cycloalkyl-C₁₋₆-alkylene, a mono- or bicyclic C₆₋₁₀-aryl, a mono- or bicyclic C₅₋₁₀-heteroaryl and a mono- or bicyclic, saturated or unsaturated C₃₋₁₀ heterocycle, which may optionally be substituted by one or more groups selected from among OH, halogen, C₁₋₆-alkyl, C₆₋₁₀-aryl, wherein **R^{2.2} and R^{2.3}** independently of one another are H or are selected from among halogen, C₁₋₆-alkyl, mono- or bicyclic C₃₋₁₀ cycloalkyl, C₆₋₁₀-aryl-C₁₋₆-alkylene, C₅₋₁₀-heteroaryl-C₁₋₆-alkylene, mono- or bicyclic C₆₋₁₀-aryl, mono- or bicyclic, saturated or unsaturated C₃₋₁₀ heterocycle, mono- or bicyclic C₅₋₁₀-heteroaryl, CO-NH₂, CO-NHCH₃, CO-N(CH₃)₂, SO₂(C₁-C₂-alkyl), CO-R^{2.1} and COOR^{2.1}, which may optionally be substituted by one or more groups selected from among OH, halogen, C₁₋₆-alkyl, C₆₋₁₀-aryl and COOR^{2.1}, or
**R²** denotes a mono- or polycyclic C₃₋₁₀-cycloalkyl, which may optionally be bridged by one or more C₁₋₃-alkyl groups and which may optionally be mono- or polysubstituted by OH or halogen or by one or more groups selected from among branched or unbranched C₁₋₆-alkanol, OR^{2.1}, COOR^{2.1}, SO₂NR^{2.2}R^{2.3}, C₃₋₁₀ heterocycle, C₆₋₁₀-aryl, C₁₋₆-alkyl, C₆₋₁₀-aryl-C₁₋₆-alkylene, C₅₋₁₀-heteroaryl-C₁₋₅-alkylene, mono- or bicyclic C₃₋₁₀-cycloalkyl and NR^{2.2}R^{2.3}, which may optionally be substituted by one or more groups selected from among OH, OR^{2.1}, oxo, halogen, C₁₋₆-alkyl, C₆₋₁₀-aryl and NR^{2.2}R^{2.3}, or
**R²** denotes a mono- or polycyclic C₆₋₁₀-aryl, which may optionally be substituted by OH, SH or halogen or by one or more groups selected from among OR^{2.1}, COOR^{2.1}, NR^{2.2}R^{2.3}, CH₂-NR^{2.2}R^{2.3},C₃₋₁₀-cycloalkyl, C₃₋₁₀ heterocycle, C₁₋₆-alkyl, C₆₋₁₀-aryl-C₁₋₆-alkylene, C₃₋₁₀ heterocycle-C₁₋₆-alkylene, C₅₋₁₀-heteroaryl-C₁₋₆-alkylene, C₆₋₁₀-aryl, SO₂-CH₃, SO₂-CH₂CH₃ and SO₂-NR^{2.2}R^{2.3}, which may in turn optionally be substituted by one or more groups selected from among OH, OR^{2.1}, oxo, halogen, C₁₋₆-alkyl, C₆₋₁₀-aryl and NR^{2.2}R^{2.3}, or
R² denotes a group selected from among mono- or bicyclic, saturated or unsaturated C₃₋₁₀ heterocycle and a mono- or bicyclic C₅₋₁₀-heteroaryl, which includes 1 to 4 heteroatoms selected from among S, O and N and may optionally be substituted by one or more groups selected from among halogen, OH, oxo and SH or by one or more groups selected from among OR^{2.1}, SR^{2.1}, COOR^{2.1}, COR^{2.1}, C₁₋₆-alkanol, C₃₋₁₀-cycloalkyl, C₆₋₁₀-aryl, C₁₋₆-alkyl, C₆₋₁₀-aryl-C₁₋₆-alkylene, C₅₋₁₀-heteroaryl-C₁₋₆-alkylene, C₅₋₁₀ heterocycle, C₅₋₁₀-heteroaryl, C₁₋₆-alkanol and NR^{2.2}R^{2.3}, which may in turn optionally be substituted by one or more groups selected from among OH, OR^{2.1}, oxo, halogen, C₁₋₆-alkyl, C₆₋₁₀-aryl and NR^{2.2}R^{2.3},
or **NR¹R²** together denotes a heterocyclic C₄₋₇ ring, which may optionally be bridged, which contains 1, 2 or 3 heteroatoms selected from among N, O and S and which may optionally be substituted by one or more groups selected from among OH, OR^{2.1}, C₁₋₆-alkanol, oxo, halogen, C₁₋₆-alkyl, C₆₋₁₀-aryl, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2,2-}SO₂-C₁₋₃-alkyl, CH₂-NR^{2,2-}SO₂-NR^{2.2}R^{2.3}, CH₂-NR^{2,2-}CO-NR^{2.2}R^{2.3}, CO-NR^{2.2}R^{2.3}, CH₂-NR^{2.2}R^{2.3} and NR^{2.2}R^{2.3},
as well as pharmacologically acceptable salts, diastereomers, enantiomers, racemates, hydrates or solvates thereof.

3. Compounds of formula **1** according to claim 1 and 2, wherein
**X** is SO,
**R¹** is H, C¹⁻⁶-alkyl, C⁶⁻¹⁰-aryl, C₆₋₁₀-aryl-C₁₋₆-alkylene or C₅₋₁₀-heteroaryl-C₁₋₆-alkylene;
**R²** is H or C₁₋₆-alkyl, which may optionally be substituted by halogen and which may optionally be substituted by one or more groups selected from among OR^{2.1}, COOR^{2,1},CONR^{2.2}R^{2.3}, SR^{2.1}, phenyl, a mono- or bicyclic C₅₋₁₀ heterocycle, C₅₋₆-heteroaryl, a mono- or bicyclic C₅₋₁₀-cycloalkyl, CH₂-NR^{2.2}R^{2.3} and NR^{2.2}R^{2.3}, which may in turn optionally be substituted by one or more groups selected from among OH, halogen, OR^{2.1}, oxo, C₁₋₆-alkyl, phenyl, COOR^{2.1}, CH₂-NR^{2.2}R^{2.3} and NR^{2.2}R^{2.3}, wherein **R^{2.1}** is H or a group selected from among C₁₋₆-alkyl, C₁₋₆-alkanol, C₁₋₃-haloalkyl, a mono- or bicyclic C₅₋₁₀ cycloalkyl, a phenyl-C₁₋₆-alkylene, a C₅₋₆-heteroaryl-C₁₋₆-alkylene, C₅₋₁₀-heterocycle-C₁₋₆-alkylene, C₅₋₁₀-cycloalkyl-C₁₋₆-alkylene, phenyl, a mono- or bicyclic C₅₋₁₀-heteroaryl and a mono- or bicyclic, saturated or unsaturated C₅₋₁₀ heterocycle, which may optionally be substituted by one or more groups selected from among OH, halogen, C₁₋₆-alkyl and phenyl, wherein **R^{2.2}** and **R^{2.3}** independently of one another are H or a group selected from among halogen, C₁₋₆-alkyl, mono- or bicyclic C₅₋₁₀ cycloalkyl, phenyl-C₁₋₆-alkylene, C₅₋₆-heteroaryl-C₁₋₆-alkylene, phenyl, mono or bicyclic C₅₋₁₀ heterocycle, mono- or bicyclic C₅₋₆-heteroaryl, CO-NH₂, CO-NHCH₃, CO-N(CH₃)₂, SO₂(C₁-C₂-alkyl), CO-R^{2.1} and COOR^{2.1}, which may optionally be substituted by one or more groups selected from among OH, halogen, C₁₋₆-alkyl, phenyl and COOR^{2.1}, **R²** or denotes a mono- or polycyclic C₅₋₁₀-cycloalkyl, which may optionally be bridged by one or more C₁₋₃-alkyl groups and which may optionally be mono- or polysubstituted by OH or halogen or by one or more groups selected from among branched or unbranched C₁₋₃-alkanol, OR^{2.1}, COOR^{2.1}, SO₂NR^{2.2}R^{2.3}, C₅₋₁₀ heterocycle, phenyl, C₁₋₆-alkyl, phenyl-C₁₋₆-alkylene, C₅₋₆-heteroaryl-C₁₋₆-alkylene, mono- or bicyclic C₅₋₁₀-cycloalkyl and NR^{2.2}R^{2.3}, which may optionally be substituted by one or more groups selected from among OH, OR^{2.1}, oxo, halogen, C₁₋₆-alkyl, phenyl and NR^{2.2}R^{2.3}, or
R² denotes a phenyl, which may optionally be substituted by OH, SH or halogen or by one or more groups selected from among OR^{2.1}, COOR^{2.1}, NR^{2.2}R^{2.3}, CH₂-NR^{2.2}R^{2.3}, C₅₋₁₀-cycloalkyl, C₅₋₁₀ heterocycle, C₁₋₆-alkyl, phonyl-C₁₋₆-alkylene, C₅₋₁₀ heterocycle-C₁₋₆-alkylene, C₅₋₆-heteroaryl-C₁₋₆-alkylene, phenyl, SO₂-CH₃, SO₂-CH₂CH₃ and SO₂-NR^{2.2}R^{2.3}, which may in turn optionally be substituted by one or more groups selected from among OH, OR^{2.1}, oxo, halogen, C₁₋₆-alkyl, C₆₋₁₀-aryl and NR^{2.2}R^{2.3}, or
R² denotes a group selected from among mono or bicyclic, saturated or unsaturated C₅₋₁₀ heterocycle and a mono- or bicyclic C₅₋₆-heteroaryl, which contains 1 to 4 heteroatoms selected from among S, O and N and may optionally be substituted by one or more groups selected from among halogen, OH, oxo and SH or by one or more groups selected from among OR^{2.1}, SR^{2.1}, COOR^{2.1}, COR^{2.1}, C₁₋₆-alkanol, C₃₋₁₀-cycloalkyl, phenyl, C₁₋₆-alkyl, phenyl-C₁₋₆-alkylene, C₅₋₆-heteroaryl-C₁₋₆-alkylene, C₅₋₁₀ heterocycle, C₅₋₆-heteroaryl, C₁₋₆-alkanol and NR^{2.2}R^{2.3}, which may in turn optionally be substituted by one or more groups selected from among OH, OR^{2.1}, oxo, halogen, C₁₋₆-alkyl, phenyl and NR^{2.2}R^{2.3},
or **NR¹R²** together denotes a heterocyclic C₄₋₇, ring, which may optionally be bridged, which contains 1, 2 or 3 heteroatoms selected from among N, O and S and which may optionally be substituted by one or more groups selected from among OH, OR^{2.1}, C₁₋₆-alkanol, oxo, halogen, C₁₋₆-alkyl, C₆₋₁₀-aryl, COOR^{2.1}, CH₂-NR^{2.2}-COO-R^{2.1}, CH₂-NR^{2.2-}CO-R^{2.1}, CH₂- NR^{2,2}-CO-CH₂-NR^{2.2}R^{2.3}, CH₂-NR^{2,2}-SO₂-C₁₋₃-alkyl, CH₂-NR^{2,2}SO₂-NR^{2.2}R^{2.3}, CH₂-NR^{2,1-}CO-NR^{2.2}R^{2.3}, CO-NR^{2.2}R^{2.3}, CH₂-NR^{2.2}R^{2.3} and NR^{2.2}R^{2.3},
as well as pharmacologically acceptable salts, diastereomers, enantiomers, racemates, hydrates or solvates thereof.

4. Compounds of formula **1** according to claim 1 to 3, wherein
**R¹** is H or methyl,
as well as pharmacologically acceptable salts, diastereomers, enantiomers, racemates, hydrates or solvates thereof.

5. Compounds of formula **1** according to claim 1 to 3, wherein
**NR¹R²** together denotes a pyrrolidine ring, which may optionally be substituted by one or more groups selected from among OH, OR^{2.1}, CH₂-OH, CH₂-CH₂-OH, oxo, Cl, F, Br, methyl, ethyl, propyl, phenyl, COOR^{2.1}, CH₂-NR^{2,2}-COO-R^{2.1}, CH₂-NR^{2,2}-CO-R^{2.1}, CH₂-NR^{2,2}-CO-CH₂-NR^{2.2}R^{2.3}, CH₂-NR^{2,2-}SO₂-C₁₋₃-alkyl, CH₂-NR^{2,2-}SO₂-NR^{2.2}R^{2.3}, CH₂-NR^{2,2-}CO-NR^{2.2}R^{2.3}, CO-NR^{2.2}R^{2.3}, CH₂-NR^{2.2}R^{2.3} and NR^{2.2}R^{2.3},
as well as pharmacologically acceptable salts, diastereomers, enantiomers, racemates, hydrates or solvates thereof.

6. Compounds of formula **1** according to claim 1 to 4, wherein
**R²** denotes phenyl which is mono- or polysubstituted by OH, SH or halogen or by one or more groups selected from among OR^{2.1}, COOR^{2.1}, NR^{2.2}R^{2.3}, CH₂-NR^{2.2}R^{2.3},C₅₋₁₀-cycloalkyl, C₅₋₁₀ heterocycle, C₁₋₆-alkyl, phenyl-C₁₋₆-alkylene, C₅₋₁₀ heterocycle-C₁₋₆-alkylene, C₅₋₆-heteroaryl-C₁₋₆-alkylene, phenyl, SO₂-CH₃, SO₂-CH₂CH₃ and SO₂-NR^{2.2}R^{2.3} at any desired position, which may in turn optionally be substituted by one or more groups selected from among OH, OR^{2.1}, oxo, halogen, C₁₋₆-alkyl, phenyl and NR^{2.2}R^{2.3},
as well as pharmacologically acceptable salts, diastereomers, enantiomers, racemates, hydrates or solvates thereof.

7. Compounds of formula **1** according to claim 1 to 4, wherein
**R²** is phenyl which may be substituted in at least one of the two meta positions by OH, SH or halogen or by one or more groups selected from among OR^{2.1}, COOR^{2.1}, NR^{2.2}R^{2.3}, CH₂-NR^{2.2}R^{2.3}, C₅₋₁₀-cycloalkyl, C₅₋₁₀ heterocycle, C₁₋₆-alkyl, phenyl-C₁₋₆-alkylene, C₅₋₁₀ heterocycle-C₁₋₆-alkylene, C₅₋₆-heteroaryl-C₁₋₆-alkylene, phenyl, SO₂-CH₃, SO₂-CH₂CH₃ and SO₂-NR^{2.2}R^{2.3}, which may in turn optionally be substituted by one or more groups selected from among OH, OR^{2.1}, oxo, halogen, C₁₋₆-alkyl, phenyl and NR^{2.2}R^{2.3},
as well as pharmacologically acceptable salts, diastereomers, enantiomers, racemates, hydrates or solvates thereof.

8. Compounds of formula **1** according to claim 7, wherein
**R²** is phenyl which is substituted in at least one of the two meta positions by one or more groups selected from among methyl, F, Cl, OH, OR^{2.1}, COOR^{2.1}, NH₂ and N(CH₃)₂,
as well as pharmacologically acceptable salts, diastereomers, enantiomers, racemates, hydrates or solvates thereof.

9. Compounds of formula **1** according to one of claims 1 to 4, wherein
**R²** is C₁₋₆-alkyl, which may optionally be substituted by halogen and which may optionally be substituted by one or more groups selected from among OR^{2.1}, COOR^{2.1}, CONR^{2.2}R^{2.3}, SR^{2.1}, phenyl, a mono- or bicyclic C₅₋₁₀ heterocycle, C₅₋₆-heteroaryl, a mono- or bicyclic C₅₋₁₀-cycloalkyl, CH₂-NR^{2.2}R^{2.3} and NR^{2.2}R^{2.3}, wherein the group from the second group may in turn be substituted by one or more groups selected from among OH, halogen, OR^{2.1}, oxo, C₁₋₆-alkyl, phenyl, COOR^{2.1}, CH₂-NR^{2,2}R^{2.1} and NR^{2.2}R^{2.3},
as well as pharmacologically acceptable salts, diastereomers, enantiomers, racemates, hydrates or solvates thereof.

10. Compounds of formula 1 according to one of claims 1 to 4 and 9, wherein
**R²** is methyl, ethyl or propyl,
as well as pharmacologically acceptable salts, diastereomers, enantiomers, racemates, hydrates or solvates thereof.

11. Compounds of formula **1** according to one of claims 1 to 4, wherein
**R²** is C₁₋₆-alkyl, which is substituted by one or more groups selected from among OH, COOR^{2.1}, CON(CH₃)₂, C₁₋₆-alkyl, phenyl, cyclopropyl and NR^{2.2}R^{2.3}, which may in turn optionally be substituted by one or more groups selected from among OH, fluorine, chlorine, bromine, iodine, OR^{2.1}, oxo, C₁₋₆-alkyl, phenyl, C₁₋₃-alkanol, CH₂-NR^{2,2}R^{2.3} and NR^{2,2}R^{2.3},
as well as pharmacologically acceptable salts, diastereomers, enantiomers, racemates, hydrates or solvates thereof.

12. Compounds of formula **1** according to one of claims 1 to 4, wherein
**R²** is C₁₋₆-alkyl, which is substituted by one or more groups selected from among OH, phenyl, COOR^{2.1}, NH₂, wherein the phenyl in turn may optionally be substituted by one or more groups selected from among OH, fluorine, chlorine, bromine, iodine, OR^{2.1}, C₁₋₆-alkyl, CH₂-NH₂, CH₂(CH₃)₂, NH₂ and N(CH₃)₂,
as well as pharmacologically acceptable salts, diastereomers, enantiomers, racemates, hydrates or solvates thereof.

13. Compounds of formula **1** according to one of claims 1 to 4 and 11 to 12, wherein
**R²** is a group according to formula **2** 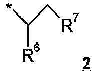
wherein **R⁷** is OH or NH₂ and
wherein **R⁶** is a group selected from among C₁₋₆-alkyl, C₅₋₁₀-heteroaryl and C₆-₁₀-aryl, preferably phenyl, which may optionally be substituted by one or more groups selected from among halogen, OH, COOR^{2.1}, OR^{2.1}, NH₂, C₁₋₆-alkyl, C₁₋₆-haloalkyl and C₁₋₆-alkanol,
as well as pharmacologically acceptable salts, diastereomers, enantiomers, racemates, hydrates or solvates thereof.

14. Compounds of formula **1** according to claim 13, wherein
**R²** is a group according to formula **2** 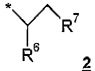
wherein **R⁷** is OH or NH₂ and
**R⁶** is methyl, ethyl, propyl, isopropyl.

15. Compounds of formula **1** according to one of claims 1 to 4, wherein
**R²** is a monocyclic C₃₋₇-cycloalkyl ring, which may be substituted in the spiro position by a group selected from among -OH, -CH₂-OH, -CH₂-CH₂-OH, branched or unbranched C₃₋₆-alkanol, -OR^{2.1}, methyl, ethyl, propyl, isopropyl, butyl, isobutyl and halogen, wherein R^{2.1} may be selected from among methyl, ethyl, propyl, isopropyl, butyl, isobutyl.

16. Compounds of formula **1** according to one of claims 1 to 4, wherein
**R²** denotes a group selected from among monocyclic, saturated three, four, five, six or seven-membered heterocycle with 1, 2 or 3 heteroatoms each selected from among N, O and S, which may optionally be substituted by one or more groups selected from among fluorine, chlorine, bromine, OH, oxo and SH or by one or more groups selected from among OR^{2.1}, SR^{2.1}, COOR^{2.1}. COR^{2.1}, C₁₋₆-alkanol, C₃₋₁₀-cycloalkyl, phenyl, C₁₋₆-alkyl, phenyl-C₁₋₆-alkylene, C₅₋₁₀-heteroaryl-C₁₋₆-alkylene, C₅₋₁₀ heterocycle, C₅₋₁₀-heteroaryl and NR^{2.2}R^{2.3}, which may in turn optionally be substituted by one or more groups selected from among OH, OR^{2.1}, oxo, halogen, C₁₋₆-alkyl, phenyl and NR^{2.2}R^{2.3}, wherein
R^{2.1}, denotes H or a group selected from among C₁₋₆-alkyl, C₁₋₆-alkanol. C₁₋₃-haloalkyl, mono- or bicyclic C₃₋₁₀ cycloalkyl, phenyl-C₁₋₆-alkylene, mono- or bicyclic C₅₋₁₀-heteroaryl-C₁₋₆-alkylene, C₃₋₁₀ heterocycle-C₁₋₆-alkylene, C₃₋₇-cycloalkyl-C₁₋₆-alkylene, phenyl, a mono- or bicyclic C₅₋₁₀-heteroaryl and a monocyclic, saturated or unsaturated, five, six or seven-membered heterocycle with 1, 2 or 3 heteroatoms selected from among N, O and S, which may optionally be substituted by one or more groups selected from among OH, halogen, C₁₋₆-alkyl and phenyl, wherein **R^{2.2}** and **R^{2.3}** independently of one another are H or a group selected from among halogen, C₁₋₆-alkyl, mono- or bicyclic C₃₋₁₀ cycloalkyl, phenyl-C₁₋₆-alkylene, C₅₋₁₀-heteroaryl-C₁₋₆-alkylene, phenyl, mono or bicyclic C₃₋₁₀ heterocycle, mono- or bicyclic C₅₋₁₀-heteroaryl, CO-NH₂, CO-NHCH₃, CO-N(CH₃)₂, SO₂(C₁-C₂-alkyl), CO-R^{2.1}, and COOR^{2.1}, which may optionally be substituted by one or more groups selected from among OH, halogen, C₁₋₆-alkyl, phenyl and COOR^{2.1}.

17. Compounds of formula **1** according to one of claims 1 to 16, wherein
**R³** denotes fluorine, chlorine, bromine, iodine or CN,
as well as pharmacologically acceptable salts, diastereomers, enantiomers, racemates, hydrates or solvates thereof.

18. Compounds of formula **1** according to one of claims 1 to 16, wherein
**R³** denotes the group -CO-NR^{3.1}R^{3.2},
wherein **R^{3.1}** and **R^{3.2}** independently of one another are H or groups selected from among C₁₋₆-alkyl, C₂₋₆-alkenyl, C₂₋₆-alkynyl, C₆₋₁₀-aryl; C₆₋₁₀-aryl-C₁₋₆-alkylene, C₅₋₁₀-heteroaryl-C₁₋₆-alkylene, C₅₋₁₀-heteroaryl-C₁₋₆-alkynylene, C₅₋₁₀-heteroaryl-C₁₋₆-alkenylene, mono- or bicyclic, C₅₋₁₀ heterocycle, C₅-₁₀ heterocycle-C₁₋₆-alkylene and mono- or bicyclic C₅₋₁₀-heteroaryl, wherein the group may in each case optionally be substituted by one or more groups selected from among OH, oxo, halogen, C₁₋₆-alkyl and O-C₁₋₆-alkyl,
as well as pharmacologically acceptable salts, diastereomers, enantiomers, racemates, hydrates or solvates thereof.

19. Compounds of formula **1** according to one of claims 1 to 16 and 18, wherein
R³ denotes the group -CO-NR^{3.1}R^{3.2},
wherein **R^{3.1}** and **R^{3.2}** independently of one another are H or groups selected from among C₁₋₆-alkyl, phenyl; phenyl-C₁₋₆-alkylene, C₅₋₆-heteroaryl-C₁₋₆-alkylene, C₅₋₆-heteroaryl-C₁₋₆-alkynylene, C₅₋₆-heteroaryl-C₁₋₆-alkenylene, mono- or bicyclic, C₅₋₁₀ heterocycle, C₆₋₁₀ heterocycle-C₁₋₆-alkylene and mono- or bicyclic C₅₋₁₀-heteroaryl , wherein the group may in each case optionally be substituted by one or more groups selected from among OH, oxo, halogen, C₁₋₆-alkyl and O-C₁₋₆-alkyl,
as well as pharmacologically acceptable salts, diastereomers, enantiomers, racemates, hydrates or solvates thereof.

20. Compounds of formula **1** according to one of claims 1 to 16, wherein
**R³** denote the group -NR^{3.3}-CO-R^{3,4},
wherein **R^{3.3}** is H or a group selected from among C₁₋₆-alkyl, C₂₋₆-alkenyl, C₂₋₆-alkynyl, C₆₋₁₀-aryl; C₆₋₁₀-aryl-C₁₋₆-alkylene, C₅₋₁₀-heteroaryl-C₁₋₆-alkylene, C₃₋₁₀ heterocycle and a C₄₋₁₀- heteroaryl, which may optionally be substituted by one or more groups selected from among OH, OR^{2.1}, oxo, NH₂, NR^{2.2}R^{2.3}, halogen, C₁₋₆-alkyl and C₆₋₁₀-aryl, and
wherein **R^{3.4}** is H or a group selected from among C₁₋₆-alkyl, C₂₋₆-alkenyl, C^{2.6}-alkynyl, C₁₋₆-alkanol, OR^{2.1}, CH₂-O-CO-C₁₋₆-alkyl, CH₂NR^{2.2}R^{2.3}, NR^{2.2}R^{2.3}, C₆₋₁₀-aryl; C₅₋₁₀-aryl-C₁₋₆-alkylene, C₅₋₁₀-heteroaryl-C₁₋₆-alkylene, mono- or bicyclic, saturated or unsaturated C₃₋₁₀ heterocycle with 1, 2 or 3 heteroatoms selected from among O, S and N and a mono- or bicyclic C₅₋₁₀- heteroaryl with 1, 2 or 3 heteroatoms selected from among O, S and N, which may optionally be substituted by one or more groups selected from among OH, OR^{2.1}, oxo, NH₂, NR^{2.2}R^{2.3}, halogen, C₁-₆-alkyl and C₆₋₁₀-aryl,
as well as pharmacologically acceptable salts, diastereomers, enantiomers, racemates, hydrates or solvates thereof.

21. Compounds of formula **1** according to one of claims 1 to 16 and 20, wherein
R³ denotes the group -NR^{3.3}-CO-R^{3.4},
wherein R^{3.3} is H or a group selected from among C₁₋₆-alkyl, phenyl; phenyl-C₁₋₆-alkylene, C₅₋₁₀-heteroaryl-C₁₋₆-alkylene, C₅₋₁₀-heterocycle and a C₅₋₁₀-heteroaryl, which may optionally be substituted by one or more groups selected from among OH, OR^{2.1}, oxo, NH₂, N(CH₃)₂, halogen, C₁₋₆-alkyl and phenyl, and
wherein R^{3.4} is H or a group selected from among C₁₋₆-alkyl, C₁₋₆-alkanol, OR^{2.1}, CH₂-O-CO-C₁₋₆-alkyl, CH₂-NH₂, CH₂-N(CH₃)₂, NH₂, N(CH₃)₂, phenyl; phenyl-C₁₋₆-alkylene, C₅₋₁₀-heteroaryl-C₁₋₆-alkylene, mono- or bicyclic, saturated or unsaturated C₅₋₁₀ heterocycle with 1, 2 or 3 heteroatoms selected from among N; S and O and a mono- or bicyclic C₅₋₁₀-heteroaryl with 1, 2 or 3 heteroatoms selected from among N, S and O, which may optionally be substituted by one or more groups selected from among OH, OR^{2.1}, oxo, NH₂, N(CH₃)₂, halogen, C₁₋₆-alkyl and phenyl,
as well as pharmacologically acceptable salts, diastereomers, enantiomers, racemates, hydrates or solvates thereof.

22. Compounds No. 1 to 123 of formula 1 according to one of claims 1 to 4 or 6 to 21, wherein
X denotes SO and
wherein R¹ is H and
wherein R² and R³ are combined like in compounds No. 1-123 in the following table
| compound-No. | R² | R³ |
|---|---|---|
| 1. | 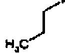 | 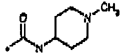 |
| 2. | 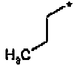 | 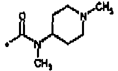 |
| 3. | 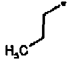 | 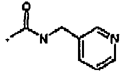 |
| 4. | 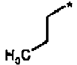 | 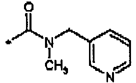 |
| 5. | 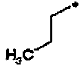 | 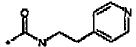 |
| 6. | 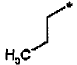 | 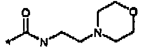 |
| 7. | 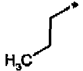 | 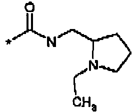 |
| 8. | 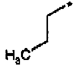 | 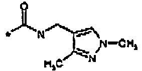 |
| 9. | 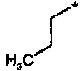 | 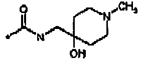 |
| 10. | 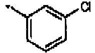 | 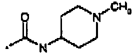 |
| 11. | 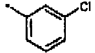 | 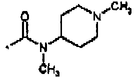 |
| 12. | 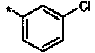 | 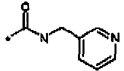 |
| 13. | 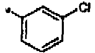 | 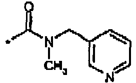 |
| 14. | 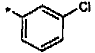 | 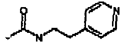 |
| 15. | 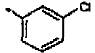 | 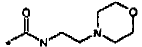 |
| 16. | 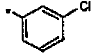 | 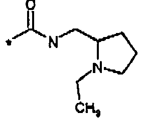 |
| 17. | 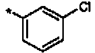 | 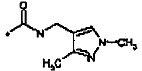 |
| 18. | 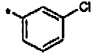 | 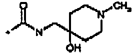 |
| 19. | 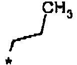 | 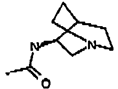 |
| 20. | 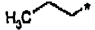 | Cl |
| 21. | 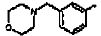 | Cl |
| 22. | 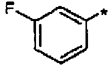 | Cl |
| 23. | 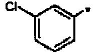 | Cl |
| 24. | 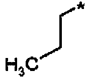 | 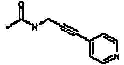 |
| 25. | 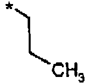 | 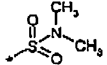 |
| 26. | 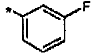 | 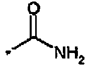 |
| 27. | 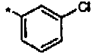 | 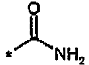 |
| 28. | 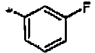 | 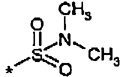 |
| 29. | 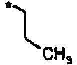 | 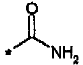 |
| 30. | 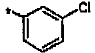 | 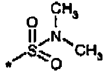 |
| 31. | 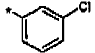 | 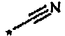 |
| 32. | 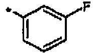 | 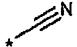 |
| 33. | 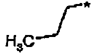 | 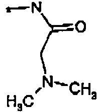 |
| 34. | 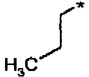 | 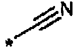 |
| 35. | 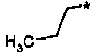 | 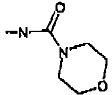 |
| 36. | 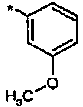 | Cl |
| 37. | 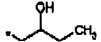 | Cl |
| 38. | 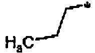 | 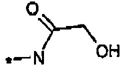 |
| 39. | 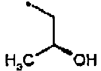 | Cl |
| 40. | 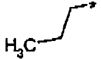 | 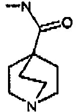 |
| 41. | 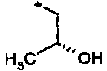 | Cl |
| 42. | 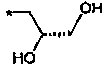 | Cl |
| 43. | 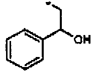 | Cl |
| 44. | 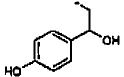 | Cl |
| 45. | 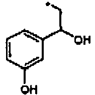 | Cl |
| 46. | 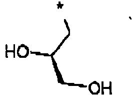 | Cl |
| 47. | 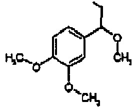 | Cl |
| 48. | 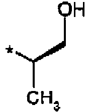 | Cl |
| 49. | 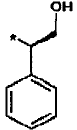 | Cl |
| 50. | 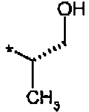 | Cl |
| 51. | 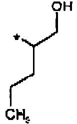 | Cl |
| 52. | 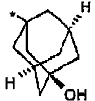 | Cl |
| 53. | 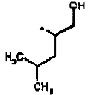 | Cl |
| 54. | 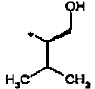 | Cl |
| 55. | 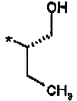 | Cl |
| 56. | 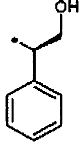 | Cl |
| 57. | 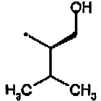 | Cl |
| 58. | 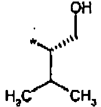 | Cl |
| 59. | 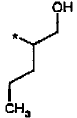 | Cl |
| 60. | 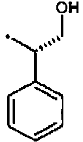 | Cl |
| 61. | 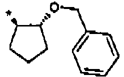 | Cl |
| 62. | 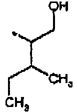 | Cl |
| 63. | 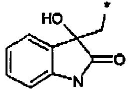 | Cl |
| 64. | 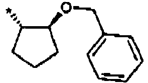 | Cl |
| 65. | 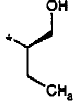 | Cl |
| 66. | 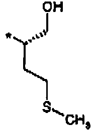 | Cl |
| 67. | 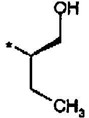 | Cl |
| 68. | 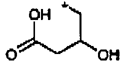 | Cl |
| 69. | 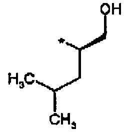 | Cl |
| 70. | 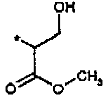 | Cl |
| 71. | 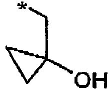 | Cl |
| 72. | 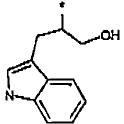 | Cl |
| 73. | 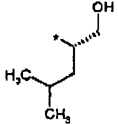 | Cl |
| 74. | 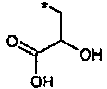 | Cl |
| 75. | 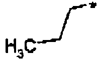 | 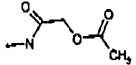 |
| 76. | 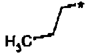 | 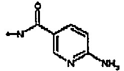 |
| 77. | 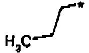 | 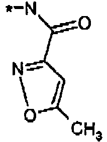 |
| 78. | 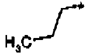 | 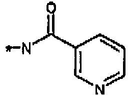 |
| 79. | 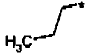 | 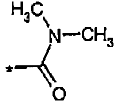 |
| 80. | 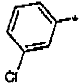 | 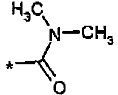 |
| 81. | 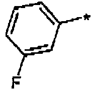 | 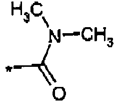 |
| 82. | 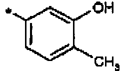 | Cl |
| 83. | 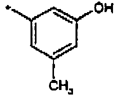 | Cl |
| 84. | 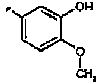 | Cl |
| 85. | 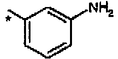 | Cl |
| 86. | 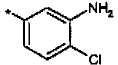 | Cl |
| 87. | 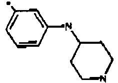 | Cl |
| 88. | 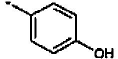 | Cl |
| 89. | 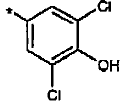 | Cl |
| 90. | 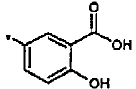 | Cl |
| 91. | 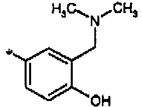 | Cl |
| 92. | 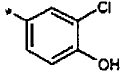 | Cl |
| 93. | 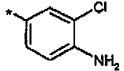 | Cl |
| 94. | 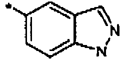 | Cl |
| 95. | 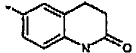 | Cl |
| 96. | 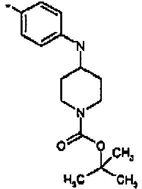 | Cl |
| 97. | 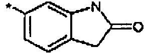 | Cl |
| 98. | 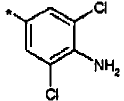 | Cl |
| 99. | 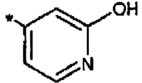 | Cl |
| 100. | 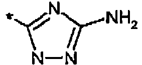 | Cl |
| 101. | 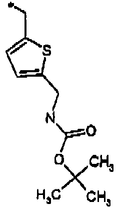 | Cl |
| 102. | 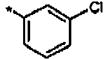 | 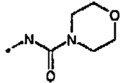 |
| 103. | 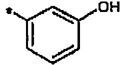 | Cl |
| 104. | 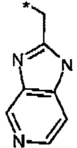 | Cl |
| 105. | 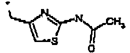 | Cl |
| 106. | 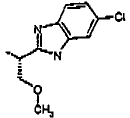 | Cl |
| 107. | 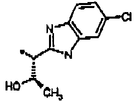 | Cl |
| 108. | 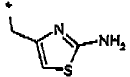 | Cl |
| 109. | 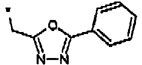 | Cl |
| 110. | 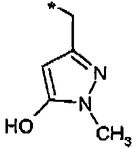 | Cl |
| 111. | 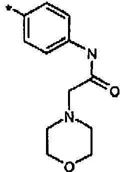 | Cl |
| 112. | 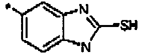 | Cl |
| 113. | 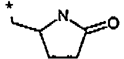 | Cl |
| 114. | 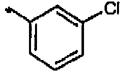 | 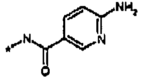 |
| 115. | 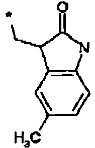 | Cl |
| 116. | 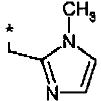 | Cl |
| 117. | 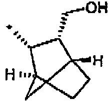 | Cl |
| 118. | 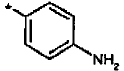 | Cl |
| 119. | 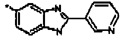 | Cl |
| 120. | 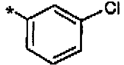 | 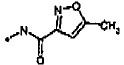 |
| 121. | 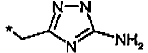 | Cl |
| 122. | 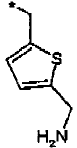 | Cl |
| 123. | 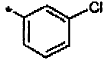 | 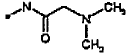 |

23. | compound-No. | R² | R³ |
|---|---|---|
| 131 | 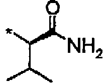 | Cl |
| 132 | 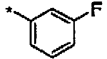 | 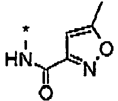 |
| 133 | 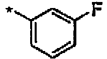 | 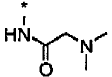 |
| 134 | 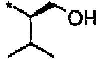 | 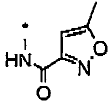 |
| 135 | 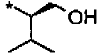 | 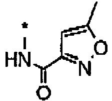 |
| 136 | 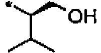 | 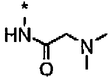 |
| 137 | 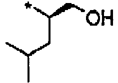 | 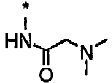 |
| 138 | 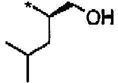 | 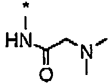 |
| 139 | 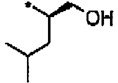 | 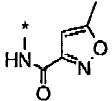 |
| 140 | 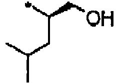 | 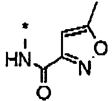 |
| 141 | 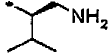 | Cl |
| 142 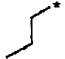 | 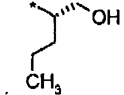 | Cl |
| 143 | 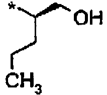 | Cl |
| 144 | 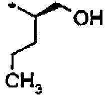 | Cl |
| 145 | 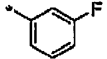 | 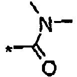 |
| 146 | 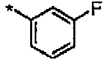 | 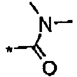 |
| 147 | 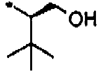 | Cl |
| 148 | 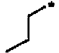 | CN |
| 149 | 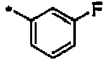 | 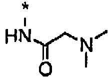 |
| 150 | 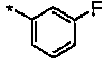 | 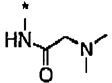 |
| 151 | 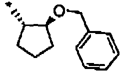 | Cl |
| 152 | 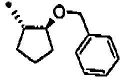 | Cl |
| 153 | 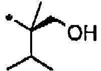 | Cl |
| 154 | 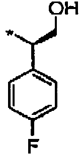 | Cl |
| 155 | 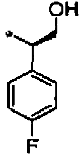 | Cl |
| 156 | 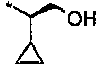 | Cl |
| 157 | 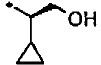 | Cl |
| 158 | 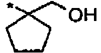 | Cl |
| 159 | 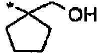 | Cl |
| 160 | 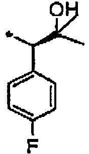 | Cl |
| 161 | 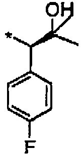 | Cl |
| 162 | 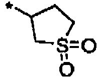 | Cl |
| 163 | 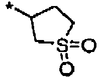 | Cl |
| 164 | 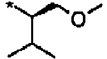 | Cl |
| 165 | 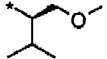 | Cl |
| 166 | 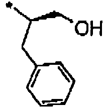 | Cl |
| 167 | 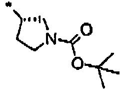 | Cl |
| 168 | 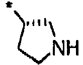 | Cl |
| 169 | 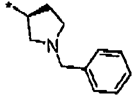 | Cl |
| 170 | 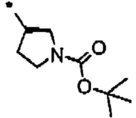 | Cl |
| 171 | 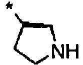 | Cl |
| 172 | 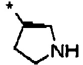 | Cl |
| 173 | 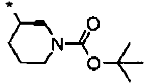 | Cl |
| 174 | 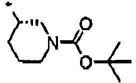 | Cl |
| 175 | 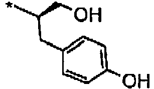 | Cl |
| 176 | 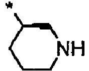 | Cl |
| 177 | 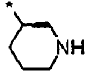 | Cl |
| 178 | 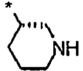 | Cl |
| 179 | 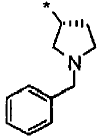 | Cl |
| 180 | 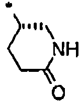 | Cl |
| 181 | 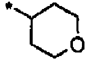 | 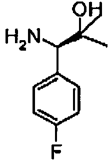 |
| 182 | 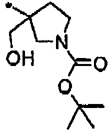 | Cl |
| 183 | 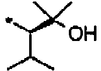 | Cl |
| 184 | 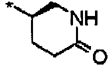 | Cl |
| 185 | 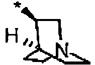 | Cl |
| 186 | 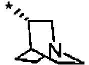 | Cl |
| 187 | 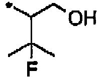 | Cl |
| 188 | 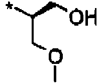 | Cl |
| 189 | 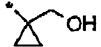 | Cl |
| 190 | 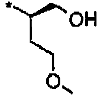 | Cl |
| 191 | 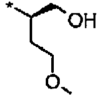 | Cl |
| 192 | 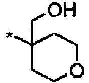 | Cl |
| 193 | 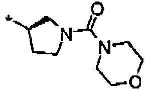 | Cl |
| 194 | 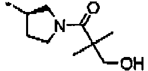 | Cl |
| 195 | 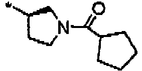 | Cl |
| 196 | 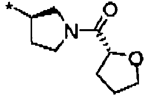 | Cl |
| 197 | 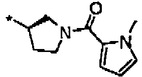 | Cl |
| 198 | 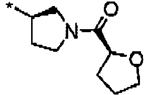 | Cl |
| 199 | 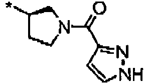 | Cl |
| 200 | 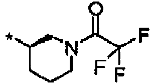 | Cl |
| 201 | 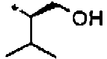 | OH |
| 202 | 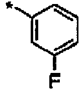 | 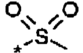 |
| 203 | 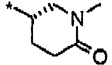 | Cl |
| 204 | 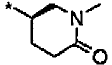 | Cl |
| 205 | 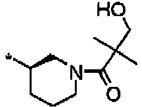 | Cl |
| 206 | 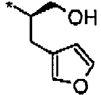 | Cl |
| 207 | 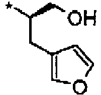 | Cl |
| 208 | 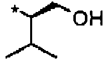 | Br |
| 209 | 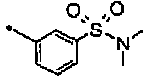 | Cl |
| 210 | 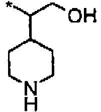 | Cl |
| 211 | 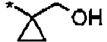 | Br |
| 212 | 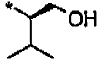 | 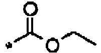 |
| 213 | 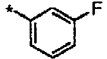 | 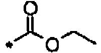 |
| 214 | 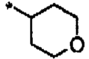 | 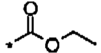 |
| 215 | 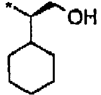 | Cl |
| 216 | 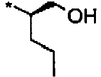 | Br |
| 217 | 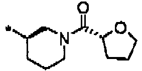 | Cl |

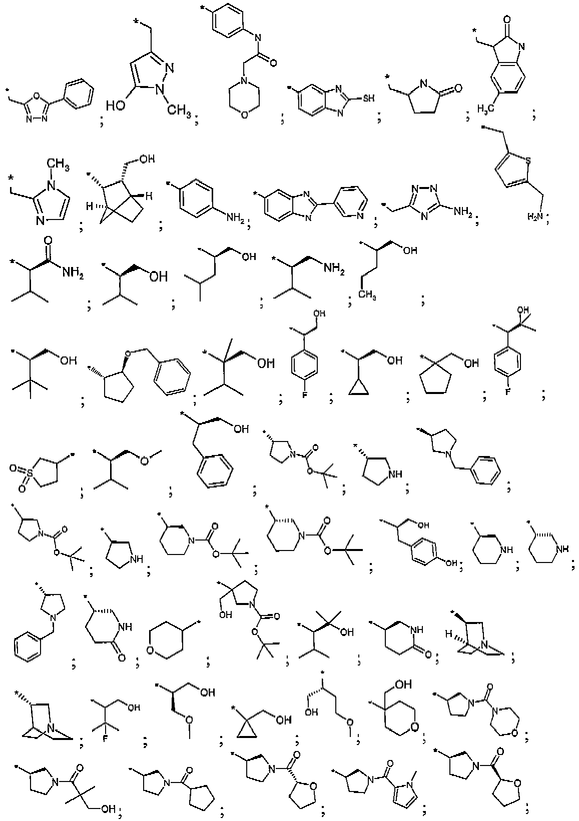
as well as pharmacologically acceptable salts, diastereomers, enantiomers, racemates, hydrates or solvates thereof.

24. Compounds of formula 1 according to one of claims 1 to 3 or 5, wherein X denotes SO and wherein NR¹R² is selected from among 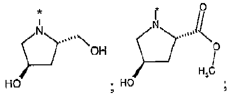 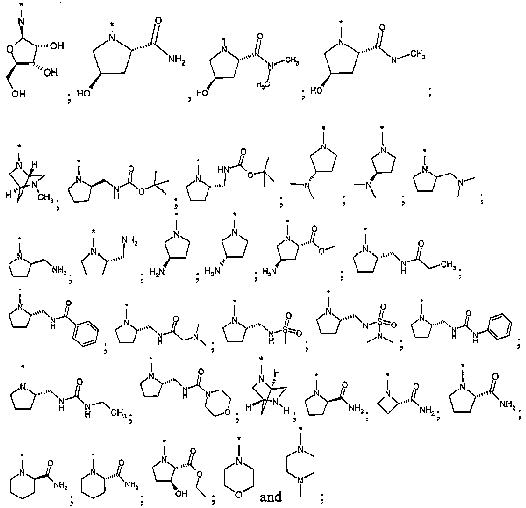 and wherein
R³ is Cl,
as well as pharmacologically acceptable salts, diastereomers, enantiomers, racemates, hydrates or solvates thereof.

25. Compounds
of formula 4 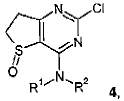 of formula 5 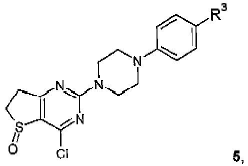 of formula 6 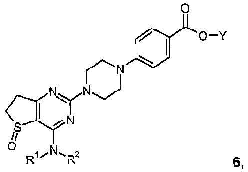 of formula 7 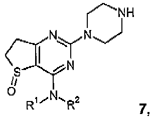 of formula 8 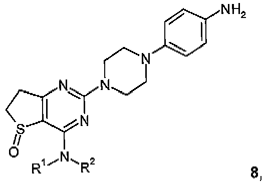 wherein R¹, R² and R³ are defined according to one of claims 1 to 23, and wherein Y denotes H, methyl or ethyl,
as well as pharmacologically acceptable salts, diastereomers, enantiomers, racemates, hydrates or solvates thereof.

26. Compounds according to one of claims 1 to 23 as medicaments.

27. Use of compounds according to one of claims 1 to 23, for preparing a medicament for the treatment of respiratory or gastrointestinal complaints or diseases, and also inflammatory diseases of the joints, skin or eyes, cancers, as well as diseases of the peripheral or central nervous system.

28. Use of compounds according to one of claims 1 to 23 for preparing a medicament for the prevention and treatment of respiratory or pulmonary diseases which are accompanied by increased mucus production, inflammation and/or obstructive diseases of the airways.

29. Use of compounds according to one of claims 1 to 23 for preparing a medicament for the treatment of inflammatory and obstructive diseases such as COPD, chronic sinusitis, asthma, Crohn's disease, ulcerative colitis and inflammatory diseases of the gastrointestinal tract.

30. Use of compounds according to one of claims 1 to 23 for preparing a medicament for the prevention and treatment of diseases of the peripheral or central nervous system such as depression, bipolar or manic depression, acute and chronic anxiety states, schizophrenia, Alzheimer's disease, Parkinson's disease, acute and chronic multiple sclerosis or acute and chronic pain as well as injuries to the brain caused by stroke, hypoxia or craniocerebral trauma.

## Revendications

1. Composés de formule 1, 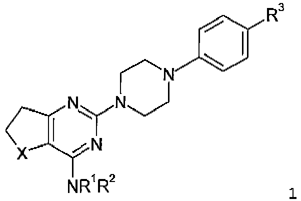 dans laquelle
X est SO ;
R¹ est H, alkyle en C₁ à C₆, alcényle en C₂ à C₆, alcynyle en C₂ à C₆, aryle en C₆ à C₁₀, (aryle en C₆ à C₁₀) alkylène en C₁ à C₆ ou (hétéroaryle en C₅ à C₁₀)-alkylène en C₁ à C₆ ;
R² est H ou bien un reste choisi dans le groupe constitué par alkyle en C₁ à C₁₀, alcényle en C₂ à C₆ et alcynyle en C₂ à C₆, qui peut le cas échéant être substitué par un halogène et qui peut le cas échéant être substitué par un ou plusieurs restes choisis dans le groupe constitué par OR^{2.1}, COOR^{2.1}, CONR^{2.2}R^{2.2}, SR^{2.1}, aryle en C₆ à C₁₀, hétérocycle en C₃ à C₁₀ mono- ou bicyclique, hétéroaryle en C₅ à C₁₀ mono- ou bicyclique, cycloalkyl, en C₃ à C₁₀ mono- ou bicyclique, CH₂-NR^{2.2}R^{2.3} et NR^{2.2}R^{2.3}, qui peut le cas échéant être substitué lui aussi par un ou plusieurs restes choisis dans le groupe constitué par OH, halogène, OR^{2.1}, oxo, alkyle en C₁ à C₆, aryle en C₆ à C₁₀, COOR^{2.1}, CH₂-NR^{2.2}R^{2.3} et NR^{2.2}R^{2.3},
où R^{2.1} est H ou un reste choisi dans le groupe constitué par alkyle en C₁ à C₆, alcanol en C₁ à C₆, haloalkyle en C₁ à C₃, cycloalkyle en C₃ à C₁₀ mono- ou bicyclique, (aryle en C₆ à C₁₀) alkylène en C₁ à C₆, (hétéroaryle en C₅ à C₁₀ mono- ou bicyclique)alkylène en C₁ à C₆, (hétérocycle en C₃ à C₁₀)alkylène en C₁ à C₆, (cycloalkyle en C₃ à C₁₀)alkylène en C₁ à C₆, aryle en C₆ à C₁₀ mono- ou bicyclique, hétéroaryle en C₅ à C₁₀ mono-ou bicyclique et hétérocycle mono- ou bicyclique, saturé ou insaturé, qui peut le cas échéant être substitué par un ou plusieurs restes choisis dans le groupe constitué par OH, halogène, alkyle en C₁ à C₆ et aryle en C₆ à C₁₀,
où R^{2.2} et R^{2.3} sont indépendamment l'un de l'autre H ou un reste choisi dans le groupe constitué par halogène, alkyle en C₁ à C₆, cycloalkyle en C₃ à C₁₀ mono- ou bicyclique, (aryle en C₆ à C₁₀)alkylène en C₁ à C₆, (hétéroaryle en C₅ à C₁₀)alkylène en C₁ à C₆, aryle en C₆ à C₁₀ mono- ou bicyclique, hétérocycle en C₃ à C₁₀ mono- ou bicyclique, hétéroaryle en C₅ à C₁₀ mono- ou bicyclique, CO-NH₂, CO-NHCH₃, CO-N(CH₃)₂, SO₂-alkyle en C₁-C₂, CO-R^{2.1} et COOR^{2.1}, qui peut le cas échéant être substitué par un ou plusieurs restes choisis dans le groupe constitué par OH, halogène, alkyle en C₁ à C₆, aryle en C₆ à C₁₀ et COOR^{2.1},
ou
R² est un cycloalkyle en C₃ à C₁₀ mono- ou polycyclique qui peut le cas échéant être ponté par un ou plusieurs groupes alkyle en C₁ à C₃ et qui peut le cas échéant être substitué par un reste choisi dans le groupe constitué par alcanol en C₁ à C₆ ramifié ou non ramifié, OR^{2.1}, COOR^{2.1}, SO₂NR^{2.2}R^{2.3}, hétérocycle en C₃ à C₁₀, aryle en C₆ à C₁₀, alkyle en C₁ à C₆, (aryle en C₆ à C₁₀)alkylène en C₁, à C₆, (hétéroaryle en C₅ à C₁₀)-alkylène en C₁ à C₆, cycloalkyle en C₃ à C₁₀ mono- ou bicyclique et NR^{2.2}R^{2.3}, qui peut le cas échéant être substitué par un ou plusieurs restes choisis dans le groupe constitué par OH, OR^{2.1}, oxo, halogène, alkyle en C₁ à C₆, aryle en C₆ à C₁₀ et NR^{2.2}R^{2.3},
ou
R² est un aryle en C₆ à C₁₀ mono- ou polycyclique, qui peut le cas échéant être substitué par OH, SH ou un halogène ou par un ou plusieurs restes choisis dans le groupe constitué par OR^{2.1}, COOR^{2.1}, NR^{2.2}R^{2.3}, CH₂-NR^{2.2}R^{2.3}, cycloalkyle en C₃ à C₁₀, hétérocycle en C₃ à C₁₀, alkyle en C₁ à C₆, (aryle en C₆ à C₁₀)alkylène en C₁ à C₆, (hétérocycle en C₃ à C₁₀) alkylène en C₁ à C₆, (hétéroaryle en C₅ à C₁₀)alkylène en C₁ à C₆, aryle en C₆ à C₁₀, SO₂-CH₃, SO₂-CH₂CH₃ et SO₂-NR^{2.2}R^{2.3}, qui peut le cas échéant être substitué lui aussi par un ou plusieurs restes choisis dans le groupe constitué par OH, OR^{2.1}, oxo, halogène, alkyle en C₁ à C₆, aryle en C₆ à C₁₀ et NR^{2.2}R^{2.3},
ou
R² est un reste choisi dans un groupe constitué par un hétérocycle en C₃ à C₁₀ mono- ou bicyclique, saturé ou insaturé, et un hétéroaryle en C₅ à C₁₀ mono-ou bicyclique contenant de 1 à 4 hétéxoatomes choisis parmi S, 0 et N et peut le cas échéant être substitué par un ou plusieurs restes choisis dans le groupe constitué par halogène, OH, oxo et SH ou par un ou plusieurs restes choisis dans le groupe constitué par OR^{2.1}, SR^{2.1}, COOR^{2.1}, COR^{2.1}, alcanol en C₁ à C₆, cycloalkyle en C₃ à C₁₀, aryle en C₆ à C₁₀, alkyle en C₁ à C₆, (aryle en C₆ à C₁₀)alkylène en C₁ à C₆, (hétéroaryle en C₅ à C₁₀) alkylène en C₂ à C₆, hétérocycle en C₅ à C₁₀, hétéroaryle en C₅ à C₁₀, alcanol en C₁ à C₆ et NR^{2.2}R^{2.3}, qui peut le cas échéant être substitué lui aussi par un ou plusieurs restes choisis dans le groupe constitué par OH, OR^{2.1}, oxo, halogène, alkyle en C₂ à C₆, aryle en C₆ à C₁₀ et NR^{2.2}R^{2.3},
ou
NR¹R² dans son ensemble représente un noyau hétérocyclique en C₄ à C₇ qui peut le cas échéant être ponté, qui contient 1, 2 ou 3 hétéroatomes choisis dans le groupe constitué par N, O et S, et qui peut le cas échéant être substitué par un ou plusieurs restes choisis dans le groupe constitué par OH, OR^{2.1}, alcanol en C₁ à C₆, oxo, halogène, alkyle en C₁ à C₆, aryle en C₆ à C₁₀, COOR^{2.1}, CH₂-NR^{2.2}-COO-R^{2.1}, CH₂-NR^{2.2}-CO-R^{2.1}, CH₂-NR^{2.2}-CO-CH₂,-NR^{2.2}R^{2.3}, CH₂-NR^{2.2}-SO₂-alkyle en C₁ à C₃, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} et NR^{2.2}R^{2.3},
et dans laquelle
R³ est choisi dans le groupe constitué par un un atome de fluor, chlore, brome, iode, un groupe hydroxy, SO₂-CH₃, COOR^{2.1}, nitrile et (hétérocycle en C₃ à C₁₀)-alkylène en C₁ à C₆, l'hétérocycle en C₃ à C₁₀ pouvant être mono- ou bicyclique et le cas échéant substitué par un reste choisi dans le groupe constitué par OH, halogène, oxo, alkyle en C₁ à C₆ et aryle en C₆ à C₁₀,
ou
un reste choisi dans le groupe constitué par alkyle en C₁ à C₆, alcényle en C₂ à C₆, alcynyle en C₂ à C₆, aryle en C₆ à C₁₀, (aryle en C₆ à C₁₀)alkylène en C₁ à C₆, (hétéroaryle en C₅ à C₁₀)alkylène en C₁ à C₆, hétérocycle en C₃ à C₁₀ et cycloalkyle en C₃ à C₁₀, qui peut le cas échéant être substitué par un reste choisi dans le groupe constitué par OH, halogène, oxo, alkyle en C₁ à C₆ et aryle en C₆ à C₁₀,
ou bien
R³ représente le groupe -CO-NR^{3.1}R^{3.2},
où R^{3.1} et R^{3.2} sont indépendamment l'un de l'autre H ou des restes choisis dans le groupe constitué par alkyle en C₁ à C₆, alcényle en C₂ à C₆, alcynyle en C₂ à C₆, aryle en C₆ à C₁₀, (aryle en C₆ à C₁₀)alkylène en C₁ à C₆, (hétéroaryle en C₅ à C₁₀)alkylène en C₁ à C₆, (hétéroaryle en C₅ à C₁₀)alcynylène en C₁ à C₆, (hétéroaryle en C₅ à C₁₀)alcénylène en C₁ à C₆, hétérocycle en C₃ à C₁₀ mono- ou bicyclique, (hétérocycle en C₃ à C₁₀)alkylène en C₁ à C₆ et hétéroaryle en C₅ à C₁₀ mono- ou bicyclique, chaque reste pouvant le cas échéant être substitué par un ou plusieurs restes choisis dans le groupe constitué par OH, oxo, halogène, alkyle en C₁ à C₆ et O-alkyle en C₁ à C₆,
ou bien
R³ représente le groupe -NR^{3.3}-CO-R^{3.4},
où R^{3.3} est H ou un reste choisi dans le groupe constitué par alkyle en C₁ à C₆, alcényle en C₂ à C₆, alcynyle en C₂ à C₆, aryle en C₆ à C₁₀, (aryle en C₆ à C₁₀)alkylène en C₁ à C₆, (hétéroaryle en C₅ à C₁₀)-alkylène en C₁ à C₆, hétérocycle en C₃ à C₁₀ et hétéroaryle en C₅ à C₁₀, qui peut le cas échéant être substitué par un ou plusieurs restes choisis dans le groupe constitué par OH, OR^{2.1}, oxo, NH₂, NR^{2.2}R^{2.3}, halogène, alkyle en C₁ à C₆ et aryle en C₆ à C₁₀, et
R^{3.4} est H ou un reste choisi dans le groupe constitué par alkyle en C₁ à C₆, alcényle en C₂ à C₆, alcynyle en C₂ à C₆, alcanol en C₁ à C₆, OR^{2.1}, CH₂-O-CO-alkyle en C₁ à C₆, CH²-NR^{2.2}R^{2.3}, NR^{2.2}R^{2.3}, aryle en C₆ à C₁₀, (aryle en C₆ à C₁₀)alkylène en C₁ à C₆, (hétéroaryle en C₅ à C₁₀)alkylène en C₁ à C₆, hétérocycle en C₃ à C₁₀ mono- ou bicyclique, saturé, partiellement saturé ou insaturé, contenant 1, 2 ou 3 hétéroatomes du groupe S, O et N, et hétéroaryle en C₅ à C₁₀ mono- ou bicyclique contenant 1, 2 ou 3 hétéroatomes du groupe S, 0 et N, qui peut le cas échéant être substitué par un ou plusieurs restes choisis dans le groupe constitué par OH, OR^{2.1} oxo, NH₂, NR^{2.2}R^{2.3}, halogène, alkyle en C₁ à C₆ et aryle en C₆ à C₁₀,
ou bien
R³ représente un groupe sulfonamide SO₂-NR^{3.5}R^{3.6} le cas échéant mono- ou di-N-substitué,
où R^{3.5} et R^{3.6} peuvent être chacun indépendamment l'un de l'autre alkyle en C₁ à C₆ ou aryle en C₆ à C₁₀,
ainsi que leurs sels, diastéréoisomères, énantiomères, racémates, hydrates ou solvates compatibles au plan pharmacologique.

2. Composés de formule 1 selon la revendication 1, dans laquelle
X est SO ;
R¹ est H, alkyle en C₁ à C₆, aryle en C₆ à C₁₀.
(aryle en C₆ à C₁₀)alkylène en C₁ à C₆ ou (hétéroaryle en C₅ à C₁₀)alkylène en C₁ à C₆ ;
R² est H ou un alkyle en C₁ à C₆ qui peut le cas échéant être substitué par un halogène et qui peut le cas échéant être substitué par un ou plusieurs restes choisis dans le groupe constitué par OR^{2.1}, COOR^{2.1}, CONR^{2.2}R^{2.3}, SR^{2.1}, aryle en C₆ à C₁₀, hétérocycle en C₃ à C₁₀ mono- ou bicyclique, hétéroaryle en C₅ à C₁₀ mono-ou bicyclique, cycloalkyle en C₃ à C₁₀ mono- ou bicyclique, CH₂-NR^{2.2}R^{2.3} et NR^{2.2}R^{2.3}, qui peut le cas échéant être substitué lui aussi par un ou plusieurs restes choisis dans le groupe constitué par OH, halogène, OR^{2.1}, oxo, alkyle en C₁ à C₆, aryle en C₆ à C₁₀, COOR^{2.1}, CH₂-NR^{2.2}R^{2.3} et NR^{2.2}R^{2.3},
où R^{2.1} est H ou un reste choisi dans le groupe constitué par alkyle en C₁ à C₆, alcanol en Ci à C₆, haloalkyle en C₁ à C₃, cycloalkyle en C₃ à C₁₀ mono- ou bicyclique, (aryle en C₆ à C₁₀)alkylène en C₁ à C₆ ou (hétéroaryle en C₅ à C₁₀)alkylène en C₁ à C₆, (hétérocycle en C₃ à C₁₀)alkylène en C₁ à C₆, (cycloalkyle en C₃ à C₁₀)alkylène en C₁ à C₆, aryle en C₆ à C₁₀ mono- ou bicyclique, hétéroaryle en C₅ à C₁₀ mono-ou bicyclique et hétérocycle en C₃ à C₁₀ mono- ou bicyclique, saturé ou insaturé, qui peut le cas échéant être substitué par un ou plusieurs restes choisis dans le groupe constitué par OH, halogène, alkyle en C₁ à C₆, aryle en C₆ à C₁₀,
où R^{2.2} et R^{2.3} sont indépendamment l'un de l'autre H ou choisis dans le groupe constitué par halogène, alkyle en C₁ à C₆, cycloalkyle en C₃ à C₁₀ mono- ou bicyclique, (aryle en C₆ à C₁₀)alkylène en C₁ à C₆, (hétéroaryle en C₅ à C₁₀)alkylène en C₁ à C₆, aryle en C₆ à C₁₀ mono- ou bicyclique, hétérocycle en C₃ à C₁₀ mono-ou bicyclique, saturé ou insaturé, hétéroaryle en C₅ à C₁₀ mono- ou bicyclique, CO-NH₂, CO-NHCH₃, CO-N(CH₃)₂, SO₂-alkyle en C₁-C₂, CO-R^{2.1} et COOR^{2.1}, qui peut le cas échéant, être substitué par un ou plusieurs restes choisis dans le groupe constitué par OH, halogène, alkyle en C₁ à C₆, aryle en C₆ à C₁₀ et COOR^{2.1},
ou
R² est un cycloalkyle en C₃ à C₁₀ mono- ou polycyclique qui peut le cas échéant être ponté par un ou par plusieurs groupes alkyle en C₁ à C₃ et qui peut le cas échéant être mono- ou polysubstitué par OH ou un halogène ou par un ou plusieurs restes choisis dans le groupe constitué par alcanol en C₁ à C₆ ramifié ou non ramifié, OR^{2.1}, COOR^{2.1}, SO₂NR^{2.2}R^{2.3}, hétérocycle en C₃ à C₁₀, aryle en C₆ à C₁₀, alkyle en C₁ à C₆, (aryle en C₆ à C₁₀)alkylène en C₁ à C₆, (hétéroaryle en C₅ à C₁₀)-alkylène en C₁ à C₆, cycloalkyle en C₃ à C₁₀ mono- ou bicyclique, et NR^{2.2}R^{2.3}, qui peut le cas échéant être substitué par un ou plusieurs restes choisis dans le groupe constitué par OH, OR^{2.1}, oxo, halogène, alkyle en C₁ à C₆, aryle en C₆ à C₁₀ et NR^{2.2}R^{2.3},
ou
R² est un aryle en C₆ à C₁₀ mono- ou polycyclique qui peut le cas échéant être substitué par OH, SH ou un halogène ou par un ou plusieurs restes choisis dans le groupe constitué par OR²⁻¹, COOR^{2.1}, NR^{2.2}R^{2.3}, CH₂-NR^{2.2}R^{2.3}, cycloalkyle en C₃ à C₁₀, hétérocycle en C₃ à C₁₀, alkyle en C₁ à C₆, (aryle en C₆ à C₁₀)alkylène en C₁ à C₆, (hétérocycle en C₃ à C₁₀)alkylène en C₁ à C₆, (hétéro-aryle en C₅ à C₁₀)alkylène en C₁ à C₆, aryle en C₆ à C₁₀, SO₂-CH₃, SO₂-CH₂CH₃ et SO₂-NR^{2.2}R^{2.3}, qui peut le cas échéant être substitué lui aussi par un ou plusieurs restes choisis dans le groupe constitué par OH, OR^{2.1}, oxo, halogène, alkyle en C₁ à C₆, aryle en C₆ à C₁₀ et NR^{2.2}R^{2.3},
ou
R² est un reste choisi dans un groupe constitué par un hétérocycle en C₃ à C₁₀ mono- ou bicyclique, saturé ou insaturé, et un hétéroaryle en C₅ à C₁₀ mono-ou bicyclique contenant de 1 à 4 hétéroatomes choisis parmi S, O et N et peut le cas échéant être substitué par un ou plusieurs restes du groupe halogène, OH, oxo et SH ou par un ou plusieurs restes choisis dans le groupe constitué par OR^{2.1}, SR^{2.1}, COOR^{2.1}, COR^{2.1}, alcanol en C₁ à C₆, cycloalkyle en C₃ à C₁₀, aryle en C₆ à C₁₀, alkyle en C₁ à C₆, (aryle en C₆ à C₁₀)alkylène en C₁ à C₆, (hétéroaryle en C₅ à C₁₀)alkylène en C₁ à C₆, hétérocycle en C₅ à C₁₀, hétéroaryle en C₅ à C₁₀, alcanol en C₁ à C₆ et NR²²R^{2.3}, qui peut le cas échéant être substitué lui aussi par un ou plusieurs restes choisis dans le groupe constitué par OH, OR^{2.1}, oxo, halogène, alkyle en C₁ à C₆, aryle en C₆ à C₁₀ et NR^{2.2}R^{2.3},
ou NR¹R² dans son ensemble représente un noyau hétérocyclique en C₄ à C₇ qui peut le cas échéant être ponté, qui contient 1, 2 ou 3 hétéroatomes choisis dans le groupe constitué par N, O et S, et qui peut le cas échéant être substitué par un ou plusieurs restes choisis dans le groupe constitué par OH, OR^{2.1}, alcanol en C₁ à C₆, oxo, halogène, alkyle en C₁ à C₆, aryle en C₆ à C₁₀, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-alkyle en C₁ à C₃, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} et NR^{2.2}R^{2.3},
ainsi que leurs sels, diastéréoisomères, énantiomères, racémates, hydrates ou solvates compatibles au plan pharmacologique.

3. Composés de formule 1 selon la revendication 1 et la revendication 2, dans laquelle
X est SO ;
R¹ est H, alkyle en C₁ à C₆, aryle en C₆ à C₁₀, (aryle en C₆ à C₁₀)alkylène en C₁ à C₆ ou (hétéroaryle en C₅ à C₁₀)alkylène en C₁ à C₆ ;
R² est H ou un alkyle en C₁ à C₆ qui peut le cas échéant être substitué par un halogène et qui peut le cas échéant être substitué par un ou plusieurs restes choisis dans le groupe constitué par OR^{2.1}, COOR^{2.1}, CONR^{2.2}R^{2.3}, SR^{2.1,} phényle, hétérocycle en C₅ à C₁₀ mono-ou bicyclique, hétéroaryle en C₅-C₆, cycloalkyle en C₅ à C₁₀ mono- ou bicyclique, CH₂-NR^{2.2}R^{2.3} et NR^{2.2}R^{2.3}, qui peut le cas échéant être substitué lui aussi par un ou plusieurs restes choisis dans le groupe constitué par OH, halogène, OR^{2.1} oxo, alkyle en C₁ à C₆, phényle, COOR^{2.1}, CH₂-NR^{2.2}R^{2.3} et NR^{2.2}R^{2.3},
où R^{2.1} est H ou un reste choisi dans le groupe constitué par alkyle en C₁ à C₆, alcanol en C₁ à C₆, haloalkyle en C₁ à C₃, cycloalkyle en C₅ à C₁₀ mono- ou bicyclique, phénylalkylène en C₁ à C₆, (hétéroaryle en C₅-C₆)alkylène en C₁ à C₆, (hétérocycle en C₅ à C₁₀)-alkylène en C₁ à C₆, (cycloalkyle en C₅ à C₁₀)alkylène en C₁ à C₆, phényle, hétéroaryle en C₅ à C₁₀ mono- ou bicyclique et hétérocycle en C₅ à C₁₀ mono- ou bicyclique, saturé ou insaturé, qui peut le cas échéant être substitué par un ou plusieurs restes choisis dans le groupe constitué par OH, halogène, alkyle en C₁ à C₆ et phényle,
où R^{2.2} et R^{2.3} sont indépendamment l'un de l'autre H ou un reste choisi dans le groupe constitué par halogène, alkyle en C₁ à C₆, cycloalkyle en C₅ à C₁₀ mono- ou bicyclique, phénylalkylène en C₁ à C₆, (hétéroaryle en C₅ à C₁₀)alkylène en C₁ à C₆, phényle, hétérocycle en C₅ à C₁₀ mono- ou bicyclique, hétéroaryle en C₅-C₈ mono- ou bicyclique, CO-NH₂, CO-NHCH₃, CON(CH₃)₂, SO₂-alkyle en C₁-C₂, CO-R^{2.1} et COOR^{2.1}, qui peut le cas échéant être substitué par un ou plusieurs restes choisis dans le groupe constitué par OH, halogène, alkyle en C₁ à C₆, phényle et COOR^{2.1},
ou
R² est un cycloalkyle en C₅ à C₁₀ mono- ou polycyclique qui peut le cas échéant être ponté par un ou plusieurs groupes alkyle en C₁ à C₃ et qui peut le cas échéant être mono- ou polysubstitué par OH ou un halogène ou par un ou plusieurs restes choisis dans le groupe constitué par alcanol en C₁ à C₃ ramifié ou non ramifié, OR^{2.1}, COOR^{2.1}, SO₂NR^{2.2}R^{2.3} hétérocycle en C₅ à C₁₀, phényle, alkyle en C₁ à C₆, phénylalkylène en C₁ à C₆, (hétéroaryle en C₅-C₆)alkylène en C₁ à C₆, cycloalkyle en C₅ à C₁₀ mono- ou bicyclique et NR^{2.2}R^{2.3}, qui peut le cas échéant être substitué par un ou plusieurs restes choisis dans le groupe constitué par OH, OR^{2.1}, oxo, halogène, alkyle en C₁ à C₆, phényle et NR^{2.2}R^{2.3},
ou
R² est un phényle qui peut le cas échéant être substitué par OH, SH ou un halogène ou par un ou plusieurs restes choisis dans le groupe constitué par OR^{2.1}, COOR^{2.1}, NR^{2.2}R^{2.3}, CH₂-NR^{2.2}R^{2.3}, cycloalkyle en C₅ à C₁₀, hétérocycle en C₅ à C₁₀, alkyle en C₁ à C₆, phénylalkylène en C₁ à C₆, (hétérocycle en C₅ à C₁₀)alkylène en C₁ à C₆, (hétéroaryle en C₅-C₆)alkylène en C₁ à C₆, phényle, SO₂-CH₃, SO₂-CH₂CH₃ et SO₂-NR^{2.2}R^{2.3}, qui peut le cas échéant être substitué lui aussi par un ou plusieurs restes choisis dans le groupe constitué par OH, OR^{2.1}, oxo, halogène, alkyle en C₁ à C₆, aryle en C₆ à C₁₀ et NR^{2.2}R^{2.3},
ou
R² est un reste choisi dans un groupe constitué par un hétérocycle en C₅ à C₁₀ mono- ou bicyclique, saturé ou insaturé, et un hétéroaryle en C₅-C₆ mono- ou bicyclique contenant de 1 à 4 hétéroatomes choisis parmi S, O et N, et peut le cas échéant être substitué par une ou plusieurs restes du groupe halogène, OH, oxo et SH ou par un ou plusieurs restes choisis dans le groupe constitué par OR^{2.1}, SR^{2.1}, COOR^{2.1}, COR^{2.1}, alcanol en C₁ à C₆, cycloalkyle en C₃ à C₁₀, phényle, alkyle en C₁ à C₆, phénylalkylène en C₁ à C₆, (hétéroaryle en C₅-C₆)alkylène en C₁ à C₆, hétérocycle en C₅ à C₁₀, hétéroaryle en C₅-C₆, alcanol en C₁ à C₆ et NR^{2.2}R^{2.3}, qui peut le cas échéant être substitué lui aussi par un ou plusieurs restes choisis dans le groupe constitué par OH, OR^{2.1}, oxo, halogène, alkyle en C₁ à C₆, phényle et NR^{2.2}R^{2.3},
ou NR¹R² dans son ensemble représente un noyau hétérocyclique en C₄ à C₇ qui peut le cas échéant être ponté, qui contient 1, 2 ou 3 hétéroatomes choisis dans le groupe constitué par N, O et S, et qui peut le cas échéant être substitué par un ou plusieurs restes choisis dans le groupe constitué par OH, OR^{2.1}, alcanol en C₁ à C₆, oxo, halogène, alkyle en C₁ à C₆, aryle en C₆ à C₁₀, COOR^{2.1}, CH₂-NR^{2.2}-COO-R^{2.1}, CH₂-NR^{2.2}-CO-R^{2.1}, CH₂-NR^{2.2}-CO-CH₂-NR^{2.2}R^{2.3}, CH₂-NR^{2.2}-SO₂-alkyle en C₁ à C₃, CH₂-NR^{2.2}-SO₂-NR^{2.2}R^{2.3}, CH²-NR^{2.2}-CO-NR^{2.2}R^{2.3}, CO-NR^{2.2}R^{2.3}, CH₂-NR^{2.2}R^{2.3} et NR^{2.2}R^{2.3},
ainsi que leurs sels, diastéréoisomères, énantiomères, racémates, hydrates ou solvates compatibles au plan pharmacologique.

4. Composés de formule 1 selon les revendications 1 à 3, dans laquelle
R¹ est H ou méthyle,
ainsi que leurs sels, diastéréoisomères, énantiomères, racémates, hydrates ou solvates compatibles au plan pharmacologique.

5. Composés de formule 1 selon les revendications 1 à 3, dans laquelle
NR¹R² dans son ensemble représente un noyau pyrrolidine qui peut le cas échéant être substitué par un ou plusieurs restes choisis dans le groupe constitué par OH, OR^{2.1}, CH₂-OH, CH₂-CH₂-OH, oxo, Cl, F, Br, méthyle, éthyle, propyle, phényle, COOR^{2.1}, CH₂-NR^{2.2}-COOR^{2.1}, CH₂-NR^{2.2}-CO-R^{2.1}, CH₂-NR^{2.2}-CO-CH₂-NR^{2.2}R^{2.3}, CH₂-NR^{2.2}-SO₂-alkyle en C₁ à C₃, CH₂-NR^{2.2}-SO₂-NR^{2.2}R^{2.3} CH₂-NR^{2.2}-CO-NR^{2.2}R^{2.3}, CO-NR^{2.2}R^{2.3}, CH₂-NR^{2.2}R^{2.3} et NR^{2.2}R^{2.3},
ainsi que leurs sels, diastéréoisomères, énantiomères, racémates, hydrates ou solvates compatibles au plan pharmacologique.

6. Composés de formule 1 selon les revendications 1 à 4, dans laquelle
R² est un phényle qui est mono- ou polysubstitué dans une position quelconque par OH, SH ou un halogène ou par un ou plusieurs restes choisis dans le groupe constitué par OR^{2.1}, COOR^{2.1}, NR^{2.2}R^{2.3}, CH₂-NR^{2.2}R^{2.3}, cycloalkyle en C₅ à C₁₀, hétérocycle en C₅ à C₁₀, alkyle en C₁ à C₆, phénylalkylène en C₁ à C₆, (hétérocycle en C₅ à C₁₀) alkylène en C₁ à C₆, (hétéroaryle en C₅-C₆)-alkylène en C₁ à C₆, phényle, SO₂-CH₃, SO₂-CH₂CH₃ et SO₂-NR^{2.2}R^{2.3}, qui peut le cas échéant être substitué lui aussi par un ou plusieurs restes choisis dans le groupe constitué par OH, OR^{2.1} oxo, halogène, alkyle en C₁ à C₆, phényle et NR^{2.2}R^{2.3},
ainsi que leurs sels, diastéréoisomères, énantiomères, racémates, hydrates ou solvates compatibles au plan pharmacologique.

7. Composés de formule 1 selon les revendications 1 à 4, dans laquelle
R² est un phényle qui peut être substitué dans l'une au moins des deux positions méta par OH, SH ou un halogène ou par un ou plusieurs restes choisis dans le groupe constitué par OR^{2.1}, COOR^{2.1}, NR^{2.2}R^{2.3}, CH₂-NR^{2.2}R^{2.3}, cycloalkyle en C₅ à C₁₀, hétérocycle en C₅ à C₁₀, alkyle en C₁ à C₆, phénylalkylène en C₁ à C₆, (hétérocycle en C₅ à C₁₀) alkylène en C₁ à C₆, (hétéroaryle en C₅-C₆) - alkylène en C₁ à C₆, phényle, SO₂-CH₃, SO₂-CH₂CH₃ et SO₂NR^{2.2}R^{2.3}, qui peut le cas échéant être substitué lui aussi par un ou plusieurs restes choisis dans le groupe constitué par OH, OR^{2.1}, oxo, halogène, alkyle en C₁ à C₆, phényle et NR^{2.2}R^{2.3},
ainsi que leurs sels, diastéréoisomères, énantiomères, racémates, hydrates ou solvates compatibles au plan pharmacologique.

8. Composés de formule 1 selon la revendication 7, dans laquelle
R² est un phényle qui est substitué dans l'une au moins des deux positions méta par un ou plusieurs restes choisis dans le groupe constitué par méthyle, F, Cl, OH, OR^{2.1}, COOR^{2.1}, NH₂ et N(CH₃)₂,
ainsi que leurs sels, diastéréoisomères, énantiomères, racémates, hydrates ou solvates compatibles au plan pharmacologique.

9. Composés de formule 1 selon l'une des revendications 1 à 4, dans laquelle
R² est un alkyle en C₁ à C₆ qui peut le cas échéant être substitué par un halogène et qui peut le cas échéant être substitué par un ou plusieurs restes choisis dans le groupe constitué par OR^{2.1}, COOR^{2.1}, CONR^{2.2}R^{2.3}, SR^{2.1}, phényle, hétérocycle en C₅ à C₁₀ mono-ou bicyclique, hétéroaryle en C₅-C₆, cycloalkyle en C₅ à C₁₀ mono- ou bicyclique, CH₂-NR^{2.2}R^{2.3} et NR^{2.2}R^{2.3}, le reste du deuxième groupe pouvant être substitué lui aussi par un ou plusieurs restes choisis dans le groupe constitué par OH, halogène, OR^{2.1}, oxo, alkyle en C₁ à C₆, phényle, COOR^{2.1}, CH₂-NR^{2.2}R^{2.3} et NR^{2.2}R^{2.3},
ainsi que leurs sels, diastéréoisomères, énantiomères, racémates, hydrates ou solvates compatibles au plan pharmacologique.

10. Composés de formule 1 selon l'une des revendications 1 à 4 et 9, dans laquelle
R² est méthyle, éthyle ou propyle,
ainsi que leurs sels, diastéréoisomères, énantiomères, racémates, hydrates ou solvates compatibles au plan pharmacologique.

11. Composés de formule 1 selon l'une des revendications 1 à 4, dans laquelle
R² est un alkyle en C₁ à C₆ qui est substitué par un ou plusieurs restes choisis dans le groupe constitué par OH, COOR^{2.1}, CON(CH₃)₂, alkyle en C₁ à C₆, phényle, cyclopropyle et NR^{2.2}R^{2.3}, qui peut être le cas échéant substitué lui aussi par un ou plusieurs restes choisis dans le groupe constitué par OH, fluor, chlore, brome, iode, OR^{2.1}, oxo, alkyle en C₁ à C₆, phényle, alcanol en C₁ à C₃, CH₂-NR^{2.2}R^{2.1} et NR^{2.2}R^{2.3},
ainsi que leurs sels, diastéréoisomères, énantiomères, racémates, hydrates ou solvates compatibles au plan pharmacologique.

12. Composés de formule 1 selon l'une des revendications 1 à 4, dans laquelle
R² est un alkyle en C₁ à C₆ qui est substitué par un ou plusieurs restes choisis dans le groupe constitué par OH, phényle, COOR^{2.1}, NH₂, le phényle pouvant le cas échéant être substitué lui aussi par un ou plusieurs restes choisis dans le groupe constitué par OH, fluoro, chloro, bromo, iodo, OR^{2.1}, alkyle en C₁ à C₆, CH₂-NH₂, CH₂(CH₃)₂, NH₂ et N(CH₃)₂,
ainsi que leurs sels, diastéréoisomères, énantiomères, racémates, hydrates ou solvates compatibles au plan pharmacologique.

13. Composés de formule 1 selon l'une des revendications 1 à 4 et 11 à 12, dans laquelle
R² est un reste de formule 2 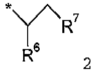 dans laquelle
R⁷ est OH ou NH₂, et
R⁶ est un reste choisi dans le groupe constitué par alkyle en C₁ à C₆, hétéroaryle en C₅ à C₁₀ et aryle en C₆ à C₁₀, de préférence phényle, qui peut le cas échéant être substitué par un ou plusieurs restes choisis dans le groupe constitué par halogène, OH, COOR^{2.1}, OR^{2.1}, NH₂, alkyle en C₁ à C₆, haloalkyle en C₁ à C₆ et alcanol en C₁ à C₆,
ainsi que leurs sels, diastéréoisomères, énantiomères, racémates, hydrates ou solvates compatibles au plan pharmacologique.

14. Composés de formule 1 selon la revendication 13, dans laquelle
R² est un reste de formule 2 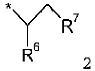 dans laquelle R⁷ est OH ou NH₂, et R⁶ est méthyle, éthyle, propyle, isopropyle.

15. Composés de formule 1 selon l'une des revendications 1 à 4, dans laquelle
R² est un noyau cycloalkyle en C₃ à C₇ monocyclique qui peut être substitué en position spiro par un reste choisi dans le groupe constitué par -OH, -CH₂-OH, -CH₂-CH₂-OH, alcanol en C₃ à C₆ ramifié ou non ramifié, -OR^{2.1}, méthyle, éthyle, propyle, isopropyle, butyle, isobutyle et halogène, R^{2.1} pouvant être choisi dans le groupe constitué par méthyle, éthyle, propyle, isopropyle, butyle, isobutyle,

16. Composés de formule 1 selon l'une des revendications 1 à 4, dans laquelle
R² est une reste choisi dans un groupe constitué par un hétérocycle monocyclique saturé à trois, quatre, cinq, six ou sept termes, contenant 1, 2 ou 3 hétéroatomes choisis chacun dans le groupe constitué par N, O et S, qui peut le cas échéant être substitué par un ou plusieurs restes choisis dans le groupe constitué par fluor, chlore, brome, OH, oxo et SH ou par un ou plusieurs restes choisis dans le groupe constitué par OR^{2.1}, SR^{2.1}, COOR^{2.1}, COR^{2.1}, alcanol en C₁ à C₆, cycloalkyle en C₃ à C₁₀, phényle, alkyle en C₁ à C₆, phényl-alkylène en C₁ à C₆, (hétéroaryle en C₅ à C₁₀) alkylène en C₁ à C₆, hétérocycle en C₅ à C₁₀, hétéroaryle en C₅ à C₁₀ et NR^{2.2}R^{2.3}, qui peut le cas échéant être substitué lui aussi par un ou plusieurs restes choisis dans le groupe constitué par OH, OR^{2.1}, oxo, halogène, alkyle en C₁ à C₆, phényle et NR^{2.2}R^{2.3},
où R^{2.1} est H ou un reste choisi dans le groupe constitué par alkyle en C₁ à C₆, alcanol en C₁ à C₆, haloalkyle en C₁ à C₃, cycloalkyle en C₃ à C₁₀ mono- ou bicyclique, phénylalkylène en C₁ à C₆, (hétéroaryle en C₅ à C₁₀ mono- ou bicyclique)alkylène en C₁ à C₆, (hétérocycle en C₃ à C₁₀)alkylène en C₁ à C₆, (cycloalkyle en C₃ à C₇)alkylène en C₁ à C₆, phényle, hétéroaryle en C₅ à C₁₀ mono- ou bicyclique et hétérocycle monocyclique saturé ou insaturé à cinq, six ou sept termes contenant 1, 2 ou 3 hétéroatomes du groupe constitué par N, O et S, qui peut le cas échéant être substitué par un ou plusieurs restes choisis dans le groupe constitué par OH, halogène, alkyle en C₁ à C₆ et phényle,
où R^{2.2} et R^{2.3} sont indépendamment l'un de l'autre H ou un reste choisi dans le groupe constitué par halogène, alkyle en C₁ à C₆, cycloalkyle en C₃ à C₁₀ mono- ou bicyclique, phénylalkylène en C₁ à C₆, (hétéroaryle en C₅ à C₁₀) alkylène en C₁ à C₆, phényle, hétérocycle en C₃ à C₁₀ mono- ou bicyclique, hétéroaryle en C₅ à C₁₀ mono- ou bicyclique, CO-NH₂, CO-NHCH₃, CON(CH₃)₂, SO₂-alkyle en C₁-C₂, CO-R^{2.1} et COOR^{2.1}, qui peut le cas échéant être substitué par un ou plusieurs restes choisis dans le groupe constitué par OH, halogène, alkyle en C₁ à C₆, phényle et COOR^{2.1}.

17. Composés de formule 1 selon l'une des revendications 1 à 16, dans laquelle
R³ est fluor, chlore, brome, iode ou CN,
ainsi que leurs sels, diastéréoisomères, énantiomères, racémates, hydrates ou solvates compatibles au plan pharmacologique.

18. Composés de formule 1 selon l'une des revendications 1 à 16, dans laquelle
R³ représente le groupe -CO-NR^{3.1}R^{3.2},
où R^{3.1} et R^{3.2} sont indépendamment l'un de l'autre H ou des restes choisis dans le groupe constitué par alkyle en C₁ à C₆, alcényle en C₂ à C₆, alcynyle en C₂ à C₆, aryle en C₆ à C₁₀, (aryle en C₆ à C₁₀)alkylène en C₁ à C₆, (hétéroaryle en C₅ à C₁₀)alkylène en C₁ à C₆, (hétéroaryle en C₅ à C₁₀) alcynylène en C₁ à C₆, (hétéroaryle en C₅ à C₁₀) alcénylène en C₁ à C₆, hétérocycle en C₅ à C₁₀ mono- ou bicyclique, (hétérocycle en C₅ à C₁₀)-alkylène en C₁ à C₆ et hétéroaryle en C₅ à C₁₀ mono- ou bicyclique, chaque reste pouvant le cas échéant être substitué par un ou plusieurs restes choisis dans le groupe constitué par OH, oxo, halogène, alkyle en C₁ à C₆ et O-alkyle en C₁ à C₆,
ainsi que leurs sels, diastéréoisomères, énantiomères, racémates, hydrates ou solvates compatibles au plan pharmacologique.

19. Composés de formule 1 selon l'une des revendications 1 à 16 et 18, dans laquelle
R³ représente le groupe -CO-NR^{3.1}R^{3.2},
où R^{3.1} et R^{3.2} sont indépendamment l'un de l'autre H ou des restes choisis dans le groupe constitué par alkyle en C₁ à C₆, phényle, phénylalkylène en C₁ à C₆, (hétéroaryle en C₅-C₆)alkylène en C₁ à C₆, (hétéroaryle en C₅-C₆)alcynylène en C₁ à C₆, (hétéroaryle en C₅-C₆)-alcénylène en C₁ à C₆, hétérocycle en C₅ à C₁₀ mono- ou bicyclique, (hétérocycle en C₅ à C₁₀)alkylène en C₁ à C₆ et hétéroaryle en C₅ à C₁₀ mono- ou bicyclique, chaque reste pouvant le cas échéant être substitué par un ou plusieurs restes choisis dans le groupe constitué par OH, oxo, halogène, alkyle en C₁ à C₆ et O-alkyle en C₁ à C₆,
ainsi que leurs sels, diastéréoisomères, énantiomères, racémates, hydrates ou solvates compatibles au plan pharmacologique.

20. Composés de formule 1 selon l'une des revendications 1 à 16, dans laquelle
R³ représente le groupe -NR^{3.3}-CO-R^{3.4},
où R^{3.3} est H ou un reste choisi dans le groupe constitué par alkyle en C₁ à C₆, alcényle en C₂ à C₆, alcynyle en C₂ à C₆, aryle en C₆ à C₁₀, (aryle en C₆ à C₁₀)alkylène en C₁ à C₆, (hétéroaryle en C₅ à C₁₀)-alkylène en C₁ à C₆, hétérocycle en C₃ à C₁₀ et hétéroaryle en C₅ à C₁₀, qui peut le cas échéant être substitué par un ou plusieurs restes choisis dans le groupe constitué par OH, OR^{2.1}, oxo, NH₂, NR^{2.2}R^{2.3}, halogène, alkyle en C₁ à C₆ et aryle en C₆ à C₁₀, et
R^{3.4} est H ou un reste choisi dans le groupe constitué par alkyle en C₁ à C₆, alcényle en C₂ à C₆, alcynyle en C₂ à C₆, alcanol en C₁ à C₆, OR^{2.1}, CH₂-O-CO-alkyle en C₁ à C₆, CH₂NR^{2.2}R^{2.3}, NR^{2.2}R^{2.3}, aryle en C₆ à C₁₀, (aryle en C₆ à C₁₀) alkylène en C₁ à C₆, (hétéroaryle en C₅ à C₁₀) alkylène en C₁ à C₆, hétérocycle en C₃ à C₁₀ mono- ou bicyclique, saturé ou insaturé contenant 1, 2 ou 3 hétéroatomes du groupe O, S et N, et hétéroaryle en C₅ à C₁₀ mono- ou bicyclique contenant 1, 2 ou 3 hétéroatomes du groupe O, S et N, qui peut le cas échéant être substitué par un ou plusieurs restes choisis dans le groupe constitué par OH, OR^{2.1}, oxo, NH₂, NR^{2.2}R^{2.3}, halogène, alkyle en C₁ à C₆ et aryle en C₆ à C₁₀,
ainsi que leurs sels, diastéréoisomères, énantiomères, racémates, hydrates ou solvates compatibles au plan pharmacologique.

21. Composés de formule 1 selon l'une des revendications 1 à 16 et 20, dans laquelle
R³ représente le groupe -NR^{3.3}-CO-R^{3.4},
où R^{3.3} est H ou un reste choisi dans le groupe constitué par alkyle en C₁ à C₆, phényle, phénylalkylène en C₁ à c₆, (hétéroaryle en C₅ à C₁₀)alkylène en C₁ à C₆, hétérocycle en C₅ à C₁₀ et hétéroaryle en C₅ à C₁₀, qui peut le cas échéant être substitué par un ou plusieurs restes choisis dans le groupe constitué par OH, OR^{2.1}, oxo, NH₂, N(CH₃)₂, halogène, alkyle en C₁ à C₆ et phényle, et
R^{3.4} est H ou un reste choisi dans le groupe constitué par alkyle en C₁ à C₆, alcanol en C₁ à C₆, OR^{2.1}, CH₂-O-CO-alkyle en C₁ à C₆, CH₂-NH₂, CH₂-N(CH₃)₂, NH₂, N(CH₃)₂, phényle, phénylalkylène en C₁ à C₆, (hétéroaryle en C₅ à C₁₀)alkylène en C₁ à C₆, hétérocycle en C₅ à C₁₀ mono- ou bicyclique, saturé ou insaturé contenant 1, 2 ou 3 hétéroatomes du groupe N, S et O, et hétéroaryle en C₅ à C₁₀ mono- ou bicyclique contenant 1, 2 ou 3 hétéroatomes du groupe N, S et O, qui peut le cas échéant être substitué par un ou plusieurs restes choisis dans le groupe constitué par OH, OR^{2.1}, oxo, NH₂, N(CH₃)₂, halogène, alkyle en C₁ à C₆ et phényle,
ainsi que leurs sels, diastéréoisomères, énantiomères, racémates, hydrates ou solvates compatibles au plan pharmacologique.

22. Composés numéros 1 à 123 de formule 1 selon l'une des revendications 1 à 4 ou 6 à 21, dans laquelle
| Composé numero | R² | R³ |
|---|---|---|
| 1. | 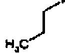 | 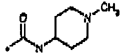 |
| 2. | 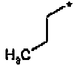 | 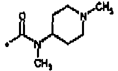 |
| 3. | 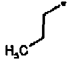 | 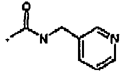 |
| 4. | 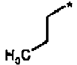 | 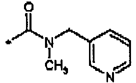 |
| 5. | 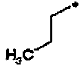 | 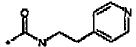 |
| 6. | 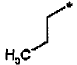 | 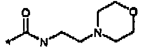 |
| 7. | 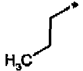 | 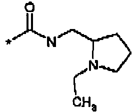 |
| 8. | 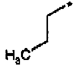 | 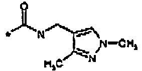 |
| 9. | 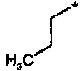 | 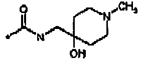 |
| 10. | 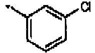 | 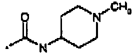 |
| 11. | 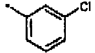 | 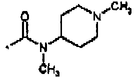 |
| 12. | 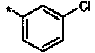 | 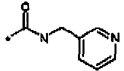 |
| 13. | 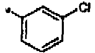 | 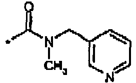 |
| 14. | 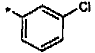 | 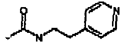 |
| 15. | 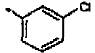 | 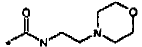 |
| 16. | 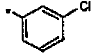 | 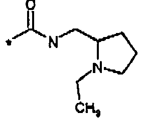 |
| 17. | 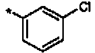 | 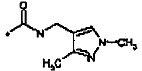 |
| 18. | 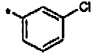 | 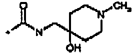 |
| 19. | 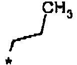 | 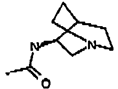 |
| 20. | 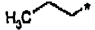 | Cl |
| 21. | 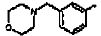 | Cl |
| 22. | 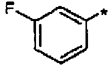 | Cl |
| 23. | 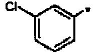 | Cl |
| 24. | 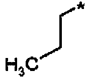 | 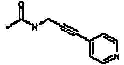 |
| 25. | 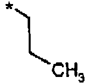 | 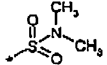 |
| 26. | 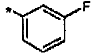 | 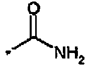 |
| 27. | 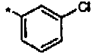 | 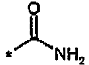 |
| 28. | 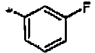 | 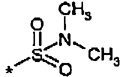 |
| 29. | 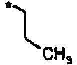 | 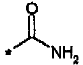 |
| 30. | 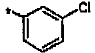 | 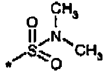 |
| 31. | 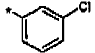 | 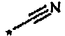 |
| 32. | 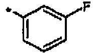 | 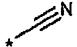 |
| 33. | 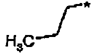 | 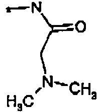 |
| 34. | 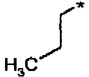 | 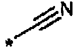 |
| 35. | 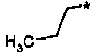 | 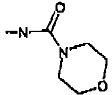 |
| 36. | 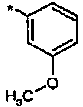 | Cl |
| 37. | 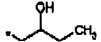 | Cl |
| 38. | 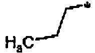 | 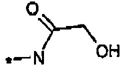 |
| 39. | 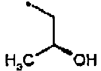 | Cl |
| 40. | 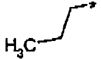 | 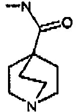 |
| 41. | 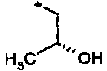 | Cl |
| 42. | 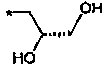 | Cl |
| 43. | 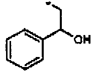 | Cl |
| 44. | 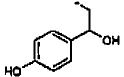 | Cl |
| 45. | 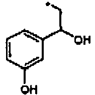 | Cl |
| 46. | 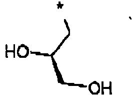 | Cl |
| 47. | 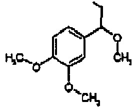 | Cl |
| 48. | 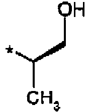 | Cl |
| 49. | 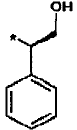 | Cl |
| 50. | 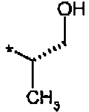 | Cl |
| 51. | 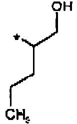 | Cl |
| 52. | 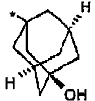 | Cl |
| 53. | 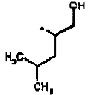 | Cl |
| 54. | 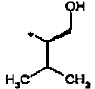 | Cl |
| 55. | 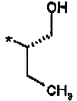 | Cl |
| 56. | 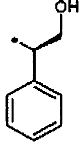 | Cl |
| 57. | 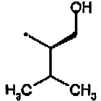 | Cl |
| 58. | 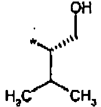 | Cl |
| 59. | 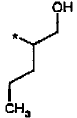 | Cl |
| 60. | 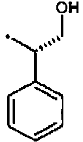 | Cl |
| 61. | 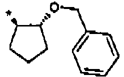 | Cl |
| 62. | 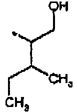 | Cl |
| 63. | 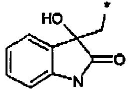 | Cl |
| 64. | 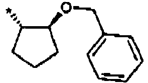 | Cl |
| 65. | 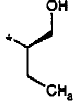 | Cl |
| 66. | 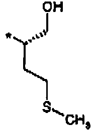 | Cl |
| 67. | 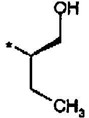 | Cl |
| 68. | 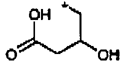 | Cl |
| 69. | 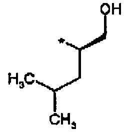 | Cl |
| 70. | 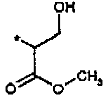 | Cl |
| 71. | 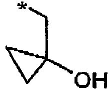 | Cl |
| 72. | 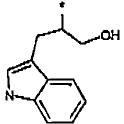 | Cl |
| 73. | 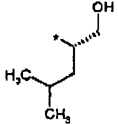 | Cl |
| 74. | 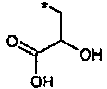 | Cl |
| 75. | 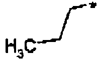 | 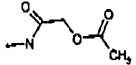 |
| 76. | 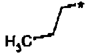 | 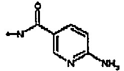 |
| 77. | 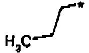 | 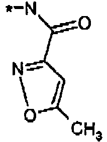 |
| 78. | 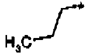 | 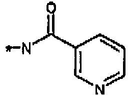 |
| 79. | 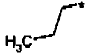 | 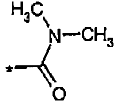 |
| 80. | 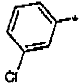 | 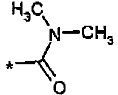 |
| 81. | 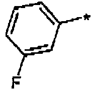 | 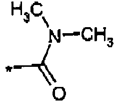 |
| 82. | 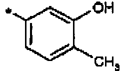 | Cl |
| 83. | 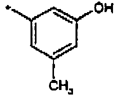 | Cl |
| 84. | 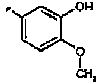 | Cl |
| 85. | 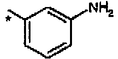 | Cl |
| 86. | 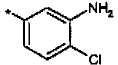 | Cl |
| 87. | 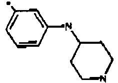 | Cl |
| 88. | 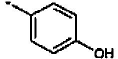 | Cl |
| 89. | 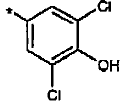 | Cl |
| 90. | 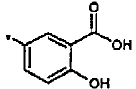 | Cl |
| 91. | 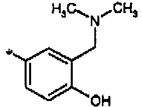 | Cl |
| 92. | 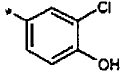 | Cl |
| 93. | 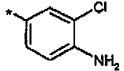 | Cl |
| 94. | 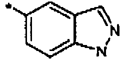 | Cl |
| 95. | 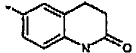 | Cl |
| 96. | 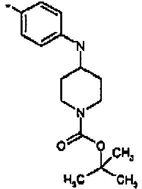 | Cl |
| 97. | 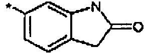 | Cl |
| 98. | 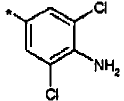 | Cl |
| 99. | 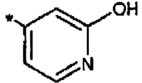 | Cl |
| 100. | 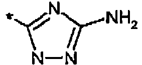 | Cl |
| 101. | 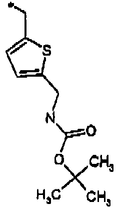 | Cl |
| 102. | 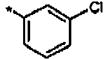 | 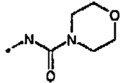 |
| 103. | 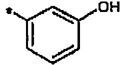 | Cl |
| 104. | 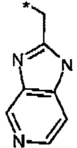 | Cl |
| 105. | 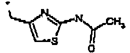 | Cl |
| 106. | 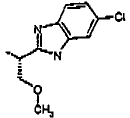 | Cl |
| 107. | 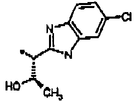 | Cl |
| 108. | 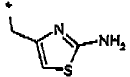 | Cl |
| 109. | 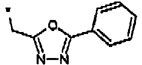 | Cl |
| 110. | 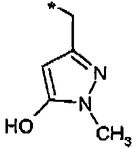 | Cl |
| 111. | 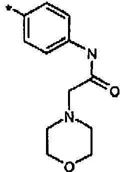 | Cl |
| 112. | 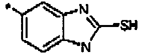 | Cl |
| 113. | 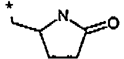 | Cl |
| 114. | 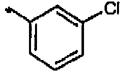 | 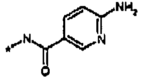 |
| 115. | 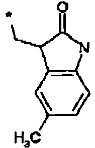 | Cl |
| 116. | 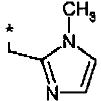 | Cl |
| 117. | 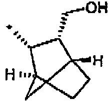 | Cl |
| 118. | 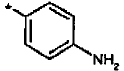 | Cl |
| 119. | 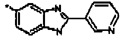 | Cl |
| 120. | 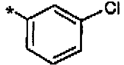 | 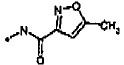 |
| 121. | 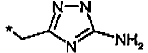 | Cl |
| 122. | 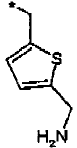 | Cl |
| 123. | 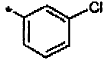 | 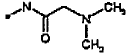 |

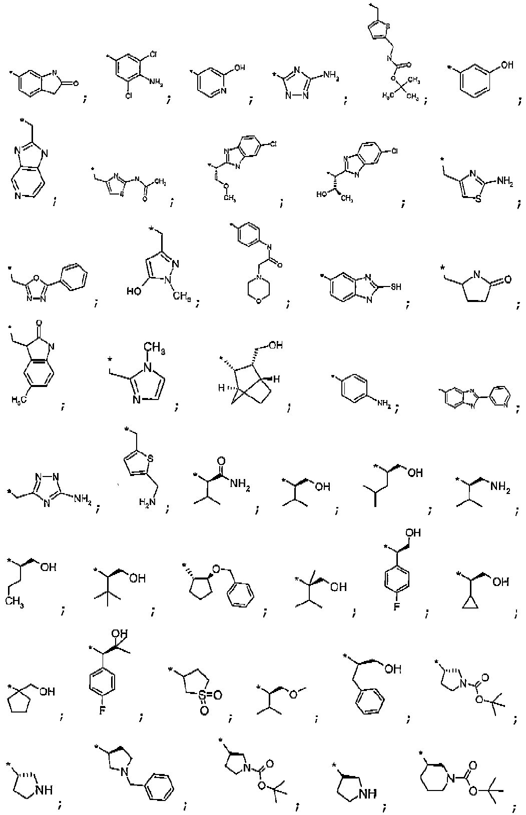
ainsi que leurs sels, diastéréoisomères, énantiomères, racémates, hydrates ou solvates compatibles au plan pharmacologique.

23. Composés numeros 131 à 217 de formule 1 selon l'une des revendications 1 à 4 ou 6 à 21, dans laquelle
X représente SO, et
R¹ représente H ; et
dans laquelle R² et R³ sont combinés selon les composés numeros 131 à 217 dans le tableau suivant
| Composé numero | R² | R³ |
|---|---|---|
| 131 | 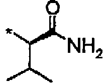 | Cl |
| 132 | 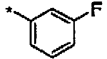 | 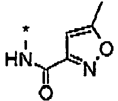 |
| 133 | 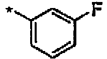 | 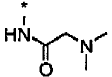 |
| 134 | 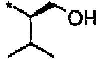 | 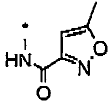 |
| 135 | 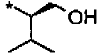 | 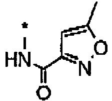 |
| 136 | 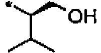 | 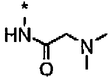 |
| 137 | 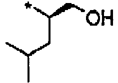 | 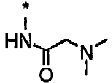 |
| 138 | 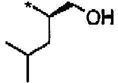 | 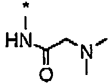 |
| 139 | 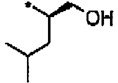 | 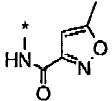 |
| 140 | 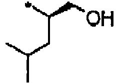 | 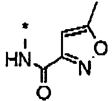 |
| 141 | 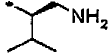 | Cl |
| 142 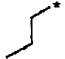 | 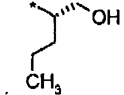 | Cl |
| 143 | 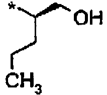 | Cl |
| 144 | 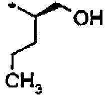 | Cl |
| 145 | 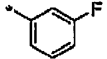 | 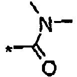 |
| 146 | 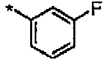 | 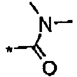 |
| 147 | 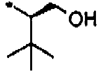 | Cl |
| 148 | 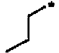 | CN |
| 149 | 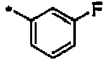 | 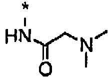 |
| 150 | 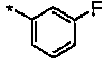 | 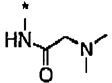 |
| 151 | 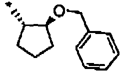 | Cl |
| 152 | 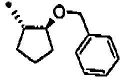 | Cl |
| 153 | 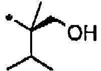 | Cl |
| 154 | 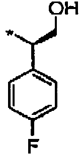 | Cl |
| 155 | 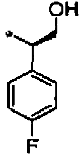 | Cl |
| 156 | 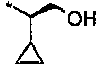 | Cl |
| 157 | 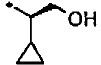 | Cl |
| 158 | 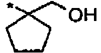 | Cl |
| 159 | 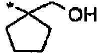 | Cl |
| 160 | 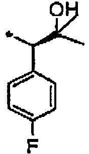 | Cl |
| 161 | 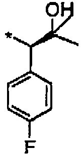 | Cl |
| 162 | 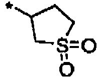 | Cl |
| 163 | 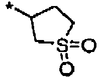 | Cl |
| 164 | 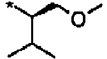 | Cl |
| 165 | 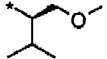 | Cl |
| 166 | 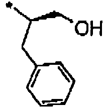 | Cl |
| 167 | 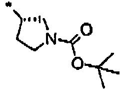 | Cl |
| 168 | 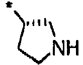 | Cl |
| 169 | 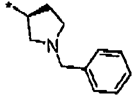 | Cl |
| 170 | 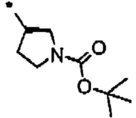 | Cl |
| 171 | 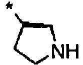 | Cl |
| 172 | 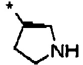 | Cl |
| 173 | 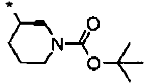 | Cl |
| 174 | 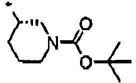 | Cl |
| 175 | 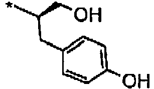 | Cl |
| 176 | 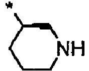 | Cl |
| 177 | 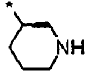 | Cl |
| 178 | 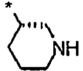 | Cl |
| 179 | 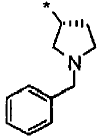 | Cl |
| 180 | 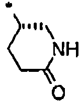 | Cl |
| 181 | 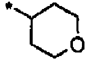 | 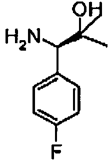 |
| 182 | 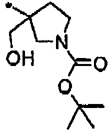 | Cl |
| 183 | 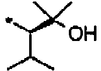 | Cl |
| 184 | 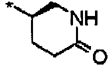 | Cl |
| 185 | 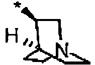 | Cl |
| 186 | 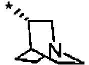 | Cl |
| 187 | 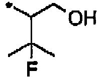 | Cl |
| 188 | 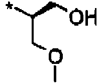 | Cl |
| 189 | 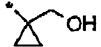 | Cl |
| 190 | 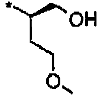 | Cl |
| 191 | 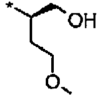 | Cl |
| 192 | 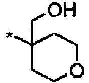 | Cl |
| 193 | 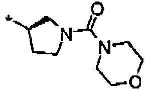 | Cl |
| 194 | 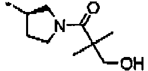 | Cl |
| 195 | 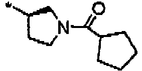 | Cl |
| 196 | 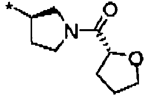 | Cl |
| 197 | 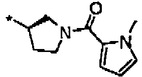 | Cl |
| 198 | 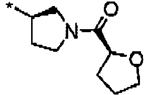 | Cl |
| 199 | 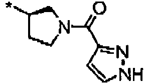 | Cl |
| 200 | 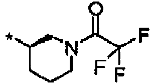 | Cl |
| 201 | 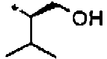 | OH |
| 202 | 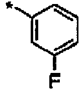 | 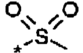 |
| 203 | 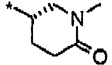 | Cl |
| 204 | 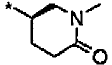 | Cl |
| 205 | 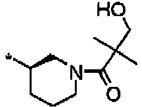 | Cl |
| 206 | 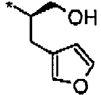 | Cl |
| 207 | 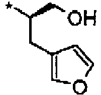 | Cl |
| 208 | 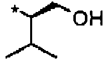 | Br |
| 209 | 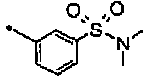 | Cl |
| 210 | 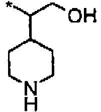 | Cl |
| 211 | 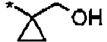 | Br |
| 212 | 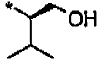 | 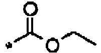 |
| 213 | 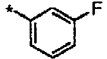 | 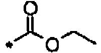 |
| 214 | 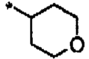 | 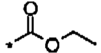 |
| 215 | 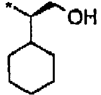 | Cl |
| 216 | 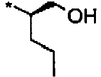 | Br |
| 217 | 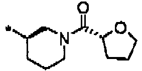 | Cl |
ainsi que leurs sels, diastéréoisomères, énantiomères, racémates, hydrates ou solvates compatibles au plan pharmacologique.

24. Composés de formule 1 selon l'une des revendications 1 à 3 ou 5, dans laquelle
X représente SO, et
NR¹R² est choisi dans le groupe constitué par 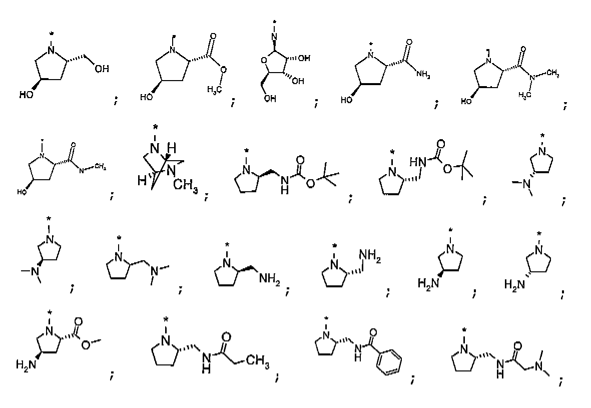 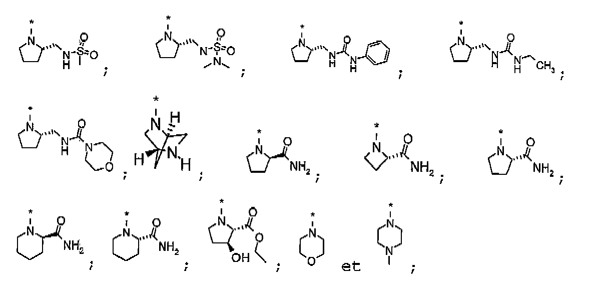 et dans laquelle
R³ est Cl,
ainsi que leus sels, diastéréoisomères, énantiomères, racémates, hydrates ou solvates compatibles au plan pharmacologique.

25. Composés de formule 4 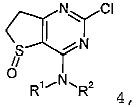 de formule 5 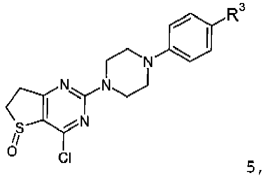 de formule 6 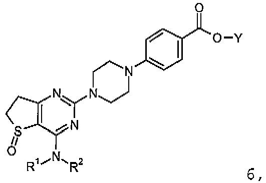 de formule 7 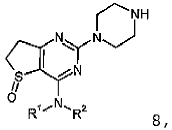 de formule 8 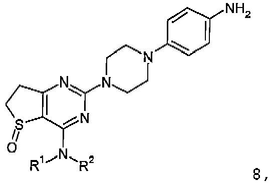 dans lesquelles
R¹, R² et R³ sont tels que définis dans l'une des revendications 1 à 23 ; et
Y est H, méthyle ou éthyle,
ainsi que leurs sels, diastéréoisomères, énantiomères, racémates, hydrates ou solvates compatibles au plan pharmacologique.

26. Composés selon l'une des revendications 1 à 23 en tant que médicaments.

27. Utilisation de composés selon l'une des revendications 1 à 23 pour préparer un médicament destiné au traitement de troubles ou affections des voies respiratoires ou du système gastro-intestinal, d'affections inflammatoires des articulations, de la peau ou des yeux, d'affections cancéreuses ainsi que d'affections du système nerveux périphérique ou central.

28. Utilisation de composés selon l'une des revendications 1 à 23 pour préparer un médicament destiné à la prévention et au traitement d'affections des voies respiratoires ou des poumons associées à une production accrue de mucosités, à des inflammations et/ou à des affections obstructives des voies respiratoires.

29. Utilisation de composés selon l'une des revendications 1 à 23 pour préparer un médicament destiné au traitement d'affections inflammatoires et obstructives telles que la BPCO, la sinusite chronique, l'asthme, l'iléite régionale, la rectocolite hémorragique, ainsi que d'affections inflammatoires du système gastro-intestinal.

30. Utilisation de composés selon l'une des revendications 1 à 23 pour préparer un médicament destiné à la prévention et au traitement d'affections du système nerveux périphérique ou central telles que la dépression, la dépression bipolaire ou maniaque, les états anxieux aigus et chroniques, la schizophrénie, la maladie d'Alzheimer, la maladie de Parkinson, la sclérose en plaques aiguë et chronique ou les états douloureux aigus et chroniques, ainsi que des lésions cérébrales provoquées par un accident vasculaire, une hypoxie ou un traumatisme crânio-cérébral.
